# EUROPEAN PATENT APPLICATION

(11) **EP 2 060 565 A1**
(43) Date of publication of application: **20.05.2009**
(21) Application number: 07022287.2
(22) Date of filing: 16.11.2007
(51) Int. Cl.: C07D 213/68, C07D 239/94, C07D 401/04, C07D 401/12, C07D 401/14, C07D 403/12, C07D 405/04, C07D 409/04, C07D 409/12, C07D 409/14, C07D 417/04, C07D 417/14, A61K 31/517, A61P 35/00

(54) **Novel bifunctional compounds which inhibit protein kinases and histone deacetylases**

(71) Applicant: 4SC AG, 82152 Planegg - Martinsried (DE)
(72) Inventor: Bär, Thomas, 78479 Reichenau (DE); Maier, Thomas, 78333 Stockach (DE); Beckers, Thomas, 78464 Konstanz (DE); Ciossek, Thomas, 88213 Ravensburg (DE); Mahboobi, Siavosh, 93051 Regensburg (DE); Sellmer, Andreas, 93138 Lappersdorf/Hainsacker (DE)
(74) Representative: Vossius & Partner

(57) **Abstract**

The present invention relates to a bifunctional compound of formula I or its pharmaceutically acceptable salts or solvates

A-L-B (I)

wherein A is a histone deacetylase (HDAC) inhibitory moiety, L is a single bond or a linker group and B is a protein kinase inhibitory moiety. The bifunctional compound according to formula (I) is useful for the treatment of malignant and non-malignant neoplasia and diseases related to abnormal cell growth.

## Description

### Field of the invention

The invention relates to novel bifunctional compounds and pharmaceutically acceptable salts thereof, which are used in the pharmaceutical industry for the production of pharmaceutical compositions, which may be useful for the treatment of malignant and non-malignant neoplasias and diseases related to abnormal cell growth.

### Technical background

Transcriptional regulation in cells is a complex biological process, involving multiprotein complexes with transcription factors, coactivators and repressors, receptors as well as platform / DNA binding proteins as functional subunits. One basic principle in transcriptional regulation is based on the posttranslational modification of histone proteins, namely histone proteins H2A/B, H3 and H4 forming the octameric histone core complex. The complex N-terminal modifications at lysine residues by acetylation or methylation and at serine residues by phosphorylation constitute part of the so called "histone code" (Strahl & Ellis, Nature 403, 41-45, 2000). In a simple model, acetylation of positively charged lysine residues decreases affinity to negatively charged DNA, which now becomes accessible for the entry of transcription factors.

Histone acetylation and deacetylation is catalysed by histone acetyltransferases (HATs) and histone deacetylases (HDACs). In many cases, HDACs are associated with transcriptional repressor complexes, switching chromatin to a transcriptionally inactive, silent structure (Marks et al. Nature Cancer Rev 1, 194-202, 2001). The opposite holds true for HATs which are frequently associated with transcriptional activator complexes. Nevertheless, opposite functions for HATs and HDACs have been described in the literature. The cAMP response element binding protein (CBP) and p300 as HATs contain the transcriptional repressor domain CRD1 (cell cycle regulatory domain 1), allowing these proteins to act as transcriptional repressors (Snowden et al. Mol Cell Biol 20, 2676-2686, 2000). Transcriptional signatures of HDAC inhibitors show a similar proportion of induced and repressed genes. In one study, HDAC inhibition abrogates interferon-induced gene transcription presumably by antagonizing the co-activator function of HDAC1 for the interferon stimulated gene factor 3 (ISGF3; Nusinzon & Horvath, Science STKE aug 2005). In melanoma cells, interaction of NfκB p65 with STAT1 is dependent on STAT1 acetylation. HDAC inhibitor or interferon α mediated STAT1 hyperacetylation causes the cytoplasmatic retention of NfκB, finally leading to repression of NFκB regulated genes (Krämer et al. Gen Develop 20, 473-485, 2006).

Four different classes of HDACs have been described so far, namely class I (HDAC 1-3, 8) with Mr = 42-55 kDa primarily located in the nucleus and sensitive towards inhibition by Trichostatin A (TSA), class II (HDAC 4-7, 9, 10) and class IV (HDAC11) with Mr = 120-130kDa and TSA sensitivity as well as class III (Sir2 homologes) which are quite distinct by their NAD⁺ dependency and TSA insensitivity (Ruijter et al. Biochem.J. 370, 737-749, 2003; Khochbin et al. Curr Opin Gen Dev 11, 162-166, 2001; Verdin et al. Trends Gen 19, 286-293, 2003). HATs and HDACs exist in large complexes together with transcription factor and platform proteins in cells (Fischle et al. Mol Cell 9, 45-47, 2002). Surprisingly, only about 2% of all genes are regulated by histone acetylation (von Lint et al. Gene Expression 5, 245-253, 1996). Studies with the HDAC inhibitor (HDI) suberoylanilid-hydroxamic acid (SAHA) in multiple myeloma cells showed that these transcriptional changes can be grouped into distinct functional gene classes important for e.g. regulation of apoptosis or proliferation (Mitsiades et al. Proc Natl Acad Sci 101, pp540, 2004).

Substrates different to histone proteins exist. For HDACs these include transcription factors like p53, STAT proteins and TFII E, α-tubulin as a major protein of microtubles, or chaperones like heat shock protein 90 (Hsp90; Johnstone & Licht, Cancer Cell 4, 13-18, 2003). Therefore the correct name for HDACs would be lysine-specific protein deacetylases. As a consequence of these findings, inhibitors of HDACs affect not only chromatin structure and gene transcription but also protein function and stability by regulating protein acetylation in general. This function of HDACs in protein acetylation might also be important for understanding of immediate gene repression by treatment with HDls (von Lint et al. Gene Expression 5, 245-253, 1996). In this regard, proteins involved in oncogenic transformation, apoptosis regulation and malignant cell growth are of particular importance.

Different protein publications highlight the pathophysiological importance of reversible histone acetylation for cancer drug development (reviewed by Kramer et al. Trends Endocrin Metabol 12, 294-300, 2001; Marks et al. Nature Cancer Rev 1, 194-202, 2001; Minucci & Pelicci, Nature Rev Canc 6, 38-51, 2006):
- Mutations of CBP as a HAT are associated with Rubinstein-Taybi syndrome, a cancer predisposition (Murata et al. Hum Mol Genet 10, 1071-1076, 2001),
- Aberrant recruitment of HDAC1 activity by transcription factors in acute promyelocytic leukemia (APL) is mediated by the PML-retinoic acid receptor α fusion gene (He et al. Nat genet 18, 126-135, 1998)
- Aberrant recruitment of HDAC activity by the overexpressed BCL6 protein was shown in non-Hodgkins lymphoma (Dhordain et al. Nuceic Acid Res 26, 4645-4651, 1998)
- Aberrant recruitment of HDAC activity by the AML-ETO fusion protein was shown for acute myelogenous leukemia (AML M2 subtype; Wang et al. Proc Natl Acad Sci USA 95, 10860-10865, 1998). In this AML subtype, the recruitment of HDAC1 activity causally leads to gene silencing, a differentiation block and oncogenic transformation.

HDAC1 gene knock-out in mice showed that HDAC1 has a profound function in embryonal stem cell proliferation by repressing cyclin-dependent kinase inhibitors p21^{waf1} and p27^{kip1} (Lagger et al. Embo J. 21, 2672-2681, 2002). Since p21^{wat1} is induced by HDIs in many cancer cell lines, HDAC1 might be a crucial component in cancer cell proliferation as well as survival. Initial siRNA based gene-knock down experiments in HeLa cells support this hypothesis (Glaser et al. 310, 529-536, 2003). HDAC2 is overexpressed in colon carcinoma upon constitutive activation of the wnt/β-catenin/TCF signalling pathways by loss of a functional adenomatosis polyposis coli (APC) protein, as reported by Zhu et al. (Cancer Cell 5, 455-463, 2004). A high expression of HDAC1, 2 and 3 in prostate adenocarcinomas was shown by immunohistochemistry, with HDAC2 as an independent prognostic factor for patient survival and HDAC1 / 2 correlating positively with tumor grade (Roeske et al, EORTC-NCI-AACR meeting Prague 2006, Abstract 350)

On the molecular level, a pleithora of published data with various HDAC inhibitors like Trichostatin A (TSA) or SAHA showed that many cancer relevant genes are up- or down regulated. These include p21^{waf1}, Cyclin E, transforming growth factor β (TGFβ), p53 or the von Hippel-Lindau (VHL) tumor suppressor gene, which are upregulated, whereas e.g. Bcl-XL, bcl2, hypoxia inducible factor (HIF)1α, vascular endothelial growth factor (VEGF) and cyclin A/D are down-regulated by HDAC inhibition (reviewed by Kramer et al. Trends Endocrin Metabol 12, 294-300, 2001).

HDIs transiently arrest cells at G1 and G2/M within the cell cycle and deplete S-phase cells, as shown for Depsipeptide as an example (Sandor et al., British J Cancer 83, 817-825, 2000). The interaction of HDAC3 with the mitotic kinase Aurora B was shown recently (Li et al. Genes Dev. 20:2566-79, 2006). In this study, phosphorylation of histone H3 at S¹⁰ by Aurora B was dependent on HDAC3 mediated N-terminal histone H3 deacetylation, giving a hint to the partial M-phase arrest seen by many HDIs.

HDAC inhibitory compounds induce p53 and caspase3/8 independent apoptosis and have broad anti-tumor activity. Anti-angiogenic activity was described also, which might be related to down-regulation of VEGF and HIF1α. In summary, HDAC inhibition affects tumor cells at different molecular levels and multiple cellular proteins are targeted.

Interestingly, HDAC inhibitors were found to induce cellular differentiation and this pharmacological activity might contribute to their anti-cancer activity as well. For example it was shown that SAHA induces differentiation of breast cancer cell lines, exemplified by resynthesis of milk fat membrane globule protein (MFMG), milk fat globule protein and lipid (Munster et al. Cancer Res. 61, 8492, 2001). The same was shown for the benzamide analog MS275 (Beckers et al. Int. J. Cancer 121, 1138, 2007).

There is a growing rationale for synergism of HDAC inhibitors with chemotherapeutic as well as target specific cancer drugs. For example, synergism was shown for (i) SAHA with the kinase / cdk inhibitor flavopiridol (Alemenara et al. Leukemia 16, 1331-1343, 2002) or with the death receptor DR4/5 ligand TRAIL (Butler et al. Int J Cancer 119, 944-54, 2006; Sonnemann et al. Invest New drugs 23, 90-109, 2005), (ii) for

LAQ-824 with the bcr-abl kinase inhibitor Imatinib in CML cells (Nimmanapalli et al. Cancer Res. 63, 5126-5135, 2003) or the KDR / VEGFR2 kinase inhibitor PTK787/ZK222584 in angiogenesis (Qian et al. Cancer Res. 64, 6626-34, 2004), (iii) for SAHA and Trichostatin A (TSA) with etoposide (VP16), cisplatin and doxorubicin (Kim et al. Cancer Res. 63, 7291-7300, 2003), for TSA in combination with retinoid acid in acute myeloid leukemia / AML (Ferrara et al. Canc Res 61, 2-7, 2001)(iv) for LBH589 with the Hsp90 inhibitor 17-allyl-amino-demethoxy-geldanamycin (17-DMAG; George et al. Blood online, Oct.28, 2004) or the proteasome inhibitor bortezomib /Velcade (Maiso et al. Canc Res 66, 5781-5789, 2006, (iiv) PXD101 with 5-FU in colon cancer models (Tumber et al. Canc Chem Pharmacol nov. 2006) and Taxol or Carboplatin in ovarian carcinoma models (Qian et al. Mol Canc ther 5, 2086-95). Also it was shown that HDAC inhibition causes reexpression of estrogen or androgen receptors in breast and prostate cancer cells with the potential to resensitize these tumors to anti-hormone therapy (Yang et al. Cancer Res. 60, 6890-6894, 2000; Nakayama et al. Lab Invest 80, 1789-1796, 2000). Finally, histone deacetylase inhibitors sensitize towards cellular radiation responses as reviewed recently (Karagiannis & El-Osta, Oncogene 25, 3885-93, 2006).

HDIs from various chemical classes were described in the literature with four most important classes, namely (i) hydroxamic acid analogs, (ii) benzamide analogs, (iii) cyclic peptides / peptolides and (iv) fatty acid analogs. A comprehensive summary of known HDAC inhibitors was published by various authors (Miller et al. J Med Chem 46, 5097-5116, 2003; Dokmanovic & Marks, J Cell Biochem 96, 293-304, 2005; Drummond et al. Ann Rev Pharmacol Toxicol 45, 495-528, 2005). There is only limited data published regarding specificity of these histone deacetylase inhibitors (Beckers et al. Int. J. Cancer 121, 1138, 2007). In general most hydroxamate based HDI are not specific regarding class I and II HDAC enzymes. For example, TSA inhibits HDACs 1, 3, 4, 6 and 10 with IC₅₀ values around 20 nM, whereas HDAC8 was inhibited with IC₅₀ = 0.49 µM (Tatamiya et al, AACR Annual Meeting 2004, Abstract #2451). But there are exceptions like the experimental HDI Tubacin, selective for the class II enzyme HDAC 6 (Haggarty et al. Proc Natl. Acad. Sci. USA 100, 4389-4394, 2003). In addition, data on class I selectivity of benzamide HDIs are emerging. MS-275 inhibited class I HDAC1 and 3 with IC₅₀ = 0.51 µM and 1.7 µM, respectively. In contrast class II HDACs 4, 6, 8 and 10 were inhibited with IC₅₀ values of >100µM, >100µM, 82.5 µM and 94.7 µM, respectively (Tatamiya et al, AACR Annual Meeting 2004, Abstract #2451). Comparable data were published by Hu et al. with inhibition of IC₅₀ = 0.3 µM, 8 µM and >100 µM for HDAC1, 3 and 8, respectively (Hu et al. J Pharmacol Exp Therap 307, 720-28, 2003). Finally, the benzamide analog MGCD0103 inhibited HDAC1, 2, 3 and 11 with IC₅₀ from 0.1-2 µM and HDACs 4 to 8 with IC₅₀ values >20 µM (Kalita et al. AACR-NCI-EORTC Conference Philadelphia 2005; Abstract C216),

Clinical studies in cancer patients with HDAC inhibitors are on-going, namely with SAHA (Zolinza™ by Merck Inc.; Kelly et al. J. Clin. Oncol. 23, 3923-31, 2005), CRA-024781 (Pharmacyclics Inc.; Buggy et al., Mol. Canc. Therap. 5, 1309-17, 2006), ITF-2357 (Italfarmaco; J. Hepatology 42, 210-17, 2005), Valproic acid (Topotarget; Göttlicher et al. EMBO J. 20, 6969-78, 2001), FK228 / Depsipeptide (Gloucester Pharmaceuticals / NC; Nakajima et al. Exp. Cell Res. 241, 126-33, 19981), MS275 (syn SNDX-275; Berlex-Schering / Syndax; Ryan et al. J. Clin. Oncol. 23, 3912-22, 2005), NVP LBH-589 (Novartis; Remiszewski et al. J. Med. Chem. 46, 4609-24, 2003), PXD-101 (Topotarget / Curagen ; Plumb et al. Mol. Canc. Therap. 2, 721-28, 2003), and MGCD0103 (Methylgene Inc.; Kalita et al. AACR-EORTC-NCI meeting 2005, Abstract C216). These studies showed evidence of clinical efficacy, highlighted recently by partial and complete responses with FK228 / Depsipeptide in patients with peripheral T-cell lymphoma (Plekarz et al. Blood, 98, 2865-2868, 2001) and approval in this indication of SAHA (Zolinza™) by Merck Inc., (Biotechn. 25, 17-18, 2007; Olsen et al. J. Clin. Oncol. 25, 3109, 2007).

Various publications also showed possible medical use of HDAC inhibitors in diseases different to cancer. These diseases include systemic lupus erythematosus (Mishra et al. J. Clin. Invest. 111, 539-552, 2003; Reilly et al. J. Immunol. 173, 4171-4178, 2004), rheumatoid arthritis (Chung et al. Mol. Therapy 8, 707-717, 2003; Nishida et al. Arthritis & Rheumatology 50, 3365-3376, 2004), inflammatory diseases (Leoni et al. Proc. Natl. Acad. Sci. USA 99, 2995-3000, 2002), neurodegenerative diseases like Huntington's disease (Steffan et al. Nature 413, 739-743, 2001, Hockly et al. Proc. Natl. Acad. Sci. USA 100(4):2041-6, 2003), cardiac hyperthrophy (Kong et al., Circulation 113, 2579-88, 2006), muscle dystrophy (Minetti et al. Nat. Med. 12, 1147-50, 2006), adipositas (Lagace & Nachtigal, J. Biol. Chem. 279, 18851-860, 2004) and diabetes (Gray & DeMeyts, Diabetes Metab. Res. Rev. 21, 416-33, 2005).

Cancer chemotherapy was established based on the concept that cancer cells with uncontrolled proliferation and a high proportion of cells in mitosis are killed preferentially. Standard cancer chemotherapeutic drugs finally kill cancer cells upon induction of programmed cell death ("apoptosis") by targeting basic cellular processes and molecules, namely RNA/DNA (alkylating and carbamylating agents, platin analogs and topoisomerase inhibitors), metabolism (drugs of this class are named anti-metabolites) as well as the mitotic spindle apparatus (stabilizing and destabilizing tubulin inhibitors). HDIs constitute a new class of anti-cancer drugs with differentiation and apoptosis inducing activity. By targeting histone deacetylases, HDIs affect histone (protein) acetylation and chromatin structure, inducing a complex transcriptional reprogramming, exemplified by reactivation of tumor suppressor genes and repression of oncogenes. Non-histone targets important for cancer cell biology have been described, including heat-shock-protein 90 (Hsp90; Bali et al. J. Biol. Chem. 280, 26729-734, 2005), α-tubulin (Hubbert et al. Nature 417, 455-58, 2002), STAT1 or STAT3 (Yuan et al. Science 307, 269-273, 2005; Krämer et al. Gen & Develop 20, 473-485, 2006) or the p53 tumor suppressor protein (Mol. Cell 24, 807-808, 2006). The medical use of HDIs might not be restricted to cancer therapy, since efficacy in animal models for e.g., rheumatoid arthritis, neurodegeneration, cardiac hyperthrophy and muscle dystrophy has been shown.

Another therapeutic approach is based on inhibition of protein kinases having pathopyhsiological relevance. In particular, inhibition of protein kinases is an effective therapeutic approach for the treatment of a wide range of human malignacies (Dancey et al. Nat. Drug Discovery 2, 296-313, 2003). Protein kinases are key regulators in many cellular processes like signal transduction, proliferation, cell cycle regulation, differentiation and survival / apoptosis as well as pathophysiological alterations within these processes causing diseases. Thus, protein kinases constitute an important target class for therapeutic intervention (P.Cohen, Nature Rev. Drug Discovery 1, 309, 2002; T.G. Cross, et al., Exp. Cell Res. 256, 34-41, 2000). They can be categorized regarding their substrate specificity, namely enzymes specific for tyrosine and / or serine / threonine residues.

As extensively documented for the BCR-ABL oncogene in Imatinib (Gleevec / ST1571 / CGP57148) treated chronic myeloid leukemia (CML) patients, clinical resistance caused by multiple mutations affecting Imatinib binding to the bcr-abl kinase protein have been observed (Shah et al. Cancer Cell 2, 117-125, 2002; Daub et al. Cancer Res. 63, 6395-6404, 2004). One frequent mutation found is T³¹⁵I within the ATP-binding site of BCR/ABL, abrogating a direct hydrogen bond of Imatinib with T³⁰⁴ of bcr-abl (Shah et al. Cancer Cell 2, 117-125, 2002, Talpaz et al. N. Engl. J. Med. 354, 2531-2541, 2006; Branford et al. Blood 99, 3472-3475, 2002). In vitro studies of resistance to Imatinib indicate additional mechanisms of resistance, including overexpression of BCR/ABL caused by gene amplification and increased Imatinib efflux mediated by the multidrug resistance P-glycoprotein. A strategy to circumvent drug resistance of CML patients towards Imatinib therapy is examplified by two drugs recently approved for CML therapy: the abl/src dual kinase inhibitor Dasatinib (BMS-354825; Lombardo et al. J Med Chem 47, 6658-6661, 2004; Talpaz et al. N. Engl. J. Med. 354, 2531-2541, 2006) and the Imatinib analog Nilotinib (AMN-107; Weisberg et al. Cancer Cell 7, 129-141, 2005; Kantarjian et al. N. Engl. J. Med. 354, 2542-2551, 2006). Whereas Nilotinib is a more potent, close analog of Imatinib also binding to the inactive abl kinase, Dasatinib as a 2-aminopyridinyl-thiazol analog inhibits abl and src kinases in the active and inactive conformation with high potency. Both agents are effective towards most clinically relevant bcr-abl mutants with the exception of the T³¹⁵I mutant (Weisberg et al. Cancer Cell 7, 129-141, 2005; Talpaz et al. N. Engl. J. Med. 354, 2531-2541, 2006).

Another, still preclinical strategy was examplified by combination of Imatinib or Dasatinib with inhibitors of histone deacetylases. Cotreatment with LAQ824, a close analog of LBH589 or SAHA as HDIs increased Imatinib and Dasatinib induced apoptosis of K562 and LAMA-84 blast crisis CML cell lines (Yu et al. Cancer Res. 63, 2118-26, 2003; Fiskus et al. Clin. Canc. Res. 12, 5869-5878, 2006). The combination showed additive or synergistic effects which were explained by down-regulation of wild-type and mutant Bcr-Abl T³¹⁵I protein (Nimmanapalli et al. Canc Res 63, 5126-5135,2003).

A second important kinase target are the members of the epidermal growth factor receptor (EGF-R) familiy, namely EGF-R (HER1), HER2 / c-erbB2 and their heterodimerization partners HER3 and HER4. Approved drugs for treatment of advanced non-small cell lung and breast cancer inhibiting EGFR and HER2 receptor protein kinases are Erlotinib (Tarceva), Gefitinib (Iressa) and Lapatinib (Tykerb). These selective competitive kinase inhibitors have shown clinical efficacy in particular in combination therapy (e.g. Lapatinib / Tykerb in combination with Capecitabine /Xeloda). Beside small molecule protein kinase inhibitors, monoclonal antibodies adressing EGFR or HER2 have been developed (summarized and compared to small molecule kinase inhibitors in Imai & Takaoka Nature Rev. Cancer 6, 724, 2006). Erbitux (C225 / Cetuximab) is a monoclonal antibody specific for the EGFR/HER1 protein, approved for the treatment of colorectal carcinoma in combination with Irinotecan (Campthotecin). Herceptin (Trastuzumab) is a monoclonal antibody targeting HER2, approved for treatment of advanced breast cancer patients in monotherapy or combination therapy with Taxotere. These examples ilustrate the pathyphysiologal relevance of adressing EGFR and/or HER2 in cancer treatment. Drug resistance developed under therapy with these agents includes mutation of the EGF-R by treatment with Gefitinib (Sharma et al. Nature Rev. Cancer 7, 169, 2007), up-regulation of estrogen receptor (ER) by treatment with Lapatinib (Xia et al. Proc Natl Acad Sci 103, 7795, 2006) or usage of alternative signaling pathways like c-met overexpression under Gefitinib therapy (Enbgelman et al. 316, 1039, 2007). Therefore combination of EGFR /HER2 protein kinase inhibitors with cytotoxic or targeted agents is a highly useful clinical strategy to lower the risk of immediate drug resistance. In this regard, a combination of the HDAC inhibitor SAHA with the EGFR kinase inhibitors Gefitinib was shown to have synergistic activity in head and neck cancer (Bruzesse et al. EORTC/NCI/AACR conference Philadelphia 2005).

### Object of the Invention

The object of the present invention is to provide novel single compounds that are effective as active pharmaceutical ingredients of medicaments, particularly compounds that have an anti-proliferative and/or cancer cell apoptosis inducing activity and hence are useful in the treatment of e.g. benign and malignant neoplasias and other diseases that are sensitive to histone deacetylase and/or protein kinase inhibitor therapy. The compounds of the present invention are bifunctional compounds that are inhibitors of both, protein kinases and HDACs. It has been surprisingly found that such bifunctional compounds, which are described in greater detail below, differ profoundly from prior art compounds and have advantageous pharmacological properties. It seems that compounds of the present invention are indeed effective in blocking both protein kinases and HDACs which could not be predicted in view of the fact that it was unknown whether the respective targets of the inhibitory moieties of the inventive compounds tolerate both pharmacological properties, so that one single compound can indeed inhibit more than one target class / enzyme family. Moreover, it was unclear and not predictable if a sufficient degree of binding affinity / inhibitory potency of the compounds of the present invention to both target classes, protein kinases and HDACs, at therapeutically reasonable concentrations could be achieved, thus allowing for a significant functional inhibition of both enzymes.

### Summary of the invention

Thus, the present invention relates to a bifunctional compound of formula I or its pharmaceutically acceptable salts or solvates

A-L-B (I)

wherein A is a histone deacetylase (HDAC) inhibitory moiety, L is a single bond or a linker group and B is a protein kinase inhibitory moiety. The bifunctional compound according to formula (I) is useful as an active ingredient for the treatment of malignant and non-malignant neoplasia and diseases related to abnormal cell growth.

In a preferred embodiment, the HDAC inhibitory moiety A according to formula (I) contains (i) a hydroxamic acid group, and (ii) a benzamide group. A particularly preferred moiety A can be selected from the following groups: 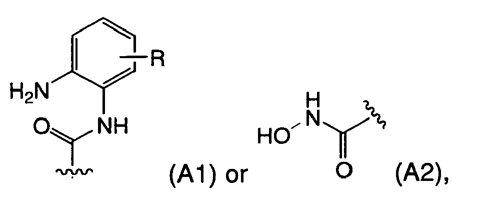 wherein R is hydrogen, halogen, (C₁-C₄)alkyl, (C₁-C₄)alkoxy, thienyl, or N(R¹²)R¹³
wherein R¹² and R¹³ independently of one another are hydrogen, (C₁-C₄)alkyl, (C₁-C₄)alkylcarbonyl, or R¹² and R¹³ together and with inclusion of the nitrogen atom to which they are bonded form an azetidinyl-, pyrrolidinyl-, piperidinyl-, piperazinyl-, 4-methylpiperazinyl-, morpholinyl- or thiomorpholinyl-ring; more specifically R is is hydrogen, halogen, (C₁-C₄)alkyl, (C₁-C₄)alkoxy, thienyl, or N(R¹²)R¹³ wherein R¹² and R¹³ independently of one another are hydrogen, (C₁-C₄)alkyl, or R¹² and R¹³ together and with inclusion of the nitrogen atom to which they are bonded form a pyrrolidinyl ring; even more specifically R is hydrogen, fluorine, chlorine, methyl, ethyl, propyl, isopropyl, methoxy, ethoxy, thienyl, N(R¹²)R¹³ wherein R¹² and R¹³ independently of one another are hydrogen, methyl, ethyl, or R¹² and R¹³ together and with inclusion of the nitrogen atom to which they are bonded form a pyrrolidinyl-ring. In a preferred embodiment R is hydrogen.

The group L according to formula (I) can be a single bond or a linker group that separates the moiety A and the moiety B. L is preferably selected from
(a) a straight or branched C₁-C₆ alkylene group, a C₂-C₆ alkenylene group, a C₂-C₆ alkinylene group, a C₃-C₆ cycloalkylene group, each of which may optionally be interrupted by -0-, -S-, -COO-, -NHCO- or arylene,
(b) a group of the formula

   D-Ar-E
wherein D and E may be the same or different and are selected from a bond, a straight or branched C₁-C₆ alkylene group, C₂-C₆ alkenylene group or C₂-C₆ alkinylene group, a C₃-C₆ cycloalkylene group, an amide group, a sulfinyl group, a sulfonyl group, -O-, -NH-, -N(C₁-C₆ alkyl)- and -S-; Ar is an aryl group with 5-10 carbon atoms, an alkylaryl group wherein alkyl is C₁-C₆ and aryl is C₅-C₁₀, a heteroaryl group with 5-10 carbon atoms and 1-3 heteroatoms, selected from O, S and N.

In a further preferred embodiment, the linker group L is an ethylene group, a trans-ethenylene group, or L is

D-Ar-E

wherein D is selected from a bond or a trans-ethenylene group, and E is selected from a bond, an amide group, a sulfonyl group, and -O-, and

Ar is selected from a phenyl group, a benzyl group, a pyridinyl group, a pyrimidinyl group, a thienyl group or a pyrrolyl group.

Preferably, the protein kinase inhibitory moiety B of the compound according to formula (I) has an enzyme specificity for at least one kinase selected from (i) tyrosine kinase ,(ii) serine kinase and (iii) dual-specificity kinases. In a further preferred embodiment the protein kinase inhibitory moiety B of the compound according to formula (I) inhibits the bcr-abl, PDGFR, HER1 and/or HER2 protein kinases as well as mutants of these protein kinases, including but not limited to point mutations and fusion proteins. The protein kinase inhibitory moiety B is preferably selected from 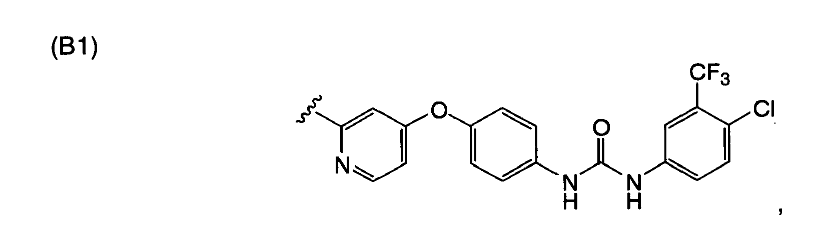 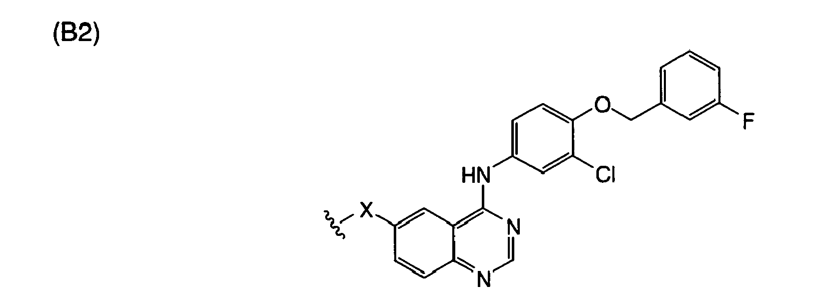 wherein X is a 5- or 6-membered carbocyclic or heterocyclic aromatic ring moiety, preferably a 2-, 3-, or 4-pyridyl or phenyl, or a 2- or 3-pyrrolyl, furanyl or thienyl, 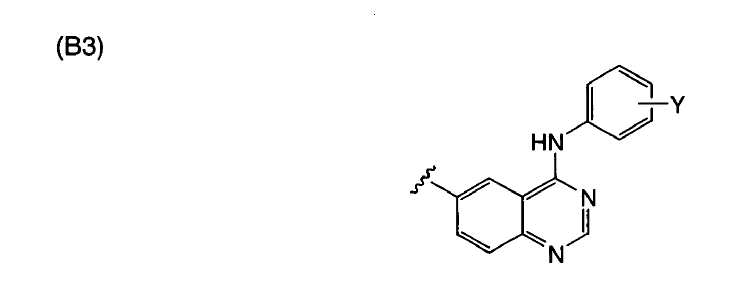 wherein Y is F, Cl, Br, I, an optionally substituted C₁-C₆ alkyl group, an optionally substituted C₂-C₆ alkenyl group, an optionally substituted C₂-C₆ alkinyl group, and 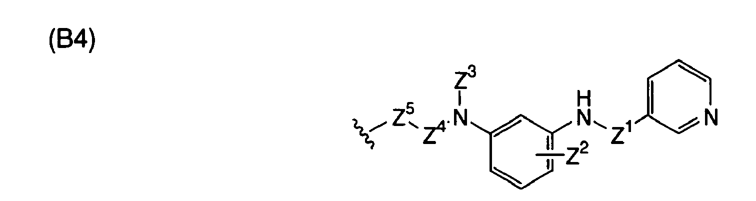 wherein Z¹ is a 5- or 6-membered aromatic or heteroaromatic ring, Z² is a hydrogen atom, a C₁-C₆ straight, branched alkyl group or a halogen atom, Z³ is a hydrogen atom, a C₁-C₆ straight or branched alkyl group, or a C₃-C₆ cycloalkyl group, Z⁴ is a -CH₂- group or a -CO- group, and Z⁵ is a 5- or 6-membered aromatic or heteroaromatic ring.

In a particularly preferred embodiment, the protein kinase inhibitory moiety B is selected from 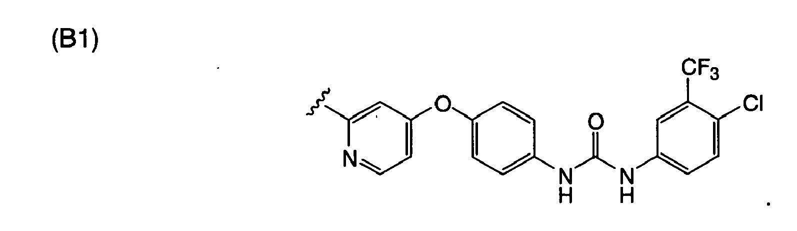 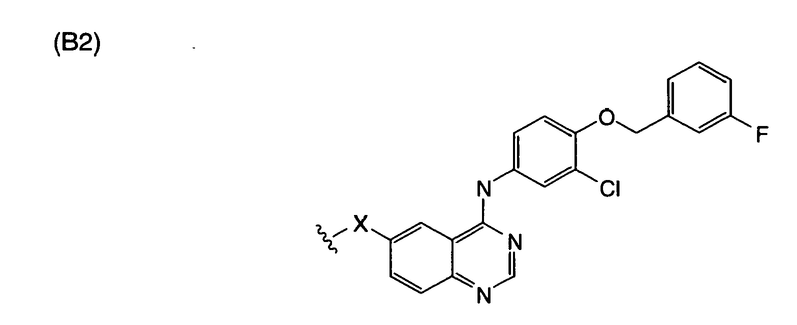 wherein X is a furanyl moiety, a thienyl moiety, or a phenyl moiety, 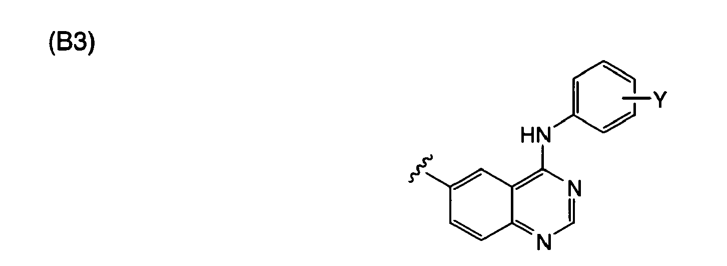 wherein Y is Br or an ethinyl group, 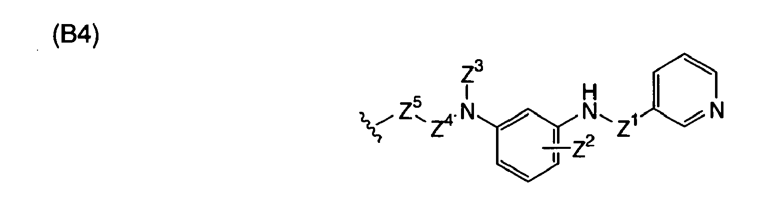 wherein Z¹ is a pyrimidinyl moiety or a thiazolyl moiety,
Z² is selected from a hydrogen atom or a methyl group or a chlorine atom,
Z³ is selected from a hydrogen atom or a methyl group,
Z⁴ is a -CH₂- group or a -CO- group, and
Z⁵ is a phenyl moiety, a pyrimidyl moiety or a thienyl moiety.

### Definitions

"Histone deacetylase" (HDAC) means an enzyme with an activity towards the ε-acetyl group of lysine residues within a substrate protein. HDAC substrates are histone H2A, H2B, H3 or H4 proteins and isoforms as well as substrate proteins different to histones like, but not limited to, heat shock protein 90 (Hsp90), α-tubulin, STAT1 or STAT3 and the tumor suppressor protein p53. In particular histone deacetylases catalyse the hydrolysis of the ε-acetyl group of lysine residues within these substrate proteins, forming the free amino group of lysine.

Inhibition of histone deacetylase by compounds according to this invention means inhibiting the activity and function of one or more HDAC isoenzymes, in particular isoenzymes selected from the so far known histone deacetylases, namely HDAC 1, 2, 3 and 8 (class I) and HDAC 4, 5, 6, 7, 10 (class II), HDAC 11 (class IV) as well as the NAD+ dependent class III (Sir2 homologes). In a preferred embodiment this inhibition is at least about 50%, more preferable at least 75% and still more preferable above 90% of the original HDAC activity. Preferably, this inhibition is specific to a defined histone deacetylase class (eg HDAC class I enzymes), a selection of isoenzymes of highest pathophysiological relevance (eg HDAC 1, 2, 3 enzymes) or a single isoenzyme (eg the HDAC 1, HDAC 2 or HDAC 3 enzyme). The term histone deacetylase inhibitory compound or moiety is used to identify a compound or moiety capable of interacting with a histone deacetylase and inhibiting its activity, in particular its enzymatic activity by at least 50%. In this context "head group" defines the residues within a histone deacetylase inhibitor responsible for interacting with the active site of the enzyme, eg the Zn²⁺ ion.

The inhibition of histone deacetylases can be determined in biochemical assays of various formats and sources of enzymatic activity. HDAC activity is used either derived from nuclear or cellular extracts or by heterologous expression of a defined HDAC isoenzyme in E.coli, insect cells or mammalian cells. Since HDAC isoenzymes are active in multiprotein complexes and form homo- and heterodimeres, nuclear extracts derived from human cancer cells, for example the human cervical carcinoma cell line HeLa, are preferred. These nuclear extracts contain class I and class II enzymes, but are enriched in class I enzymes. For expression of recombinant HDAC isoenzymes, mammalian expression systems like HEK293 cells are preferred. Nevertheless, heterologous expression of HDAC isoenzymes in insect cells alone or coexpressed with relevant human cofactors is a suitable alternative approach.The HDAC isoenzyme is expressed as a fusion protein with an affinity tag, like the FLAG epitope. By affinity chromatography, the tagged protein is purified alone or in complex with endogenous / co-expressed proteins (eg other HDAC isoenzmyes and coactivators / platform proteins).

The biochemical assays are well described and well known to persons skilled in the art. As substrates, histone proteins, peptides derived from histone proteins or other HDAC substrates as well as acetylated lysine mimetics are used. Preferred promiscous HDAC substrates are for example the tripeptide Ac-NH-GGK(Ac) or the lysine mimetic Boc-K(Ac), also known as Flour-de-Lys and commercially available by Biomol, coupled with the fluorophore 7-amino-4-methylcoumarin (AMC).

For the purposes of defining a histone deacetylase (HDAC) inhibitory moiety according to the present invention, reference is made to the fluorimetric rHDAC1 activity assay described below. Compounds according to the present invention containing such moieties should have an IC₅₀ of at most 1µM in this assay, more preferably an IC₅₀ <1µM.

"Protein kinase" means an enzyme with an activity towards the hydroxyl group of tyrosine, serine or threonine residues within a substrate protein. In particular protein kinases catalyse the hydrolysis the γ-phosphate of ATP and transfer of this phosphate on the hydroxyl group of tyrosine, serine or threonine residues forming a phosphate ester linkage.

Inhibition of protein kinases and analogous terms means inhibiting the activity and function of one or more protein kinases, in particular protein kinases selected from a group of kinases with pathophysiological importance for human therapy, in particular cancer treatment. Within this group of kinases, the abl protein kinase, respective fusions proteins like BCR-abl or BCR-abl mutants, PDGFR, members of the EGFR family like EGFR/HER1 and/or HER2 as well as mutants thereof including point mutants and fusion proteins are of particular importance.

Interesting in the context of this invention are also those compounds that are selective in inhibition of the protein kinase BCR-abl or BCR-abl mutants, EGFR familiy members EGFR and / or HER2 or a selection of protein kinases most relevant or causative involved in a disease state, in particular in cancer, like those kinases mentioned before and in addition protein kinases like c-kit, PDGFR, VEGFR2 / KDR, c-met, Plk1 or PKB/Akt. This means that those compounds exhibit greater inhibition against said protein kinase(s), when compared to the compounds inhibition of the activity of other house-keeping protein kinases like protein kinase A (PKA) where inhibition might cause unspecific toxicity and reduce the therapeutic window of said compounds.

For the purposes of defining a protein kinase inhibitory moiety according to the present invention, reference is made to the biochemical protein kinase activity assays described in the experimental section. Compounds according to the present invention containing such moieties should have an IC₅₀ of at most 1 µM and more preferable an IC₅₀ <1µM in this assay.

"Linker" as mentioned above can either be a single bond or an organic group that links the HDAC inhibitory moiety A and the protein kinase inhibitory moiety B, without substantially affecting their respective pharmacological activities. The linker serves to provide a distance between the moiety A and the moiety B.

"Chimeric or bifunctional" are terms used interchangeably to describe a compound that combines two pharmacological activities in one molecule. In this invention, chimeric / bifunctional compounds inhibit protein kinases and histone deacetylases in respective biochemical and / or cellular assays.

"Cellular activity" of a histone deacetylase inhibitor means any cellular effect related to histone deacetylase inhibition, like inhibition of histone deacetylase activity as measured by using a cellular permeable HDAC substrate, protein hyperacetylation, transcriptional repression and activation, induction of apoptosis, differentiation and /or cytotoxicity.

Cellular activity and analogous terms of a protein kinase or HDAC inhibitor means any cellular effect related to protein kinase or HDAC inhibition, in particular dephosphorylation or hyperacetylation, respectively, of defined substrate proteins causing, as an example, induction of apoptosis.

The term "induction of apoptosis" and analogous terms are used to identify a compound which excecutes programmed cell death in cells contacted with that compound. Apoptosis is defined by complex biochemical events within the contacted cell, such as the activation of cystein specific proteinases ("caspases") and the fragmentation of chromatin. Induction of apoptosis in cells contacted with the compound might not necessarily coupled with inhibition of cell proliferation or cell differentiation. Preferably, the inhibition of proliferation, induction of differentiation and/or induction of apoptosis is specific to cells with aberrant cell growth.

"Cytotoxicity" in general means arresting proliferation and/or inducing apoptotic cell death in vitro in mammalian cells, in particular human cancer cells.

"Cell cycle independent mode of action" means no or only weak discrimination between proliferating (cycling) and non-proliferating (arrested) cells regarding cytotoxicity / apoptosis induction. As a consequence of a cell cycle independent mode of action, pharmacologically active compounds kill proliferating and dormant neoplastic cells.

Assays for quantification of cell proliferation, percentage of cells in respective cell cycle stages, apoptosis or differentiation are well known to experts and state of the art. For example, metabolic activity which is linked to cellular proliferation is quantified using the Alamar Blue / Resazurin assay (O'Brian et al. Eur J Biochem 267, 5421-5426, 2000) and induction of apoptosis is quantified by measurement of chromatin fragmentation with the cell death detection ELISA (Roche Biochemicals). Distribution of cells in respective stages of the cell cycle (G1, G2, G2/M, sub G1) is quantified by flow cytometry after DNA staining with e.g. propidiumiodide.

Examples for cellular assays for the determination of hyperacetylation of HDAC substrates are given by measuring core histone acetylation using specific antibodies by Western blotting, reporter gene assays using respective responsive promoters or promoter elements (eg the p21 promotor or the sp1 site as responsive element) or finally by image analysis again using acetylation specific antibodies for core histone proteins. Cellular enzymatic HDAC activity can be quantified by using the cell permeable HDAC substrate Boc-K(Ac)-AMC /Flour-deLys).

Examples for cellular assays for protein kinase inhibition are given by measuring substrate phosphorylation after treatment with the compounds by various means, including SDS-PAGE / Western blot analysis, Bioplex / bead suspension assays and ELISAs. Cellular activity and analogous terms of a protein kinase inhibitor means any cellular effect related to protein kinase inhibition, in particular dephosphorylation of defined substrate proteins causing, as an example, induction of apoptosis.

A "neoplasia" is defined by cells displaying aberrant cell proliferation and/or survival and/or a block in differentiation. The term neoplasia includes "benign neoplasia" which is described by hyperproliferation of cells, incapable of forming an aggressive, metastasizing tumor in vivo, and, in contrast, "malignant neoplasia" which is described by cells with multiple cellular and biochemical abnormalities, capable of forming a systemic disease, for example forming tumor metastasis in distant organs.

### Detailed description of the invention

The present invention is directed to bifunctional compounds that are inhibitiors of both, protein kinases and HDACs. It has been surprisingly found that the bifunctional compounds of the present invention and their pharmaceutically acceptable salts and solvates differ profoundly from prior art compounds and are effective inhibitors of histone deacetylases and protein kinases with surprising and advantageous properties.

Compounds according to this invention can be therapeutically useful and hence industrially applicable due to their HDAC and /or protein kinase inhibitory, anti-proliferative and/or apoptosis inducing activity. Such activities may be beneficial in the therapy of diseases responsive thereto, such as e.g. any of those diseases mentioned herein.

The compounds according to the present invention are preferably used for the treatment of malignant neoplasia, also described as cancer, characterized by tumor cells finally metastasizing into distinct organs or tissues. Examples of malignant neoplasia treated with the bifunctional compounds according to the present invention include solid and hematological tumors. Solid tumors are exemplified by tumors of the breast, bladder, bone, brain, central and peripheral nervus system, colon, endocrine glands (e.g. thyroid and adrenal cortex), esophagus, endometrium, germ cells, head and neck, kidney, liver, lung, larynx and hypopharynx, mesothelioma, ovary, pancreas, prostate, rectum, renal, small intestine, soft tissue, testis, stomach, skin, ureter, vagina and vulva. Malignant neoplasia include inherited cancers exemplified by Retinoblastoma and Wilms tumor. In addition, malignant neoplasia include primary tumors in said organs and corresponding secondary tumors in distant organs ("tumor metastases"). Hematological tumors are exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, cancers of unknown primary site as well as AIDS related malignancies.

It is to be noted that a cancer disease as well as a malignant neoplasia does not necessarily require the formation of metastases in distant organs. Certain tumors exert devastating effects on the primary organ itself through their aggressive growth properties. These can lead to the destruction of the tissue and organ structure finally resulting in failure of the assigned organ function.

Neoplastic cell proliferation might also affect normal cell behaviour and organ function. For example the formation of new blood vessels, a process described as neovascularization, is induced by tumors or tumor metastases. Compounds according to the invention can be therapeutically useful for treatment of pathophysiological relevant processes caused by benign or neoplastic cell proliferation, such as but not limited to neovascularization by unphysiological proliferation of vascular endothelial cells. Drug resistance is of particular importance for the frequent failure of standard cancer therapeutics. This drug resistance is caused by various cellular and molecular mechanisms like overexpression of drug efflux pumps, mutation within the cellular target protein, overexpression of functional related targets and constitutive activation of alternative signaling pathways, . The commercial applicability of compounds according to the present invention is not limited to 1^{st} line treatment of patients. Patients with resistance to cancer chemotherapeutics or target specific anti-cancer drugs can be also amenable for treatment with the compounds of the present invention for e.g. 2^{nd} or 3^{rd} line treatment cycles. Examples are given by CML patients with mutant BCR-abl fusion proteins (eg T³¹⁵I resistant to standard therapy with Glivec / Imatinib, acute myeloid leukemia (AML) patients with the PML-RAR fusion protein resistant to standard therapy with retinoids, or patients with 2^{nd} site mutated EGFR (T⁷⁹⁰M or D⁷⁶¹Y) protein who are resistant towards Gefitinib or Erlotinib therapy. Also the compounds of the present invention are useful for treating patients with a resistance not caused by a mutation of the direct target, eg those cancer patients with constitutive activated alternative pathways, eg the constitutive activation of the Pi3K/Akt pathway by c-met overexpression.

The invention relates, in its most general aspect to a compound of formula I or its pharmaceutically acceptable salts or solvates

A-L-B (I)

wherein A is a histone deacetylase (HDAC) inhibitory moiety, L is a single bond or a linker group and B is a protein kinase inhibitory moiety. Preferred compounds according to formula (I) are further defined in that the HDAC inhibitory moiety A contains (i) a hydroxamic acid group, and (ii) a benzamide group.

The HDAC inhibitory moiety A of the bifunctional compound can for example be selected from 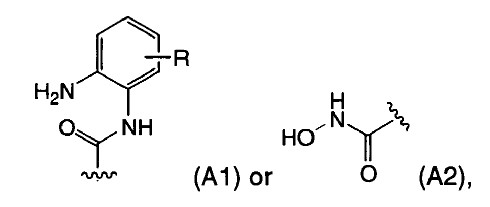 wherein R is hydrogen, halogen, (C₁-C₄)alkyl, (C₁-C₄)alkoxy, thienyl, or N(R¹²)R¹³
wherein R¹² and R¹³ independently of one another are hydrogen, (C₁-C₄)alkyl, (C₁-C₄)alkylcarbonyl, or R¹² and R¹³ together and with inclusion of the nitrogen atom to which they are bonded form an azetidinyl-, pyrrolidinyl-, piperidinyl-, piperazinyl-, 4-methylpiperazinyl-, morpholinyl- or thiomorpholinyl-ring;
more specifically R is is hydrogen, halogen, (C₁-C₄)alkyl, (C₁-C₄)alkoxy, thienyl, or N(R¹²)R¹³ wherein R¹² and R¹³ independently of one another are hydrogen, (C₁-C₄)alkyl, or R¹² and R¹³ together and with inclusion of the nitrogen atom to which they are bonded form a pyrrolidinyl- ring; even more specifically R is hydrogen, fluorine, chlorine, methyl, ethyl, propyl, isopropyl, methoxy, ethoxy, thienyl, N(R¹²)R¹³ wherein R¹² and R¹³ independently of one another are hydrogen, methyl, ethyl, or R¹² and R¹³ together and with inclusion of the nitrogen atom to which they are bonded form a pyrrolidinyl-ring. In a preferred embodiment of (A1), R is hydrogen.

In another specific embodiment of this invention, A is a HDAC inhibitory moiety derived (e.g. by abstraction of a hydrogen atom) from any of the following HDAC inhibitors: SAHA (Zolinza™ by Merck Inc.), CRA-024781, ITF-2357, Valproic acid, FK228 / Depsipeptide, MS275, NVP LBH-589, PXD-101, and MGCD0103.

The group L of formula (I) as depicted above is selected from
(L1) a single bond, a straight or branched C₁-C₆ alkylene group, a C₂-C₆ alkenylene group, a C₂-C₆ alkinylene group, a C₃-C₆ cycloalkylene group, each of which may optionally be interrupted by -O-, -S-, -COO-, -NHCO- or arylene,
(L2) a group of the formula

D-Ar-E

wherein D and E may be the same or different and are selected from a bond, a straight or branched C₁-C₆ alkylene group, C₂-C₆ alkenylene group or C₂-C₆ alkinylene group, a C₃-C₆ cycloalkylene group, an amide group, a sulfinyl group, a sulfonyl group, -O-, -NH-, -N(C₁-C₆ alkyl)- and -S-;

Ar is an aryl group with 5-10 carbon atoms, an alkylaryl group wherein alkyl is C₁-C₆ and aryl is C₅-C₁₀, a heteroaryl group with 5-10 carbon atoms and 1-3 heteroatoms, selected from O, S and N.

In a preferred embodiment, the linker group L is (L3) an ethylene group, (L4) a trans-ethenylene group, or

D-Ar-E

wherein D is selected from a bond or a trans-ethenylene group, and E is selected from a bond, an amide group, a sulfonyl group, and -O-, and Ar is selected from a phenyl group, a benzyl group, a pyridinyl group, a pyrimidinyl group, a thienyl group or a pyrrolyl group.

The protein kinase inhibitory moiety B from (i) tyrosine kinase ,(ii) serine kinase and (iii) dual-specificity kinases. In a preferred embodiment the protein kinase inhibitory moiety B of the compound according to formula (I) inhibits the bcr-abl kinase protein or the EGFR and/or HER2 receptor kinase proteins.

The protein kinase inhibitory moiety B can be selected from the following groups (B1)-(B4): 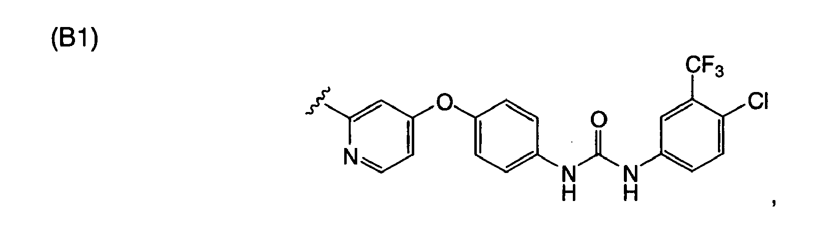 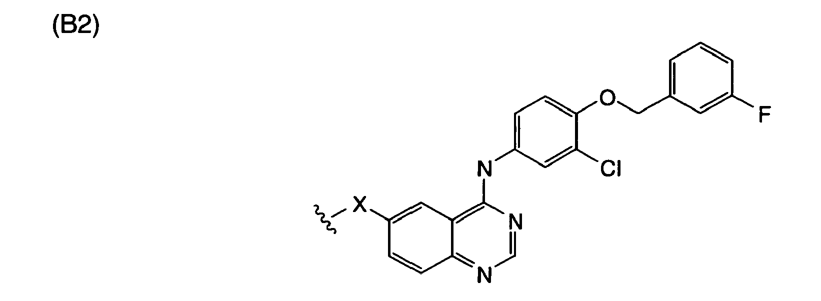 wherein X is a 5- or 6-membered carbocyclic or heterocyclic aromatic ring; 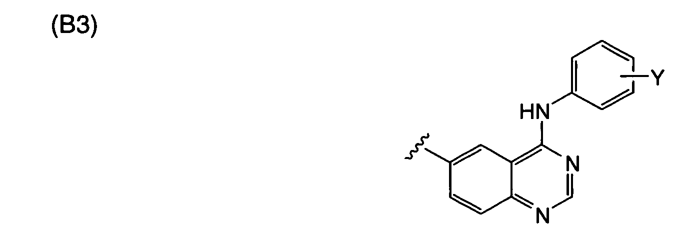 wherein Y is F, Cl, Br, I, an optionally substituted C₁-C₆ alkyl group, an optionally substituted C₂-C₆ alkenyl group, an optionally substituted C₂-C₆ alkinyl group, and 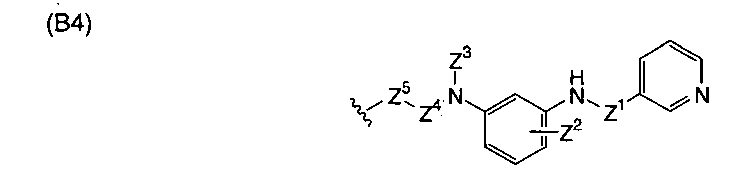 wherein Z¹ is a 5- or 6-membered aromatic or heteroaromatic ring,
Z² is a hydrogen atom, a C₁-C₆ straight or branched alkyl group, or a halogen atom, Z³ is a hydrogen atom, a C₁-C₆ straight or branched alkyl group, or a C₃-C₆ cycloalkyl group,
Z⁴ is a -CH₂ group or a -CO- group, and
Z⁵ is a 5- or 6-membered aromatic or heteroaromatic ring.

In a preferred embodiment, the protein kinase inhibitory moiety B is selected from 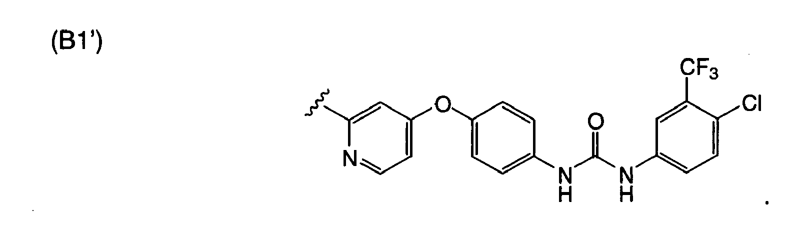 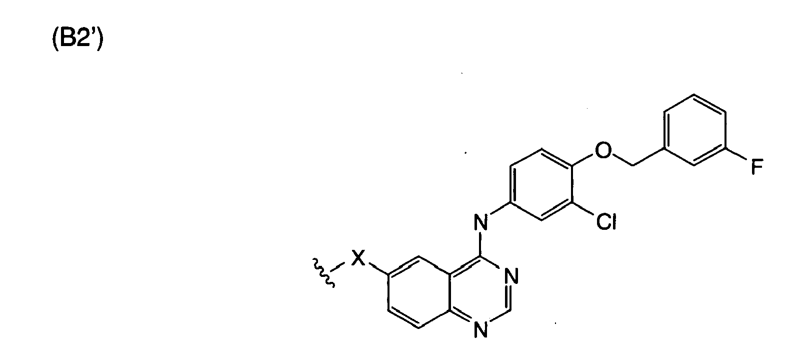 wherein X is a furanyl moiety, a thienyl moiety, or a phenyl moiety. 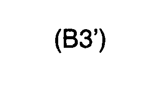 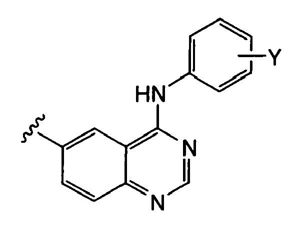 wherein Y is Br or an ethinyl group; 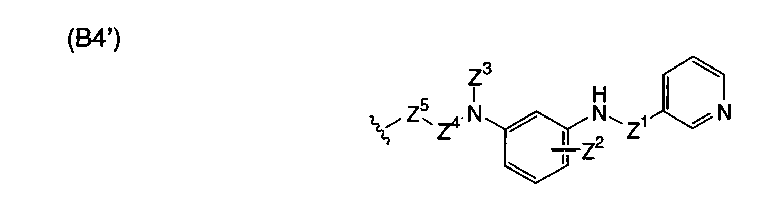 wherein Z¹ is a pyrimidinyl moiety or a thiazolyl moiety,
Z² is selected from a hydrogen atom, a methyl group or a chlorine atom,
Z3 is selected from a hydrogen atom or a methyl group,
Z⁴ is a -CH₂- group or a -CO- group, and
Z⁵ is a phenyl moiety, a pyrimidyl moiety or a thienyl moiety.

The moiety B may also be represented by the following formula (B3") 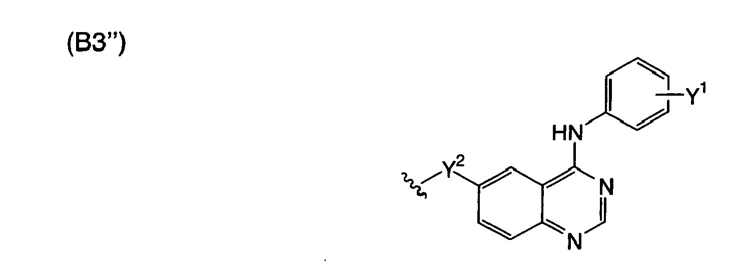 wherein Y¹ has the same meaning as Y in (B3), and Y² is selected from a -NHCO-Ar moiety, a -OCH₂-Ar moiety, and a -SO₂-Ar moiety,
Ar is an aryl group with 5-10 carbon atoms, an alkylaryl group wherein alkyl is C1-C6 and aryl is C5-C10, a heteroaryl group with 5-10 carbon atoms and 1-3 heteroatoms, selected from O, S and N.

In a preferred mode, compound (B3) as depicted above bears substituent Y in meta-position. Substitutent Z² of compound (B4) is preferably in ortho-position of the ring.

In another more specific aspect of the present invention, the moiety B can be a protein kinase inhibitory moiety derived (e.g. by abstraction of a hydrogen atom) from any of the following protein kinase inhibitors: Imatinib, Dasatinib, Nilotinib, Erlotinib, Gefitinib, Lapatinib, Sunitinib, Sorafenib, Vatalanib, Vandetanib, Pazopanib.

In a preferred aspect, the present invention is directed to a compound of formula (I)

A-L-B (I)

or its pharmaceutically acceptable salts or solvates, wherein A is a HDAC inhibitory moiety selected from the groups (A1) and (A2) as defined above, L is selected from (L1), (L2), (L3) and (L4) as defined above, and B is selected from (B1), (B2), (B3) and (B4) as defined above. In yet a further aspect hereof, B is selected from (B1), (B2'), (B3') and (B4') as defined above

As illustrated in the table below, A, L and B can be permutated to give the follwing groups of substances, each of which forms a further aspect of the present invention:

| **Group No.** | **A** | **L** | **B** |
|---|---|---|---|
| **1** | A1 | L1 | B1 |
| **2** | A1 | L1 | B2 |
| **3** | A1 | L1 | B3 |
| **4** | A1 | L1 | B4 |
| **5** | A1 | L2 | B1 |
| **6** | A1 | L2 | B2 |
| **7** | A1 | L2 | B3 |
| **8** | A1 | L2 | B4 |
| **9** | A2 | L1 | B1 |
| **10** | A2 | L1 | B2 |
| **11** | A2 | L1 | B3 |
| **12** | A2 | L1 | B4 |
| **13** | A2 | L2 | B1 |
| **14** | A2 | L2 | B2 |
| **15** | A2 | L2 | B3 |
| **16** | A2 | L2 | B4 |
| **17** | A1 | L3 | B1 |
| **18** | A1 | L3 | B2 |
| **19** | A1 | L3 | B3 |
| **20** | A1 | L3 | B4 |
| **21** | A1 | L4 | B1 |
| **22** | A1 | L4 | B2 |
| **23** | A1 | L4 | B3 |
| **24** | A1 | L4 | B4 |
| **25** | A2 | L3 | B1 |
| **26** | A2 | L3 | B2 |
| **27** | A2 | L3 | B3 |
| **28** | A2 | L3 | B4 |
| **29** | A2 | L4 | B1 |
| **30** | A2 | L4 | B2 |
| **31** | A2 | L4 | B3 |
| **32** | A2 | L4 | B4 |

To give just two examples for illustration, a compound of Group No. 1 as defined by the above table may be a compound of formula (I) wherein: A is 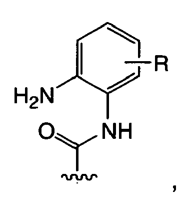 wherein R is, e.g., hydrogen, L is, e.g. a butylene group, and B is 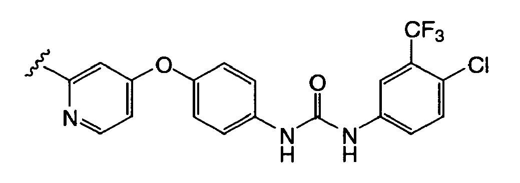 Compound No. 15 represents a compound of formula (I) wherein: A is 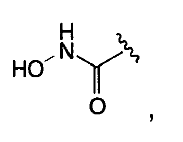 L is a group of the formula D-Ar-E
wherein D and E may be C₁-C₆ alkylene groups and Ar is a phenylene group, and B is 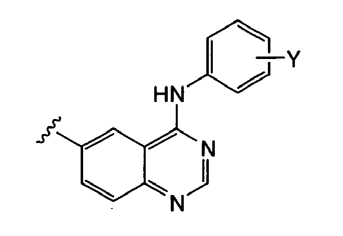 wherein Y is F, Cl, Br, I, an optionally substituted C₁-C₆ alkyl group, an optionally substituted C₂-C₆ alkenyl group, an optionally substituted C₂-C₆ alkinyl group. For example, Y may be chlorine. In a further preferred embodiment A is either 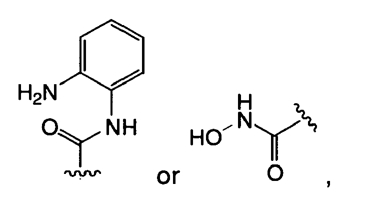 L is a group selected from an ethylene group, a trans-ethenylene group, or

D-Ar-E

wherein D, E and Ar are as defined above, and B is selected from 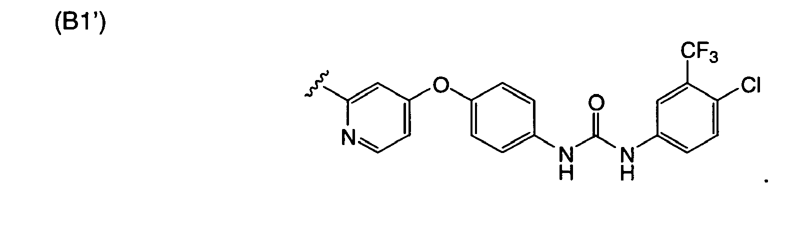 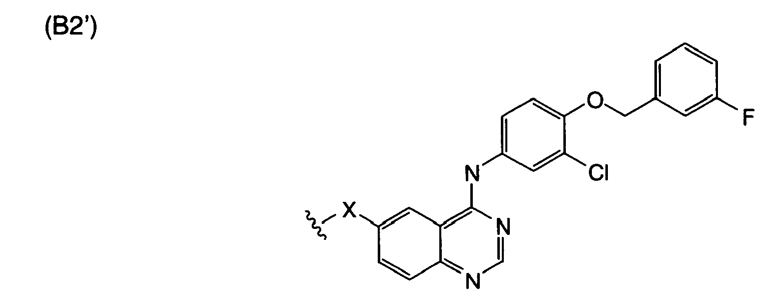 wherein X is a furanyl moiety, a thienyl moiety, or a phenyl moiety, 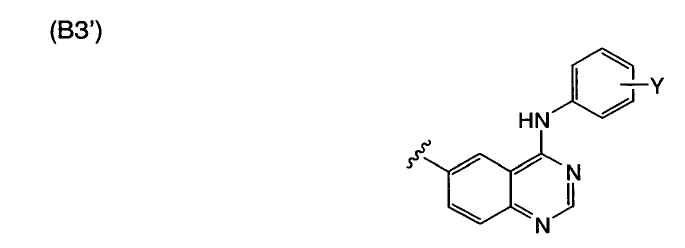 wherein Y is Br, or an ethinyl group, and 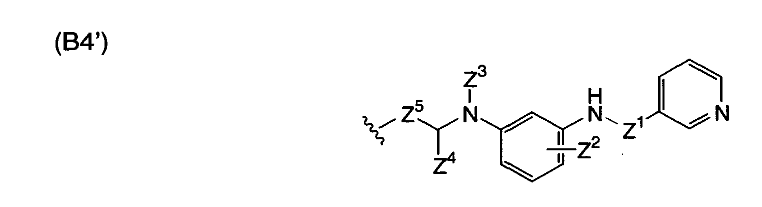 wherein Z¹ is a pyrimidinyl moiety or a thiazolyl moiety,
Z² is selected from a hydrogen atom, a methyl group or a chlorine atom,
Z3 is selected from a hydrogen atom or a methyl group,
Z⁴ is a hydrogen atom or a carbonyl group, and
Z⁵ is a phenyl moiety, a pyrimidine moiety or a thienyl moiety.

In a preferred embodiment, the compounds according to the present invention, i.e. the compounds according to formula (I) or any preferred sub-set thereof, are in crystalline form. The term "compound of the present invention" as used here and in the following sections is to include the pharmaceutically acceptable salts and solvates of such compounds, unless the context specifies otherwise.

Exemplary compounds of the present invention include the following substances and pharmaceutically acceptable salts thereof:

**Table 1: Concordance list of the most important synthesized compounds**

| **No.** | **No. in schemes & ex. part** | **Name** | **Structure** |
|---|---|---|---|
| [1] | | *E*-3-(4-{4-[3-(Chloro-trifluoromethyl-phenyl)-ureido]-phenoxy}-pyridin-2-yl)-N-hydroxy-acrylamide | 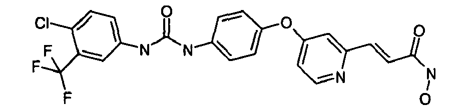 |
| [2] | 6a | *E*-3-(5-{4-[3-Chloro-4-(3-fluorobenzyloxy)-phenylamino]quinazolin-6-yl}furan-2-yl)-*N*-hydroxy-acrylamide hydrochloride monohydrate | 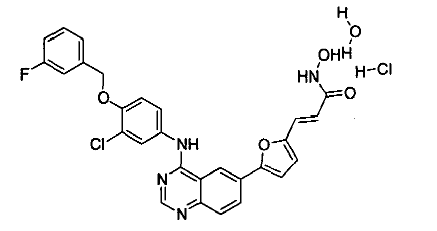 |
| [3] | 7b | 5-{4-[3-Chloro-4-(3-fluorobenzyloxy)phenylamino]-quinazolin-6-yl}thiophene-2-carboxylic acid (2-aminophenyl)amide | 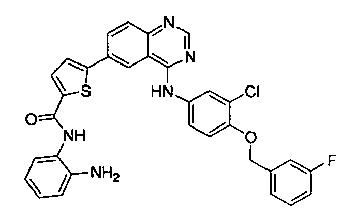 |
| [4] | 8b | 5-{4-[3-Chloro-4-(3-fluorobenzyloxy)phenyl-amino]quinazolin-6-yl}thiophene-2-carboxylic acid hydroxyamide hydrochloride dihydrate | 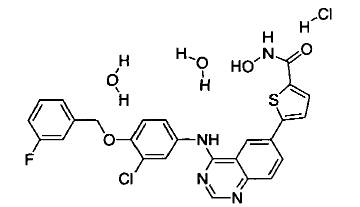 |
| [5] | 7a | 5-{4-[3-Chloro-4-(3-fluorobenzyloxy)phenylamino]-quinazolin-6-yl}furan-2-carboxylic acid (2-amino-phenyl)amide | 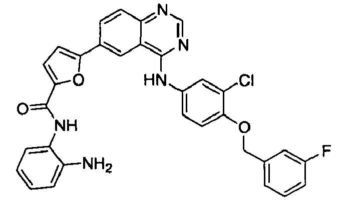 |
| [6] | 8a | 5-{4-[3-Chloro-4-(3-fluorobenzyloxy)phenylamino]-quinazolin-6-yl}furan-2-carboxylic acid hydroxyamide hydrochloride monohydrate | 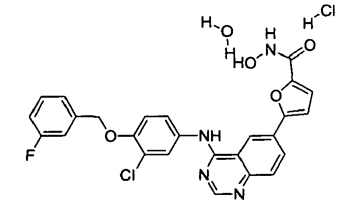 |
| [7] | 6b | *E*-3-(5-{4-[3-Chloro-4-(3-fluorobenzyloxy)-phenylamino]quinazolin-6-yl}thiophene-2-yl)-*N*-hydroxy-acrylamide hydrochloride monohydrate | 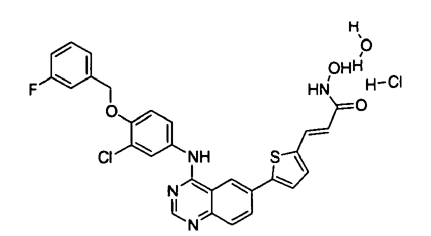 |
| [8] | 6d | *E*-3-(4-(4-(3-chloro-4-(3-fluorobenzyloxy)phenylamino)quinazolin-6-yl)phenyl)-*N*-hydroxyacrylamide hydrochloride monohydrate | 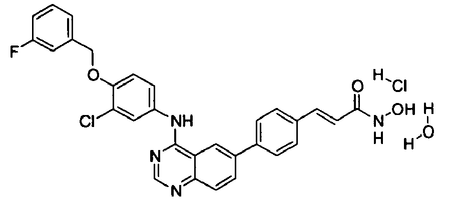 |
| [9] | 6c | *E*-3-(3-(4-(3-chloro-4-(3-fluorobenzyloxy)phenylamino )quinazolin-6-yl)phenyl)-*N*-hydroxy-acrylamide | 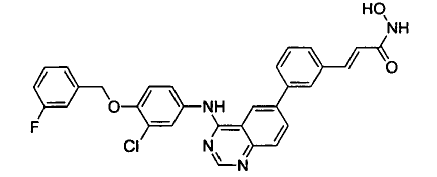 |
| [10] | 7e | *E*-*N*-(2-aminophenyl)-3-(4-(4-(3-chloro-4-(3-fluorobenzyloxy)phenylamino )quinazolin-6-yl)phenyl)acrylamide | 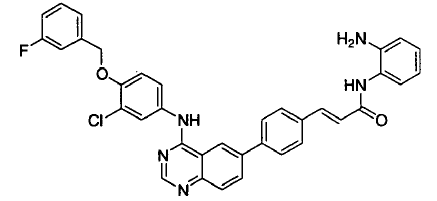 |
| [11] | 7d | *E*-*N*-(2-aminophenyl)-3-(3-(4-(3-chloro-4-(3-fluorobenzyloxy)phenylamino)-quinazolin-6-yl)phenyl)acrylamide | 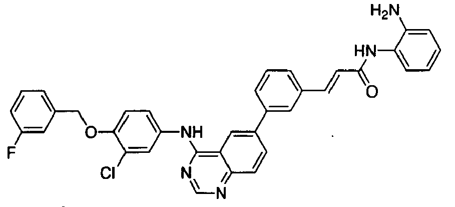 |
| [12] | 7f | *N*-(2-aminophenyl)-3-(4-(4-(3-chloro-4-(3-fluorobenzyloxy)phenylamino)quinazolin-6-yl)phenyl)propanamide | 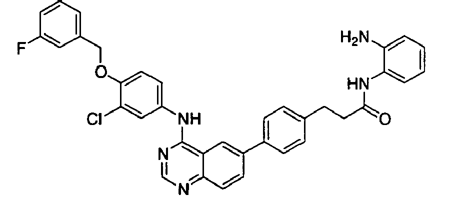 |
| [13] | 7c | *E*-*N*-(2-aminophenyl)-3-(5-(4-(3-chloro-4-(3-fluorobenzyloxy)phenylamino )quinazolin-6-yl)furan-2-yl)acrylamide | 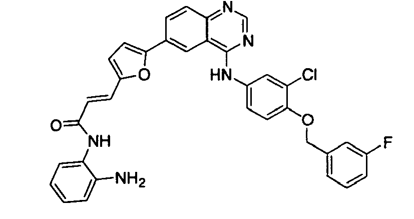 |
| [14] | | *N*-(2-Amino-phenyl)-*N*-[4-methyl-3-(4-pyridin-3-yl-pyrimidin-2-ylamino)-phenyl]-terephthalamide compound with trifluoroacetic acid | 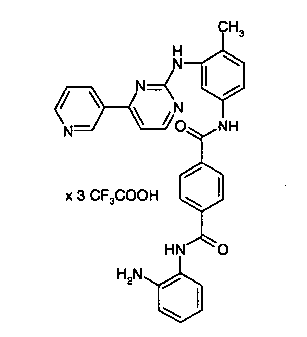 |
| [15] | | *N*-(2-Amino-phenyl)-4-{[4-methyl-3-(4-pyridin-3-yl-pyrimidin-2-ylamino)-phenylamino]-methyl}-benzamide | 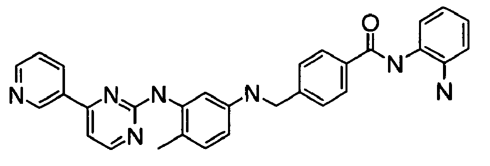 |
| [16] | | (*E*)-*N*-Hydroxy-3-(4-{[4-methyl-3-(4-pyridin-3-yl-pyrimidin-2-ylamino)-phenylamino]-methyl}-phenyl)-acrylamide | 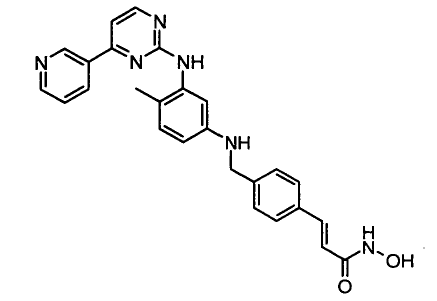 |
| [17] | 59a | *N*¹-hydroxy-*N*⁴-(3-(4-(pyridin-3-yl)thiazol-2-ylamino)phenyl)terephthalamide | 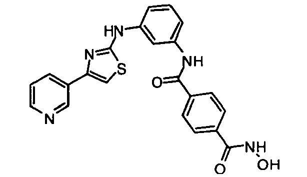 |
| [18] | 46a | *N*¹-(2-aminophenyl)-*N*⁴-(3-(4-(pyridin-3-yl)thiazol-2-ylamino)-phenyl)terephthalamide hydrate | 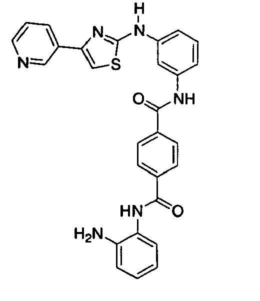 |
| [19] | 53b | *E*-4-(3-(hydroxyamino)-3-oxoprop-1-enyl)-*N*-(4-methyl-3-(4-(pyridin-3-yl)thiazol-2-ylamino)phenyl)benzamide | 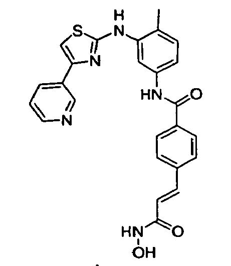 |
| [20] | 59b | *N*¹-hydroxy-*N*⁴-(4-methyl-3-(4-(pyridin-3-yl)thiazol-2-ylamino)phenyl)terephthalam ide | 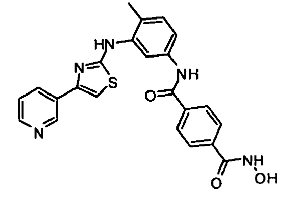 |
| [21] | 46b | *N*¹-(2-aminophenyl)-*N*⁴-(4-methyl-3-(4-(pyridin-3-yl)thiazol-2-ylamino)phenyl)terephthalamide | 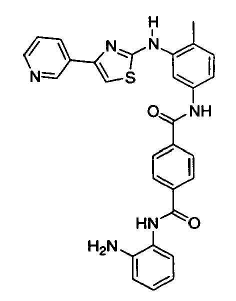 |
| [22] | 53a | *E*-4-(3-(hydroxyamino)-3-oxoprop-1-enyl)-N-(3-(4-(pyridin-3-yl)thiazol-2-ylamino)phenyl)benzamide | 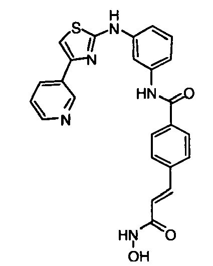 |
| [23] | 44 | *N*²-(2-aminophenyl)-*N*⁵-(4-methyl-3-(4-(pyridin-3-yl)pyrimidin-2-ylamino)phenyl)pyridine-2,5-dicarboxamide | 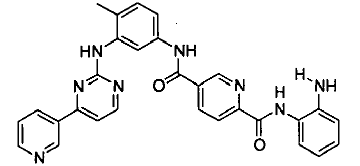 |
| [24] | 47a | *N*²-(2-aminophenyl)-*N*⁵-(3-(4-(pyridin-3-yl)thiazol-2-ylamino)phenyl)pyridine-2,5-dicarboxamide | 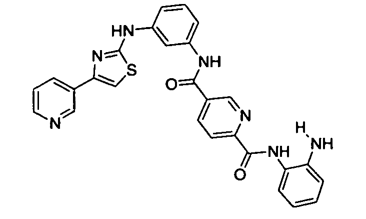 |
| [25] | 47b | *N*²-(2-aminophenyl)-*N*⁵-(3-(4-(pyridin-3-yl)thiazol-2-ylamino)-phenyl)pyridine-2,5-dicarboxamide | 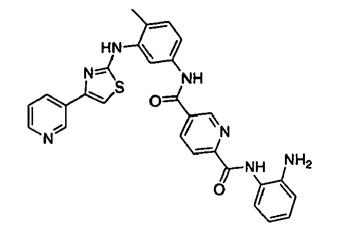 |
| [26] | 45 | *N*²-(2-aminophenyl)-*N*⁵-(4-methyl-3-(4-(pyridin-3-yl)pyrimidin-2-ylamino)phenyl)thiophene-2,5-dicarboxamide | 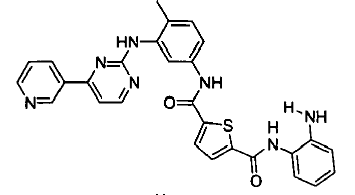 |
| [27] | 48b | *N*²-(2-aminophenyl)-*N*⁵-(4-methyl-3-(4-(pyridin-3-yl)thiazol-2-ylamino)phenyl)thiophene-2,5-dicarboxamide | 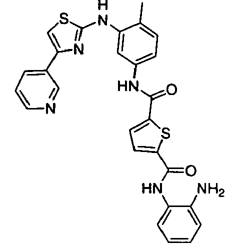 |
| [28] | 48a | *N*²-(2-aminophenyl)-*N*⁵-(3-(4-(pyridin-3-yl)thiazol-2-ylamino)phenyl)thiophene-2,5-dicarboxamide | 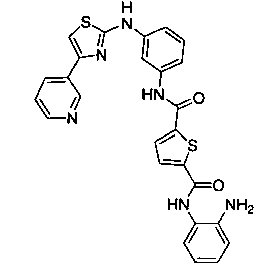 |
| [29] | | *N*-(2-Amino-phenyl)-*N*-[4-methyl-3-(4-pyridin-3-yl-pyrimidin-2-ylamino)-phenyl]-terephthalamide | 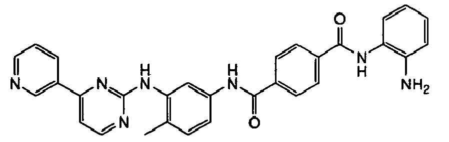 |
| [30] | | *N*-(2-Amino-phenyl)-*N*-methyl-*N*-[3-(4-pyridin-3-yl-pyrimidin-2-ylamino)-phenyl]-terephthalamide | 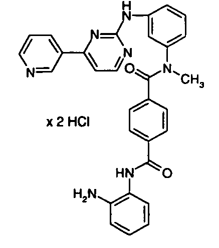 |
| [31] | | *N*-(2-Amino-phenyl)-*N*-[3-(4-pyridin-3-yl-pyrimidin-2-ylamino)-phenyl]-terephthalamide | 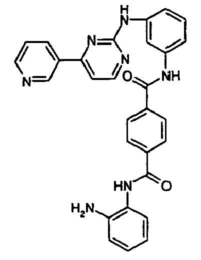 |
| [32] | | *N*-(2-Amino-phenyl)-*N*-[4-chloro-3-(4-pyridin-3-yl-pyrimidin-2-ylamino)-phenyl]-terephthalamide | 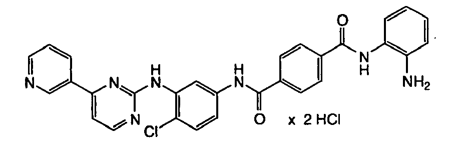 |
| [33] | 54b | *E*-5-(3-(hydroxyamino)-3-oxoprop-1-enyl)-*N*-(4-methyl-3-(4-(pyridin-3-yl)thiazol-2-ylamino)phenyl)thiophene-2-carboxamide | 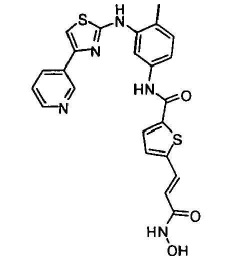 |
| [34] | 54a | *E*-5-(3-(hydroxyamino)-3-oxoprop-1-enyl)-*N*-(3-(4-(pyridin-3-yl)thiazol-2-ylamino)phenyl)thiophene-2-carboxamide | 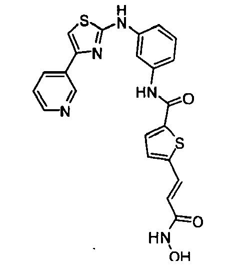 |
| [35] | | N-(2-Amino-phenyl)-N'-[4-chloro-3-(4-pyridin-3-yl-pyrimidin-2-ylamino)-phenyl]-N'-methyl-terephthalamide | 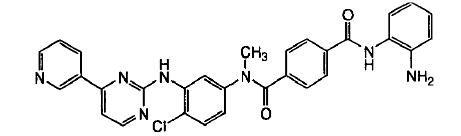 |
| [36] | | *N*²-(2-aminophenyl)-*N*⁵-(4-(3-ethynylphenylamino)-quinazolin-6-yl)pyridine-2,5-dicarboxamide | 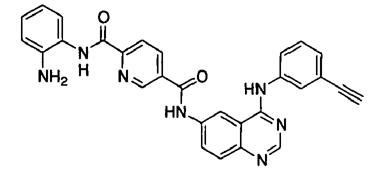 |
| [37] | | *N*-(2-Amino-phenyl)-*N*'-[4-(3-ethynyl-phenylamino)-quinazolin-6-yl]-terephthalamide | 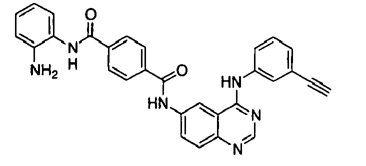 |
| [38] | | Thiophene-2,5-dicarboxylic acid 2-[(2-amino-phenyl)-amide]-5-{[4-(3-ethynyl-phenylamino)-quinazolin-6-yl]-amide} | 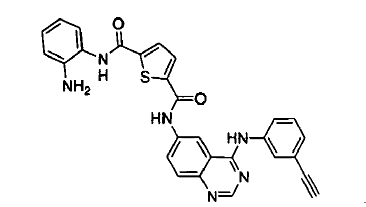 |
| [39] | | Pyridine-2,5-dicarboxylic acid 2-[(2-amino-phenyl)-amide]-5-{[4-(3-ethynyl-phenylamino)-quinazolin-7-yl]-amide} | 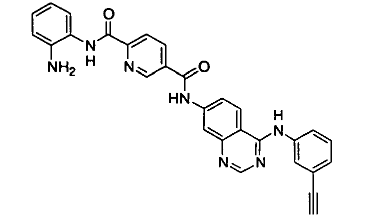 |
| [40] | | Thiophene-2,5-dicarboxylic acid 2-[(2-amino-phenyl)-amide] 5-{[4-(3-ethynyl-phenylamino)-quinazolin-7-yl]-amide} | 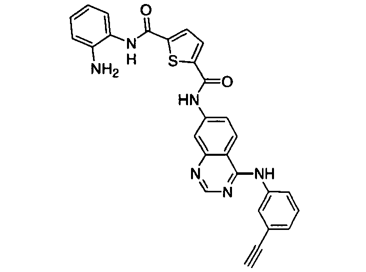 |
| [41] | | *N*-(2-Amino-phenyl)-*N*-[4-(3-ethynyl-phenylamino)-quinazolin-7-yl]-terephthalamide | 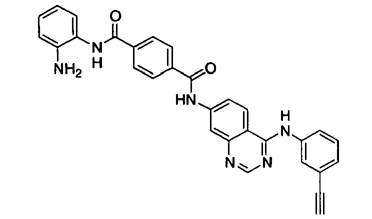 |
| [42] | | *E*-3-(3-(2-aminophenylamino)-3-oxoprop-1-enyl)-*N*-(4-(3-ethynylphenylamino)quinazol in-7-yl)benzamide | 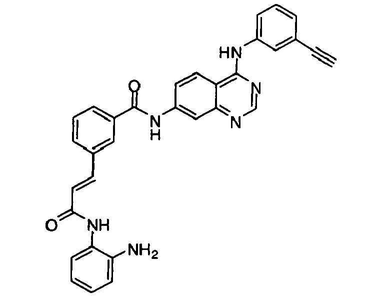 |
| [43] | | *N*-(2-aminophenyl)-4-((4-(3-bromophenylamino)quinazoli n-6-yloxy)-methyl)benzamide | 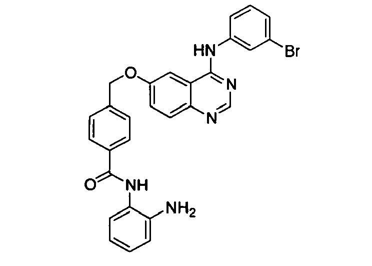 |
| [44] | | *N*-(2-aminophenyl)-4-((4-(3-ethynylphenylamino)-quinazolin-6-yloxy)methyl)benzamide | 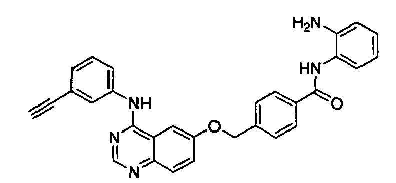 |
| [45] | | 3-{1-[4-(3-Ethynylphenylamino)quinazol ine-6-sulfonyl]-1*H*-pyrrol-3-yl}-*N*-hydroxy-acrylamide | 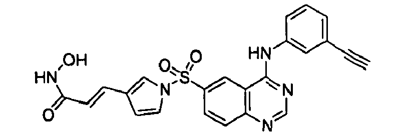 |
| [46] | | *N*-(2-Amino-phenyl)-3-{1-[4-(3-ethynylphenylamino)quinazol ine-6-sulfonyl]-1*H*-pyrrol-3-yl}acryl-amide | 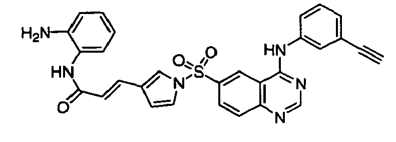 |

The most preferred compounds of the present invention are selected from the following list:

| | |
|---|---|
| [1] | *E*-3-(4-{4-[3-(Chloro-trifluoromethyl-phenyl)-ureido]-phenoxy}-pyridin-2-yl)-N-hydroxy-acrylamide |
| [2] | *E*-3-(5-{4-[3-Chloro-4-(3-fluorobenzyloxy)-phenylamino]quinazolin-6-yl}furan-2-yl)-*N*-hydroxy-acrylamide hydrochloride monohydrate |
| [9] | *E*-3-(3-(4-(3-chloro-4-(3-fluorobenzyloxy)phenylamino)quinazolin-6-yl)phenyl)-*N*-hydroxy-acrylamide |
| [39] | Pyridine-2,5-dicarboxylic acid 2-[(2-amino-phenyl)-amide]-5-{[4-(3-ethynyl-phenylamino)-quinazolin-7-yl]-amide} |
| [43] | *N*-(2-aminophenyl)-4-((4-(3-bromophenylamino)quinazolin-6-yloxy)-methyl)benzamide |
| [44] | *N*-(2-aminophenyl)-4-((4-(3-ethynylphenylamino)-quinazolin-6-yloxy)methyl)benzamide |
| [45] | 3-{1-[4-(3-Ethynylphenylamino)quinazoline-6-sulfonyl]-1*H*-pyrrol-3-yl}-*N*-hydroxy-acrylamide |
| [46] | *N*-(2-Amino-phenyl)-3-{1-[4-(3-ethynylphenylamino)quinazoline-6-sulfonyl]-1*H*-pyrrol-3-yl}acryl-amide |

and pharmaceutically acceptable salts or solvates thereof.

### Synthetic Procedures

The the compound of formula I or its pharmaceutically acceptable salts thereof can be obtained by the following strategy:

### Sorafenib chimeras:

Sorafenib chimeras can be obtained by processes known in the art and by the process outlined in the experimental part.

### Lapatinib chimeras:

Ring closure of 2-amino-5-iodo-benzoic acid (**9**) according to Nishino et al (S. Process for producing 3,4-dihydroquinazolin-4-one derivatives, 2003) formed the 1*H-*quinazolin-4-one system **10.** Chlorination with phosphorous oxychloride and one pot S_{*N*-}reaction⁷ by 3-chloro-4-(3-fluoro-benzyloxy)phenylamine (**15**), yielded the desired [3-chloro-4-(3-fluorobenzyloxy)phenyl]-(6-iodo-quinazolin-4-yl)amine (**16**) as a central intermediate. The aniline derivative **15** was easily accessible from its nitro-precursor **14** by catalytic reduction with hydrogen over sulfided platinum. The nitro-precursor **14** itself was prepared according to Wallace et. al. (Preparation of cyanoguanidines and cyanoamidines as ErbB2 and EGFR inhibitors, 2005) by S_{NAr}-reaktion of 2-chloro-1-fluoro-4-nitrobenzene and (3-fluorophenyl)methanol in DMF solution.

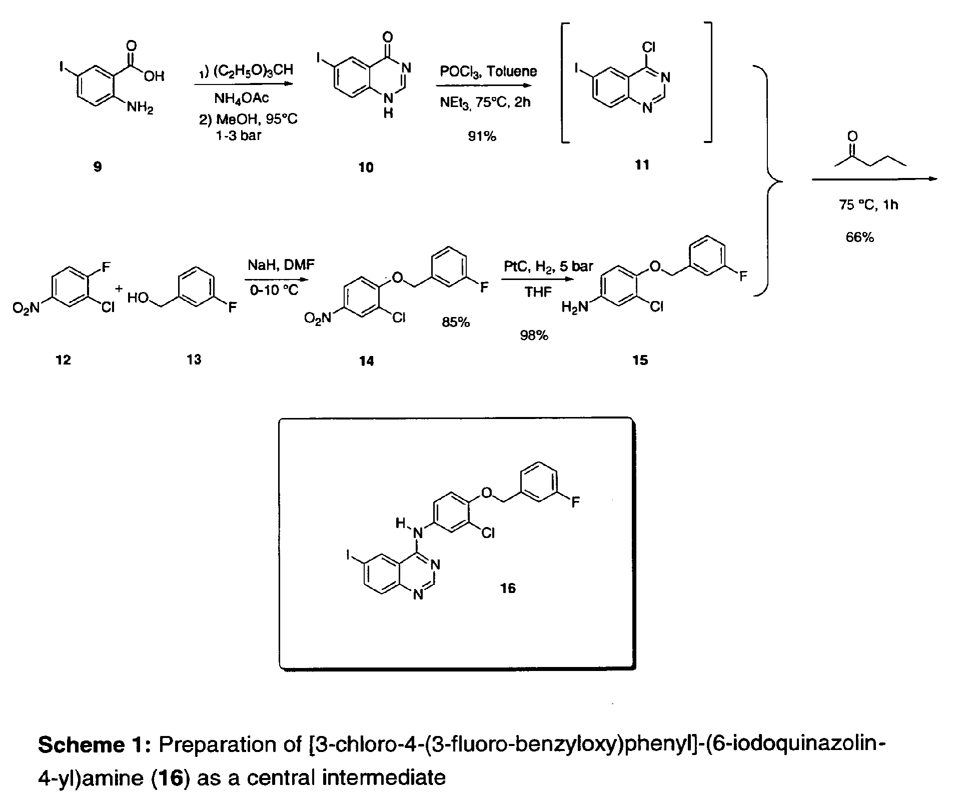

The desired furanyl- and thienyl- and phenyl-N-hydroxy-acrylamides **6a-6d [2, 7, 8, 9]** (Scheme 2) were obtained in the following from **16** by suzuki coupling with the respective formylboronic acids **17a-17d** analogously to Hosoya et al. (Bioorg. Med. Chem. 2003, 11, 663-673), Wittig olefination and deprotection of the resulting acrylic acid *tert*-butyl esters (**19a-19d**) with trifluoro acetic acid. Amidation of the acrylic acids **20a-20d** with commercially available NH₂OTHP (O-(tetrahydropyran-2-yl) hydroxylamine) by use of BOP ((benzotriazol-1-yloxy)tris-(dimethylamino) phosphonium-hexafluorophosphat) as coupling reagent and cleavage of the tetrahydro-pyran-2-yl protected acrylamides **21a-21d** led to the N-hydroxy-acrylamides **6a-6d [2, 7, 8, 9].**

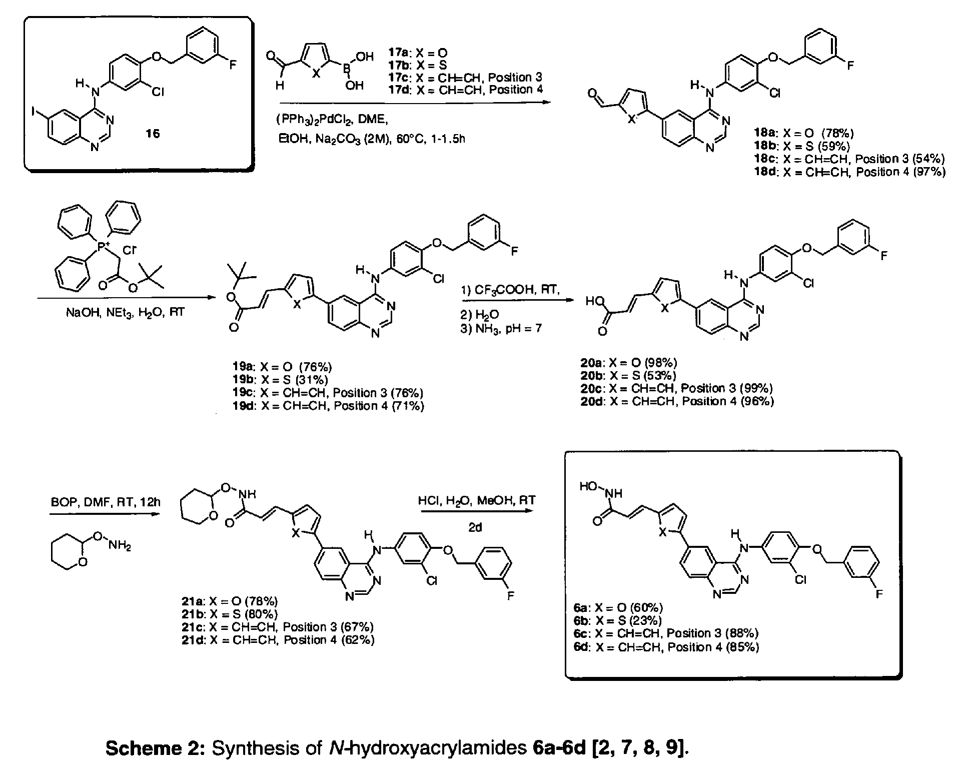

In an analogous manner the carboxylic acid (2-aminophenyl)amides **7a [5]** and **7b [3]** as well as the carboxylic acid hydroxyamides **8a [6]** and **8b [4]** were prepared as shown in scheme 3 by amidation of the carboxylic acids with NH₂OTHP or the mono protected phenylendiamine **25,** followed by deprotection with trifluoro acetic acid (for details cf. experimental part). The mono protected phenylendiamine **25** itself was easily accessible by reaction of *o*-phenylendiamine (**24**) with BOC₂O in THF solution.

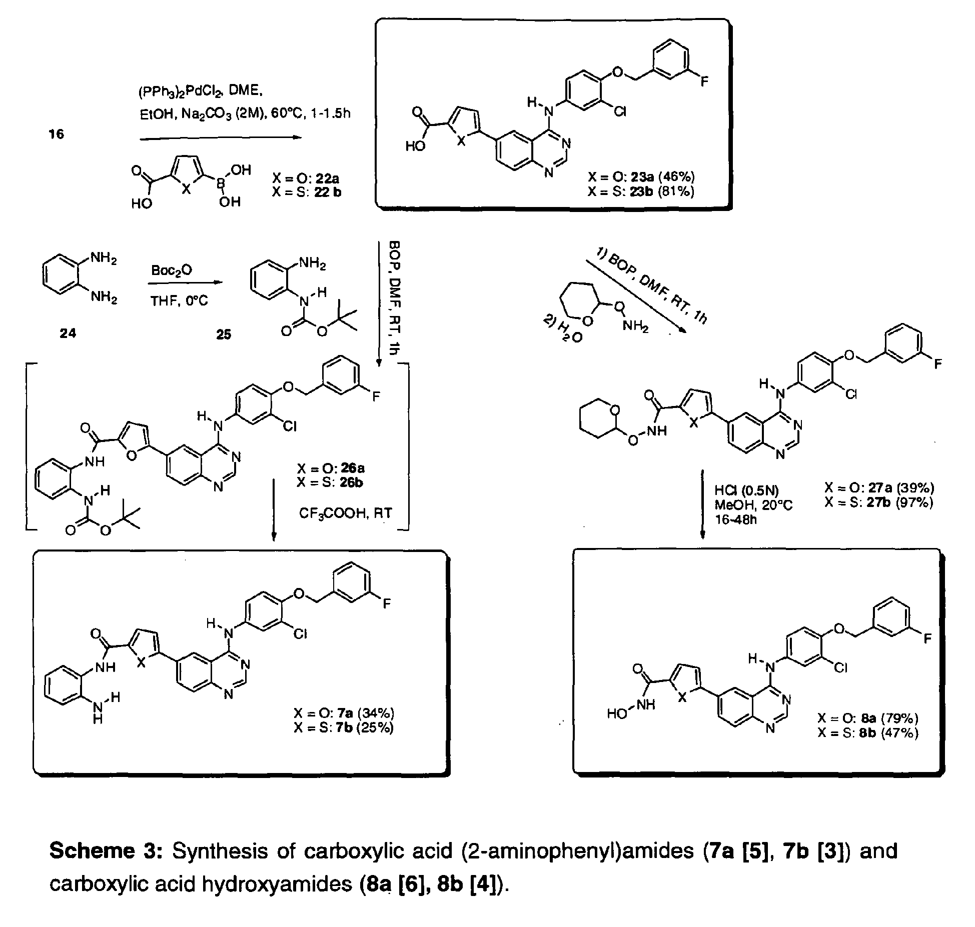

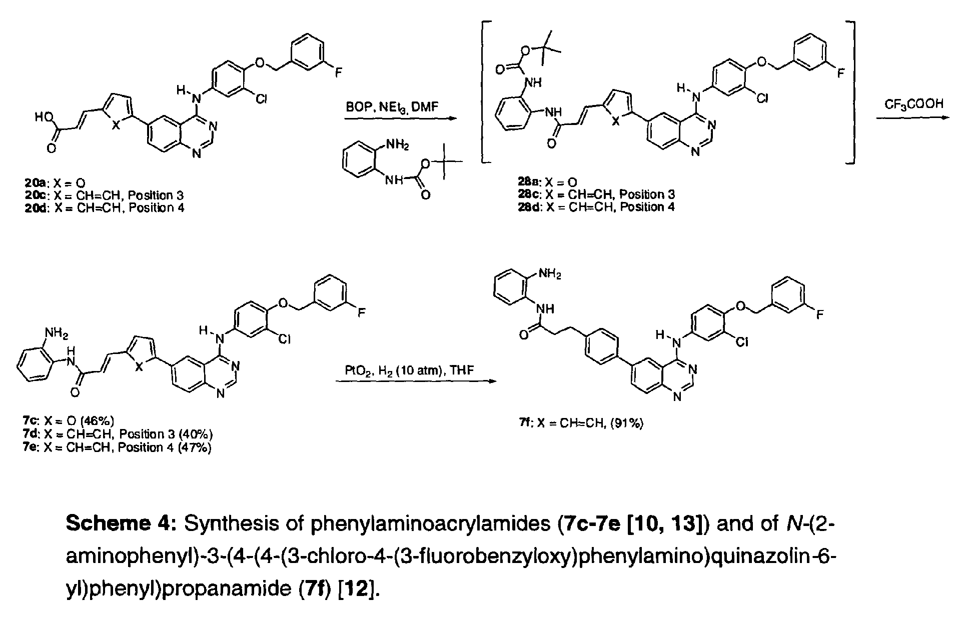

The *(E)*-*N*-(2-aminophenyl)-3-(furan-2-yl)acrylamide **7c [13]** as well as the *N*-(2-aminophenyl)cinnamamides **7d [11]** and **7e [10]** also were obtained in a similar manner by amidation of the acrylic acids **20a-20d** with the mono protected phenylendiamine **25** and deprotection of the *tert*-butyl carbamates (**28a, 28c** and **28d**) with trifluoro acetic acid. Selective catalytic hydrogenation in presence of a benzyloxy- and an aryl chlorine-group by use of PtO₂ yielded the *N*-(2-aminophenyl)-3-phenylpropanamide **7f [12]** without cleavage in addition.

### Imatinib chimera

Catalytic reduction with hydrogen of the respective *N*-(3-nitrophenyl)-4-pyrimidin-2-amine (**30a** and **30b**) or the corresponding *N*-(3-nitrophenyl)thiazol-2-amines (**31a** and **31b**) by use of Pd/C yielded the desired primary arylamines (**32a, 32b** and **33a, 33b**), which were used as central intermediates for the amidation with the suitable protected and substituted carboxylic acids (**34, 35, 36, 49, 50** and **55**). Amidation hereby was performed either by transformation of the corresponding carboxylic acid (**34, 35, 36** and **55** ) to its carboxylic acid chloride in a mixture of pyridine / thionyl chloride and addition of the primary arylamino compound (**32a, 32b** and **33a, 33b**), or mediated by BOP (1-benzotriazolyloxy-tris-(dimethylamino)phosphonium-hexafluorophosphate) as a coupling reagent. Deprotection of the *tert*-butyl-2-benzamidocarbamates (**37-42b**) with trifluoro acetic acid, respectively of the *N-*(tetrahydro-2*H*-pyra*N*-2-yloxy)cinnamamides (**51a-52b**) or *N*-(tetrahydro-2*H*-pyran-2-yloxy)benzamides with hydrochloric acid led to the desired *N*-(2-arylamino)benzamides (**43-48b**) and N-hydroxycinnamamides (**53a-54b**), respectively hydroxamic acids (**59a-59b**).

The *N*-(3-nitrophenyl)-4-pyrimidin-2-amines (**30a, 30b**) used for synthesis as described above were prepared as described earlier (Zimmerann et al., Bioorg. Med. Chem. Lett. 1996, 6, 1221-1226; Szakacs et al., J. Med. Chem. 2005, 48, 249-255). The *N*-(3-nitrophenyl)thiazol-2-amines (**31a, 31b**) were prepared analogously by reaction of 2-bromo-1-(pyridin-3-yl)ethanone hydrobromide (**62**) and the respective phenylthiourea derivatives (**61a, 61b**), which were easily available from 3-nitroaniline (**60a**) and 2-methyl-5-nitroaniline (**60b**) in two steps by a modification of the method described by Rasmussen et al. (Synthesis 1988, 6, 456-459) (Scheme 6).

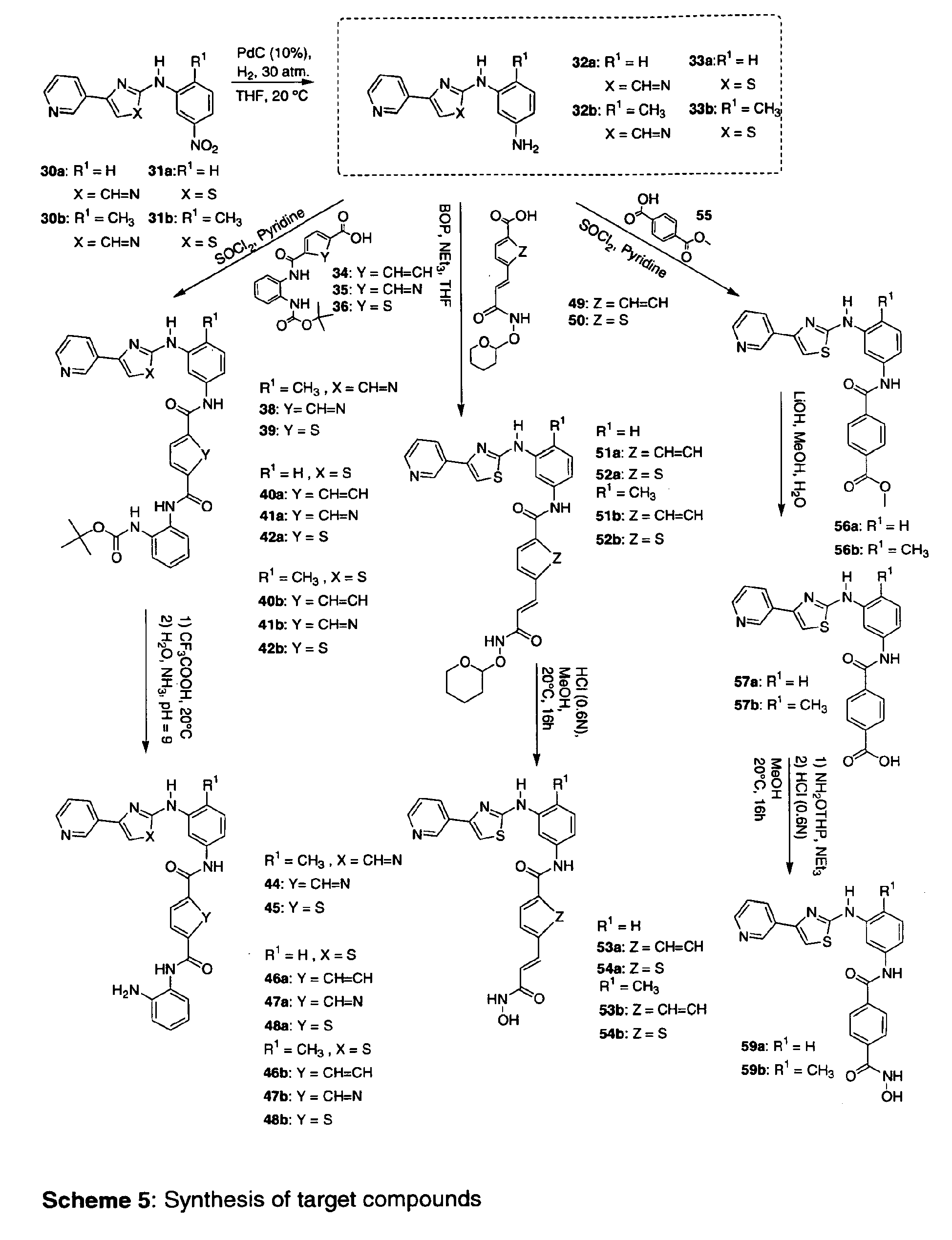

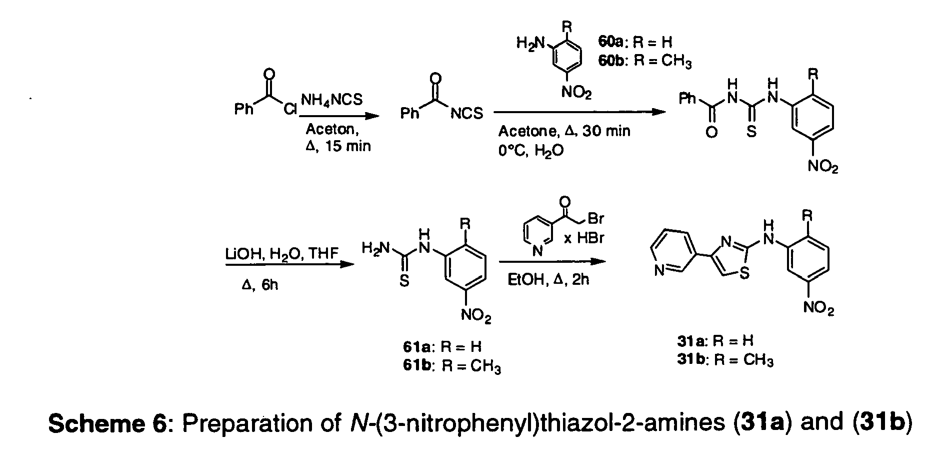

The substituted carboxylic acids, 4-(2-(*tert*-butoxycarbonylamino)phenylcarbamoyl) benzoic acid (**34**) and 6-(2-(*tert*-butoxycarbonylamino)phenylcarbamoyl)nicotinic acid (**35**), which were used for the central amidation step as described in scheme 5 were prepared from the corresponding methoxycarbonyl aroylic acids (**62, 63**), by reaction with *tert*-butyl 2-aminophenylcarbamate (**64**) followed by selective alkaline cleavage of the methylester-group of **65** and **66** with LiOH (Scheme 7).

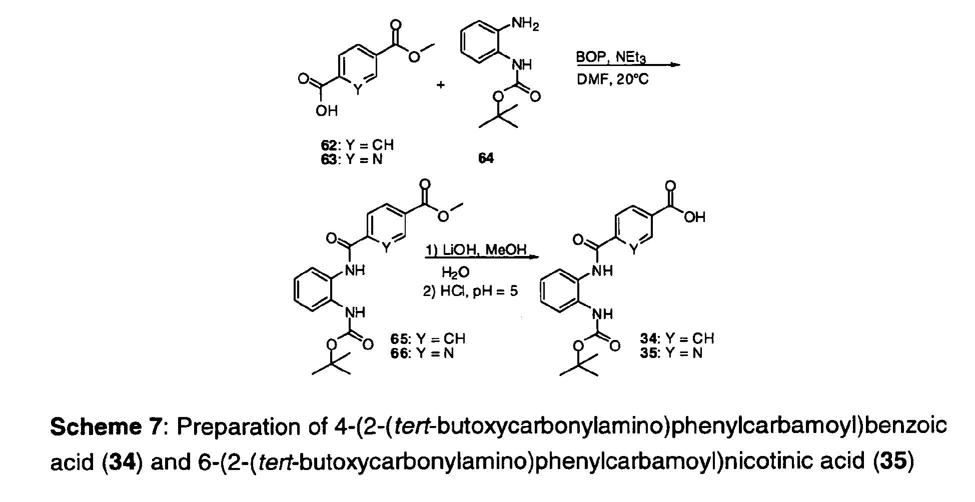

Following the same synthetic strategy, 5-(2-(*tert*-butoxycarbonylamino)phenylcarbamoyl)thiophene-2-carboxylic acid (**36**) was prepared from 5-(methoxycarbonyl)thiophene-2-carboxylic acid (**71**). Compound **71** hereby was obtained from thiophene-2-carbaldehyde (**67**) by transformation to 2-(thiophen-2-yl)-1,3-dioxolane (**68**), lithiation with *n*-BuLi, introduction of the carboxylic acid and cleavage of the 1,3-dioxolane in one step, followed by esterification of **69** with methyl iodide.

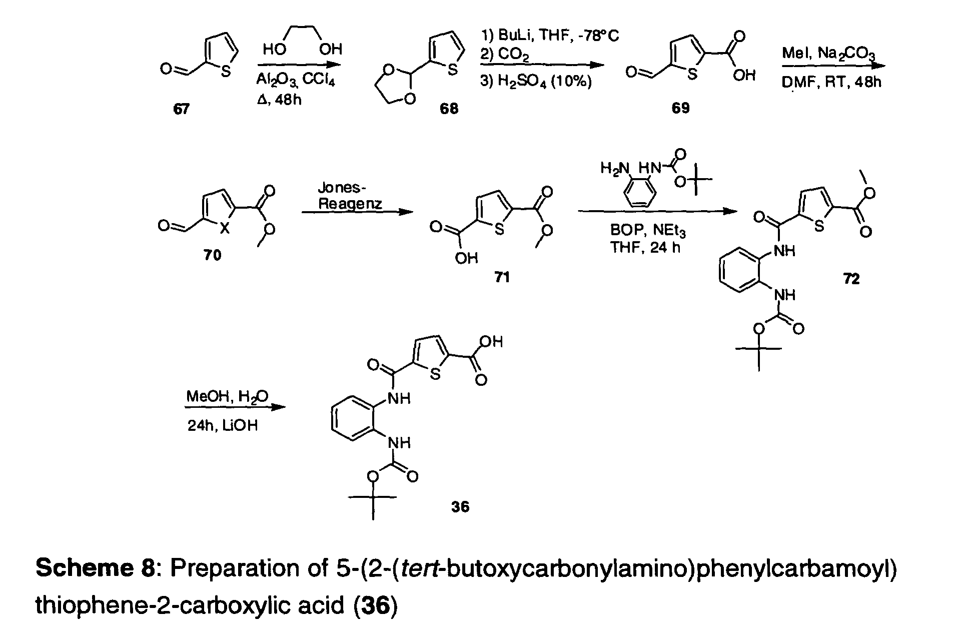

The N-hydroxycinnamamide-precursors (**50** and **49**) were obtained from the aldehydes **70** and **73** by an aldol-condensation with malonic acid, in a mixture of pyridine/piperidine, amidation of the resulting cinnamic acids (**74, 75**) with NH₂OTHP (O-(tetrahydro-2*H*-pyran-2-yl)hydroxylamine) by use of BOP as coupling reagent, and alkaline cleavage of the methyl ester group of **76,** respectively **77.**

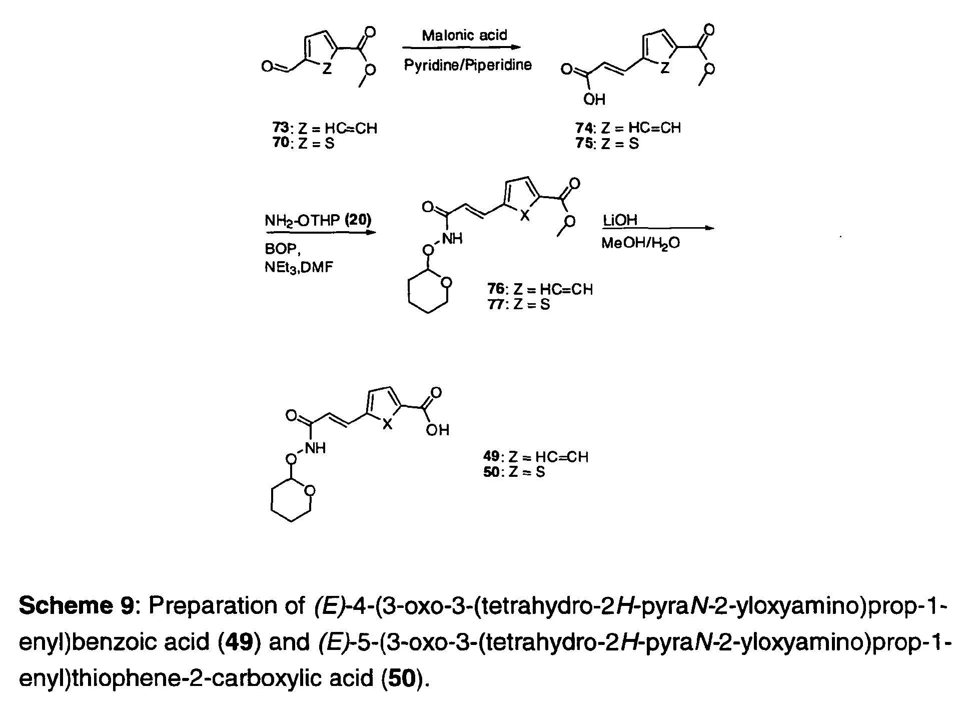

### Erlotinib chimera

The Erlotinib chimera containing an amide substructure were synthesized as follows: Building up the 6-nitroquinazolin-4(3*H*)-one and 7-nitroquinazolin-4(3*H*)-one from 2-amino-5-nitrobenzonitrile, respectively from 2-amino-4-nitro-benzoic acid as reported earlier was followed by chlorination and subsequent S_{NAr}-reaction of the resulting 4-chloronitroquinazolines with 3-ethynylaniline in a one pot synthesis according to the procedure described by *Nishino et al*. The *N*-(3-ethynylphenyl)-6-nitroquinazolin-4-amine and *N*-(3-ethynylphenyl)-7-nitroquinazolin-4-amine obtained this way were reduced to their corresponding amino derivatives by use of iron in acetic acid analogous Rachid et al.(J. Med. Chem. 2003, 46, 4313-4321) to the corresponding 6-aminoquinazolin-4(3*H*)-one and 7-aminoquinazolin-4(3*H*)-one, which were used as central intermediates for the amidation with the suitable protected and substituted carboxylic acids (see part 2-Imatinib hybrides). Amidation hereby was performed either by transformation of the corresponding carboxylic acid to its carboxylic acid chloride in a mixture of pyridine / thionyl chloride and addition of the primary arylamino compounds or mediated by BOP (1-benzotriazolyloxy-tris-(dimethylamino)phosphoniumhexafluorophosphate) as a coupling reagent.

Deprotection of the *tert*-butyl 2-benzamidocarbamates with formic acid, led to the desired target compounds.

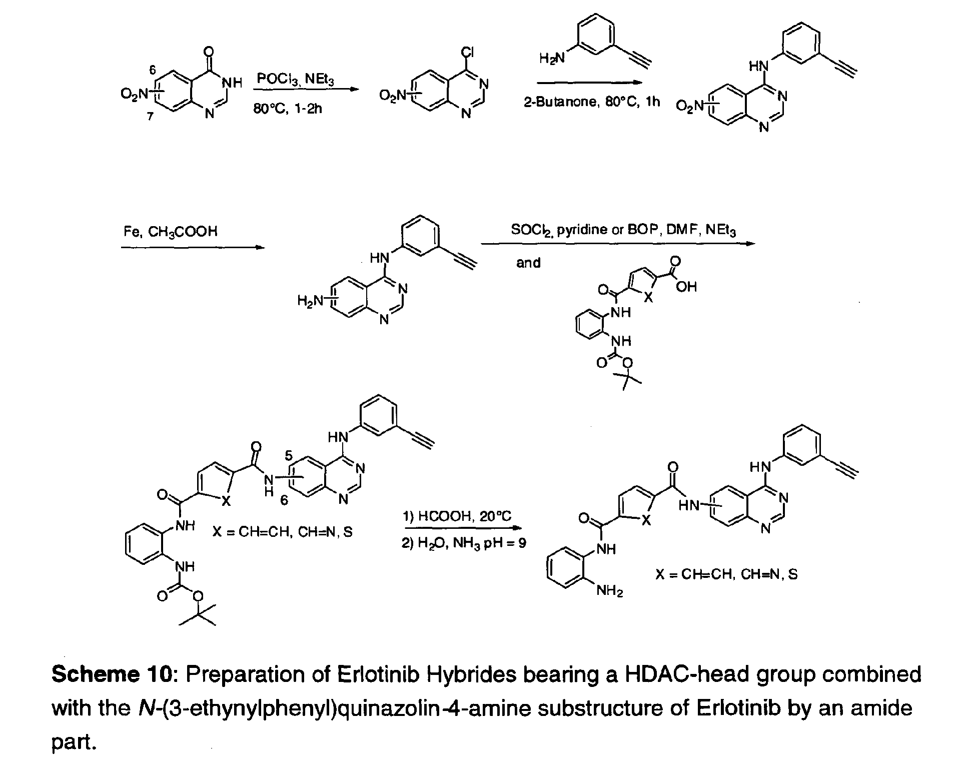

The substituted carboxylic acids, 4-(2-(*tert*-butoxycarbonylamino)phenylcarbamoyl) benzoic acid and 6-(2-(*tert*-butoxycarbonylamino)phenylcarbamoyl)nicotinic acid, which were used for the central amidation step as described in scheme 10 were prepared from the corresponding methoxycarbonyl aroylic acids, by reaction with *tert-*butyl 2-aminophenylcarbamate followed by selective alkaline cleavage of the methylester-group with LiOH (Scheme 11).

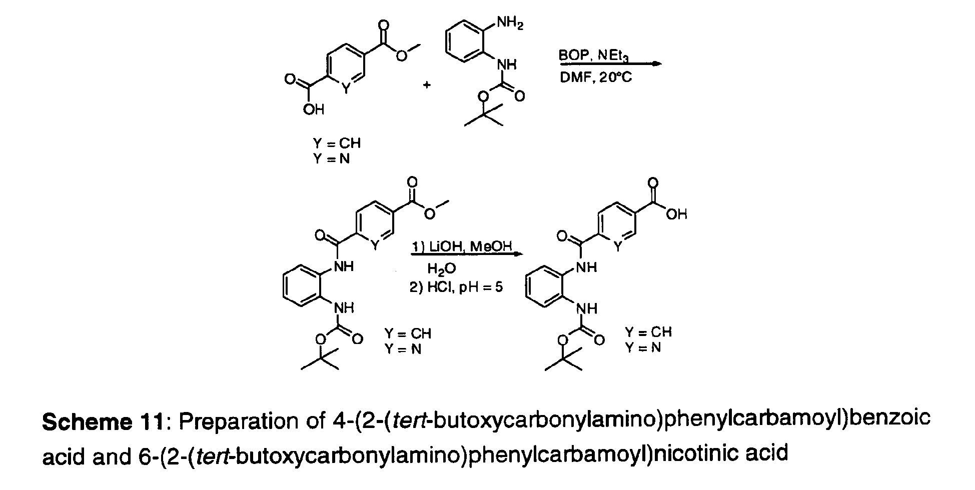

Following the same synthetic synthesis, 5-(2-(*tert*-butoxycarbonylamino)phenylcarbamoyl)thiophene-2-carboxylic acid was prepared from 5-(methoxycarbonyl) thiophene-2-carboxylic acid (Scheme 12). 5-(Methoxycarbonyl)thiophene-2-carboxylic acid hereby was obtained from thiophene-2-carbaldehyde by transformation to 2-(thiophen-2-yl)-1,3-dioxolane, lithiation with *n*-BuLi, introduction of the carboxylic acid and cleavage of the 1,3-dioxolane in one step, followed by esterification of the carboxylic acid with methyl iodide. 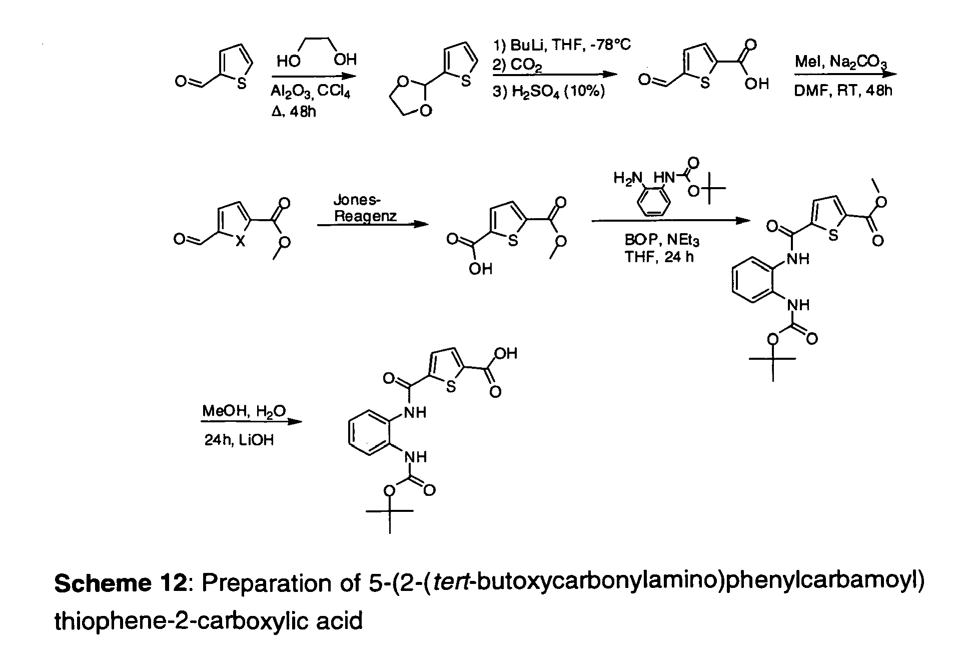 For the synthesis of *N*-(2-aminophenyl)-4-((4-(3-ethynylphenylamino)quinazolin-6-yloxy)methyl)benzamide, a compound combining the *N*-(3-ethynylphenyl)quinazolin-4-amine substructure of Erlotinib and linking the HDAC-head group by an ether, the synthetic way has been modified. The 6-methoxyquinazolin-4(3*H*)-one system was prepared according to Nishino et al. from 2-amino-5-methoxybenzoic acid, followed by the one pot synthesis of *N*-(3-bromophenyl)-6-methoxyquinazolin-4-amine from 6-methoxyquinazolin-4(3*H*)-one and 3-bromoaniline analogous Nishino et al.

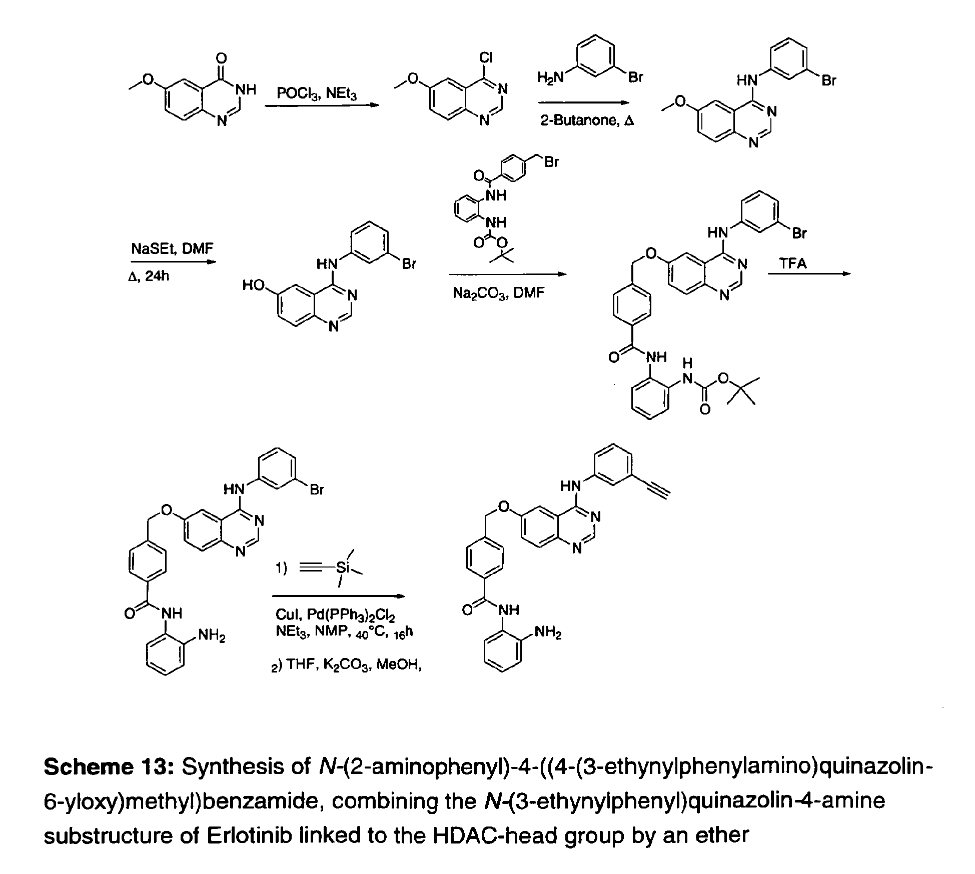

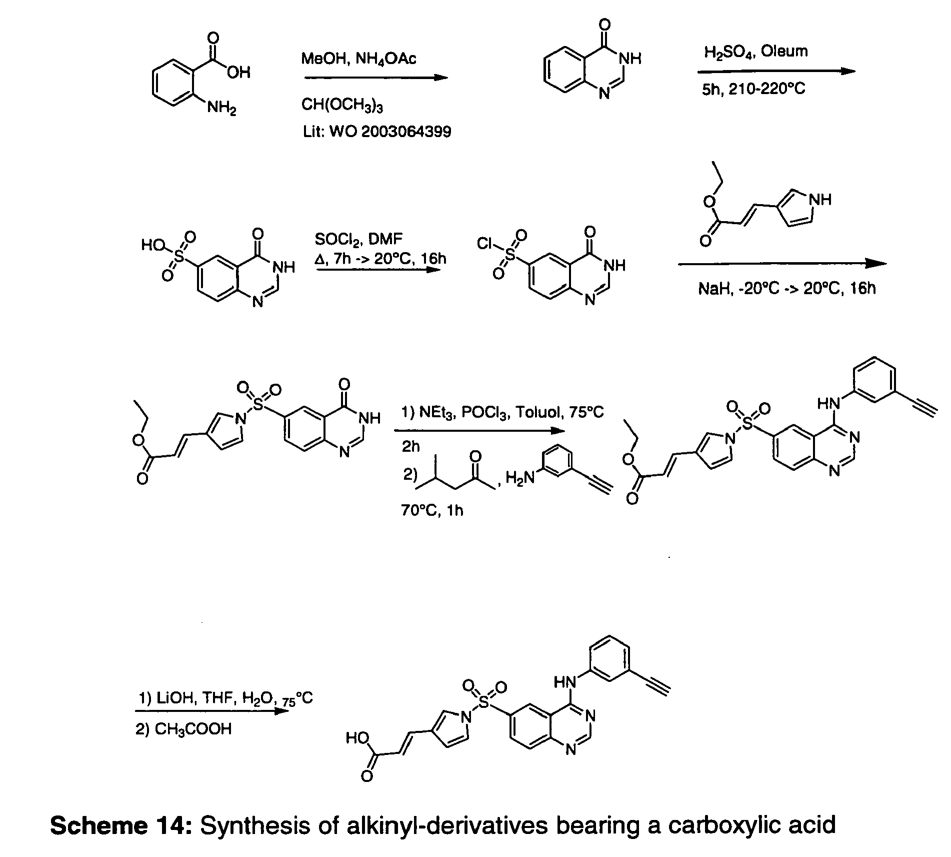

Anthranilic acid was transformed via various steps to the corresponding sulfuryl chloride. This species was reacted with pyrrolylacrylate to form the sulfonylpyrrol derivative. Subsequent activation of the ketone and transformation resulted in the alkinyl-derivative bearing a free carboxylic acid.

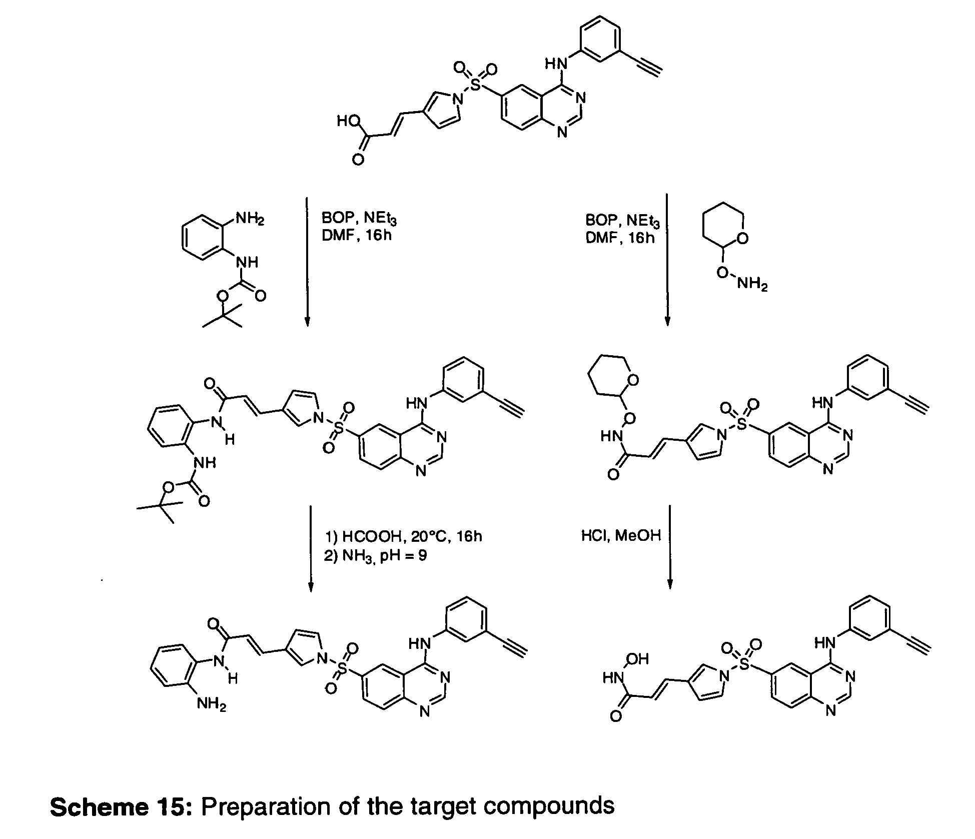

The free carboxylic acid was transferred to the aminophenyl derivative or the hydroxamic acid derivative by activation with BOP and subsequent reaction with the respective protected building block. In the last step the boc- or the THP-protection group were cleaved by acidic treatment.

The invention is further directed to a pharmaceutical composition comprising a compound according to formula (I) or any preferred subset thereof as defined herein, and a pharmaceutically acceptable carrier.

In a further embodiment, the invention is directed to a compound of formula (I) for use in a method for the treatment of malignant or non-malignant neoplasias or non-malignant diseases different from cellular neoplasia. Such diseases include
(i) arthropathies and osteopathological conditions or diseases such as rheumatoid arthritis, osteoarthritis, gout, polyarthritis, and psoriatic arthritis,
(ii) autoimmune diseases like systemic lupus erythematosus and transplant rejection,
(iii) hyperproliferative diseases such as smooth muscle cell proliferation including vascular proliferative disorders, atherosclerosis, restenosis and proliferative fibrosis such as lung fibrosis,
(iv) acute and chronic inflammatory conditions or diseases and dermal conditions such as psoriasis, ulcerative colitis, Crohn's disease, allergic rhinitis, allergic dermatitis, cystic fibrosis, chronic obstructive bronchitis and asthma, and
(v) endometriosis, uterine fibroids, endometrial hyperplasia and benign prostate hyperplasia.

The malignant neoplasia is a disease selected from solid and hematological tumors, myelodysplastic syndrome, plasma cell neoplasia, paraneoplastic syndromes, cancers of unknown primary site as well as AIDS related malignancies.

The invention is further directed to a pharmaceutical composition comprising a combination of a compound of formula (I) and a further anti-cancer agent. The anti-cancer agent is selected from the group of 5 FU, actinomycin D, ABARELIX, ABCIXIMAB, ACLARUBICIN, ADAPALENE, ALEMTUZUMAB, ALTRETAMINE, AMINOGLUTETHIMIDE, AMIPRILOSE, AMRUBICIN, ANASTROZOLE, ANCITABINE, ARTEMISININ, AZATHIOPRINE, BASILIXIMAB, BENDAMUSTINE, BEVACIZUMAB, BEXXAR, BICALUTAMIDE, BLEOMYCIN, BORTEZOMIB, BROXURIDINE, BUSULFAN, CAMPATH, CAPECITABINE, CARBOPLATIN, CARBOQUONE, CARMUSTINE, CETRORELIX, CHLORAMBUCIL, CHLORMETHINE, CISPLATIN, CLADRIBINE, CLOMIFENE, CYCLOPHOSPHAMIDE, DACARBAZINE, DACLIZUMAB, DACTINOMYCIN, DASATINIB, DAUNORUBICIN, DECITABINE, DESLORELIN, DEXRAZOXANE, DOCETAXEL, DOXIFLURIDINE, DOXORUBICIN, DROLOXIFENE, DROSTANOLONE, EDELFOSINE, EFLORNITHINE, EMITEFUR, EPIRUBICIN, EPITIOSTANOL, EPTAPLATIN, ERBITUX, ERLOTINIB, ESTRAMUSTINE, ETOPOSIDE, EXEMESTANE, FADROZOLE, FINASTERIDE, FLOXURIDINE, FLUCYTOSINE, FLUDARABINE, FLUOROURACIL, FLUTAMIDE, FORMESTANE, FOSCARNET, FOSFESTROL, FOTEMUSTINE, FULVESTRANT, GEFITINIB, GENASENSE, GEMCITABINE, GLIVEC, GOSERELIN, GUSPERIMUS, HERCEPTIN, IDARUBICIN, IDOXURIDINE, IFOSFAMIDE, IMATINIB, IMPROSULFAN, INFLIXIMAB, IRINOTECAN, IXABEPILONE, LANREOTIDE, LAPATINIB, LETROZOLE, LEUPRORELIN, LOBAPLATIN, LOMUSTINE, LUPROLIDE, MELPHALAN, MERCAPTOPURINE, METHOTREXATE, METUREDEPA, MIBOPLATIN, MIFEPRISTONE, MILTEFOSINE, MIRIMOSTIM, MITOGUAZONE, MITOLACTOL, MITOMYCIN, MITOXANTRONE, MIZORIBINE, MOTEXAFIN, MYLOTARG, NARTOGRASTIM, NEBAZUMAB, NEDAPLATIN, NILUTAMIDE, NIMUSTINE, NILOTINIB, OCTREOTIDE, ORMELOXIFENE, OXALIPLATIN, PACLITAXEL, PALIVIZUMAB, PANITUMUMAB, PATUPILONE, PAZOPANIB, PEGASPARGASE, PEGFILGRASTIM, PEMETREXED, PENTETREOTIDE, PENTOSTATIN, PERFOSFAMIDE, PIPOSULFAN, PIRARUBICIN, PLICAMYCIN, PREDNIMUSTINE, PROCARBAZINE, PROPAGERMANIUM, PROSPIDIUM CHLORIDE, RALOXIFEN, RALTITREXED, RANIMUSTINE, RANPIRNASE, RASBURICASE, RAZOXANE, RITUXIMAB, RIFAMPICIN, RITROSULFAN, ROMURTIDE, RUBOXISTAURIN, SARGRAMOSTIM, SATRAPLATIN, SIROLIMUS, SOBUZOXANE, SORAFENIB, SPIROMUSTINE, STREPTOZOCIN, SUNITINIB, TAMOXIFEN, TASONERMIN, TEGAFUR, TEMOPORFIN, TEMOZOLOMIDE, TENIPOSIDE, TESTOLACTONE, THIOTEPA, THYMALFASIN, TIAMIPRINE, TOPOTECAN, TOREMIFENE, TRAIL, TRASTUZUMAB, TREOSULFAN, TRIAZIQUONE, TRIMETREXATE, TRIPTORELIN, TROFOSFAMIDE, UREDEPA, VALRUBICIN, VATALANIB, VANDETANIB, VERTEPORFIN, VINBLASTINE, VINCRISTINE, VINDESINE, VINORELBINE, VOROZOLE and ZEVALIN.

The invention further relates to the use of the compounds according to this invention for inhibiting histone deacetylase and/or kinase activity in cells and tissues, causing hyperacetylation / hypophosphorylation of respective substrate proteins and as functional consequence for example the induction or repression of gene expression, the disassembly or inactivation of protein complexes, inhibition of signaling cascades, induction of protein degradation, cell cycle arrest, induction of differentiation and/or induction of apoptosis.

The invention further relates to a method for inhibiting, treating, ameliorating or preventing cellular neoplasia by adminstration of an effective amount of a compound according to this invention to a mammal, in particular a human in need of such treatment.

The invention further provides a method for treating a mammal, in particular a human, bearing a disease different to cellular neoplasia, which is sensitive to histone deacetylase inhibitor and/or protein kinase inhibitor therapy and includes the non malignant diseases) as mentioned above. The method comprises administering to said mammal a pharmacologically active and therapeutically effective and tolerable amount of a compound according to this invention.

Compounds according to the present invention may also be useful for treatment, prevention or amelioration of the diseases of benign and malignant behavior as described herein, such as, for example, (hyper)proliferative diseases and/or disorders responsive to induction of apoptosis and/or disorders responsive to cell differentiation, e.g. benign or malignant neoplasia, particularly cancer, such as e.g. any of those cancer diseases described above.

In view of their properties, functions and usabilities mentioned herein, the compounds according to the present invention are expected to be distinguished by valuable and desirable effects related therewith, such as e.g. by low toxicity, superior bioavailability in general (such as e.g. good enteral absorption), superior therapeutic window, absence of significant side effects, and/or further beneficial effects related to their therapeutic and pharmaceutical suitability.

Crystalline compounds according to this invention, e.g. crystalline salts according to this invention, are expected to have desirable physicochemical properties and such properties may beneficially influence the stability, as well as the chemical and pharmaceutical processing, formulating and mechanical handling on a commercial scale. Thus, these crystalline compounds may be particularly suited for the manufacture of commercially viable and pharmaceutically acceptable drug compositions or dosage forms.

The present invention provides compounds according to this invention isolated in purified or substantially pure form, such as e.g. greater than about 50%, more precisely about 60%, more precisely about 70%, more precisely about 80%, more precisely about 90%, more precisely about 95%, more precisely about 97%, more precisely about 99% wt purity as determined by art-known methods.
Some of the compounds according to the present invention can be present in more than one diastereomeric or enantiomeric form, owing to one or several chiral centers in the molecule. Where applicable, the present invention includes any such stereoisomers, particularly the pure R- or S-enantiomers, or any mixtures thereof. The present invention also includes polymorphs of the compounds of the present invention.

In a further aspect, the compounds according to the present invention are provided in a pharmaceutically acceptable dosage form. Thus, the present invention provides compounds according to this invention in solid or liquid pharmaceutically acceptable dosage forms, particularly solid oral dosage forms, such as tablets and capsules, as well as suppositories and other pharmaceutical dosage forms.

The present invention further includes a method for the treatment of mammals, including humans, which are suffering from one of the abovementioned conditions, illnesses, disorders or diseases. The method is characterized in that a pharmacologically active and therapeutically effective and tolerable amount of one or more of the compounds according to this invention, which function by inhibiting histone deacetylases and protein kinases and -in general- by modulating protein acetylation and phosphorylation, induce various cellular effects, in particular induction or modulation of gene expression, arresting cell proliferation, inducing cell redifferentiation and/or inducing apoptosis, is administered to the subject in need of such treatment.

The invention further includes a method for treating diseases and/or disorders responsive or sensitive to the inhibition of histone deacetylases and / or protein kinases, particularly those diseases mentioned above, such as e.g. cellular neoplasia or diseases different to cellular neoplasia as indicated above, in mammals, including humans, suffering therefrom comprising administering to said mammals in need thereof a pharmacologically active and therapeutically effective and tolerable amount of one or more of the compounds according to the present invention.

The present invention further includes a therapeutic method useful to modulate protein acetylation and phosphorylation, gene expression, cell proliferation, cell differentiation and/or apoptosis in vivo in diseases mentioned above, in particular cancer, comprising administering to a subject in need of such therapy a pharmacologically active and therapeutically effective and tolerable amount of one or more of the compounds according to this invention, which function by inhibiting histone deacetylases and/or protein kinases.

The present invention further provides a method for regulating endogenous or heterologous promotor activity by contacting a cell with a compound according to this invention.

The invention further includes a method for treating diseases, particularly those diseases mentioned above, in mammals, including humans, suffering therefrom comprising administering to said mammals in need thereof a therapeutically effective and tolerable amount of one or more of the compounds according to the present invention and, optionally, one or more further therapeutic agents, such as e.g. those mentioned below. These further agents can be administered simultaneously, sequentially or separately with the compounds of the present invention.

The invention further relates to the use of the compounds according to the present invention for the preparation of pharmaceutical compositions for the treatment and/or prophylaxis of the diseases, disorders, illnesses and/or conditions as mentioned herein.

The invention further relates to the use of the compounds according to the present invention for the production of pharmaceutical compositions which are employed for the treatment and/or prophylaxis of diseases and/or disorders responsive or sensitive to the inhibition of histone deacetylases and/or protein kinases, particularly those diseases mentioned above, such as e.g. cellular neoplasia or diseases different to cellular neoplasia as indicated above.

The invention further relates to the use of the compounds according to the present invention for the production of pharmaceutical compositions having histone deacetylase inhibitory and / or kinase inhibitory activity.

The invention further relates to the use of the compounds according to the present invention for the production of pharmaceutical compositions for inhibiting or treating cellular neoplasia, such as e.g. benign or malignant neoplasia, e.g. cancer.

The invention further relates to the use of the compounds according to the present invention for the production of pharmaceutical compositions which can be used for treating, preventing or ameliorating of diseases responsive to arresting aberrant cell growth, such as e.g. (hyper)proliferative diseases of benign or malignant behaviour, such as e.g. any of those diseases mentioned herein, particularly cancer, such as e.g. any of those cancer diseases described herein above.

The invention further relates to the use of the compounds according to the present invention for the production of pharmaceutical compositions which can be used for treating, preventing or ameliorating of disorders responsive to induction of apoptosis, such as e.g. any of those diseases mentioned herein, particularly cancer, such as e.g. any of those cancer diseases described herein above.

The invention further relates to the use of the compounds according to the present invention for the production of pharmaceutical compositions which can be used for treating, preventing or ameliorating of disorders responsive to induction of differentiation, such as e.g. any of those diseases mentioned herein, particularly cancer, such as e.g. any of those cancer diseases described herein above.

The invention further relates to the use of the compounds according to the present invention for the production of pharmaceutical compositions which can be used for treating, preventing or ameliorating of benign or malignant neoplasia, particularly cancer, such as e.g. any of those cancer diseases described herein above.

The invention further relates to the use of the compounds according to the present invention for the production of pharmaceutical compositions for the treatment of a disease different to a cellular neoplasia and sensitive to histone deacetylase inhibitor and/or protein kinase inhibitor therapy, such as the non-malignant diseases mentioned before.

The invention further relates to the use of the compounds according to the present invention for the production of pharmaceutical compositions for inhibiting histone deacetylase activity and/or protein kinase activity in the treatment of diseases responsive to said inhibition or to the functional consequences thereof.

The invention further relates to a method for treating, preventing or ameliorating the diseases, disorders, illnesses and/or conditions mentioned herein in a mammal, in particular a human patient, comprising administering a pharmacologically active and therapeutically effective and tolerable amount of one or more compounds according to the present invention to said mammal in need thereof.

The invention further relates to the compounds according to this invention for use in the treatment and/or prophylaxis of non-malignant diseases, especially the diseases mentioned above.

The invention further relates to a combination comprising one or more of the compounds according to this invention and a pharmaceutically acceptable diluent, excipient and/or carrier, e.g. for treating, preventing or ameliorating (hyper)proliferative diseases of benign or malignant behaviour and/or disorders responsive to induction of apoptosis, such as, for example, benign or malignant neoplasia, e.g. cancer, such as e.g. any of those cancer diseases described herein above.

The invention further relates to pharmaceutical compositions according to this invention having histone deacetylases and/or protein kinase inhibitory activity.

The invention further relates to pharmaceutical compositions according to this invention having apoptosis inducing activity.

The invention further relates to pharmaceutical compositions according to this invention having anti-proliferative activity.

The invention further relates to pharmaceutical compositions according to this invention having cell differentiation inducing activity.

The invention further relates to the use of a pharmaceutical composition comprising one or more of the compounds according to this invention and a pharmaceutically acceptable carrier or diluent in the manufacture of a pharmaceutical product, such as e.g. a commercial package, for use in the treatment and/or prophylaxis of the diseases as mentioned.

Additionally, the invention relates to an article of manufacture, which comprises packaging material and a pharmaceutical agent contained within said packaging material, wherein the pharmaceutical agent is therapeutically effective by inhibiting histone deacetylases and / or protein kinases, ameliorating the symptoms of a histone deacetylase or protein kinase mediated disorder, and wherein the packaging material comprises a label or package insert which indicates that the pharmaceutical agent is useful for preventing or treating histone deacetylase / protein kinase mediated disorders, and wherein said pharmaceutical agent comprises one or more compounds according to the invention. The packaging material, label and package insert otherwise parallel or resemble what is generally regarded as standard packaging material, labels and package inserts for pharmaceuticals having related utilities.

The pharmaceutical compositions according to this invention are prepared by processes which are known per se and familiar to the person skilled in the art. As pharmaceutical compositions, the compounds of the invention (= active compounds) are either employed as such, or preferably in combination with suitable pharmaceutical auxiliaries and/or excipients, e.g. in the form of tablets, coated tablets, capsules, caplets, suppositories, powders, patches (e.g. as TTS), emulsions, suspensions, gels or solutions, the active compound content advantageously being between 0.1 and 95% and where, by the appropriate choice of the auxiliaries and/or excipients, a pharmaceutical administration form (e.g. a delayed release form or an enteric form) exactly suited to the active compound and/or to the desired onset of action can be achieved.

The person skilled in the art is familiar with auxiliaries, vehicles, excipients, diluents, carriers or adjuvants which are suitable for the desired pharmaceutical formulations, preparations or compositions on account of his/her expert knowledge. In addition to solvents, gel formers, ointment bases and other active compound excipients, for example antioxidants, dispersants, emulsifiers, preservatives, solubilizers, colorants, complexing agents or permeation promoters, can be used.

Depending upon the particular disease, to be treated or prevented, additional therapeutic active agents, which are normally administered to treat or prevent that disease, may optionally be coadministered with the compounds according to the present invention. As used herein, additional therapeutic agents that are normally administered to treat or prevent a particular disease are known as appropriate for the disease being treated.

For example, the compounds according to this invention may be combined with standard therapeutic agents or radiation used for treatment of the diseasesas mentioned before.

In one particular embodiment the compounds according to this invention may be combinded with one or more art-known anti-cancer agents, such as e.g. with one or more art-known chemotherapeutic and/or target specific anti-cancer agents, e.g. with one or more of those described below, and/or radiation.

Examples of known chemotherapeutic anti-cancer agents frequently used in combination therapy include, but not are limited to (i) alkylating/carbamylating agents such as Cyclophosphamid (Endoxan®), Ifosfamid (Holoxan®), Thiotepa (Thiotepa Lederle®), Melphalan (Alkeran®), or chloroethylnitrosourea (BCNU); (ii) platinum derivatives like cis-platin (Platinex® BMS), oxaliplatin (Eloxatin®), or carboplatin (Cabroplat® BMS); (iii) antimitotic agents / tubulin inhibitors such as vinca alkaloids (vincristine, vinblastine, vinorelbine), taxanes such as Paclitaxel (Taxol®), Docetaxel (Taxotere®) and analogs as well as new formulations and conjugates thereof like the nanoparticle formulation Abraxane™ with paclitaxel bound to albumin, epothilones such as Epothilone B (Patupilone®), Azaepothilone (Ixabepilone®) or ZK-EPO, a fully synthetic epothilone B analog; (iv) topoisomerase inhibitors such as anthracyclines (exemplified by Doxorubicin / Adriblastin®), epipodophyllotoxines (examplified by Etoposide / Etopophos®) and camptothecin and camptothecin analogs (exemplified by Irinotecan / Camptosar® or Topotecan / Hycamtin®); (v) pyrimidine antagonists such as 5-fluorouracil (5-FU), Capecitabine (Xeloda®), Arabinosylcytosine / Cytarabin (Alexan®) or Gemcitabine (Gemzar®); (vi) purin antagonists such as 6-mercaptopurine (Puri-Nethol®), 6-thioguanine or fludarabine (Fludara®) and finally (vii) folic acid antagonists such as methotrexate (Farmitrexat®) or Pemetrexed (Alimta®).

Examples of target specific anti-cancer drug classes used in experimental or standard cancer therapy include but are not limited to (i) kinase inhibitors such as e.g. Imatinib (Glivec®), ZD-1839 / Gefitinib (Iressa®), Bay43-9006 (Sorafenib, Nexavar®), SU11248 / Sunitinib (Sutent®) or OSI-774 / Erlotinib (Tarceva®), Nilotinib (AMN-107), Dasatinib (Sprycel®), Lapatinib (Tykerb®), or, see also below, Vatalanib, Vandetanib (Zactima®) or Pazopanib; (ii) proteasome inhibitors such as PS-341 /Bortezumib (Velcade®); (iii) heat shock protein 90 inhibitors like 17-allylaminogeldanamycin (17-AAG) or 17-dimethylaminogeldanamycin (17-DMAG) ; (iv) vascular targeting agents (VTAs) like combretastatin A4 phosphate or AVE8062 /AC7700 and anti-angiogenic drugs like the VEGF antibodies, such as Bevacizumab (Avastin®), or KDR tyrosine kinase inhibitors such as PTK787 / ZK222584 (Vatalanib) or Vandetanib (Zactima®) or Pazopanib; (v) monoclonal antibodies such as Trastuzumab (Herceptin®) or Rituximab (MabThera / Rituxan®) or Alemtuzumab (Campath®) or Tositumomab (Bexxar®) or C225/ Cetuximab (Erbitux®) or Avastin (see above) or Panitumumab (Vectibix®) as well as mutants and conjugates of monoclonal antibodies, e.g. Gemtuzumab ozogamicin (Mylotarg®) or Ibritumomab tiuxetan (Zevalin®), and antibody fragments; (vi) oligonucleotide based therapeutics like G-3139 / Oblimersen (Genasense®) or the DNMT1 inhibitor MG98; (vii) Toll-like receptor / TLR 9 agonists like Promune®, TLR 7 agonists like Imiquimod (Aldara®) or Isatoribine and analoges thereof, or TLR 7/8 agonists like Resiquimod as well as immunostimulatory RNA as TLR 7/8 agonists; (viii) protease inhibitors (ix) hormonal therapeutics such as anti-estrogens like Tamoxifen (Nolvaed®) or Raloxifen (Evista®), anti-androgens like Flutamide (Drogenil®) or Bicalutamide (Casode®), LHRH analogs like Leuprolide, Goserelin or Triptorelin and aromatase inhibitors like Letrozole (Femara®) and Anastrozole (Arimedex®).

Other known target specific anti-cancer agents which can be used for combination therapy include bleomycin, retinoids such as all-trans retinoic acid (ATRA), DNA methyltransferase inhibitors such as 5-Aza-2' -deoxycytidine (Decitabine Dacogen®) and 5-Azacytidine (Vidaza®), alanosine, cytokines such as interleukin-2, interferons such as interferon α2 or interferon-γ, death receptor agonists, such as TRAIL, DR4/5 agonistic antibodies, FasL and TNF-R agonists (e.g. TRAIL receptor agonists like mapatumumab or lexatumumab), and finally histone deacetylase inhibitors different to the compounds according to this invention such as SAHA (Zolinza®), PXD101, MS275 (SNDX275), MGCD0103, Depsipeptide / FK228, NVP-LBH589, CRA / PCI 24781, ITF2357, SB939, Valproic acid (VPA) and butyrates.

Exemplary anti-cancer agents for use in combination with the compounds according to this invention in the therapies mentioned herein are as mentioned above. The anti-cancer agents mentioned herein above as combination partners of the compounds according to this invention are meant to include pharmaceutically acceptable derivatives thereof, such as e.g. their pharmaceutically acceptable salts.

The person skilled in the art is aware on the base of his/her expert knowledge of the kind: total daily dosage(s) and administration form(s) of the additional therapeutic agent(s) coadministered. Said total daily dosage(s) can vary within a wide range.

In practicing the present invention and depending on the details, characteristics or purposes of their uses mentioned above, the compounds according to the present invention may be administered in combination therapy separately, sequentially, simultaneously, concurrently or chronologically staggered (e.g. as combined unit dosage forms, as separate unit dosage forms or a adjacent discrete unit dosage forms, as fixed or non-fixed combinations, as kit-of-parts or as admixtures) with one or more standard therapeutics, in particular art-known chemotherapeutic and/or target specific anti-cancer agents, such as e.g. any of those mentioned above.

Thus, a further aspect of the present invention is a combination or pharmaceutical composition comprising a first active ingredient, which is a compound according to this invention, a second active ingredient, which is an art-known standard therapeutic, in particular art-known chemotherapeutic or target specific anti-cancer agent, such as one of those mentioned above, and optionally a pharmacologically acceptable carrier, diluent and/or excipient for sequential, separate, simultaneous or chronologically staggered use in therapy in any order, e.g. to treat, prevent or ameliorate in a patient diseases responsive to HDAC inhibitor and / or protein kinase inhibitor treatment, such as the diseases, disorders or illnesses mentioned, in particular cancer.

In this context, the present invention further relates to a combination comprising a first active ingredient, which is at least one compound according to this invention, and a second active ingredient, which is at least one art-known standard therapeutic, for example an art-known anti-cancer agent, such as e.g. one or more of those mentioned herein above, for separate, sequential, simultaneous, concurrent or chronologically staggered use in therapy, such as e.g. in therapy of any of those diseases mentioned herein.

The term "combination" according to this invention may be present as a fixed combination, a non-fixed combination or a kit-of-parts.

A "fixed combination" is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A "kit-of-parts" is defined as a combination wherein the said first active ingredient and the said second active ingredient are present in more than one unit. One example of a "kit-of-parts" is a combination wherein the said first active ingredient and the said second active ingredient are present separately. The components of the kit-of-parts may be administered separately, sequentially, simultaneously, concurrently or chronologically staggered.

The first and second active ingredient of a combination or kit-of-parts according to this invention may be provided as separate formulations (i.e. independently of one another), which are subsequently brought together for simultaneous, sequential, separate or chronologically staggered use in combination therapy; or packaged and presented together as separate components of a combination pack for simultaneous, concurrent, sequential, separate or chronologically staggered use in combination therapy.

The type of pharmaceutical formulation of the first and second active ingredient of a combination or kit-of-parts according to this invention can be similar, i.e. both ingredients are formulated in separate tablets or capsules, or can be different, i.e. suited for different administration forms, such as e.g. one active ingredient is formulated as tablet or capsule and the other is formulated for e.g. intravenous administration. The amounts of the first and second active ingredients of the combinations, compositions or kits according to this invention may together comprise a therapeutically effective amount for the treatment, prophylaxis or amelioration of a disease responsive or sensitive to the inhibition of histone deacetylases and / or protein kinases, particularly one of those diseases mentioned herein, e.g. benign or malignant neoplasia, particularly cancer, like any one of those cancer diseases mentioned herein.

A further aspect of the present invention is a combination comprising, in non-fixed form, one or more bifunctional compounds according to this invention or the salts thereof, and one or more art-known standard therapeutic, in particular art-known chemotherapeutic or target specific anti-cancer agents, such as those mentioned above, for sequential, separate, simultaneous or chronologically staggered use in therapy in any order, e.g. to treat, prevent or ameliorate in a patient diseases responsive to HDAC inhibitor and/or protein kinase inhibitor treatment, such as the diseases, disorders or illnesses mentioned, in particular cancer. Optionally said combination comprises instructions for its use in therapy.

A further aspect of the present invention is a combined preparation, such as e.g. a kit of parts, comprising a preparation of a first active ingredient, which is a compound according to this invention and a pharmaceutically acceptable carrier or diluent; a preparation of a second active ingredient, which is an art-known therapeutic agent, in particular an anti-cancer agent, such as e.g. one of those mentioned above, and a pharmaceutically acceptable carrier or diluent; and optionally instructions for simultaneous, sequential, separate or chronologically staggered use in therapy, e.g. to treat benign and malignant neoplasia or diseases different to cellular neoplasia responsive or sensitive to the inhibition of histone deacetylases and / or protein kinases.

A further aspect of the present invention is a kit of parts comprising a dosage unit of a first active ingredient, which is a anilid or hydroxamate derivative mentioned in above or a salt thereof, a dosage unit of a second active ingredient, which is an art-known standard therapeutic, in particular an anti-cancer agent such as e.g. one of those mentioned above, and optionally instructions for simultaneous, sequential or separate use in therapy, e.g. to treat disorders responsive or sensitive to the inhibition of histone deacetylases and/or protein kinases, such as, for example, benign or malignant neoplasia, e.g. cancer. A further aspect of the present invention is a pharmaceutical product comprising one or more compounds according to this invention, or one or more pharmaceutical compositions comprising said compounds; and one or more art-known therapeutic agents, in particular art-known anti-cancer agents, or one or more pharmaceutical compositions comprising said therapeutic agents, such as e.g. those mentioned above, for simultaneous, sequential or separate use in therapy, e.g. to treat diseases as mentioned before, in particular cancer. Optionally this pharmaceutical product comprises instructions for use in said therapy.

In this connection, the present invention further relates to combinations, compositions, formulations, preparations or kits according to the present invention having histone deacetylases and / or protein kinase inhibitory activity.

A further aspect of the present invention is a pharmaceutical composition as unitary dosage form comprising, in admixture, a first active ingredient, which is a bifunctional compound according to this invention or a salt thereof, a second active ingredient, which is an art-known standard therapeutic, in particular art-known chemotherapeutic or target specific anti-cancer agent, such as one of those mentioned above, and optionally a pharmacologically acceptable carrier, diluent or excipient.

The present invention further relates to a pharmaceutical composition comprising
(i) a first active ingredient, which is at least one compound according to this invention, and
(ii) a second active ingredient, which is at least one art-known anti-cancer agent, such as e.g. one or more of those mentioned herein above, and, optionally, a pharmaceutically acceptable carrier or diluent, for separate, sequential, simultaneous, concurrent or chronologically staggered use in therapy, such as e.g. in therapy of diseases responsive or sensitive to the inhibition of histone deacetylases and/or protein kinases particularly (hyper)proliferative diseases and/or disorders responsive to induction of apoptosis, such as e.g. any of those diseases mentioned herein, like benign or malignant neoplasia, especially cancer, particularly any of those cancer diseases described above.

The present invention further relates to a combination product comprising
(i) at least one compound according to this invention formulated with a pharmaceutically acceptable carrier or diluent, and
(ii) at least one art-known anti-cancer agent, such as e.g. one or more of those mentioned herein above, formulated with a pharmaceutically acceptable carrier or diluent.

The present invention further relates to a kit-of-parts comprising a preparation of a first active ingredient, which is a compound according to this invention, and a pharmaceutically acceptable carrier or diluent; a preparation of a second active ingredient, which is an art-known anti-cancer agent, such as one of those mentioned above, and a pharmaceutically acceptable carrier or diluent; for simultaneous, concurrent, sequential, separate or chronologically staggered use in therapy. Optionally, said kit comprises instructions for its use in therapy, e.g. to treat diseases responsive or sensitive to the inhibition of histone deacetylases and / or protein kinases, such as e.g. cellular neoplasia or diseases different to cellular neoplasia as indicated above, particularly cancer, such as e.g. any of those cancer diseases described above.

The present invention further relates to a combined preparation comprising at least one compound according to this invention and at least one art-known anti-cancer agent for simultaneous, concurrent, sequential or separate administration.

In this connection, the present invention further relates to combinations, compositions, formulations, preparations or kits according to the present invention having histone deacetylase inhibitory and/or protein kinase inhibitory activity.

Also in this connection, the present invention further relates to combinations, compositions, formulations, preparation or kits according to the present invention having anti-(hyper)proliferative and/or apoptosis inducing activity.

In addition, the present invention further relates to the use of a composition, combination, formulation, preparation or kit according to this invention in the manufacture of a pharmaceutical product, such as e.g. a commercial package or a medicament, for treating, preventing, or ameliorating diseases responsive or sensitive to the inhibition of histone deacetylases and / or protein kinases, particularly those diseases mentioned herein, such as e.g. benign or malignant neoplasia, particularly cancer.

The present invention further relates to a commercial package comprising one or more compounds of the present invention together with instructions for simultaneous, sequential or separate use with one or more chemotherapeutic and/or target specific anti-cancer agents, such as e.g. any of those mentioned herein.

The present invention further relates to a commercial package consisting essentially of one or more compounds of the present invention as sole active ingredient together with instructions for simultaneous, sequential or separate use with one or more chemotherapeutic and/or target specific anti-cancer agents, such as e.g. any of those mentioned herein.

The present invention further relates to a commercial package comprising one or more chemotherapeutic and/or target specific anti-cancer agents, such as e.g. any of those mentioned herein, together with instructions for simultaneous, sequential or separate use with one or more compounds according to the present invention.

Furthermore, also an aspect of the present invention is a method for treating diseases and/or disorders responsive or sensitive to the inhibition of histone deacetylases, e.g. (hyper)proliferative diseases and/or disorders responsive to induction of apoptosis, such as e.g. cancer, in combination therapy in a patient comprising administering a pharmacologically active and therapeutically effective and tolerable amount of a pharmaceutical combination, composition, formulation, preparation or kit as described above to said patient in need thereof.

A further aspect of the present invention is a method for treating cotherapeutically diseases responsive or sensitive to inhibiting histone deacetylases and/or protein kinases, such as e.g. those diseases as mentioned before, particularly cancer, in a patient in need of such treatment comprising administering separately, sequentially, simultaneously, concurrently, fixed or non-fixed a pharmacologically active and therapeutically effective and tolerable amount of one or more of the compounds according to the present invention and a pharmacologically active and therapeutically effective and tolerable amount of one or more art-known therapeutic agents, in particular anti-cancer agents, such as those mentioned above, to said patient.

In further addition, the present invention relates to a method for treating, preventing or ameliorating (hyper)proliferative diseases and/or disorders responsive to induction of apoptosis, such as e.g. benign or malignant neoplasia, e.g. cancer, particularly any of those cancer diseases mentioned herein, in a patient comprising administering separately, simultaneously, concurrently, sequentially or chronologically staggered to said patient in need thereof an amount of a first active compound, which is a compound according to the present invention, and an amount of at least one second active compound, said at least one second active compound being a standard therapeutic agent, particularly at least one art-known anti-cancer agent, such as e.g. one or more of those chemotherapeutic and target-specific anti-cancer agents mentioned herein, wherein the amounts of the first active compound and said second active compound result in a therapeutic effect.

In yet further addition, the present invention relates to a method for treating, preventing or ameliorating (hyper)proliferative diseases and/or disorders responsive to induction of apoptosis, such as e.g. benign or malignant neoplasia, e.g. cancer, particularly any of those cancer diseases mentioned herein, in a patient comprising administering a combination according to the present invention.

The pharmaceutical compositions, combinations, preparations, formulations, kits, products or packages mentioned above may also include more than one of the compounds according to this invention and/or more than one of the art-known standard therapeutics, in particular anti-cancer agents as mentioned.

In addition, compounds according to the present invention can be used in the pre- or post-surgical treatment of cancer.

Furthermore, compounds according to the present invention can be used in combination with radiation therapy, in particular in sensitization of cancer patients towards standard radiation therapy.

The administration of the compounds according to this invention, the combinations and pharmaceutical compositions according to the invention may be performed in any of the generally accepted modes of administration available in the art. Illustrative examples of suitable modes of administration include intravenous, oral, nasal, inhalativ, parenteral, topical, transdermal and rectal delivery. Oral and intravenous delivery are preferred.

For the treatment of dermatoses, the compounds according to the invention are in particular administered in the form of those pharmaceutical compositions which are suitable for topical application. For the production of the pharmaceutical compositions, the compounds of the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, pastes, gels or solutions.

The pharmaceutical compositions according to the invention are prepared by processes known per se. The dosage of the compounds according to the invention (= active compounds) is carried out in the order of magnitude customary for histone deacetylases or protein kinase inhibitors. Topical application forms (such as ointments) for the treatment of dermatoses thus contain the active compounds in a concentration of, for example, 0.1-99%. The customary dose in the case of systemic therapy (p.o.) may be between 0.03 and 60 mg/kg per day, (i. v.) may be between 0.03 and 60 mg/kg/h. In another embodiment, the customary dose in the case of systemic therapy (p.o.) is between 0.3 and 30 mg/kg per day, (i. v.) is between 0.3 and 30 mg/kg/h.

The choice of the optimal dosage regime and duration of medication, particularly the optimal dose and manner of administration of the active compounds necessary in each case can be determined by a person skilled in the art on the basis of his/her expert knowledge.

### Examples

The following examples serve to illustrate the present invention.

### Chemical Syntheses

### I. Synthesis of Sorafenib chimera

### (E)-3-(4-{4-[3-(Chloro-trifluoromethyl-phenyl)-ureido]-phenoxy}-pyridin-2-yl)-N-hydroxy-acrylamide

### 1-(4-Benzyloxy-phenyl)-3-(4-chloro-3-trifluoromethyl-phenyl)-urea:

15 g (4-Benzyloxy-phenylamine) is diluted in 260 ml dichloromethane under argon atmosphere. The solution is cooled down to 0°C. 18.35 g Chloro-isocyanato-trifluoromethyl-benzene are dissolved in 260 ml dichloromethane and is slowly added to the solution. The solution is stirred overnight at ambient temperature. The solid is filtered and washed with dichloromethane and dried in high vacuo. By this method 28.37 g of the title compound are obtained (Yield: 90 %).

### 1-(4-Chloro-3-trifluoromethyl-phenyl)-3-(4-hydroxy-phenyl)-urea:

26.37 g 1-(4-Benzyloxy-phenyl)-3-(4-chloro-3-trifluoromethyl-phenyl)-urea are diluted in 200 ml MeOH/THF (1:1). 2.6 g of Pd/C are added under argon atmosphere. The solution is hydrogenated at ambient temperature. The reaction is filtered and washed with dichloromethane. The compound is concentrated by vacuo and cristalyzed with ethylacetate. By this method 18.5 g of the title compound are obtained (Yield: 89 %).

### 1-[4-(2-Chloro-pyridin-4-yloxy)-phenyl]-3-(4-chloro-3-trifluoromethyl-phenyl)-urea:

470 mg NaH 60 % in mineral oil is suspended in 90 ml DMF under argon atmosphere. The suspension is cooled down to 0°C. 3 g of 1-(4-Chloro-3-trifluoromethyl-phenyl)-3-(4-hydroxy-phenyl)-urea are added and the colour of the suspension turned from white to red-brown. 1.868 g of 2-chloro-4-nitropyridine are added slowly to the reaction mixture. The reaction is stirred overnight at ambient temperature. The reaction is cooled to 0°C and poured into water, after 1.5 h it is filtrated and washed with water. The solid is dried in high vacuo. By this method 3.3 g of the title compound are obtained (Yield: 84 %).

### 1-(4-Chloro-3-trifluoromethyl-phenyl)-3-[4-(2-vinyl-pyridin-4-yloxy)-phenyl]-urea:

998 mg of 1-[4-(2-Chloro-pyridin-4-yloxy)-phenyl]-3-(4-chloro-3-trifluoro-methylphenyl)-urea are diluted in DME under argon atmosphere. 580 mg of vinylboronic-acid-dibutylester, 317 mg Dichlorobis(triphenylphosphine)-palladium(II) and 3.4 ml sodium carbonate (2M) are added and the reaction is stirred overnight at 90°C under argon atmosphere.

The reaction solution is filtrated and concentrated under reduced pressure. Ethyl acetate is added and the organic layer is washed three times with sodium hydrogen carbonate. The organic phases are combined and dried over sodium sulfate. The crude material is then purified by silica gel flash chromatography.

By this method 627 mg of the title compound are obtained (Yield: 64 %).

### 1-(4-Chloro-3-trifluoromethyl-phenyl)-3-[4-(2-formyl-pyridin-4-yloxy)-phenyl]-urea:

627 mg of 1-(4-Chloro-3-trifluoromethyl-phenyl)-3-[4-(2-vinyl-pyridin-4-yloxy)-phenyl]-urea are diluted in 12 ml dioxane and 4 ml water. 330 µl 2,6-lutidine and 2.92 ml osmiumtetroxid (2.5 % wt in 2-methyl-2-propanol solution) are added.
After 5 minutes 1.2 g of sodiumperiodate is added. The reaction is stirred for 1.5 h at ambient temperature. The reaction is poured into dichloromethane and water. The organic phase is extracted three times with water. The organic layer is dried over sodiumsulfate and concentrated by vacuo.

### (E)-3-(4-{4-[3-(Chloro-trifluoromethyl-phenyl)-ureido]-phenoxy}-pyridin-2-yl)-acrylic acid tert-butylester:

83 mg NaH (60 %) is suspended in THF. The suspension is cooled to -30°C. 0.57 ml of (Dimethoxy-phoshoryl)-acetic acid tert-butylester is added. The solution is stirred for 1 h at ambient temperature.

The reaction is cooled to -30°C again and 1-(4-Chloro-3-trifluoromethxl-phenyl)-3-[4-(2-formyl-pyridin-4-yloxy)-phenyl]-urea is added. The reaction is stirred at ambient temperature for 2 h. The reaction is poured on ammoniumchloride/water and the water phase is extracted three times with ethylacetate. The organic layer is dried over sodiumsulfate, concentrated in high vacuo and purified by silica gel flash chromatography. By this method 614 mg of the title compound are obtained (Yield: 65%).

### (E)-3-(4-{4-[3-(Chloro-trifluoromethyl-phenyl)-ureido]-phenoxy}-pyridin-2-yl)-acrylic acid:

390 mg of (*E*)-3-(4-{4-[3-(Chloro-trifluoromethyl-phenyl)-ureido]-phenoxy}-pyridin-2-yl)-acrylic acid tert-butylester is diluted in 15 ml dichloromethane. 1.3 ml trifluoroacetic acid are added and the solution is stirred at ambient temperature for 8 hours. The solvent is evaporated and coevaporated with toluene. The compound is dried in high vacuo.

### (E)-3-(4-{4-[3-(Chloro-trifluoromethyl-phenyl)-ureido]-phenoxy}-pyridin-2-yl)-N-(tetrahydro-pyran-2-yloxy)-acrylamide:

### (E)-3-(4-{4-[3-(Chloro-trifluoromethyl-phenyl)-ureido]-phenoxy}-pyridin-2-yl)-acrylic

acid, 228 mg HOBt, 781 mg EDC are solved in 62 ml DMF and 1.9 ml NEt₃. The mixture is stirred for 1 h at ambient temperature under argon atmosphere. 176 mg of *O*-(Tetrahydro-2*H*-pyran-2-yl)-hydroxylamine are added and the reaction is stirred overnight at ambient temperature under argon atmosphere. DMF is evaporated and the crude material is extracted between ethylacetate and water. The organic layer is dried over sodium sulfate, filtered and dried in high vacuo.

The crude material is purified by silica gel flash chromatography. By this method 289 mg of the title compound is obtained (Yield: 35 %).

### (E)-3-(4-{4-[3-(Chloro-trifluoromethyl-phenyl)-ureido]-phenoxy}-pyridin-2-yl)-N-hydroxy-acrylamide:

289 mg of (E)-3-(4-{4-[3-(Chloro-trifluoromethyl-phenyl)-ureido]-phenoxy}-pyridin-2-yl)-N-(tetrahydro-pyran-2-yloxy)-acrylamide are diluted in 3 ml methanol. 4.9 ml of a HCl-solution (1M) are added and the reaction is stirred at ambient temperature. The product is crystallized with ethylacetate and dried in high vacuo. By this method 41 mg of the title compound is obtained (Yield: 41 %).

### II. Synthesis of Lapatinib Chimera

### 6-Iodo-1H-quinazolin-4-one (10)

was prepared from 2-amino-5-iodo-benzoic acid (Acros) according to Boyd and Castaner (Lapatinib: oncolytic dual EGFR and erbB-2 inhibitor. Drugs for the Future 2005, 30, 1225-1239).

### 2-Chloro-1-(3-fluorobenzyloxy)-4-nitrobenzene (14)

was prepared from (3-fluoro-phenyl)-methanol **(13)**(Aldrich) and 2-chloro-1-fluoro-4-nitro-benzene **(12)** (Aldrich) according to Wallace et al. (Preparation of cyanoguanidines and cyanoamidines as ErbB2 and EGFR inhibitors. 2005). Yield (20.0g; 85%) yellow crystals. ¹H-NMR (DMSO-[D₆]): δ (ppm) = 5.41 (s, 2H), 7.18-7.25 (m, H), 7.31-7.35 (m, 2H), 7.43-7.50 (m, 2H), 8.26 (dd, 1H, J = 9.2 Hz, ⁴J = 2.8 Hz), 8.35 (d, 1H, ⁴J = 2.8 Hz).

### 3-Chloro-4-(3-fluorobenzyloxy)phenylamine (15)

Preparation analogous Wallace et al.; (Preparation of cyanoguanidines and cyanoamidines as ErbB2 and EGFR inhibitors. 2005) as follows : 2-chloro-1-(3-fluorobenzyloxy)-4-nitrobenzene **(14)** (20.0 g; 70.9 mmol) was dissolved in THF (300mL), dry sulfided platinum on carbon (Aldrich) (4.00g) were added and the mixture stirred under a hydrogen atmosphere (10 atm) over night. The platinum was filtered over cellite, washed with THF, the solvent removed under reduced pressure and the residue treated with light petrol to afford the title compound as a analytical pure solid. Yield (17.49 g; 98%). ¹H-NMR (DMSO-[D₆]): δ (ppm) = 4.98 (s, 2H, exchangeable), 5.04 (s, 2H), 6.54 (dd, 1H, J = 8.8 Hz, ⁴J = 2.6 Hz), 6.74 (d, 1H, ⁴J = 2.6 Hz), 6.92 (d, 1H, J = 8.8 Hz), 7.09-7.15 (m, 1H), 7.25-7.30 (m, 2H), 7.37-7.45 (m, 1H).

### [3-Chloro-4-(3-fluorobenzyloxy)phenyl]-(6-iodoquinazo-lin-yl)amine (16)

(Roschangar et al. ,Use of lithium N,O-dimethylhydroxylamide as an efficient in situ protecting agent for aromatic aldehydes. Tertrahedron 2002, 58, 1657-1666). Preparation analogous (Nishino et al.; Process for producing 4-aminoquinazoline compound by chlorination of quinazolin-4-one or its derivative and amination. 2003) as follows: To a mixture of 6-iodo-1*H*-quinazolin-4-one **(10)** (6.80g; 25.0 mmol), toluene (5.0 mL) and POCl₃ (27.5 mmol; 2.60 mL) carefully triethylamine (27.5 mmol; 3.81 mL) was added. The mixture was heated to 80 °C for 2 h, cooled to room temperature, a solution of 3-chloro-4-(3-fluorobenzyloxy)phenylamine **(15)** (27.50 mmol; 6.92 g) in 2-butanone (20.0 mL) added and the mixture stirred at 80 °C for another hour. The mixture was cooled to 0°C, the yellow precipitate was filtered off and added to a NaOH solution (1N; 150 mL) by stirring. After 30 min the yellow solid was filtered off, washed with water and a small amount of acetone and dried in vacuo. Yield (8.38 g; 66%) analytical pure sample. ¹H-NMR (DMSO-[D₆]): δ (ppm) = 5.26 (s, 2H), 7.15-7.22 (m, 1H), 7.27 (d, 1H, J = 9.1 Hz), 7.29-7.35 (m, 2H), 7.43-7.51 (m, 1H), 7.56 (d, 1H, J = 8.8 Hz), 7.74 (dd, 1H, J = 9.1 Hz, ⁴J = 2.5 Hz), 8.02 (d, 1H, ⁴J = 2.5 Hz), 8.12 (dd; 1H, J = 8.8 Hz, ⁴J = 1.7 Hz), 8.62 (s, 1H), 8.96 (d, ⁴J = 1.7 Hz), 9.90 (s, 1H, exchangeable).

### General Procedure for Suzuki coupling

Preparation analogous to Hosoya et al. (Danthrone Analogues Revisted: General Synthesis and Specific Functions Capable of Discriminating Two Kinds of Ca2+ Release from Sarcoplasmic Rerticulum of Mouse Skeletal Muscle. Bioorg. Med. Chem. 2003, 11, 663-673*).* A mixture of **16** (5.00 g; 9.88 mmol), the respective arylboronic acid **(17a, 17b, 22b:** Aldrich; **17c, 17d** Alfa Aesar **22a** Rare Chemicals ) (13.1 mmol), (PPh₃)₂PdCl₂ (0.35 g; 0.5 mmol), DME (30 mL), ethanol (20 mL) and 2M aqueous Na₂CO₃ (30 mL) was heated at 60 °C for 3h. After being cooled to room temperature, the crude product precipitated as a yellow solid. It was removed by filtration, washed with water and dried in vacuum over night. Crystallisation from acetone afforded the title compound in sufficient purity for further synthesis. An analytical sample was obtained column chromatography (SiO₂; CH₂Cl₂/ethyl acetate = 4:1).

### 5-{4-[3-Chloro-4-(3-fluorobenzyloxy)phenylamino]-quinazolin-6-yl}furan-2-carbaldehyde (18a)

(Roschangar et al.; Use of lithium N,O-dimethylhydroxylamide as an efficient in situ protecting agent for aromatic aldehydes. Tertrahedron 2002, 58, 1657-1666): Yield (3.65 g, 78%). mp: 229.8-234.1 °C. ¹H-NMR (DMSO-[D₆]): δ (ppm) = 5.26 (s, 2H), 7.17 (dt, 1H, J = 2.5 Hz, J = 8.5), 7.26-7.34 (m, 3H), 7.40 (d, 1H, J = 3.6 Hz), 7.43-7.50 (m, 1H), 7.68-7.74 (m, 2H), 7.84 (d, 1H, J = 8.5 Hz), 7.98 (d, 1H, J = 2.5 Hz), 8.28 (dd, 1H, J = 1.1 Hz, J = 8.8 Hz), 8.58 (s, 1H), 8.95 (d, 1H, J = 0.8 Hz), 9.66 (s, 1H), 10.10 (s, 1H, exchangeable). + p ESI m/z (%): 476 [M+H⁺]⁺⁻ (37); 474 [M+H⁺]⁺⁻(100); - p ESI m/z (%): 474 [M-H⁺]⁻ (37), 472 [M-H⁺]⁻ (100). IR (KBr): 3399, 1673 cm⁻¹.

### 5-{4-[3-Chloro-4-(3-fluorobenzyloxy)phenylamino]-quinazolin-6-yl}thiophene-2-carbaldehyde semihydrat (18b):

### Yield (2.85 g, 59%). mp: 251.8-251.9 °C. ¹H-NMR (DMSO-[D₆]): δ (ppm) = 5.28 (s, 2H, OCH₂), 7.19 (dt, 1H, J = 2.0 Hz, J = 8.7 Hz), 7.30-7.36 (m, 3H), 7.45-7.52 (m, 1H), 7.72 (dd, 1H, J = 2.5 Hz, J = 8.9 Hz), 7.85 (d, 1H, J = 8.7 Hz), 7.93 (d, 1H, J = 4.0 Hz), 7.99 (d, 1H, J = 2.5), 8.14 (d, 1H, J = 4.0 Hz), 8.29 (dd, 1H, J = 1.7 Hz, J = 8.7 Hz), 8.60 (s, 1H), 8.91 (d, 1H, J = 1.7 Hz), 9.97 (s, 1H, ), 10.02 (s, 1H, exchangeable).+ p ESI m/z (%): 492 [M+H⁺]⁺ (41), 490 [M+H⁺]⁺ (100 %); - p ESI m/z (%): 490 [M-H⁺]⁻ (42), 488 [M-H⁺]⁻ (100), 977 [2M-H⁺]⁻ (60). IR (KBr): 3279, 1703 cm⁻¹. Anal. (C₂₆H₁₇ClFN₃O₂S x ½ H₂O): C, H, N.

### 3-{4-[3-Chloro-4-(3-fluorobenzyloxy)phenylamino]-quinazolin-6-yl}benzaldehyde (18c):

mp: 243.2-247.5 °C. Yield (2.73 g, 54 %) yellow crystals. ¹H-NMR (DMSO-[D₆]): 6 (ppm) = 5.25 (s, 2H), 7.18 (dt, 1H, J = 2.2 Hz, J = 8.3 Hz), 7.28 (d, 1H, J = 9.1 Hz), 7.33 (m, 2H), 7.47 (dt, 1H, J = 6.1 Hz, J = 8.0 Hz), 7.74 (dd, 1H, J = 2.2 Hz, J = 6.7 Hz), 7.78 (t, 1H, J = 5.4 Hz), 7.86 (d, 1H, J = 8.7 Hz), 7.97 (td, 1H, J = 1.2 Hz, J = 7.6 Hz), 8.01 (d, 1H, J = 2.6 Hz), 8.21 (m, 2H), 8.38 (t, 1H, J = 1.6 Hz), 8.60 (s, 1H), 8.85 (d, 1H, J = 1.6 Hz), 9.94 (s, 1H, exchangeable), 10.14 (s, 1H). EI-MS (70eV) m/z (%): 483 (18) [M⁺⁻]⁺, 374 (100) [M-C₇H₆F^{.}]⁺. IR (KBr): 3380, 2729, 1696 cm⁻¹. Anal. (C₂₈H₁₉ClFN₃O₂ x 1/5 H₂O): C, H, N.

### 4-{4-[3-Chloro-4-(3-fluorobenzyloxy)phenylamino]-quinazolin-6-yl}benzaldehyde (18d):

mp: 236.6-238.0 °C. Yield (4.69 g, 97%). ¹H-NMR (DMSO-[D₆]): δ (ppm) = 5.26 (s, 2H), 7.18 (dt, 1H, J = 1.7 Hz, J = 8.2 Hz), 7.31 (m, 3H), 7.47 (m, 1H), 7.75 (dd, 1H, J = 2.5 Hz, J = 8.9 Hz), 7.87 (d, 1H, J = 8.7 Hz), 8.02 (d, 1H, J = 2.5 Hz), 8.10 (q, 4H, J = 8.5 Hz), 8.26 (dd, 1H, J = 1.7 Hz, J = 8.7 Hz), 8.61 (s, 1H), 8.90 (d, 1H, J = 1.6 Hz), 9.97 (s, 1H, exchangeable), 10.09 (s, 1H). EI-MS (70eV) *m*/*z* (%): 483 (15) [M⁺⁻]⁺, 374 (100) [M-C₇H₆F⁻]⁺. IR (KBr): 3317, 1677 cm⁻¹. Anal. (C₂₈H₁₉ClFN₃O₂ x ½ H₂O): C, H, N.

### 5-{4-[3-Chloro-4-(3-fluorobenzyloxy)phenylamino]-quinazolin-6-yl}furan-2-carboxylic acid (23a):

Crystallisation from methanol. Yield (2.30 g, 46%). mp: 283.7-285.2 °C. ¹H-NMR (DMSO-[D₆]): δ (ppm) = 5.27 (s, 2H), 7.19 (dt, 1H, J = 2.1 Hz, J = 8.5), 7.27-7.40 (m, 5H), 7.45-7.52 (m, 1H), 7.71 (d, 1H, J = 2.6 Hz, J = 8.7 Hz), 7.85 (d, 1H, J = 8.8 Hz), 7.99 (d, 1H, J = 2.5 Hz), 8.26 (dd, 1H, J = 1.6 Hz, J = 8.8 Hz), 8.58 (s, 1H), 8.89 (d, 1H, J = 1.4 Hz), 10.10 (s, 1H, exchangeable). CI-MS m/z (%): 488 [M]⁺⁻ (9), 453 (13), 444 (23), 409 (100), 336 (66). Anal. (C₂₆H₁₇ClFN₃O₄ x MeOH x H₂O): C, H, N.

### 5-{4-[3-Chloro-4-(3-fluorobenzyloxy)phenylamino]-quinazolin-6-yl}thiophene-2-carboxylic acid (23b):

The precipitating product was extracted with ethyl acetate, the organic layer washed with HCl (1N), dried (Na₂SO₄) and the solvent removed under reduced pressure. Yield (4.11 g, 81%). mp: 312.0-314.0 °C. ¹H-NMR (DMSO-[D₆]): δ (ppm) = 5.27 (s, 2H), 7.16-7.35 (m, 6H, 1H exchangeable), 7.44-7.48 (m, 1H), 7.53 (d, 1H, J = 3.6 Hz), 7.76 (d, 2H, J = J = 8.8 Hz), 8.05 (d, 1H, J = 2.5 Hz), 8.12 (d, 1H, J = 8.5 Hz), 8.54 (s, 1H), 8.78 (s, 1H), 10.23 (s, 1H, exchangeable). + p ESI m/z (%): 508 [M+H⁺]⁺ (42), 506 [M+H⁺]⁺ (100 %); - p ESI m/z (%): 506 [M-H⁺]⁻ (42), 504 [M-H⁺]⁻ (100). IR (KBr): 3521, 1612. Anal. (C₂₆H₁₇ClFN₃O₃S x 2HCl x H₂O): C, H, N.

### Acrylic acid tert-butyl esters (19a-d).

A mixture of the respective aryl-2-carbaldehydes **18a-18d** (1.0 mmol), (*tert-*butoxycarbonylmethyl)triphenylphosphonium chloride (0.56 g; 1.02 mmol), NaOH (0.08 g; 2.04 mmol), NEt₃ (0.3 g; 3.06 mmol) in CH₂Cl₂ (100 mL) and water (2.04 mL) was stirred at room temperature over night. The organic layer was separated, subsequently washed with sat. NH₄Cl solution (3 x 50 mL), dried (Na₂SO₄), filtered and concentrated under reduced pressure. The crude product was purified by column chromatography (DCM/EE = 1:1) and precipitated by addition of light petrol.

### 3-(5-{4-[3-Chloro-4-(3-fluorobenzyloxy)phenyl-amino]quinazolin-6-yl}furan-2-yl)acrylic acid tert-butyl ester (19a):

Yield (0.43 g, 76%) yellow crystals. mp: 201.3-201.4 °C. ¹H-NMR (DMSO-[D₆]): δ (ppm) = 1.50 (s, 9H), 5.28 (s, 2H), 6.50 (d, 1H, Jₜᵣₐₙₛ = 15.6 Hz), 7.13 (d, 1H, J = 3.6 Hz), 7.19 (dt, 1H, J = 2.4 Hz, J = 8.6 Hz), 7.28-7.36 (m, 4H), 7.44 (d, 1H, Jₜᵣₐₙₛ = 15.6 Hz), 7.45-7.52 (m, 1H), 7.72 (dd, 1H, J = 2.5 Hz, J = 8.8 Hz), 7.82 (d, 1H, J = 8.8 Hz), 7.99 (d, 1H, J = 2.5 Hz), 8.29 (dd, 1H, J = 1.5 Hz, J = 8.6 Hz), 8.57 (s, 1H), 8.87 (d, 1H, J = 1.1 Hz), 9.97 (s, 1H, exchangeable).+ p ESI m/z (%): 574 [M+H⁺]⁺ (39), 572 [M+H⁺]⁺ (100 %); - p ESI m/z (%): 572 [M-H⁺]⁻ (40), 570 [M-H⁺]⁻. IR (KBr): 2977, 1697 cm⁻¹. Anal. (C₃₂H₂₇ClFN₃O₄): C, H, N.

### 3-(5-{4-[3-Chloro-4-(3-fluorobenzyloxy)phenyl-amino]quinazolin-6-yl}thiophene-2-yl) acrylic acid tert-butyl ester (19b):

Yield (1.05 g, 31%) yellow crystals. mp: 204.1-204.4 °C. ¹H-NMR (DMSO-[D₆]): δ (ppm) = 1.49 (s, 9H), 5.28 (s, 2H), 6.21 (d, 1H, Jₜᵣₐₙₛ = 15.7 Hz), 7.19 (dt, 1H, J = 1.7 Hz, J = 8.2 Hz), 7.33 (m, 3H, aromat.), 7.49 (dt, 1H, J = 6.1 Hz, J = 8.0 Hz), 7.65 (d, 1H, J = 3.9 Hz), 7.73 (m, 3H), 7.82 (d, 1H, J = 8.7 Hz), 8.00 (d, 1H, J = 2.5 Hz), 8.17 (dd, 1H, J = 1.8 Hz, J = 8.8 Hz), 8.58 (s, 1H), 8.79 (d, 1H, J = 1.8 Hz), 9.96 (s, 1H, exchangeable). + p ESI m/z (%): 588 [M+H⁺]⁺ (100); - p ESI m/z (%): 586 [M-H⁺]⁻(100), 1173 [2M-H⁺]⁻ (50). IR (KBr): 2977, 1700 cm⁻¹. Anal. (C₃₂H₂₇ClFN₃O₃S x 1/3 H₂O) C, H, N.

### 3-(3-{4-[3-Chloro-4-(3-fluorobenzyloxy)-phenylamino]quinazolin-6-yl}phenyl)acrylic acid tert-butyl ester (19c):

Yield (2.30 g, 76 %) colorless crystals. mp: 202.9-203.0 °C. ¹H-NMR (DMSO-[D₆]): δ (ppm) = 1.51 (s, 9H), 5.27 (s, 2H), 6.73 (d, 1H, J = 16.0 Hz), 7.19 (dt, 1H, J = 2.2 Hz, J = 8.2 Hz), 7.32 (m, 3H), 7.48 (m, 1H), 7.60 (t, 1H, J = 7.7 Hz), 7.69 (d, 1H, J = 16.0 Hz), 7.76 (m, 2H), 7.87 (d, 1H, J = 8.7 Hz), 7.94 (d, 1H, J = 7.9 Hz), 8.03 (d, 1H, J = 2.6 Hz), 8.20 (m, 1H), 8.29 (dd, 1H, J = 1.7 Hz, J = 8.7 Hz), 8.60 (s, 1H), 8.83 (d, 1H, J = 1.5 Hz), 9.90 (s, 1H, exchangeable). EI-MS (70eV) *mlz* (%): 581 (18) [M⁺⁻]⁺, 236 (100) [M-C₁₁H₁₄F⁻]⁺. IR (KBr): 3379, 2982, 1676 cm⁻¹. Anal. (C₃₄H₂₉ClFN₃O₃) C, H, N.

### E-3-(4-{4-[3-Chloro-4-(3-fluorobenzyloxy)-phenylamino]quinazolin-6-yl}phenyl)acrylic acid tert-butyl ester (19d);

Yield (4.00 g, 71 %). mp: 211.9-212.0 °C. ¹H-NMR (DMSO-[D₆]): δ (ppm) = 1.50 (s, 9H), 5.26 (s, 2H), 6.64 (d, 1H, J = 16.0 Hz), 7.19 (dt, 1H, J = 1.8, J = 8.2 Hz), 7.29 (d, 1H, J = 9.0 Hz), 7.34 (m, 2H), 7.47 (m, 1H), 7.63 (d, 1H, J = 16.0 Hz), 7.75 (dd, 1H, J = 2.6 Hz, J = 8.9 Hz), 7.85 (d, 1H, J = 8.8 Hz), 7.92 (dd, 4H, J = 8.6 Hz, J = 20.2 Hz), 8.02 (d, 1H, J = 2.6 Hz), 8.23 (dd, 1H, J = 1.7 Hz, J = 8.8 Hz), 8.59 (s, 1H), 8.85 (d, 1H, J = 1.4 Hz), 9.95 (s, 1H, exchangeable). EI-MS (70eV) m/z (%): 581 (20) [M⁺⁻]⁺, 416 (100) [M-C₁₁H₁₄F⁻]⁺. IR (KBr): 3444, 2932, 1709 cm⁻¹. Anal. (C₃₄H₂₉ClFN₃O₃) C, H, N.

### Synthesis of acrylic acids (20a-20d) by cleavage of the acrylic acid tert-butyl esters:

The respective acrylic acid *tert*-butyl ester (3.50 mmol) was dissolved in 10 mL TFA. The mixture was stirred at 20°C for 30 min, added to water (50 mL) by stirring, the yellow precipitate filtered off, washed with water and dried in vacuo.

### E-3-(5-{4-[3-Chloro-4-(3-fluorobenzyloxy)-phenylamino]quinazolin-6-yl}furan-2-yl)acrylic acid (20a):

Yield (0.18 g, 99%) yellow crystals. mp: 268.8-268.9 °C. ¹H-NMR (DMSO-[D₆]): δ (ppm) = 5.27 (s, 2H), 6.53 (d, 1H, Jₜᵣₐₙₛ = 15.9 Hz), 7.11 (d, 1H, J 0 3.6 Hz). 7.18 (dt, 1H, J = 2.0 Hz, J = 8.6 Hz), 7.26-7.35 (m, 4H, aromat.), 7.41-7.50 (m, 1H), 7.46 (d, 1 H, Jₜᵣₐₙₛ = 15.9 Hz), 7.73 (dd, 1H, J = 2.5 Hz, J = 8.9 Hz), 7.82 (d, 1H, J = 8.8 Hz), 8.00 (d, 1H, J = 2.5 Hz), 8.27 (dd, 1H, J = 1.6 Hz, J = 8.8 Hz), 8.57 (s, 1H), 8.86 (d, 1H, J = 1.4 Hz), 9.96 (s, 1H, exchangeable), 12.45 (s, 1H, exchangeable). + p ESI m/z (%): 518 [M+H⁺]⁺ (37), 516 [M+H⁺]⁺ (100); - p ESI m/z (%): 516 [M-H⁺]⁻ (40), 514 [M+H⁺]⁺ (100). IR (KBr): 3406, 1673 cm⁻¹. Anal. (C₂₈H₁₉ClFN₃O₄) C, H, N.

### E-3-(5-{4-[3-Chloro-4-(3-fluorobenzyloxy)phenyl-amino]quinazolin-6-yl}thiophene-2-yl)acrylic acid (20b):

Yield: (0.70 g, 53%) yellow crystals. mp: 251.7 °C. ¹H-NMR (DMSO-[D₆]): δ (ppm) = 5.31 (s, 2H), 6.24 (d, 1H, Jₜᵣₐₙₛ = 15.7 Hz), 7.20 (m, 1H), 7.34 (m, 3H), 7.49 (m, 1H), 7.66 (m, 2H), 7.79 (m, 2H), 7.90 (m, 2H), 8.37 (dd, 1H, J = 1.7 Hz, J = 8.8 Hz), 8.86 (d, 1H, J = 4.3 Hz), 8.92 (d, 1H, J = 1.5 Hz), 11.16 (s, 1H,exchangeable), 12.53 (s, 1 H, exchangeable).+ p ESI m/z (%): 532 [M+H⁺]⁺⁻ (100); - p ESI m/z (%): 530 [M-H⁺]⁻(100), 1064 [2M-H⁺]⁻ (10), 644 [M + TFA⁻]⁻ (15). IR (KBr) : 1672 cm⁻¹. Anal. (C₂₈H₁₉ClFN₃O₃S) C, H, N.

### E-3-(3-{4-[3-Chloro-4-(3-fluorobenzyloxy)-phenylamino]quinazolin-6-yl}-phenyl)acrylic acid (20c):

Yield (0.78 g, 99 %) orange crystals. mp: 275.1-275.2 °C. ¹H-NMR (DMSO-[D₆]): δ (ppm) = 5.29 (s, 2H), 6.73 (d, 1H, J = 16.0 Hz), 7.19 (dt, 1H, J = 2.2 Hz, J = 8.3 Hz), 7.33 (m, 3H), 7.48 (m, 1H), 7.62 (t, 1H, J = 7.7 Hz), 7.71 (m, 2H), 7.80 (d, 1H, J = 7.8 Hz), 7.92 (m, 2H), 7.96 (d, 1H, J = 2.5 Hz), 8.19 (s, 1H), 8.42 (dd, 1H, J = 1.6 Hz, J = 8.8 Hz), 8.83 (s, 1H), 8.94 (d, 1H, J = 1.2 Hz), 10.88 (s, 1H, exchangeable), 12.58 (s, 1H, exchangeable). ES-MS (DCM/MeOH + 10 mmol/l NH₄Ac) *m*/*z*(%): 526 (100) [M+H⁺⁻]⁺. IR (KBr): 3442, 2934, 1671 cm⁻¹. Anal. (C₃₀H₂₁ClFN₃O₃ x ¾ TFA) C, H, N.

### E-3-(4-{4-[3-Chloro-4-(3-fluorobenzyloxy)-phenylamino]quinazolin-6-yl}phenyl)acrylic acid (20d):

Yield (1.12 g, 96 %) yellow crystals from propa*N*-2-one/ethyl acetate. mp: 278.8-280.2 °C. ¹H-NMR (DMSO-[D₆]): δ (ppm) = 5.29 (s, 2H), 6.67 (d, 1H, J = 16.0 Hz), 7.20 (dt, 1H, J = 1.8 Hz, J = 8.3 Hz), 7.33 (m, 3H), 7.48 (m, 1H), 7.68 (dd, 2H, J = 6.7 Hz, J = 9.2 Hz), 7.94 (m, 6H), 8.38 (dd, 1H, J = 1.6 Hz, J = 8.8 Hz), 8.79 (s, 1H), 8.95 (d, 1H, J = 1.2 Hz), 10.73 (s, 1H, exchangeable), 12.51 (s, 1H, exchangeable). ES-MS (DCM/MeOH + 10 mmol/l NH₄Ac) m/z (%): 526 (100) [M+H⁺⁻]⁺. IR (KBr): 3442, 2939, 1670 cm⁻¹. Anal. (C₃₀H₂₁ClFN₃O₃ x 3/2 TFA) C, H, N.

### (2-Aminophenyl)carbamic acid tert-butyl ester (25):

**25** was prepared according to Petatis and Patel (Synthesis of piperazinones and benzopiperazinones from 1,2-diamines and organoboronic acids. Tertrahedron Lett. 2000, 41, 9607-9611) as follwows: *o*-Phenylendiamin **(24)** (5.04g; 50.0 mmol) was dissolved in THF (25.0 mL), the mixture cooled to 0°C and BOC₂O, dissolved in the same amount of THF was added dropwise within 15 min. The solution was allowed to warm up to room temperature over night, the mixture added to water (250 mL) by stirring. After 2 hours the precipitating product was removed by filtration. Purification by cc (SiO₂; CH₂Cl₂, EE 10: 1) and crystallisation from light petrol yielded the desired analytical pure compound. Yield: (6.20 g; 59%) colourless crystals. ¹H-NMR (DMSO-[D₆]): δ (ppm) = 1.51 (s, 9H), 3.51 (s, br. 2H, exchangeable), 6.26 (s, br., 1H, exchangeable), 6.75-6.81 (m, 2H), 6.96-7.02 (m, 1H), 7.24-7.28 (m, 1H). General Procedure for amidation by use of BOP as coupling reagent - Preparation of compounds **21a-21d, 26a-26b** and **27a-27b.** A mixture of the respective carboxylic acid (1.15 mmol), BOP (0.53 g; 1.20 mmol), the respective amine (2-aminophenyl)carbamic acid *tert*-butyl ester (Petatis and Patel; Synthesis of piperazinones and benzopiperazinones from 1,2-diamines and organoboronic acids. Tertrahedron Lett. 2000, 41, 9607-9611) (25) or O-tetrahydropyran-2-yl)hydroxylamine (Aldrich)) (1.15 mmol) and NEt₃ (0.24 g; 2.40 mmol) in DMF (10 mL) was stirred at room temperature for 12h. The solution was added to water (50 mL) by stirring, the precipitating product filtered off, washed with water and dried in vacuo. Crystallisation from the respective solvent given afforded the pure compound as yellow crystals.

### E-3-(5-{4-[3-Chloro-4-(3-fluorobenzyloxy)-phenylamino]quinazolin-6-yl}furan-2-yl)-N-(tetrahydropyraN-2-yloxy)acrylamide (21a):

Crystallisation from acetone. Yield: (0.55 g, 78%). mp: 232.8-232.9 °C. ¹H-NMR (DMSO-[D₆]): δ (ppm) = 1.54 (s, br., 3H), 1.70 (s, br., 3H), 3.54 (d, br., 1H, J = 11.0 Hz), 3.92-4.02 (m, br., 1H), 4.93 (s, 1H), 5.28 (s, 2H), 6.55 (d, 1H, Jₜᵣₐₙₛ = 15.6 Hz), 7.04 (d, 1H, J = 3.3 Hz), 7.16-7.52 (m, 7H), 7.74 (dd, 1H, J = 8.5 Hz, J = 1.4 Hz), 7.84 (d, 1H, J = 8.8 Hz), 8.01 (d, 1H, J = 2.5 Hz), 8.22 (dd, 1H, J = 8.8 Hz, J = 1.4 Hz), 8.58 (s, 1H), 8.86 (s, 1H), 9.98 (s, 1H,exchangeable), 11.30 (s, 1H, exchangeable).+ p ESI m/z (%): 617 [M+H⁺]⁺ (39), 615 [M+H⁺]⁺ (100), ; - p ESI m/z (%): 615 [M-H⁺]⁻(37), 613 [M-H⁺]⁻ (100). IR (KBr) : 3295, 2950, 1651 cm⁻¹. Anal. (C₃₃H₂₈ClFN₄O₅ x ½ H₂O) C, H, N.

### E-3-(5-{4-[3-Chloro-4-(3-fluorobenzyloxy)-phenylamino]quinazolin-6-yl}thiophene-2-yl)-N-(tetrahydro-pyran-2-yloxy)acrylamide (21b):

Crystallisation from MeOH. Yield: (0.49 g, 80%). mp: 165.7-165.8 °C. ¹H-NMR (DMSO-[D₆]): δ (ppm) = 1.57 (s, br., 3H), 1.74 (s, br., 3H), 3.55 (d, 1H, J = 11.3 Hz), 3.97 (m, 1H), 4.93 (m, 1H), 5.29 (s, 2H), 6.32 (d, 1H, Jₜᵣₐₙₛ = 15.4 Hz), 7.18 (m, 1H), 7.33 (m, 3H), 7.50 (m, 2H), 7.72 (m, 3H), 7.84 (d, 1H, J = 8.7 Hz), 7.99 (d, 1H, J = 2.6 Hz), 8.23 (dd, 1H, J = 1.6 Hz, J = 8.8 Hz), 8.68 (s, 1H), 8.89 (d, 1H, J = 1.4 Hz), 10.50 (s, 1H, exchangeable), 11.33 (s, 1H, exchangeable).+ p ESI m/z (%): 631 [M+H⁺]⁺ (100), 1264 [2M+H⁺]⁺ (5); - p ESI m/z (%): 629 [M-H⁺]⁻ (100), 1262 [2M-H⁺]⁻ (14). IR (KBr): 3443, 2952, 1657 cm⁻¹. Anal. (C₃₃H₂₈ClFN₄O₄S x 3/2 H₂O) C, H, N.

### (E)3-(3-(4-(3-chloro-4-(3-fluorobenzyloxy)-phenylamino)quinazolin-6-yl)phenyl)-N-(tetrahydro-2H-pyran-2-yloxy)acrylamide (21c):

Yield (0.24 g, 67 %) colourless crystals. Purification by cc (SiO₂, ethyl acetate, CH₂Cl₂ 1:1) and crystallization from ethyl acetate. ¹H-NMR (DMSO-[D₆]): δ (ppm) = 1.55 (s, 3H), 1.71 (s, 3H), 3.54 (m, 1H), 3.96 (m, 1 H), 4.94 (s, 1H), 5.27 (s, 2H), 6.68 (d, 1H, J = 15.8 Hz), 7.19 (dt, 1H, J = 2.2 Hz, J = 8.2 Hz), 7.32 (m, 3H), 7.48 (m, 1H), 7.64 (m, 3H), 7.76 (dd, 1H, J = 2.5 Hz, J = 8.9 Hz), 7.88 (d, 1H, J = 8.7 Hz), 7.92 (m, 1H), 8.03 (d, 1H, J = 2.5 Hz), 8.07 (s, 1H), 8.25 (dd, 1H, J = 1.7 Hz, J = 8.7 Hz), 8.61 (s, 1H), 8.85 (d, 1H, J = 1.1 Hz), 9.95 (s, 1H, exchangeable), 11.29 (s, 1H, exchangeable).
ES-MS (DCM/MeOH + 10 mmol/l NH₄Ac) *m*/*z* (%): 625 (100) [M+H⁺⁻]⁺. IR (KBr): 3444, 2872, 1668 cm⁻¹. Anal. (C₃₅H₃₀ClFN₄O₄) C, H, N.

### (E)-3-(4-(4-(3-chloro-4-(3-fluorobenzyloxy)-phenylamino)quinazolin-6-yl)phenyl)-N-(tetrahydro-2H-pyran-2-yloxy)acrylamide (21d):

Yield (0.31 g, 62 %) colourless crystals. Purification by cc (SiO₂, ethyl acetate) and crystallization from ethyl acetate. ¹H-NMR (DMSO-[D₆]): δ (ppm) = 1.55 (s, 3H), 1.71 (s, 3H), 3.56 (m, 1H), 3.97 (m, 1H), 4.94 (s, 1H), 5.27 (s, 2H), 6.61 (d, 1H, J = 15.8 Hz), 7.19 (dt, 1H, J = 1.8 Hz, J = 8.2 Hz), 7.32 (m, 3H), 7.48 (m, 1H), 7.58 (d, 1H, J = 15.5 Hz), 7.77 (m, 3H), 7.87 (d, 1H, J = 8.7 Hz), 7.97 (d, 2H, J = 8.4 Hz), 8.03 (d, 1H, J = 2.5 Hz), 8.25 (dd, 1H, J = 1.7 Hz, J = 8.8 Hz), 8.60 (s, 1H), 8.86 (d, 1H, J = 1.5 Hz), 9.95 (s, 1H, exchangeable), 11.28 (s, 1H, exchangeable). ES-MS (DCM/MeOH + 10 mmol/l NH₄Ac) *m*/*z* (%): 625 (100) [M+H⁺⁻]⁺. IR (KBr): 3443, 2870, 1666 cm⁻¹. Anal. (C₃₅H₃₀ClFN₄O₄ x 3/2 H₂O) C, H, N.

### 5-{4-[3-Chloro-4-(3-fluorobenzyloxy)phenyl-amino]quinazolin-6-yl}furan-2-carboxylic acid (tetrahydropyran-2-yloxy)amide (27a):

The precipitating product was extracted with methylene chloride (100mL), the organic layer washed with water (3 x 30 mL), dried (Na₂SO₄), purified by cc (SiO₂; ethyl acetate) and crystallized from ethyl acetate. Yield (0.27 g, 39%). mp: 220.7-221.1 °C. ¹H-NMR (DMSO-[D₆]): δ (ppm) = 1.57 (s, br. 3H), 1.75 (s, br. 3H), 3.55 (d, br. 1H, J = 11.2 Hz), 4.06-4.15 (m, 1H), 5.02 (s, 1H), 5.27 (s, 2H), 7.15-7.23 (m, 2H), 7.29-7.35 (m, 4H), 7.44-7.51 (m, 1H), 7.72 (dd, 1H, J = 2.6 Hz, J = 8.9 Hz), 7.86 (d, 1H, J = 8.8 Hz), 8.00 (d, 1H, J = 2.5 Hz), 8.39 (dd, 1H, J = 1.6 Hz, J = 8.8 Hz), 8.59 (s, 1H), 8.90 (d, 1H, J = 1.4 Hz), 9,94 (s, 1H, exchangeable), 11.78 (s, 1H, exchangeable). ).+ p ESI m/z (%): 591 [M+H⁺]⁺ (38), 598 [M+H⁺]⁺ (100); - p ESI m/z (%): 591 [M-H⁺]⁻ (38), 589 [M-H⁺]⁻ (100). IR (KBr): 3320, 2945, 1652 cm⁻¹. Anal. (C₃₁H₂₆ClFN₄O₅): C, H, N.

### 5-{4-[3-Chloro-4-(3-fluorobenzyloxy)phenyl-amino]quinazolin-6-yl}thiophene-2-carboxylic acid (tetrahydropyran-2-yloxy)amide (27b):

Yield (0.68 g, 97%). mp: 249.6-249.7 °C. ¹H-NMR (DMSO-[D₆]): δ (ppm) = 1.57 (s, br. 3H), 1.74 (s, br. 3H), 3.55 (d, br. 1H, J = 11.2 Hz), 4.06-4.15 (m, 1H), 4.99 (s, 1H), 5.28 (s, 2H), 7.19 (t, 1H, J = 7.6 Hz), 7.33 (t, 2H, J = 8.0 Hz), 7.39-7.57 (m, 2H), 7.69-7.88 (m, 3H), 7.95-8.00 (m, 2H), 8.26 (d, 1H, J = 9.1 Hz), 8.64 (s, 1H), 8.83 (d, 1 H, J = 1.4 Hz), 10.23 (s, 1H, exchangeable), 11.81 (s, 1H, exchangeable). + p ESI m/z (%): 607 [M+H⁺]⁺ (42), 605 [M+H⁺]⁺ (100 %); - p ESI m/z (%): 605 [M-H⁺]⁻ (42), 603 [M-H⁺]⁻ (100). IR (KBr): 3417, 2943, 1660 cm⁻¹. Anal. (C₃₁H₂₆ClFN₄O₄S): C , H, N.

### Synthesis of benzamides 7a-7e [3, 5, 10, 11, 13]:

The benzamides **7a-7e [3, 5, 10, 11, 13]** were prepared from the corresponding carboxylic acids **20a, 20c, 20d** and **23a, 23b** by coupling with (2-aminophenyl)carbamic acid *tert*-butyl ester according to the general procedure by use of BOP as coupling reagent as described above and cleavage of the crude carbamic acid *tert*-butyl esters with trifluoroacetate as described above for the acrylic acid *tert-*butyl esters. The title products were purified by cc (SiO₂; ethyl acetate) and crystallized from ethyl acetate by addition of light petrol.

### 5-{4-[3-Chloro-4-(3-fluorobenzyloxy)phenylamino]-quinazolin-6-yl}furan-2-carboxylic acid (2-amino-phenyl)amide (7a) [5]:

Yield overall (0.16 g, 34%) yellow crystals. mp: 250 °C (decomp.). ¹H-NMR (DMSO-[D₆]): δ (ppm) = 5.00 (s, br. 2H, exchangeable), 5.27 (s, 2H), 6.56-6.65 (m, 1H), 6.81 (d, 1H, 8.0 Hz), 7.01 (dt, 1H, J = 7.7 Hz, J = 1.0 Hz), 7.15-7.22 (m, 2H), 7.28-7.36 (m, 4H), 7.43-7.51 (m, 2H), 7.88 (d, 1H, J = 8.8 Hz), 8.02 (d, 1H, J = 2.5 Hz), 8.49 (d, 1H, J = 8.8 Hz), 8.60 (s, 1H), 8.96 (d, 1H, J = 1.1 Hz), 9.73 (s, 1H, exchangeable), 9.93 (s, 1H, exchangeable). + p ESI m/z (%): 582 [M+H⁺]⁺ (39), 580 [M+H⁺]⁺ (100). IR (KBr): 3321 cm⁻¹. Anal. (C₃₂H₂₃ClFN₅O₃ x 3/2 H₂O): C, H, N.

### 5-{4-[3-Chloro-4-(3-fluorobenzyloxy)phenylamino]-quinazolin-6-yl}thiophene-2-carboxylic acid (2-aminophenyl)amide (7b) [3]:

Yield overall (0.12 g, 25%) yellow crystals. mp: 236.9-237.0 °C. ¹H-NMR (DMSO-[D₆]): δ (ppm) = 4.97 (s, br. 2H, exchangeable), 5.27 (s, 2H), 6.62 (dt, 1H, J = 7.6 Hz, J = 1.3Hz), 6.80 (dd, 1H, J = 8.0 Hz, J = 1.3Hz), 7.00 (dt, 1H J = 7.6 Hz, J = 1.0 Hz), 7.16-7.22 (m, 2H), 7.29-7.36 (m, 3H), 7.45-7.52 (m, 1H), 7.74 (dd, 1H, J = 8.9 Hz, J = 2.7 Hz), 7.80 (d, 1H, J = 3.8 Hz), 7.84 (d, 1H, J = 8.6 Hz), 7.99 (d, 1H, J = 2.7 Hz), 8.07 (d, 1H, J = 3.8 Hz), 8.25 (dd, 1H, J = 8.6 Hz. J = 1.7 Hz), 8.58 (s, 1H), 8.84 (s, 1H), 9.79 (s, 1H, exchangeable), 9.99 (s, 1H, exchangeable). + p ESI m/z (%): 598 [M+H⁺]⁺ (43), 596 [M+H⁺]⁺ (100). IR (KBr): 3255, 1641 cm⁻¹. Anal. (C₃₂H₂₃ClFN₅O₂S): C, H, N.

### (E)-N-(2-aminophenyl)-3-(5-(4-(3-chloro-4-(3-fluorobenzyloxy)phenylamino)quinazolin-6-yl)furan-2-yl)acrylamide (7c) [13]:

Yield overall (0.29 g, 46 %) yellow crystals. ¹H-NMR (DMSO-[D₆]): δ (ppm) = 5.01 (s, 2H, exchangeable), 5.28 (s, 2H), 6.58 (dt, 1H, J = 1.3 Hz, J = 7.7 Hz), 6.76 (dd, 1H, J = 1.3 Hz, J = 8.0 Hz), 6.91 (m, 1H), 7.01 (m, 2H), 7.19 (dt, 1H, J = 2.5 Hz, J = 8.7 Hz), 7.26 (d, 1H, J = 3.5 Hz), 7.33 (m, 3H), 7.45 (m, 3H), 7.75 (td, 1H, J = 2.6 Hz, J = 8.9 Hz), 7.87 (d, 1H, J = 8.8 Hz), 8.03 (m, 1H), 8.25 (m, 1H), 8.59 (s, 1H), 8.91 (m, 1H), 9.51 (s, 1H, exchangeable), 10.06 (s, 1H, exchangeable). ES-MS (DCM/MeOH + 10 mmol/l NH₄Ac) *m*/*z* (%): 606 (100) [M+H⁺⁻]⁺. IR (KBr): 3375, 1617, 1530 cm⁻¹. Anal. (C₃₄H₂₅ClFN₅O₃): C, H, N.

### (E)-N-(2-aminophenyl)-3-(3-(4-(3-chloro-4-(3-fluorobenzyloxy)phenylamino)-quinazolin-6-yl)phenyl)acrylamide (7d) [11]:

Yield overall (0.12 g, 40 %) yellow crystals. ¹H-NMR (DMSO-[D₆]): δ (ppm) = 4.97 (s, 2H, exchangeable), 5.27 (s, 2H), 6.59 (dt, 1H, J = 1.4 Hz, J = 7.8 Hz), 6.76 (dd, 1H, J = 1.3 Hz, J = 8.0 Hz), 6.93 (dt, 1H, J = 1.4 Hz, J = 7.8 Hz), 7.06 (d, 1H, J = 15.8 Hz), 7.19 (dt, 1H, J = 1.8 Hz, J = 8.2 Hz), 7.32 (m, 3H), 7.38 (dd, 1H, J = 1.0 Hz, J = 7.9 Hz), 7.48 (dt, 1H, J = 6.0 Hz, J = 8.0 Hz), 7.64 (t, 1H, J = 7.6 Hz), 7.71 (m, 2H), 7.76 (dd, 1H, J = 2.6 Hz, J = 9.0 Hz), 7.91 (t, 1H, J = 7.8 Hz), 8.04 (d, 1H, J = 2.6 Hz), 8.11 (s, 1H), 8.26 (dd, 1H, J = 1.6 Hz, J = 8.7 Hz), 8.62 (s, 1H), 8.86 (d, 1H, J = 1.2 Hz), 9.44 (s, 1H, exchangeable), 9.94 (s, 1H, exchangeable). ES-MS (DCM/MeOH + 10 mmol/l NH₄Ac) *m*/*z* (%): 616 (100) [M+H⁺⁻]⁺. IR (KBr): 3380, 3028, 1930, 1660 cm⁻¹. Anal. (C₃₆H₂₇ClFN₅O₂): C, H, N.

### (E)-N-(2-aminophenyl)-3-(4-(4-(3-chloro-4-(3-fluorobenzyloxy)phenylamino)quinazolin-6-yl)phenyl)acrylamide (7e) [10]:

Yield overall (0.29 g, 47 %) yellow crystals. ¹H-NMR (DMSO-[D₆]): δ (ppm) = 4.99 (s, 2H, exchangeable), 5.27 (s, 2H), 6.60 (dt, 1H, J = 1.4 Hz, J = 7.8 Hz), 6.77 (dd, 1H, J = 1.3 Hz, J = 8.0 Hz), 6.94 (dt, 1H, J = 1.4 Hz, J = 8.0 Hz), 7.01 (d, 1H, J = 15.7 Hz), 7.19 (dt, 1H, J = 1.8 Hz, J = 8.2 Hz), 7.33 (m, 4H), 7.48 (dt, 1 H, J = 6.0 Hz, J = 8.0 Hz), 7.65 (d, 1H, J = 15.7 Hz), 7.77 (dd, 1H, J = 2.6 Hz, J = 9.0 Hz), 7.82 (d, 1 H, J = 8.3 Hz), 7.87 (d, 1H, J = 8.7 Hz), 8.00 (d, 2H, J = 8.4 Hz), 8.03 (d, 1H, J = 2.6 Hz), 8.26 (dd, 1H, J = 1.5 Hz, J = 8.8 Hz), 8.61 (s, 1H), 8.87 (d, 1H, J = 1.2 Hz), 9.44 (s, 1H, exchangeable), 9.96 (s, 1H, exchangeable). ES-MS (DCM/MeOH + 10 mmol/l NH₄Ac) *m*/*z* (%): 616 (100) [M+H⁺⁻]⁺. IR (KBr): 3414, 1656 cm⁻¹. Anal. (C₃₆H₂₇ClFN₅O₂ x 1/5 H₂O): C, H, N.

### Preparation of 7f [12] by catalytic hydrogenation of 7e [10]:

To a solution of 76 mg 7f [12] (12.3 µmol) in THF (30.0 mL) 15.2 mg PtO (83%Pt) were added and the mixture stirred in a hydrogen atmosphere at a pressure of 10 bar for 12 h. The catalyst was removed by filtration over a short chromatography column (SiO₂; THF) and the solvent removed under reduced pressure. The remaining solid was dissolved in propa*N*-2-one (30 mL), ethyl acetate was added (30.0 mL), most of the solvent removed under reduced pressure and the precipitating product removed by filtration.

### N-(2-aminophenyl)-3-(4-(4-(3-chloro-4-(3-fluorobenzyloxy)phenylamino)quinazolin-6-yl)phenyl)propanamide (7f) [12]:

Yield: (69 mg, 91%) yellow crystals. mp: 217.3-120.2 °C. ¹H-NMR (DMSO-[D₆]): δ (ppm) = 2.71 (t, 2H, J = 7.5Hz), 3.01 (t, 2H, J = 7.5Hz), 4.81 (s, 2H, exchangeable), 5.27 (s, 2H), 6.54 (dt, 1H, J = 1.2 Hz, J = 7.7 Hz), 6.71 (dd, 1H, J = 1.1 Hz, J = 7.9 Hz), 6.89 (dt, 1H, J = 1.4 Hz, J = 7.9 Hz), 7.15 (dd, 1H, J = 1.1 Hz, J = 7.8 Hz), 7.21 (dd, 1H, J = 2.2 Hz, J = 8.8 Hz), 7.30 (d, 2H, J = 9.0 Hz), 7.34 (m, 2H), 7.46 (d, 2H, J = 8.0 Hz), 7.76 (dd, 1H, J = 2.4 Hz, J = 9.0 Hz), 7.83 (d, 2H, J = 3.3 Hz), 7.86 (d, 1H, J = 3.9 Hz), 8.03 (d, 1H, J = 2.5 Hz), 8.20 (dd, 1H, J = 1.6 Hz, J = 8.8 Hz), 8.59 (s, 1H), 8.79 (d, 1H, J = 1.3 Hz), 9.16 (s, 1H, exchangeable), 9.91 (s, 1H, exchangeable). ES-MS (DCM/MeOH + 10 mmol/l NH₄Ac) *m*/*z* (%): 618 (100) [M+H⁺⁻]⁺. HR-PI-EI-MS Calcd. for C₃₆H₂₉ClFN₅O₂ [MH⁺⁻]: 617.1994; Found 617.1989. IR (KBr): 3318, 2852, 1645 cm⁻¹.

### Synthesis of N-hydroxy-acrylamides (6a-6d [2, 7, 8, 9], 8a [6] and 8b [4] from the respective (tetrahydropyran-2-yloxy)amide -precursors

To a stirred solution of the corresponding (tetrahydropyran-2-yloxy)amide (0.5 mmol) in MeOH (50 mL) was added 1N HCl (50 mL). The mixture was stirred at room temperature over night. Half of the solvent was removed under reduced pressure, the precipitating product was filtered off, crystallized from MeOH and dried in vacuo.

### E-3-(5-{4-[3-Chloro-4-(3-fluorobenzyloxy)-phenylamino]quinazolin-6-yl}furan-2-yl)-N-hydroxy-acrylamide hydrochloride monohydrate (6a) [2]:

Yield: (0.20 g, 60 %) yellow crystals. mp: 190.9-192.0 °C. ¹H-NMR (DMSO-[D₆]): δ (ppm) = 5.32 (s, 2H), 6.62 (d, 1H, Jₜᵣₐₙₛ = 15.6 Hz), 7.01 (d, 1H, J = 3.6 Hz), 7.19 (dt, 1H, J = 2.2 Hz, J = 8.9 Hz), 7.20-7.32 (m, 4H), 7.45-7.53 (m, 2H), 7.72 (dd, 1H, J = 2.5 Hz, J = 8.8 Hz), 7.95 (d, 1H, J = 2.5 Hz), 7.98 (d, 1H, J = 8.8 Hz), 8.40 (d, 1H, J = 0.8 Hz, J = 9.1 Hz), 8.91 (s, 1H), 9.40 (d, 1H, 0.8 Hz), 10.84 (s, 1H, exchangeable), 11,98 (s, 1H, exchangeable). + p ESI m/z (%): 533 [M+H⁺]⁺ (38), [M+H⁺]⁺ (531); - p ESI m/z (%): 589 [M+Ac⁻]⁻ (91), 565 [M+Cl⁻]⁻ (51), 592 [M-H⁺]⁻ (100) .IR (KBr): 3418, 2855, 1660 cm⁻¹. Anal. (C₂₈H₂₀ClFN₄O₄ x H₂O x HCl) C, H, N, Cl.

### E-3-(5-{4-[3-Chloro-4-(3-fluorobenzyloxy)-phenylamino]quinazolin-6-yl}thiophene-2-yl)-N-hydroxy-acrylamide hydrochloride monohydrate (6b) [7]:

Yield: (0.07 g, 23%) yellow crystals. mp: 234.2°C. ¹H-NMR (DMSO-[D₆]): δ (ppm) = 5.32 (s, 2H), 6.29 (d, 1H, Jₜᵣₐₙₛ = 15,6 Hz), 7.20 (dt, 1H, J = 1.8 Hz, J = 8.3 Hz), 7.35 (m, 3H), 7.49 (m, 2H), 7.68 (m, 2H), 7.94 (m, 3H), 8.37 (m, 1H), 8.92 (s, 1H), 9.20 (s, 1H, exchangeable), 10.90 (s, 1H, exchangeable), 11,84 (s, 1H, exchangeable). - p ESI m/z (%): 547 [M+H⁺]⁻ (43), 545 [M-H⁺]⁻ (100). IR (KBr): 1612 cm⁻¹. Anal. (C₂₈H₂₀ClFN₄O₃S x HCl x H₂O) C, H, N.

### (E)-3-(3-(4-(3-chloro-4-(3-fluorobenzyloxy)phen-ylamino)quinazolin-6-yl)phenyl)-N-hydroxy-acrylamide (6c) [9]:

Yield (0.13 g, 88 %) yellow crystals. NMR (DMSO-[D₆]): δ (ppm) = 5.32 (s, 2H), 6.70 (d, 1H, J = 15.8 Hz), 7.20 (dt, 1H, J = 2.1 Hz, J = 8.3 Hz), 7.35 (m, 3H), 7.49 (dt, 1H, J = 6.1 Hz, J = 8.0 Hz), 7.61 (dd, 2H, J = 7.8 Hz, J = 15.5 Hz), 7.70 (m, 2H), 7.97 (m, 2H), 8.04 (d, 1H, J = 8.7 Hz), 8.15 (s, 1H), 8.51 (dd, 1H, J = 1.1 Hz, J = 8.8 Hz), 8.97 (s, 1H), 9.28 (d, 1H, J = 0.9 Hz, exchangeable), 10.85 (s, 1H, exchangeable), 11.89 (s, 1H, exchangeable). ES-MS (DCM/MeOH + 10 mmol/l NH₄Ac) *m*/*z* (%): 541 (100) [M+H⁺]⁺. IR (KBr): 3385, 2860, 1662 cm⁻¹. Anal. (C₃₀H₂₂ClFN₄O₃ x HCl x 2 H₂O) C, H, N.

### (E)-3-(4-(4-(3-chloro-4-(3-fluorobenzyl-oxy)phenylamino)quinazolin-6-yl)phen-yl)-N-hydroxyacrylamide hydrochloride monohydrate (6d) [8]:

Yield (0.17 g, 85 %) yellow crystals. NMR (DMSO-[D₆]): δ (ppm) = 5.32 (s, 2H), 6.61 (d, 1H, J = 15.8 Hz), 7.20 (dt, 1H, J = 2.2 Hz, J = 8.3 Hz), 7.33 (dd, 1H, J = 2.1 Hz, J = 5.9 Hz), 7.38 (d, 2H, J = 9.3 Hz), 7.51 (ddd, 1H, J = 9.6 Hz, J = 11.9 Hz, J = 13.9 Hz), 7.70 (dd, 1H, J = 2.5 Hz, J = 8.9 Hz), 7.77 (d, 2H, J = 8.3 Hz), 7.94 (d, 1H, J = 2.5 Hz), 8.03 (dd, 1H, J = 5.2 Hz, J = 8.5 Hz), 8.50 (dd, 1H, J = 1.4 Hz, J = 8.8 Hz), 8.96 (s, 1H), 9.25 (d, 1H, J = 1.0 Hz, exchangeable), 10.86 (s, 1H, exchangeable), 11.83 (s, 1H, exchangeable). ES-MS (DCM/MeOH + 10 mmol/l NH₄Ac) *m*/*z* (%): 541 (100) [M+H⁺⁻]⁺. IR (KBr): 3421, 2856, 1616 cm⁻¹. Anal. (C₃₀H₂₂ClFN₄O₃ x HCl x H₂O): C, H, N, Cl.

### 5-{4-[3-Chloro-4-(3-fluorobenzyloxy)phenylamino]-quinazolin-6-yl}furan-2-carboxylic acid hydroxyamide hydrochloride monohydrate (8a) [6]:

Yield: (0.21 g, 79%), orange crystals. mp: 245-250°C. ¹H-NMR (DMSO-[D₆]): δ (ppm) = 5.30 (s, 2H), 7.16-7.36 (m, 5H), 7.44-7.52 (m, 2H), 7.79 (dd, 1H, J = 9.1 Hz, J = 2.5 Hz), 7.94 (d, 1H, J = 8.8 Hz), 8.02 (d, 1H, J = 2.5 Hz), 8.54 (dd, 1H, J = 8.8 Hz, J = 1.4 Hz), 8.93 (s, 1H), 9.76 (s, 1H), 11.57 (s, 1H, exchangeable), 11.18 (s, 1H, exchangeable). + p ESI m/z (%): 507 [M+H⁺]⁺ (37), 505 [M+H⁺]⁺ (100). IR (KBr): 3417, 3087, 2841, 1616 cm⁻¹. Anal. (C₂₆H₁₈ClFN₄O₄ x HCl x H₂O) C, H, N, Cl.

### 5-{4-[3-Chloro-4-(3-fluorobenzyloxy)phenyl-amino]quinazolin-6-yl}thiophene-2-carboxylic acid hydroxyamide hydrochloride dihydrate (8b) [4]:

Yield: (0.23 g, 47%), orange crystals. mp: 251.4°C. ¹H-NMR (DMSO-[D₆]): δ (ppm) = 5.32 (s, 2H), 7.20 (t, 1H, J = 8.2 Hz), 7.31-7.38 (m, 3H), 7.45-7.52 (m, 1H), 7.66-7.71 (m, 2H), 7.92-7.98 (m, 3H), 8.42 (d, 1H, J = 8.8 Hz), 8.92 (s, 1H), 9.27 (s, 1H), 11.45 (s, 1H, exchangeable), 11.90 (s, 1H, exchangeable). + p ESI m/z (%): 532 [M+H⁺]⁺ (42), 521 [M+H⁺]⁺ (100). IR (KBr): 3424, 3125, 1614 cm⁻¹. Anal. (C₂₆H₁₈ClFN₄O₃S x HCl x 2H₂O) C, H, N.

### (E)-3-(3-(methoxycarbonyl)phenyl)acrylic acid:

A mixture of methyl 3-formylbenzoate (Acros) (2.50 g; 15.2 mmol), malonic acid (3.17 g; 30.0 mmol), pyridine (2.5 mL) and piperidine (1.25 mL) was heated to 80°C for 4 h. The hot solution was poured into water (20 mL) by stirring, the precipitating crystals removed by filtration, washed with a small amount of cold water and dried in vacuum. Yield 2.20 g (72%). Mp.: 191.7-192.21 °C. IR (KBr): 3073, 2635, 1729, 1633 cm⁻¹. ¹H-NMR (DMSO-[D₆]): δ (ppm) = 3.88 (s, 3H), 6.61 (d, 1H, J = 16.1 Hz), 7.58 (t, 1H, J = 7.8), 7.67 (d, 1H, J = 16.1 Hz), 7.99 (m, 2H), 8.19 (t, 1H, J = 1.5 Hz). Calcd. (C₁₁H₁₀O₄) C 64.07, H 4.89. Found C 64.07, H 4.98.

### (E)-methyl 3-(3-(2-(tert-butoxycarbonylamino)phenylamino)-3-oxoprop-1-enyl)benzoate:

To a solution of (*E*)-3-(3-(methoxycarbonyl)phenyl)acrylic acid (2.80g, 10.77 mmol) in THF (25.0 mL), triethylamine (3.05 mL, 22.0 mmol), BOP (4.87g; 11.0 mmol) and tert-butyl 2-aminophenylcarbamate (10.77 mmol; 2.08 g) were added and the mixture stirred over night at room temperature. The mixture was poured into saturated brine, extracted with ethyl acetate (3 x 100 mL), the organic layer dried (Na₂SO₄), the solvent removed and the product purified by CC (SiO₂, CH₂Cl₂, ethyl acetate 10:1). Yield 2.55 g (60 %) colorless solid. Mp.: 79.0-82.4 °C. ¹H-NMR (DMSO-[D₆]): δ (ppm) = 1.46 (s, 9H), 3.90 (s, 3H), 7.06 (d, 1H, J = 16.3 Hz), 7.14 (dt, 2H, J = 1.9 Hz, J = 7.4 Hz), 7.63 (m, 4H), 7.91. (m, 1H), 7.99 (m, 1H), 8.25 (s, 1H), 8.50 (s, 1H), 9.72 (m, 1H).

### (E)-3-(3-(2-(tert-butoxycarbonylamino)phenylamino)-3-oxoprop-1-enyl)benzoic acid:

(*E*)-methyl 3-(3-(2-(tert-butoxycarbonylamino)phenylamino)-3-oxoprop-1-enyl)benzoate (2.35g; 5.93 mmol) was suspended in methanol / water (1:1; 100 mL), 1.2 equivalents LiOH were added and the mixture stirred at 80°C for 24h. Methanol was removed under reduced pressure and the aqueous layer extracted with ethyl acetate (2 x 20 mL). The aqueous layer was cooled in an ice bath to 0-5°C, acidified with acetic acid till pH = 5 and the precipitating product removed by filtration. Crystallization from methanol/ water (2:1) afforded the product as colorless crystals. Yield: 1.33 g; 59%. Mp.: 187.1-188.0 °C. IR (KBr): 3353, 2981, 1696, 1674 cm⁻¹. ¹H-NMR (DMSO-[D₆]): δ (ppm) = 1.46 (s, 9H), 7.06 (d, 1H, J = 15.4 Hz), 7.14 (dt, 1H, J = 1.9 Hz, J = 7.3 Hz), 7.62 (m, 4H), 7.88 (d, 1 H, J = 7.8 Hz), 7.97 (td, 1 H, J = 1.4 Hz, J = 7.7 Hz), 8.24 (s, 1 H), 8.49 (s, 1 H), 9.70 (s, 1 H), 13.16 (s, 1H). Calcd. (C₂₁H₂₂N₂O₅): C 65.96 H 5.80, N 7.33. Found C 65.78, H 5.98, N 7.25.

### (E)-tert-butyl 2-(3-(3-(4-(3-ethynylphenylamino)quinazolin-7-ylcarbamoyl)phenyl)acrylamido)phenylcarbamate:

(*E*)-3-(3-(2-(tert-butoxycarbonylamino)phenylamino)-3-oxoprop-1-enyl)benzoic acid (0.40 g; 1.05 mmol) was dissolved in dry pyridine (5.0 mL), 1.2 equivalents SOCl₂ were added and the mixture stirred at room temperature for 15 min. N⁴-(3-ethynylphenyl)quinazoline-4,7-diamine (0.27g; 1.05 mmol) was added and the mixture stirred at room temperature for 16 h. The mixture was pored into water, extracted with ethyl acetate (3 x 50 mL), the organic layer dried (Na₂SO₄), the solvent removed and the product purified by CC (SiO₂, CH₂Cl₂, ethyl acetate 1:2). Yield 0.18 g (27 %) colorless solid. Mp.: 217.0-218.0 °C. IR (KBr): 3294, 2978, 1692, 1668 cm⁻¹. ¹H-NMR (DMSO-[D₆]): δ (ppm) = 1.47 (s, 9H), 4.23 (s, 1 H), 7.09 (d, 1H, J = 15.5 Hz), 7.17 (m, 2H), 7.24 (m, 1 H), 7.42 (t, 1H, J = 7.9 Hz), 7.59 (d, 1H, J = 7.9 Hz), 7.68 (m, 3H), 7.92 (t, 2H, J = 8.4 Hz), 8.03 (m, 2H), 8.11 (m, 1 H), 8.30 (s, 1 H), 8.38 (d, 1 H, J = 2.0 Hz), 8.55 (m, 2H), 8.63 (s, 1 H), 9.78 (d, 2H, J = 4.1 Hz), 10.79 (s, 1H). ES-MS (MeOH + 10 mmol/l NH₄Ac) m/z (%): 624 [M+H⁺]⁺ (100). Calcd.(C₃₇H₃₂N₆O₄ x H₂O): C 69.14 H 5.33, N 13.08. Found C 69.10, H 5.54, N 12.96.

### (E)-3-(3-(2-aminophenylamino)-3-oxoprop-1-enyl)-N-(4-(3-ethynylphenylamino)quinazolin-7-yl)benzamide [42]:

(*E*)-tert-butyl 2-(3-(3-(4-(3-ethynylphenylamino)quinazolin-7-ylcarbamoyl)phenyl)acrylamido)phenylcarbamate (0.13 g; 0.21 mmol) was dissolved in formic acid (5.0 mL) and the solution stirred over night at room temperature. The mixture was poured into water (50 mL), alkalized with concentrated ammonia (pH = 9), the precipitating product removed by filtration, washed with water and dried. Yield 0.070 g (63%). Mp.: 190.0-194.2 °C. ¹H-NMR (DMSO-[D₆]): δ (ppm) = 4.22 (s, 1H), 4.99 (s, 2H), 6.59 (dt, 1 H, J = 1.4 Hz, J = 7.9 Hz), 6.76 (dd, 1 H, J = 1.3 Hz, J = 8.0 Hz), 6.93 (dt, 1 H, J = 1.3 Hz, J = 7.7 Hz), 7.07 (d, 1 H, J = 15.8 Hz), 7.23 (m, 1 H), 7.41 (m, 2H), 7.67 (m, 2H), 7.88 (d, 1 H, J = 7.8 Hz), 7.94 (m, 1 H), 8.03 (m, 2H), 8.11 (m, 1 H), 8.28 (s, 1 H), 8.37 (d, 1 H, J = 2.0 Hz), 8.55 (d, 1 H, J = 9.2 Hz), 8.62 (s, 1 H), 9.46 (s, 1 H), 9.78 (s, 1 H), 10.79 (s, 1H). ES-MS (MeOH + 10 mmol/l NH₄Ac) m/z (%): 525 [M+H⁺]⁺ (100). Calcd.(C₃₂H₂₄N₆O₂ x 3/2 H₂O): C 69.68, H 4.93, N 15.24. Found C 69.73, H 5.17, N 15.30

### III. Synthesis of Imantinib Hybride

***N*-(3-nitrophenyl)-4-(pyridin-3-yl)pyrimidin-2-amine** was prepared as described (Zimmermann; Phenylamino-pyrimidine (PAP)-Derivatives: A new class of potent and highly selective PDGFR Autophosphorylation Inhibitors. Bioorg. & Med. Chem. Lett. 1996, 6, 1221-1226).

***N*-(2-methyl-5-nitrophenyl)-4-(pyridin-3-yl)pyrimidin-2-amine** was prepared as described (Szakacs et al.; Acid-Base Profiling of Imatinib (Gleevec) and Its Fragments. J. Med. Chem. 2005, 48, 249-255).

### 1-(3-nitrophenyl)thiourea (61a) and 1-(2-methyl-5-nitrophenyl)thiourea (61b):

**61a** and **61b** were prepared according to a modification of the procedure reported by Rasmussen et al. (Improved Procedures for the Preparation of Cycloalkyl-, Arylalkyl-, and Arylthioureas. Synthesis 1988, 6, 456-459) as follows: Benzoyl chloride (0.11 mol, 11.6 mL) was added over 5 min to a freshly prepared solution of NH₄SCN (0.11 mol, 8.37g) in dry acetone (250 mL) and the mixture is heated under reflux for 15 min. Heating was stopped and the respective nitroaniline (0.10 mol), dissolved in dry acetone (100 mL) was added as rapidly as possible maintaining a vigorous reflux. Following the addition the mixture was heated under reflux for 30 min, then poured onto an excess of cracked ice with vigorous stirring. The resulting solid was collected and liberally washed with H₂O, followed by cold H₂O/MeOH (1:1) and MeOH, giving the intermediate *N-*(nitrophenylcarbamothioyl)benzamides as white solids in quantitative yield.

The intermediates, dissolved in a mixture of LiOH (2.63 g; 0.11 mmol) and THF/H₂O (1:1; 750 mL) were heated till gentle reflux for 6 h. The organic solvent was removed under reduced pressure, the solution cooled in an ice bath, the precipitating product removed by filtration and dried. Subjection to a soxleth extraction with methylene chloride for 2 days afforded the analytical pure compounds as light yellow crystals from the methylene chloride solution.

### 1-(3-nitrophenyl)thiourea (61a):

(Esser and Pook; Cyclic guanidines. IV. Intramolecular nucleophilic aromatic substitution of hydrogen in (3-nitrophenyl)guanidines. Synthesis 1992, 6, 596-601): Yield: 0.059 mol (59 %)

### 1-(2-methyl-5-nitrophenyl)thiourea (61b):

(Hayakawa et al.; Preparation of imidazopyridine derivatives as phosphatidylinositol 3-kinase inhibitors and anticancer agents. PCT Int. Appl. WO 2001083481 A1, 2001): Yield: 0.095 mol (95 %); mp: 164.5-166°C. ¹H-NMR (DMSO-[D₆]): δ (ppm) = 2.31 (s, 3H), 7.40 (s, br. 1H, exchangeable), 7.51 (d, 1H, J = 8.5 Hz), 7.90 (s, br., 1H, exchangeable), 7.99 (d, 1H, J = 8.5 Hz), 8.28 (s, 1 H), 9.41 (s, 1H, exchangeable).

### N-(3-nitrophenyl)-5-(pyridiN-3-yl)thiazol-2-amine (31a) and N-(2-methyl-5-nitrophenyl)-5-(pyridin-3-yl)thiazol-2-amine (31b):

A mixture of 2-bromo-1-(pyridin-3-yl)ethanone (62) and 1.0 eq. of the respective thiourea derivative (**61b** or **61a**) was dissolved in the necessary amount of ethanole and stirred to reflux for 2h. After cooling to 0°C, the precipitating product was filtered off and dried *in vacuo*.

### N-(2-methyl-5-nitrophenyl)-5-(pyridin-3-yl)thiazol-2-amine hydrobromide (31b):

Yield: 16.5 mmol (93 %), yellow solid; m.p. 253 °C; IR (KBr): v (cm⁻¹)= 3453, 3252, 2019, 1509. ¹H-NMR (DMSO-[D₆]): δ (ppm) = 2.45 (s, 3H), 7.49 (d, 1H, J = 8.4 Hz), 7.82 (dd, 1H, J = 2.4 Hz, J = 8.3 Hz), 8.04 (s, 1H), 8.13 (dd, 1H, J = 5.6 Hz, J = 8.2 Hz), 8.87 (dd, 1H, J = 1.0 Hz, J = 5.7 Hz), 8.94 (m, 1H), 9.30 (d, 1H, J = 1.8 Hz), 9.47 (d, 1H, J = 2.4 Hz), 10.00 (s, 1H). Cl-MS (NH₃) *m*/*z* (%): 299 [M+H⁺]⁺ (100). Anal. (C₁₅H₁₂N₄O₂S x HBr). Calc. C 45.81, H 3.33, N 14.25; Found C 45.61, H 3.53, N 14.31.

### N-(3-nitrophenyl)-5-(pyridin-3-yl)thiazol-2-amine hydrobromide (31a):

(commercially available at e.g.: Interchim Intermediates, France): Yield: 23.9 mmol (99 %), yellow solid; m.p. 279 °C; IR (KBr): v (cm⁻¹)= 2024, 1559, 1520. ¹H-NMR (DMSO-[D₆]): δ (ppm) = 7.65 (t, 1H, J = 8.2 Hz), 7.93 (m, 4H), 8.79 (m, 2H), 8.92 (t, 1H, J = 2.2 Hz), 9.31 (d, 1H, J = 1.9 Hz), 11.03 (s, 1H), + p ESI m/z (%): 299 [M+H⁺]⁺ (100), 269 [M-NO]⁺ (65); - p ESI m/z (%): 511 [M-H⁺]⁻ (100), 512 [M-H⁺]⁻ (34), 513 [M-H⁺]⁻ (14). Anal. (C₁₄H₁₀N₄O₂S x HBr). Calc. C 44.34, H 2.92, N 14.77; Found C 44.04, H 2.54, N 14.81.

### N¹-(4-(pyridin-3-yl)pyrimidin-2-yl)benzene-1,3-diamine

(Zimmermann et al.; Potent and selective inhibitors of the ABL-kinase: phenylaminopyrimidine (PAP) derivatives. Bioorg. & Med. Chem. Lett. 1997, 7, 187-192).

### 6-Methyl-N¹-(4-(pyridin-3-yl)pyrimidin-2-yl)benzene-1,3-diamine:

(Zimmermann et al.; Potent and selective inhibitors of the ABL-kinase: phenylaminopyrimidine (PAP) derivatives. Bioorg. & Med. Chem. Lett. 1997, 7, 187-192).

### N¹-(5-(pyridin-3-yl)thiazol-2-yl)benzene-1,3-diamine (33a) and 6-methyl-N¹-(5-(pyridin-3-yl)thiazol-2-yl)benzene-1,3-diamine (33b) by catalytic reduction of the respective nitro-precursors:

A mixture of the respective nitro compound (**31b** or **31a**), Pd/C (10%) and 3 eq.
Na₂CO₃ in THF were stirred at 50 °C and 30 bar H₂-pressure for 12h. After cooling to room temperature, the catalyst, excess of Na₂CO₃ and KBr were filtered off over cellite, THF removed under reduced pressure and the crude product purified by cc (ethyl acetate/MeOH = 10/1).. In case of 33b the dihydrochloride salt was obtained from the THF solution by addition of HCl saturated diethylether.

### N¹-(5-(pyridin-3-yl)thiazol-2-yl)benzene-1,3-diamine (33a)

(commercially available at: Interchim Intermediates, France): Yield: 38.8 mmol (96 %), brown solid; m.p. 161.8 °C; IR (KBr): v (cm⁻¹)= 3352, 2360, 1468. ¹H-NMR (DMSO-[D₆]): δ (ppm) = 5.13 (s, 2H), 6.22 (m, 1 H), 6.78 (m, 1 H), 6.97 (m, 2H), 7.45 (m, 2H), 8.27 (m, 1 H), 8.51 (dd, 1H, J = 1.6 Hz, J = 4.7 Hz), 9.15 (d, 1H, J = 1.6 Hz), 10.03 (s, 1H). + p ESI m/z (%): 269 [M+H⁺]⁺ (100). Anal.(C₁₄H₁₀N₄O₂). Calc. C. 62.66, H. 4.51, N. 20.88; Found C 62.56, H 4.60, N 20.30.

### 6-Methyl-N¹-(5-(pyridin-3-yl)thiazol-2-yl)benzene-1,3-diamine dihydrochloride monohydrate (33b):

Remark: The free amine has been described by *Moussy et*. *al*, Use of c-kit inhibitors for treating type II diabetes WO 2005/016323 A2): Yield: 14.5 mmol (99 %), brown solid; m.p. 312-315 °C; IR (KBr): v (cm⁻¹)= 3428, 3240, 2845, 2619, 1530. ¹H-NMR (DMSO-[D₆]): δ (ppm) = 4.02 (s, 2H, br.), 6.97 (dd, 1H, J = 2.1 Hz, J = 8.0 Hz), 7.31 (d, 1 H, J = 8.2 Hz), 7.95 (s, 1 H), 8.01 (dd, 1 H, J = 5.5 Hz, J = 8.2 Hz), 8.60 (d, 1 H, J = 2.1 Hz), 8.76 (dd, 1 H, J = 0.9 Hz, J = 5.5 Hz), 9.12 (td, 1 H, J = 1.6 Hz, J = 3.6 Hz), 9.59 (d, 1H, J = 1.8 Hz), 9.81 (s, 1H). Cl-MS (NH₃) *m*/*z* (%): 298 [M+H⁺]⁺ (100). Anal. (C₁₅H₁₄N₄S x 2 HCl x H₂O). Calc. C 48.26, H 4.86, N 15.01; Found C 48.26, H 4.86, N 15.01.

### (E)-3-(4-(methoxycarbonyl)phenyl)acrylic acid (74) and (E)-3-(5-(methoxycarbonyl)thiophen-2-yl)acrylic acid (75):

A mixture of the respective aldehyde (**73** or **74,** 15.0 mmol), malonic acid (30.0 mmol) and pyridine/piperidine (2:1; 3.75 mL) was heated to 80°C for 3h. The hot mixture was poured into water (20 mL) by stirring and acidified with diluted HCl. The mixture was cooled in an ice bath, the precipitating crystals removed by filtration, washed with diluted HCl and dried in vacuum

### (E)-3-(4-(methoxycarbonyl)phenyl)acrylic acid (74):

(Nagao et al.; New aldose reductase inhibitors. Part I. Syntheses of (rac)3-substituted 4-methoxycarbonyl-1,3-thiazolidine-2-thiones via rearrangement of a substituted group from exo-S to N in (rac)2-substituted thio-4-methoxycarbonyl-D 2-1,3-thiazolines. Chem. & Pharm. Bull. 1988, 36, 495-508): Yield (78 %), white crystals; mp. 242.3 °C. ¹H-NMR (DMSO-[D₆]): δ (ppm) = 3.87 (s, 3H), 6.66 (d, 1H, J = 16.0 Hz), 7.64 (d, 1H, J = 16.1 Hz), 7.83 (d, 2H, J = 8.3 Hz), 7.97 (d, 2H, J = 8.3 Hz), 12.57 (s, 1H).

### (E)-3-(5-(methoxycarbonyl)thiophen-2-yl)acrylic acid (75):

Yield 58 %, brown solid; mp. 180.3°C. IR (KBr): v (cm⁻¹)= 2961, 1723. ¹H-NMR (DMSO-[D₆]): δ (ppm) = 3.83 (s, 3H), 6.39 (d, 1H, J = 15.8 Hz), 7.48 (d, 1H, J = 3.9 Hz), 7.57 (d, 1H, J = 15.8 Hz), 7.75 (d, 1H, J = 3.9 Hz). EI-MS (70eV) *m*/*z* (%): 212 [M^{+·}] (92), 181 [M-OCH₃⁻]⁺ (100). Anal.(C₉H₈O₄S) calcd. C 50.94, H 3.80; found C 50.86, H 3.77.

### Preparation of carboxylic acid methylesters (65, 66, 72, 76 and 77) by amidation with 64 or NH₂OTHP (O-(tetrahydro-2H-pyran-2-yl)hydroxylamine):

The respective methoxycarbonyl aroylic acid (10.0 mmol) (**62, 63, 74, 75 or 57a** and **57b**) was dissolved in dry THF (50.0 mL) or DMF (20.0 mL) and 1.1 eq. BOP (benzotriazolyloxy-tris-(dimethylamino)phosphoniumhexafluorophosphate), 2.2 eq. NEt₃, and 1.1 eq. of the respective amine (**64** or NH₂OTHP) were added. After stirring at room temperature for 24h, the mixture was poured into water by stirring, the precipitating product filtered off, dried in vacuum and purified by cc (CH₂Cl₂/MeOH = 10/1).

### 6-methyl- methyl 4-(2-(tert-butoxycarbonylamino)phenylcarbamoyl)-benzoate (65):

(Delorme et al. Preparation of triazinyl and other carboxamides as inhibitors of histone deacetylase. US 2005/288282 A1, 2005): Yield: 31.05 mmol (94 %), colorless crystals; m.p. 145.5-146.5 °C; IR ¹H-NMR (DMSO-[D₆]): δ (ppm) = 1.44 (s, 9H), 3.91 (s, 3H), 7.22 (dt, 1H, J = 1.8 Hz, J = 7.7 Hz), 7.15 (dt, 1H, J = 1.7 Hz, J = 7.6 Hz), 7.55 (m, 2H), 8.10 (m, 4H), 8.71 (s, 1H), 9.98 (s, 1H).

### Methyl 6-(2-(tert-butoxycarbonylamino)phenylcarbamoyl)nicotinate (66):

Yield: 9.23 mmol (84 %), colorless crystals m.p. 171.5-173.0 °C; IR (KBr): v (cm⁻¹)= 3342, 1732, 1690. ¹H-NMR (DMSO-[D₆]): δ (ppm) = 1.51 (s, 9H), 3.94 (s, 3H), 7.20 (m, 1 H), 7.28 (m, 2H), 7.97 (dd, 1 H, J = 1.3 Hz, J = 7.8 Hz), 8.31 (m, 1 H), 8.56 (dd, 1H, J = 2.1 Hz, J = 8.1 Hz), 9.08 (d, 1 H, J = 1.5 Hz), 9.19 (s, 1 H), 10.55 (s, 1 H).+ p ESI m/z (%): 372 [M+H⁺]⁺ (100). Anal. (C₁₉H₂₁N₃O₅). Calc. C 61.45, H 5.70, N 11.31; Found C 61.45, H 5.90, N 11.32.

### Methyl 5-(2-(tert-butoxycarbonylamino)phenylcarbamoyl)-thiophene-2-carboxylate (72):

(Moussy and Kinet; Use of c-kit inhibitors for treating type II diabetes. PCT Int. Appl. WO 2005/016323 A2).

### (E)-methyl 4-(3-oxo-3-(tetrahydro-2H-pyran-2-yloxyamino)prop-1-enyl)benzoate (76):

Yield: 9.8 mmol (34%), white solid; m.p. 133.0 °C; IR (KBr): v (cm⁻¹)= 2956, 1716, 1664. ¹H-NMR (DMSO-[D₆]): δ (ppm) = 1.54 (s, 3H), 1.70 (s, 3H), 3.54 (dd, 1H, J = 4.6 Hz, J = 6.9 Hz), 3.86 (s, 3H), 4.93 (s, 1 H), 6.63 (d, 1H, J = 15.9 Hz), 7.55 (d, 1H, J = 15.8 Hz), 7.72 (d, 2H, J = 8.2 Hz), 7.98 (d, 2H, J = 8.3 Hz), 11.34 (s, 1H). CI-MS (NH₃) *m*/*z* (%): 323 [M+NH₄⁺]⁺ (11), 306 [M+H⁺]⁺ (12) 223 (100). Anal.( C₁₆H₁₉NO₅) Calc. C 62.94, H 6.27, N 4.59; Found C 62.63, H 6.23, N 4.48.

### (E)-methyl 5-(3-oxo-3-(tetrahydro-2H-pyran-2-yloxyamino)prop-1-enyl)thiophene-2-carboxylate (77):

Yield: 6.8 mmol (71%), brown solid; m.p. 177.4 °C; IR (KBr): v (cm⁻¹)= 3141, 2953, 1702. ¹H-NMR (DMSO-[D₆]): δ (ppm) = 1.54 (s, 3H), 1.69 (s, 3H), 3.54 (d, 1H, J = 11.6 Hz), 3.84 (s, 3H), 3.94 (m, 1 H), 4.91 (s, 1 H), 6.43 (d, 1 H, J = 15.7 Hz), 7.48 (d, 1 H, J = 3.8 Hz), 7.66 (d, 1 H, J = 15.6 Hz), 7.76 (d, 1 H, J = 3.9 Hz), 11.32 (s, 1 H). ElMS (70eV) *m*/*z* (%): 212 [M-TetrahydropyraN-2-on⁻]⁺ (100), 312 [M^{+·}] (48). Anal.(C₁₄H₁₇NO₅S) Calc. C 54.01, H 5.50, N 4.50; Found C 54.08, H 5.71, N 4.40.

### Preparation of suitable protected and substituted carboxylic acids (34, 35, 36,

**49, 50 and 55)** by alkaline cleavage of the corresponding carboxylic acid methylesters: The respective carboxylic acid methyl esters (5.0 mmol) were dissolved in MeOH (50 mL) and 2 eq. LiOH in H₂O (50 mL) were added. After stirring at room temperature over night, MeOH was removed, the aqueous layer extracted with ethyl acetate (3 x 20 mL), cooled to 0°C and acidified with diluted acetic acid till pH = 5-6. The precipitating product was removed by filtration and dried in vacuum.

### 4-(2-(tert-butoxycarbonylamino)phenylcarbamoyl)benzoic acid (34):

(Schuppan et al.; Preparation of N-aryl benzamides as histone deacetylase inhibitors. PCT Int. Appl. WO 2004058234 A2, 2004): Yield: 18.9 mmol (68 %), colorless crystals; m.p. 189.5-191.5 °C; IR (KBr): ¹H-NMR (DMSO-[D₆]): δ (ppm) = 1.44 (s, 9H), 7.15 (dt, 1 H, J = 1.6 Hz, J = 7.6 Hz), 7.22 (dt, 1 H, J = 1.8 Hz, J = 7.7 Hz), 7.55 (dt, 2H, J = 1.5 Hz, J = 7.8 Hz), 8.07 (m, 4H), 8.73 (s, 1 H), 9.97 (s, 1 H), 13.33 (s, 1H).

### 6-(2-(tert-butoxycarbonylamino)phenylcarbamoyl)nicotinic acid (35):

Yield: 8.62 mmol (88 %), colorless crystals; m.p. 358.0-361.0 °C; IR (KBr): v (cm⁻¹)= 3345, 2986, 1689. ¹H-NMR (DMSO-[D₆]): δ (ppm) = 1.51 (s, 9H), 7.21 (dd, 1H, J = 6.6 Hz, J = 8.2 Hz), 7.28 (m, 2H), 7.98 (m, 1 H), 8.29 (d, 1 H, J = 8.2 Hz), 8.53 (dd, 1 H, J = 2.0 Hz, J = 8.1 Hz), 9.07 (d, 1H, J = 1.4 Hz), 9.18 (s, 1H), 10.55 (s, 1H), 13.74 (s, 1H). + p ESI m/z (%): 358 [M+H⁺]⁺ (100). Anal.(C₁₈H₁₉N₃O₅ x 0.5 H₂O) Calc. C 59.01; H 5.50; N 11.47; Found C 59.16, H 5.69, N 11.48.

### 5-(2-(tert-butoxycarbonylamino)phenylcarbamoyl)thiophene-2-carboxylic acid (36):

(Fertig et al.; Preparation of new mono-acylated o-phenylendiamines derivatives as HDAC inhibitors for treating cancer. PCT Int. Appl. WO 2004069803).

### (E)-4-(3-oxo-3-(tetrahydro-2H-pyran-2-yloxyamino)prop-1-enyl)benzoic acid (49):

Yield: 7.9 mmol (80.6%), white solid; m.p. 182.4 °C; IR (KBr): v (cm⁻¹)= 3222, 2946, 1686. ¹H-NMR (DMSO-[D₆]): 6 (ppm) = 1.54 (s, 3H), 1.70 (s, 3H), 3.54 (dd, 1H, J = 4.6 Hz, J = 6.9 Hz), 3.98 (m, 1 H), 4.93 (s, 1 H), 6.62 (d, 1H, J = 15.8 Hz), 7.54 (d, 1H, J = 15.7 Hz), 7.69 (d, 2H, J = 8.1 Hz), 7.96 (d, 2H, J = 8.3 Hz), 11.34 (s, 1 H). + p ESI m/z (%): 292 [M+H⁺]⁺ (100), 309 [M+NH₄⁺]⁺ (26), 583 [2M+H⁺]⁺ (18); - p ESI m/z (%): 290 [M-H⁺]⁻ (100), 581 [2M-H⁺]⁻ (36), 350 [M+CH₃COO⁻]⁻ (91). Anal.(C₁₅H₁₇NO₅ x 3/2 H₂O) Calc. C 56.60, H 6.33, N 4.40; Found C 56.24, H 6.53, N 4.38.

### (E)-5-(3-oxo-3-(tetrahydro-2H-pyran-2-yloxyamino)prop-1-enyl)thiophene-2-carboxylic acid (50):

Yield: 1.1 mmol (22.5%), yellow crystals. m.p. 176.6 °C; IR (KBr): v (cm⁻¹)= 3242, 2942, 1677. ¹H-NMR (DMSO-[D₆]): δ (ppm) = 1.54 (s, 3H), 1.69 (s, 3H), 3.54 (d, 1H, J = 11.8 Hz), 3.94 (m, 1 H), 4.91 (s, 1 H), 6.40 (d, 1 H, J = 15.6 Hz), 7.45 (d, 1 H, J = 3.8 Hz), 7.65 (m, 2H), 11.30 (s, 1H), 13.35 (s, 1H, br.). EI-MS (70eV) *m*/*z* (%): 214 [M-3,4 Dihydro-2*H*-pyran⁻]⁺ (100), 298 [M⁺⁻] (24). Anal.(C₁₃H₁₅NO₅S x ¼ H₂O) Calc. C 51.73, H 5.18, N 4.64; Found C 51.92, H 5.21, N 4.70.

### Preparation of carbamin acid-tert-butyl-esters 38, 39, 40a, 41 a, 42a, 40b, 41 b and 42b by amidation of the respective carboxylic acids:

The respective carboxylic acid was dissolved in 10 mL of dry pyridine and 1.1 eq. SOCl₂ were added. The mixture was stirred at room temperature for half an hour, and 1.0 eq. of the respective arylamine was added. After stirring at room temperature for 24h, the mixture was poured into water by stirring, the precipitating product removed by filtration, dried in vacuo and purified by cc (DCM/MeOH = 10/1).

### tert-Butyl-2-(5-(4-methyl-3-(4-(pyridiN-3-yl)-pyrimidin-2-ylamino)phenylcarbamoyl)picolin-amido)phenylcarbamate (38):

Yield: 0.92 mmol (51 %), light yellow crystals; m.p. 185.5-187.0 °C; IR (KBr): v (cm⁻¹)= 3436, 3317, 1692, 1676. ¹H-NMR (DMSO-[D₆]): δ (ppm) = 1.51 (s, 9H), 2.26 (s, 3H), 7.19 (m, 1 H), 7.28 (m, 3H), 7.46 (d, 1H, J = 5.2 Hz), 7.53 (m, 2H), 8.02 (d, 1H, J = 8.1 Hz), 8.13 (d, 1 H, J = 1.9 Hz), 8.32 (d, 1 H, J = 8.2 Hz), 8.50 (m, 1 H), 8.54 (d, 1 H, J = 5.2 Hz), 8.58 (dd, 1 H, J = 2.1 Hz, J = 8.2 Hz), 8.70 (dd, 1 H, J = 1.5 Hz, J = 4.7 Hz), 9.03 (s, 1 H), 9.12 (d, 1 H, J = 1.6 Hz), 9.18 (s, 1 H), 9.30 (d, 1 H, J = 1.8 Hz), 10.57 (s, 1 H), 10.62 (s, 1H). + p ESI m/z (%): 617 [M+H⁺]⁺ (100). Anal.(C₃₄H₃₂N₈O₄ x 0.5 H₂O) Calc. C 65.27; H 5.32; N 17.91; Found C 65.42, H 5.47, N 17.97.

### tert-butyl 2-(5-(4-methyl-3-(4-(pyridin-3-yl)pyrimidin-2-ylamino)phenylcarbamoyl)-thiophene-2-carboxamido)phenylcarbamate (39):

Yield: 0.84 mmol (61 %), colorless crystals; m.p. 206.0-208.0 °C; IR (KBr): v (cm⁻¹)= 3278, 1648, 1537. ¹H-NMR (DMSO-[D₆]): δ (ppm) = 1.46 (s, 9H), 2.24 (s, 3H), 7.15 (dt, 1 H, J = 1.6 Hz, J = 7.6 Hz), 7.23 (m, 2H), 7.46 (m, 1 H), 7.50 (dd, 2H, J = 1.2 Hz, J = 7.8 Hz), 7.55 (m, 2H), 7.94 (d, 1 H, J = 4.0 Hz), 8.07 (m, 2H), 8.50 (m, 1 H), 8.53 (d, 1 H, J = 5.2 Hz), 8.70 (dd, 1 H, J = 1.6 Hz, J = 4.8 Hz), 8.77 (s, 1 H), 9.01 (s, 1 H), 9.29 (d, 1H, J = 1.7 Hz), 9.97 (s, 1 H), 10.38 (s, 1H). ES-MS (CH₂Cl₂/CH₃OH/CH₃COONH₄) m/z (%): 622 [M+H⁺]⁺. Anal.(C₃₃H₃₁N₇O₄S x 0.75 H₂O) Calc. C 62.40; H 5.16; N 15.44; Found C 62.20, H 5.15, N15.46.

### tert-butyl 2-(4-(3-(4-(pyridin-3-yl)thiazol-2-ylamino)-phenylcarbamoyl)benzamido)phenylcarbamate hydrate (40a):

Yield: 0.8 mmol (51 %), orange solid; m.p. 229.3 °C; IR (KBr): v (cm⁻¹)= 3348, 2982, 1664, 1608, 1540. ¹H-NMR (DMSO-[D₆]): δ (ppm) = 1.46 (s, 9H), 7.20 (m, 2H), 7.35 (m, 2H), 7.49 (m, 2H), 7.55 (s, 1 H), 7.57 (s, 1 H), 7.59 (d, 1H, J = 1.5 Hz), 8.13 (m, 4H), 8.38 (m, 1 H), 8.49 (s, 1 H), 8.52 (dd, 1 H, J = 1.6 Hz, J = 4.8 Hz), 8.74 (s, 1 H,), 9.23 (d, 1 H, J = 1.7 Hz), 9.98 (s, 1 H), 10.45 (d, 1 H, J = 7.6 Hz). + p ESI m/z (%): 607 [M+H⁺]⁺ (100), 608 [M+H⁺]⁺ (39), 609 [M+H⁺]⁺ (13). Anal.(C₃₃H₃₀N₆O₄S x 3/2 H₂O). Calc. C 62.54, H 5.25, N 13.26; Found C 62.48, H 5.15, N 13.50.

### tert-butyl 2-(5-(3-(4-(pyridin-3-yl)thiazol-2-ylamino)phenylcarbamoyl)picolinamido)phenylcarbamate (41a):

Yield: 0.54 mmol (79 %), beige solid; m.p. 220.0-222.0 °C; IR (KBr): v (cm⁻¹)= 3348, 1691, 1669. ¹H-NMR (DMSO-[D₆]): δ (ppm) = 1.52 (s, 9H), 7.21 (m, 2H), 7.28 (m, 1 H), 7.32 (m, 2H), 7.35 (d, 1 H, J = 7.8 Hz), 7.45 (m, 2H), 7.57 (s, 1 H), 8.02 (m, 1 H), 8.34 (d, 1 H, J = 8.2 Hz), 8.39 (m, 1H), 8.52 (m, 1 H), 8.60 (dd, 1 H, J = 2.1 Hz, J = 8.1 Hz), 9.14 (d, 1H, J = 1.6 Hz), 9.18 (s, 1 H), 9.24 (s, 1 H), 10.46 (s, 1 H), 10.59 (s, 1 H), 10.69 (s, 1H). + p ESI m/z (%): 608 [M+H⁺]⁺ (100). Anal.(C₃₂H₂₉N₇O₄S x 0.5 H₂O) Calc. C 62.32; H 4.90; N 15.90; Found C 62.49, H 4.89, N 15.80.

### tert-butyl 2-(5-(3-(4-(pyridin-3-yl)thiazol-2-ylamino)phenyl-carbamoyl)thiophene-2-carboxamido)phenyl-carbamate semihydrate (42a):

Yield: 0.4 mmol (22%). m.p. 197.7 °C; brown substance; IR (KBr): v (cm⁻¹)= 3271, 2976, 1634, 1533 ¹H-NMR (DMSO-[D₆]): δ (ppm) = 1.46 (s, 9H), 7.18 (m, 1H), 7.25 (m, 2H), 7.36 (d, 1H, J = 6.8 Hz), 7.45 (m, 1 H), 7.50 (m, 2H), 7.57 (m, 1 H), 7.96 (d, 1H, J = 4.1 Hz), 8.10 (d, 1 H, J = 4.1 Hz), 8.38 (m, 1 H), 8.44 (m, 1 H), 8.52 (dd, 1 H, J = 1.6 Hz, J = 4.7 Hz), 8.76 (s, 1 H), 9.22 (d, 1 H, J = 1.8 Hz), 9,99 (s, 1 H), 10.45 (d, 2H, J = 4.2 Hz). + p ESI m/z (%):613 [M+H⁺]⁺ (100), 614 [M+H⁺]⁺ (36), 615 [M+H⁺]⁺ (17); - p ESI m/z (%): 611 [M-H⁺]⁻ (100), 612 [M-H⁺]⁻ (38), 613 [M-H⁺]⁻ (18). Anal.(C₃₁H₂₈N₆O₄S₂ x 1/2 H₂O) Calc. C 59.89, H 4.70, N 13.52; Found C 59.55, H 4.64, N 13.67.

### tert-butyl 2-(4-(4-methyl-3-(4-(pyridin-3-yl)thiazol-2-ylamino)phenylcarbamoyl)benzamido)phenylcarbamate semihydrate (40b):

Yield: 0.6 mmol (56%), orange solid; m.p. 239.8 °C; IR (KBr): v (cm⁻¹)= 3309, 1538. ¹H-NMR (DMSO-[D₆]): δ (ppm) = 1.46 (s, 9H), 3.34 (s, 3H), 7.21 (m, 2H), 7.35 (m, 2H), 7.50 (m, 2H), 7.54 (s, 1 H), 7.57 (s, 1 H), 7.60 (d, 1H, J = 1.5 Hz), 8.13 (m, 4H), 8.36 (m, 1 H), 8.49 (s, 1 H), 8.52 (dd, 1 H, J = 1.6 Hz, J = 4.9 Hz), 8.74 (s, 1 H), 9.23 (d, 1 H, J = 1.7 Hz), 9.99 (s, 1 H), 10.45 (d, 1 H, J = 7.6 Hz). + p ESI m/z (%): 621 [M+H⁺]⁺ (100), 521 [M+H⁺]⁺ (98), 622 [M+H⁺]⁺ (41). Anal.(C₃₄H₃₂N₆O₄S x 1/2 H₂O). Calc. C 64.85, H 5.28, N 13.35; Found C 64.78, H 5.26, N 13.27.

### tert-butyl 2-(5-(4-methyl-3-(4-(pyridin-3-yl)-thiazol-2-ylamino)phenylcarbamoyl)picolin-amido)phenylcarbamate (41b):

Yield: 0.58 mmol (71 %), colorless solid; m.p. 222.0-223.5 °C; IR (KBr): v (cm⁻¹)= 3241, 1692, 1655. ¹H-NMR (DMSO-[D₆]): δ (ppm) = 1.52 (s, 9H), 2.30 (s, 3H), 7.24 (m, 4H), 7.38 (m, 1 H), 7.43 (dd, 1 H, J = 6.2 Hz, J = 9.3 Hz), 7.51 (s, 1 H), 8.02 (d, 1 H, J = 8.1 Hz), 8.34 (m, 2H), 8.49 (dd, 1 H, J = 1.3 Hz, J = 4.8 Hz), 8.60 (dd, 1 H, J = 2.1 Hz, J = 8.2 Hz), 8.75 (d, 1H, J = 1.8 Hz), 9.17 (dd, 3H, J = 1.4 Hz, J = 17.6 Hz), 9.49 (s, 1 H), 10.58 (s, 1 H), 10.64 (s, 1H). + p ESI m/z (%): 622 [M+H⁺]⁺ (100). Anal.(C₃₃H₃₁N₇O₄S x 0.25 H₂O) Calc. C 63.29; H 5.07; N 15.66; Found C 63.25, H 5.22, N 15.50.

### tert-butyl 2-(5-(4-methyl-3-(4-(pyridin-3-yl)thiazol-2-ylamino)-phenylcarbamoyl)thiophene-2-carboxamido)phenylcarbamate semihydrate (42b):

Yield: 1.1 mmol (59 %), brown substance; m.p. 217.9 °C; IR (KBr): v (cm⁻¹)= 3292, 3064, 1697, 1644, 1537. ¹H-NMR (DMSO-[D₆]): δ (ppm) = 1.46 (s, 9H), 3.33 (s, 3H), 7.19 (m, 3H), 7.34 (m, 1 H), 7.42 (m, 1 H), 7.49 (m, 2H), 7.57 (dd, 1H, J = 1.4 Hz, J = 8.0 Hz), 7.94 (d, 1 H, J = 4.0 Hz), 8.07 (d, 1 H, J = 4.1 Hz), 8.34 (m, 1 H), 8.49 (dd, 1 H, J = 1.6 Hz, J = 4.7 Hz), 8.66 (d, 1H, J = 2.0 Hz), 8.76 (s, 1 H), 9.16 (d, 1H, J = 1.6 Hz), 9,47 (s, 1 H), 9.97 (s, 1 H), 10.40 (s, 1H). + p ESI m/z (%):627 [M+H⁺]⁺ (100), 628 [M+H⁺]⁺ (39), 629 [M+H⁺]⁺ (16); - p ESI m/z (%): 625 [M-H⁺]⁻ (100), 626 [M-H⁺]⁻ (40), 627 [M-H⁺]⁻ (17). Anal.(C₃₂H₃₀N₆O₄S₂ x 1/2 H₂O) Calc. C 60.45, H 4.91, N 13.22; Found C 60.82, H 4.99, N 13.06.

### Preparation of 44 [23], 45 [26], 46a [18], 47a [24], 48a [28], 46b [21], 47b [25] and 48b [27] by cleavage of the tert-butyl phenylcarbamate group:

The respective carbamin acid-*tert*-butyl-esters (**38, 39, 40a, 41a, 42a, 40b, 41b** and **42b**) were dissolved in TFA (5.0 mL) and stirred at room temperature for 2h. The solution was poured into water (100mL) by stirring and the mixture alkalized with NH₃ (pH = 9). The precipitating product was filtered off, washed with H₂O and dried *in vacuo*.

### N²-(2-aminophenyl)-N⁵-(4-methyl-3-(4-(pyridin-3-yl)pyrimidin-2-ylamino)phenyl)pyridine-2,5-dicarboxamide (44) [23]:

Yield: 0.43 mmol (98 %), yellow crystals; m.p. 150.5-152.5 °C; IR (KBr): v (cm⁻¹)= 3324, 1672. ¹H-NMR (DMSO-[D₆]): 6 (ppm) = 2.26 (s, 3H), 4.75 (s, 2H), 6.72 (dt, 1H, J = 1.3 Hz, J = 7.8 Hz), 6.89 (dd, 1 H, J = 1.2 Hz, J = 7.9 Hz), 7.01 (dt, 1 H, J = 1.4 Hz, J = 7.9 Hz), 7.26 (d, 1 H, J = 8.5 Hz), 7.46 (d, 1 H, J = 5.2 Hz), 7.54 (m, 3H), 8.15 (d, 1 H, J = 1.8 Hz), 8.28 (d, 1 H, J = 8.1 Hz), 8.54 (d, 2H, J = 5.1 Hz), 8.58 (m, 1 H), 8.71 (s, 1 H), 9.04 (s, 1 H), 9.22 (d, 1 H, J = 1.6 Hz), 9.31 (s, 1 H), 10.24 (s, 1 H), 10.61 (s, 1H). + p ESI m/z (%): 517 [M+H⁺]⁺ (100). Anal.(C₂₉H₂₄N₈O₂ x 1.33 H₂O) Calc. C 64.44; H 4.97; N 20.73; Found C 64.24, H 4.79, N 20.55.

### N²-(2-aminophenyl)-N⁵-(4-methyl-3-(4-(pyridin-3-yl)pyrimidin-2-ylamino)phenyl)thiophene-2,5-dicarboxamide (45) [26]:

Yield: 0.31 mmol (98 %), yellow crystals; m.p. 280.5 °C; IR (KBr): v (cm⁻¹)= 3301, 1636, 1581. ¹H-NMR (DMSO-[D₆]): δ (ppm) = 2.24 (s, 3H), 6.63 (dt, 1H, J = 1.0 Hz, J = 7.7 Hz), 6.81 (dd, 1 H, J = 1.0 Hz, J = 8.0 Hz), 7.01 (dt, 1 H, J = 1.3 Hz, J = 8.0 Hz), 7.15 (dd, 1H, J = 1.0 Hz, J = 7.7 Hz), 7.24 (d, 1H, J = 8.4 Hz), 7.46 (m, 2H), 7.54 (dd, 1 H, J = 4.8 Hz, J = 7.9 Hz), 8.02 (m, 2H), 8.08 (d, 1 H, J = 1.8 Hz), 8.52 (m, 2H), 8.70 (dd, 1 H, J = 1.4 Hz, J = 4.7 Hz), 9.01 (s, 1 H), 9.29 (d, 1 H, J = 1.7 Hz), 9.88 (s, 1 H), 10.35 (s, 1H). ES-MS (CH₂Cl₂/CH₃OH/CH₃COONH₄) m/z (%): 522 [M+H⁺]⁺.
Anal.(C₂₈H₂₃N₇O₂S) Calc. C 64.48; H 4.44; N 18.80; Found C 64.38, H 4.22, N 18.57.

### N¹-(2-aminophenyl)-N⁴-(3-(4-(pyridin-3-yl)thiazol-2-ylamino)phenyl)terephthalamide hydrate (46a) [18]:

Yield: 0.3 mmol (33%), brown solid m.p. 224.8-224.9 °C; IR (KBr): v (cm⁻¹)= 3354, 3286, 1536. ¹H-NMR (DMSO-[D₆]): δ (ppm) = 4.96 (s, 2H), 6.57 (m, 1 H), 6.94 (m, 3H), 7.19 (t, 1H, J = 8.5 Hz), 7.33 (m, 2H), 7.48 (m, 2H), 7.55 (s, 1 H), 8.13 (m, 3H), 8.37 (m, 1 H), 8.50 (m, 2H), 9.23 (d, 1H, J = 1.7 Hz), 9.81 (s, 1 H), 10.42 (d, 2H, J = 3.1 Hz). + p ESI m/z (%): 507 [M+H⁺]⁺ (100), 508 [M+H⁺]⁺ (34), 509 [M+H⁺]⁺ (10). Anal. (C₂₅H₂₂N₆O₂S x 3/2 H₂O) Calc. C 63.02, H 4.72, N 15.75; Found C 62.65, H 4.51, N 15.36.

### N²-(2-aminophenyl)-N⁵-(3-(4-(pyridin-3-yl)thiazol-2-ylamino)phenyl)pyridine-2,5-dicarboxamide (47a) [24]:

Yield: 0.39 mmol (80 %), yellow crystals; m.p. 148.0-150.0 °C; IR (KBr): v (cm⁻¹)= 3300, 1663, 1617. ¹H-NMR (DMSO-[D₆]): δ (ppm) = 5.20 (s, 2H), 6.69 (dt, 1H, J = 1.3 Hz, J = 7.8 Hz), 6.86 (dd, 1 H, J = 1.3 Hz, J = 8.0 Hz), 6.99 (dt, 1 H, J = 1.4 Hz, J = 8.0 Hz), 7.34 (m, 2H), 7.50 (m, 3H), 7.57 (s, 1 H), 8.30 (d, 1H, J = 8.2 Hz), 8.40 (m, 1 H), 8.53 (m, 2H), 8.58 (dd, 1 H, J = 2.2 Hz, J = 8.2 Hz), 9.24 (d, 2H, J = 1.7 Hz), 10.20 (s, 1 H), 10.47 (s, 1 H), 10.68 (s, 1H). EI-MS (70eV) *m*/*z* (%): 507 [M⁺⁻] (10), 489 (100), 222 (55). Anal.(C₂₇H₂₁N₇O₂S x 1.5 H₂O) Calc. C 60.66; H 4.53; N 18,34; Found C 60.85, H 4.32a, N 18.47.

### N²-(2-aminophenyl)-N⁵-(3-(4-(pyridin-3-yl)thiazol-2-ylamino)phenyl)thiophene-2,5-dicarboxamide (48a) [28]:

Yield: 0.2 mmol (66%), brown solid; m.p. 222.3 °C; IR (KBr): v (cm⁻¹)= 3345, 1721, 1647, 1573. ¹H-NMR (DMSO-[D₆]): δ (ppm) = 5.16 (s, 2H, br.), 6.63 (dt, 1H, J = 1.3 Hz, J = 7.7 Hz), 6.81 (dd, 1H, J = 1.2 Hz, J = 8.0 Hz), 7.01 (m, 1 H), 7.16 (dd, 1 H, J = 1.2 Hz, J = 7.8 Hz), 7.26 (m, 1 H), 7.34 (m, 1 H), 7.49 (m, 2H), 7.58 (s, 1 H), 8.04 (m, 2H), 8.42 (m, 2H), 8.54 (dd, 1 H, J = 1.5 Hz, J = 4.8), 9.23 (d, 1 H, J = 1.8 Hz), 9.88 (s, 1 H), 10.43 (d, 2H, J = 6.7 Hz). + p ESI m/z (%): 513 [M+H⁺]⁺ (100), 514 [M+H⁺]⁺ (33), 515 [M+H⁺]⁺ (13); - p ESI m/z (%): 511 [M-H⁺]⁻ (100), 512 [M-H⁺]⁻ (34), 513 [M-H⁺]⁻(14). Anal.(C₂₆H₂₀N₆O₂S₂ x TFA x EE) Calc. C 53.77, H 4.09, N 11.76; Found C 53.55, H 3.91, N 10.83.

### N¹-(2-aminophenyl)-N⁴-(4-methyl-3-(4-(pyridin-3-yl)thiazol-2-ylamino)phenyl)terephthalamide(46b) [21]:

Yield: 0.2 mmol (33%), orange solid; m.p. 147.1 °C; IR (KBr): v (cm-¹)= 3397, 3287, 1537. ¹H-NMR (DMSO-[D₆]): δ = 2.29 (s, 3H), 6.70 (t, 1H, J = 7.7 Hz), 6.87 (dd, 1H, J = 1.0 Hz, J = 8.0 Hz), 7.05 (m, 1 H), 7.27 (m, 2H), 7.39 (m, 1 H), 7.57 (m, 2H), 7.68 (dd, 1 H, J = 3.2 Hz, J = 6.0 Hz), 8.13 (m, 3H), 8.26 (m, 1 H), 8.49 (dd, 1 H, J = 1.4Hz, J = 8.0Hz), 8.55 (dd, 1 H, J = 1.3 Hz, J = 4.8Hz), 8.75 (m, 1 H), 9.23 (s, 1 H), 9.52 (s, 1 H), 10.40 (s, 1H). + p ESI m/z (%): 521 [M+H⁺]⁺ (100), 522 [M+H⁺]⁺ (34), 523 [M+H⁺]⁺ (11). - p ESI m/z (%): 519 [M-H⁺]⁻ (100), 520 [M-H⁺]⁻ (33), 521 [M-H⁺]⁻ (6). Anal.(C₂₉H₂₄N₆O₂S) Calc. C 66.90, H 4.65, N 16.14; Found C 66.80, H 4.53, N 16.41. 66.90; H, 4.65; N, 16.14; O, 6.15; S, 6.16

### N²-(2-aminophenyl)-N⁵-(3-(4-(pyridin-3-yl)thiazol-2-ylamino)-phenyl)pyridine-2,5-dicarboxamide (47b) [25]:

Yield: 0.69 mmol (95 %), yellow crystals; m.p. 151.0-153.5 °C; IR (KBr): v (cm⁻¹)= 3320, 1676, 1604. ¹H-NMR (DMSO-[D₆]): δ (ppm) = 4.33 (s, 2H), 6.74 (dt, 1H, J = 1.3 Hz, J = 7.7 Hz), 6.90 (dd, 1 H, J = 1.3 Hz, J = 8.0 Hz), 7.02 (m, 1H), 7.24 (d, 1H, J = 8.4 Hz), 7.38 (dd, 1H, J = 1.9 Hz, J = 8.2 Hz), 7.56 (m, 4H), 8.29 (d, 1H, J = 8.2 Hz), 8.47 (m, 1 H), 8.57 (m, 2H), 8.80 (d, 1H, J = 1.9 Hz), 9.25 (d, 2H, J = 1.6 Hz), 9.52 (s, 1H), 10.25 (s, 1H), 10.63 (s, 1H). EI-MS (70eV) *m*/*z* (%): 521 [M⁺⁻] (10), 503 (100), 281 (48), 222 (40). Anal.(C₂₈H₂₃N₇O₂S x HCl x 0.75 H₂O) Calc. C 58.84; H 4.50; N 17,15; Found C 59.02, H 4.48, N 16.96.

### N²-(2-aminophenyl)-N⁵-(4-methyl-3-(4-(pyridin-3-yl)thiazol-2-ylamino)phenyl)thiophene-2,5-dicarboxamide (48b) [27]:

Yield: 1.0 mmol from 1.0 mmol (97%), brown solid; m.p. 242.4 °C; IR (KBr): v (cm⁻¹)= 3269, 1536. ¹H-NMR (DMSO-[D₆]): δ (ppm) = 2.28 (s, 3H), 4.71 (s, 2H, breit), 6.67 (t, 1 H, J = 7.5 Hz), 6.84 (dd, 1 H, J = 1.0 Hz, J = 8.0 Hz), 6.95 (s, 1 H), 7.03 (t, 1 H, J = 7.6 Hz), 7.12 (s, 1 H), 7.29 (m, 3H), 7.56 (s, 1 H), 8.03 (m, 1 H), 8.49 (m, 1H), 8.56 (dd, 1H, J = 1.5 Hz, J = 4.9 Hz), 8.7 (d, 1 H, J = 1.9 Hz), 9.21 (d, 1 H, J = 1.7 Hz), 9.51 (s, 1 H), 9.92 (s, 1 H), 10.38 (s, 1H). + p ESI m/z (%): 527 [M+H⁺]⁺ (3); - p ESI m/z (%): 525 [M-H⁺]⁻ (8). Anal.(C₂₇H₂₂N₆O₂S₂ x 1/2 H₂O x ½ EE) Calc. C 60.10, H 4.69, N 14.50; Found C 60.50, H 4.67, N 14.58.

### Preparation of N-(tetrahydro-2H-pyran-2-yloxy)amides 51 b, 52b, 51 a and 52a by amidation of the respective carboxylic acids 49 and 50:

The respective carboxylic acid was dissolved in the necessary amount of dry DMF and 1.1 eq. BOP, 2.0 eq. NEt₃, and 1.1 eq. of the respective arylamine were added. After stirring at room temperature for 24h, the mixture was poured into water by stirring, the precipitating product removed by filtration, dried in vacuum, and purified by cc (DCM/MeOH = 10/1).

### (E)-N-(4-methyl-3-(4-(pyridin-3-yl)thiazol-2-ylamino)phenyl)-4-(3-oxo-3-(tetrahydro-2H-pyran-2-yloxyamino)prop-1-enyl)benzamide (51b):

Yield: 0.5 mmol (38 %), yellow solid; m.p. 167.5 °C; IR (KBr): v (cm⁻¹)= 3252, 2868, 1664, 1536. ¹H-NMR (DMSO-[D₆]): δ(ppm) = 1.55 (s, 3H), 1.71 (s, 3H), 2.28 (s, 3H), 3.55 (m, 1 H), 3.97 (m, 1 H), 4.94 (s, 1 H), 6.64 (d, 1H, J = 15.9 Hz), 7.20 (d, 1H, J = 8.5 Hz), 7.38 (dd, 1 H, J = 2.1 Hz, J = 8.2 Hz), 7.43 (ddd, 1 H, J = 0.6 Hz, J = 4.9 Hz, J = 8.0 Hz), 7.49 (s, 1 H), 7.57 (d, 1H, J = 15.9 Hz), 7.75 (d, 2H, J = 8.2 Hz), 8.01 (d, 2H, J = 8.4 Hz), 8.33 (m, 1 H), 8.49 (dd, 1 H, J = 1.6 Hz, J = 4.7 Hz), 8.66 (d, 1 H, J = 2.0 Hz), 9.17 (d, 1 H, J = 1.7 Hz), 9.48 (s, 1 H), 10.30 (s, 1 H), 11.33 (s, 1 H). + p ESI m/z (%): 556 [M+H⁺]⁺ (100), 557 [M+H⁺]⁺ (35), 558 [M+H⁺]⁺ (15); Anal.(C₃₀H₂₉N₅O₄S x ½ H₂O) Calc. C 63.81, H 5.36, N 12.40; Found C 63.85, H 5.41, N 12.53.

### (E)-N-(4-methyl-3-(4-(pyridin-3-yl)thiazol-2-ylamino)phenyl)-5-(3-oxo-3-(tetrahydro-2H-pyran-2-yloxyamino)prop-1-enyl)thiophene-2-carboxamide (52b):

Yield: 1.40 mmol (78 %), yellow solid; m.p. 128.4 °C; IR (KBr): v (cm⁻¹)= 2360, 1652, 1611, 1513. ¹H-NMR (DMSO-[D₆]): δ(ppm) = 1.54 (s, 3H), 1.70 (s, 3H), 2.27 (s, 3H), 3.55 (m, 1 H), 3.95 (m, 1 H), 4.91 (s, 1 H), 6.40 (d, 1H, J = 15.6 Hz), 7.20 (d, 1H, J = 8.4 Hz), 7.31 (dd, 1 H, J = 2.0 Hz, J = 8.2 Hz), 7.42 (dd, 1 H, J = 4.8 Hz, J = 7.9 Hz), 7.49 (m, 2H), 7.65 (d, 1H, J = 15.6 Hz), 7.98 (d, 1H, J = 4.0 Hz), 8.34 (m, 1 H), 8.49 (dd, 1 H, J = 1.3 Hz, J = 4.6 Hz), 8.64 (d, 1H, J = 1.9 Hz), 9.15 (d, 1H, J = 1.7 Hz), 9.46 (s, 1 H), 10.31 (s, 1 H), 11.31 (s, 1H). + p ESI m/z (%): 562 [M+H⁺]⁺ (100), 563 [M+H⁺]⁺ (35), 564 [M+H⁺]⁺ (15); Anal.(C₂₈H₂₇N₅O₄S₂ x H₂O) Calc. C 58.01, H 5.04, N 12.08; Found C 58.02, H 4.92, N 11.79.

### (E)-4-(3-oxo-3-(tetrahydro-2H-pyran-2-yloxyamino)prop-1-enyl)-N-(3-(4-(pyridin-3-yl)thiazol-2-ylamino)phenyl)benzamide (51 a):

Yield: 0.6 mmol (23%), brown substance; m.p. 157.6-157.7 °C; IR (KBr): v (cm⁻¹) = 3425, 3251, 2925, 1644, 1577. NMR (DMSO-[D₆]): δ (ppm) = 1.55 (s, 3H), 1.71 (s, 3H), 3.56 (m, 1 H), 3.98 (m, 1 H), 4.94 (s, 1 H), 6.65 (d, 1H, J = 15.8 Hz), 7.31 (m, 2H), 7.48 (m, 1 H), 7.55 (s, 1 H), 7.76 (d, 2H, J = 8.1 Hz), 8.02 (d, 2H, J = 8.3 Hz), 8.37 (td, 1 H, J = 1.9 Hz, J = 8.0 Hz), 9.23 (d, 1 H, J = 1.9 Hz), 10.35 (s, 1 H), 10.42 (s, 1 H), 11.34 (s, 1 H). + p ESI m/z (%): 542 [M+H⁺]⁺ (100), 543 [M+H⁺]⁺ (30), 544 [M+H⁺]⁺ (10). Anal.(C₂₉H₂₇N₅O₄S x 1/2 H₂O) Calc. C 63.26, H 5.13, N 12.72; Found C 53.29, H 4.72, N 11.33.

### (E)-5-(3-oxo-3-(tetrahydro-2H-pyran-2-yloxyamino)prop-1-enyl)-N-(3-(4-(pyridin-3-yl)thiazol-2-ylamino)phenyl)thiophene-2-carboxamide (52a):

Yield: 1.3 (68 %), yellow substance; m.p. 169.5 °C; IR (KBr): v (cm⁻¹)= 3471, 2927, 1613, 1536. ¹H-NMR (DMSO-[D₆]): δ (ppm) = 1.54 (s, 3H), 1.70 (s, 3H), 3.54 (m, 1H), 3.93 (m, 1 H), 4.92 (s, 1 H), 6.41 (d, 1H, J = 15.7 Hz), 7.23 (d, 1H, J = 8.6 Hz), 7.33 (t, 1 H, J = 8.0 Hz), 7.46 (dd, 2H, J = 4.4 Hz, J = 7.7 Hz), 7.50 (d, 1 H, J = 3.9 Hz), 7.56 (s, 1 H), 7.65 (d, 1 H, J = 15.7 Hz), 8.01 (d, 1 H, J = 3.9 Hz), 8.38 (m, 1 H), 8.42 (m, 1 H), 8.52 (dd, 1H, J = 1.6 Hz, J = 4.7 Hz), 9.21 (d, 1H, J = 1.7 Hz), 10.37 (s, 1 H), 10.43 (s, 1 H), 11.32 (s, 1H). + p ESI m/z (%): 548 [M+H⁺]⁺ (100), 549 [M+H⁺]⁺ (35), 550 [M+H⁺]⁺ (15); Anal.(C₂₇H₂₅N₅O₄S₂ x DCM) Calc. C 53.16, H 4.30, N 11.21; Found C 53.29, H 4.72, N 11.33.

Preparation of ***N*-hydroxyamides (53a [22], 53b [19], 54a [34], 54b [33] and 59a [17], 59b [20]) by cleavage of the respective *N*-(tetrahydro-2*H*-pyran-2-yloxy)amides (51a, 51b, 52a, 52b, and 59a [17], 59b [20])** The respective ***N*-(tetrahydro-2*H*-pyran-2-yloxy)amide** was dissolved in MeOH, 1 N HCl was added and the mixture was stirred at room temperature over night. The organic solvent was removed in vacuum, the product was filtered off, washed with a small amount of MeOH and dried in vacuum.

### (E)-4-(3-(hydroxyamino)-3-oxoprop-1-enyl)-N-(3-(4-(pyridin-3-yl)thiazol-2-ylamino)phenyl)benzamide (53a) [22]:

Yield: 0.5 mmol (83%), yellow solid; m.p. 100.6 °C; IR (KBr): v (cm⁻¹) = 3062, 1655, 1609, 1535. NMR (DMSO-[D₆]): δ (ppm) = 5.55 (s, 1 H), 6.66 (d, 1H, J = 15.9 Hz), 7.34 (m, 3H), 7.55 (d, 1H, J = 15.8 Hz), 7.79 (m, 2H), 7.98 (s, 1 H), 8.13 (m, 3H), 8.84 (m, 2H), 9.20 (m, 1 H), 9.53 (d, 1H, J = 1.3 Hz), 10.43 (s, 1 H), 10.75 (s, 1H). + p ESI m/z (%): 458 [M+H⁺]⁺ (100), 459 [M+H⁺]⁺ (30), 466 [M+H⁺]⁺ (10). Anal.(C₂₄H₁₉N₅O₃S₂ x 2 H₂O x 2 HCl) Calc. C 50.89, H 4.45, N 12.36; Found C 50.14, H 4.58, N 12.36.

### (E)-4-(3-(hydroxyamino)-3-oxoprop-1-enyl)-N-(4-methyl-3-(4-(pyridin-3-yl)thiazol-2-ylamino)phenyl)benzamide (53b) [19]:

Yield: 0.4 mmol (100 %), yellow crystals. m.p. 154.2 °C; IR (KBr): v (cm⁻¹)= 3197, 3063, 1658, 1603, 1535. ¹H-NMR (DMSO-[D₆]): δ(ppm) = 2.29 (s, 3H), 6.64 (d, 1H, J = 15.9 Hz), 7.20 (d, 1 H, J = 8.4 Hz) 7.34 (dd, 1 H, J = 2.0 Hz, J = 8.2 Hz), 7.54 (d, 1 H, J = 15.8 Hz), 7.73 (d, 2H, J = 8.3 Hz), 7.89 (s, 2H), 8.08 (m, 4H), 8.80 (dd, 1H, J = 0.9 Hz, J = 5.7 Hz), 8.95 (d, 1H, J = 1.9 Hz), 9.09 (m, 1H), 9.46 (d, 1H, J = 1.7 Hz), 9.67 (s, 1H), 10.36 (s, 1H), 10.96 (s, 1H). + p ESI m/z (%): 472 [M+H⁺]⁺ (100), 473 [M+H⁺]⁺ (35); Anal.(C₂₅H₂₁N₅O₃S x 3/2 H₂O x HCl) Calc. C 56.88, H 4.96, N 12.76; Found C 56.44, H 4.84, N 12.89.

### (E)-5-(3-(hydroxyamino)-3-oxoprop-1-enyl)-N-(3-(4-(pyridin-3-yl)thiazol-2-ylamino)phenyl)thiophene-2-carboxamide (54a) [34]:

Yield: 0.2 mmol (67%), yellow solid; m.p. 205.6 °C; IR (KBr): v (cm⁻¹)= 3241, 2136, 1610, 1537. ¹H-NMR (DMSO-[D₆]): δ (ppm) = 6.39 (d, 1H, J = 15.6 Hz), 7.30 (m, 1H), 7.33 (m, 1H), 7.43 (td, 1H, J = 2.1 Hz, J = 7.2 Hz), 7.47 (d, 1H, J = 3.9 Hz), 7.62 (d, 1H, J = 15.5 Hz), 7.88 (s, 1H), 8.02 (dd, 1H, J = 5.6 Hz, J = 8.1 Hz), 8.08 (d, 1H, J = 3.9 Hz), 8.54 (m, 1H), 8.79 (m, 1H), 9.03 (m, 1H), 9.42 (d, 1H, J = 1.6 Hz), 10.44 (s, 1H), 10.62 (s, 1H), 10.91 (s, 1H). + p ESI m/z (%): 464 [M+H⁺]⁺ (100), 465 [M+H⁺]⁺ (35), 466 [M+H⁺]⁺ (15); Anal.(C₂₂H₁₇N₅O₃S₂ x 5 H₂O) Calc. C 47.73, H 4.92, N 12.65; Found C 47.33, H 4.55, N 12.22.

### (E)-5-(3-(hydroxyamino)-3-oxoprop-1-enyl)-N-(4-methyl-3-(4-(pyridin-3-yl)thiazol-2-ylamino)phenyl)thiophene-2-carboxamide (54b) [33]:

Yield: 0.2 mmol (97%), yellow substance; m.p. 241.6 °C; IR (KBr): v (cm⁻¹)= 3206, 2134, 1601, 1537. ¹H-NMR (DMSO-[D₆]): δ (ppm) = 2.28 (s, 3H), 6.39 (d, 1H, J = 15.6 Hz), 7.20 (d, 1 H, J = 8.5 Hz), 7.36 (dd, 1 H, J = 2.0 Hz, J = 8.2 Hz), 7.45 (d, 1 H, J = 3.9 Hz), 7.61 (d, 1H, J = 15.5 Hz), 7.84 (s, 1H), 8.00 (dd, 1H, J = 5.6 Hz, J = 8.1 Hz), 8.09 (d, 1 H, J = 3.9 Hz), 8.77 (m, 2H), 9.02 (m, 1 H), 9.38 (d, 1 H, J = 1.6 Hz), 9.65 (s, 1 H), 10.44 (s, 1H), 10.91 (s, 1H). + p ESI m/z (%): 478 [M+H⁺]⁺ (100), 479 [M+H⁺]⁺ (35), 480 [M+H⁺]⁺ (15); Anal.(C₂₃H₁₉N₅O₃S₂ x 4 H₂O) Calc. C 50.26, H 4.95, N 12.74; Found C 50.35, H 4.54, N 12.77.

### N¹-hydroxy-N⁴-(3-(4-(pyridin-3-yl)thiazol-2-ylamino)phenyl)terephthalamide (59a) [17]:

Yield: 0.7 mmol (70%), brown solid; m.p. 205.5 °C; IR (KBr): v (cm⁻¹)= 3237, 1614, 1559. ¹H-NMR (DMSO-[D₆]): δ (ppm) = 7.27 (m, 1 H), 7.30 (d, 1H, J = 1.5 Hz), 7.35 (m, 1 H), 7.41 (td, 1 H, J = 1.9 Hz, J = 7.5 Hz), 7.87 (s, 1 H), 7.92 (d, 2H, J = 8.4 Hz), 8.00 (dd, 1H, J = 5.5 Hz, J = 8.1 Hz), 8.09 (d, 2H, J = 8.4 Hz) 8.74 (m, 1H), 8.78 (dd, 1H, J = 1.0 Hz, J = 5.4 Hz), 9.00 (d, 1H, J = 8.2 Hz), 9.44 (d, 1H, J = 1.8 Hz), 10.44 (s, 1H), 10.62 (s, 1 H), 11.44 (s, 1H). + p ESI m/z (%): 432 [M+H⁺]⁺ (100), 433 [M+H⁺]⁺ (27), 434 [M+H⁺]⁺ (8); - p ESI m/z (%): 490 [M+CH₃COO⁻]⁻ (53), 430 [M-H⁺]⁻ (9). Anal. (C₂₂H₁₇N₅O₃S x 2 H₂O x HCl) Calc. C 52.43, H 4.40, N 13.90; Found C 52.27, H 4.54, N 13.81.

### N¹-hydroxy-N⁴-(4-methyl-3-(4-(pyridin-3-yl)thiazol-2-ylamino)phenyl)terephthalamide (59b) [20]:

Yield: 0.25 mmol (95%), brown solid; m.p. 261.0°C; IR (KBr): v (cm⁻¹)= 3207, 1624, 1543. ¹H-NMR (DMSO-[D₆]): 6 (ppm) = 2.29 (s, 3H), 7.21 (d, 1H, J = 8.5 Hz), 7.34 (m, 1 H), 7.84 (s, 1 H), 7.90 (d, 2H, J = 8.4 Hz), 8.00 (dd, 1 H, J = 5.5 Hz, J = 8.1 Hz), 8.09 (d, 2H, J = 8.4 Hz), 8.77 (d, 1 H, J = 4.4 Hz), 8.93 (d, 1 H, J = 2.0 Hz), 9.00 (d, 1 H, J = 8.2 Hz), 9.42 (d, 1 H, J = 1.8 Hz), 9.64 (s, 1 H), 10.39 (s, 1 H), 11.44 (s, 1 H). + p ESI m/z (%): 446 [M+H⁺]⁺ (100), 447 [M+H⁺]⁺ (29), 448 [M+H⁺]⁺ (9); - p ESI m/z (%): 444 [M-H⁺]⁻ (100), 445 [M-H⁺]⁻ (21), 504 [M+CH₃COO⁻]⁻ (98). Anal. (C₂₃H₁₉N₅O₃S x 3 H₂O x HCl) Calc. C 51.54, H 4.89, N 13.07; Found C 51.41, H 5.10, N 12.83.

Preparation of **56a** and **56b** was performed as described above for **38** from **33a** or **33b** and **55**

### Methyl 4-(3-(4-(pyridin-3-yl)thiazol-2-ylamino)phenylcarbamoyl)-benzoate semihydrate (56a):

Yield: 3.7 mmol (53 %), brown solid; m.p. 237.2 °C; IR (KBr): v (cm⁻¹)= 2360, 1721, 1531. ¹H-NMR (DMSO-[D₆]): δ (ppm) = 3.91 (s, 3H), 7.28 (m, 1 H), 7.34 (t, 1H, J = 7.9 Hz), 8.11 (m, 4H), 8.38 (m, 1 H), 8.46 (t, 1 H, J = 1.8 Hz), 8.51 (dd, 1 H, J = 1.5 Hz, J = 4.8 Hz), 9.22 (d, 1H, J = 1.7 Hz), 10.42 (s, 1 H), 10.48 (s, 1H). CI-MS (NH₃) *m*/*z* (%): 431 [M+H⁺]⁺ (100). Anal.(C₂₃H₁₈N₄O₃S x 1/2 H₂O). Calc. C 62.86, H 4.36, N 12.75; Found C 63.28, H 4.04, N 12.88.

### Methyl 4-(4-methyl-3-(4-(pyridin-3-yl)thiazol-2-ylamino)phenylcarbamoyl)benzoate (56b):

Yield: 5.5 mmol (50 %), brown solid; m.p. 194.7 °C; IR (KBr): v (cm⁻¹)= 3303, 2949, 1722, 1536. ¹H-NMR (DMSO-[D₆]): δ (ppm) = 2.28 (s, 3H), 3.90 (s, 3H), 7.21 (d, 1H, J = 8.4 Hz), 7.38 (dd, 1 H, J = 2.1 Hz, J = 8.2 Hz), 7.43 (ddd, 1 H, J = 0.7 Hz, J = 4.9 Hz, J = 8.1 Hz), 7.48 (s, 1 H), 8.09 (m, 4H), 8.32 (m, 1 H), 8.48 (dd, 1 H, J = 1.6 Hz, J = 4.7 Hz), 8.64 (d, 1 H, J = 2.0 Hz), 9.16 (dd, 1 H, J = 0.7 Hz, J = 2.3 Hz), 9.46 (s, 1 H), 10.43 (s, 1H). CI-MS (NH₃) *m*/*z* (%): 445 [M+H⁺]⁺ (100). Anal.(C₂₄H₂₀N₄O₃S x 1/2 H₂O). Calc. C 63.56, H 4.67, N 12.35; Found C 63.17, H 4.56, N 12.32.

Preparation of **57a** and **57b** by cleavage of the respective methylester precursors **56a** and **56b:** The respective ester (**56b** or **56a**) was dissolved in THF and 1.1 eq. LiOH in H₂O was added. The mixture was stirred at 50°C over night, cooled to room temperature and the crude product precipitated after addition of water. The respective product was purified by cc (EE/MeOH = 20/1).

### 4-(3-(4-(pyridin-3-yl)thiazol-2-ylamino)phenylcarbamoyl)benzoic acid (57a):

Yield: 2.7 mmol from 5.0 mmol **56a** (54 %), brown solid; m.p. 298.8 °C; IR (KBr): v (cm⁻¹)= 2360, 1532, 1398. ¹H-NMR (DMSO-[D₆]): δ (ppm) = 7.28 (m, 1 H), 7.34 (t, 1H, J = 7.9 Hz), 8.11 (m, 4H), 8.38 (m, 1 H), 8.46 (t, 1 H, J = 1.8 Hz), 8.51 (dd, 1 H, J = 1.5 Hz, J = 4.8 Hz), 9.22 (d, 1H, J = 1.7 Hz), 10.42 (s, 1 H), 10.48 (s, 1H). CI-MS (NH₃) m/z(%): 417 [M+H⁺]⁺ (100). Anal. (C₂₂H₁₆N₄O₃S x 1/2 H₂O x 3/2 HCl). Calc. C 55.03, H 3.88, N 11.67; Found C 55.23, H 4.21, N 11.64.

### 4-(4-methyl-3-(4-(pyridin-3-yl)thiazol-2-ylamino)phenylcarbamoyl)benzoic acid (57b):

Yield: 2.5 mmol (47 %), brown solid; m.p. 194.7 °C; IR (KBr): v (cm⁻¹)= 3389, 3063, 1536, 1263. ¹H-NMR (DMSO-[D₆]): δ (ppm) = 3.90 (s, 3H), 7.21 (d, 1H, J = 8.4 Hz), 7.38 (dd, 1 H, J = 2.1 Hz, J = 8.2 Hz), 7.43 (ddd, 1 H, J = 0.7 Hz, J = 4.9 Hz, J = 8.1 Hz), 7.48 (s, 1 H), 8.09 (m, 4H), 8.32 (m, 1 H), 8.48 (dd, 1 H, J = 1.6 Hz, J = 4.7 Hz), 8.64 (d, 1 H, J = 2.0 Hz), 9.16 (dd, 1 H, J = 0.7 Hz, J = 2.3 Hz), 9.46 (s, 1 H), 10.43 (s, 1H). CI-MS (NH₃) m/z (%): 431 [M+H⁺]⁺ (100). Anal.(C₂₃H₁₈N₄O₃S x 6 H₂O). Calc. C 51.29, H 5.61, N 10.40; Found C 51.41, H 5.22, N 10.26.

**Formation of 58a and 58b:** The respective acid (**57b** or **57a**) was dissolved in DMF and 1.1 eq. BOP, 2 eq. NEt₃, and 1.1 eq. **NH₂OTHP** was added. After stirring at room temperature for 24h, the crude product precipitated by addition of water and was purified by cc (DCM/MeOH = 10/1).

### N¹-(4-methyl-3-(4-(pyridin-3-yl)thiazol-2-ylamino)phenyl)-N⁴-(tetrahydro-2H-pyran-2-yloxy)terephthalamide hydrate (58b):

Yield: 0.7 mmol (60%), brown solid; m.p. 184.1 °C; IR (KBr): v (cm⁻¹)= 3284, 2947, 1642. ¹H-NMR (DMSO-[D₆]): δ (ppm) = 1.57 (s, 3H), 1.74 (s, 3H), 2.28 (s, 3H), 3.58 (m, 1 H), 4.07 (m, 1 H), 5.03 (s, 1 H), 7.21 (d, 1 H, J = 8.5 Hz), 7.39 (dd, 1 H, J = 2.1, J = 8.3 Hz), 7.43 (m, 1 H), 7.48 (s, 1 H), 7.91 (d, 2H, J = 8.4 Hz), 8.06 (d, 2H, J = 8.4 Hz), 8.32 (m, 1 H), 8.49 (dd, 1H, J = 1.6 Hz, J = 4.7 Hz), 8.65 (d, 1H, J = 2.0 Hz), 9.16 (d, 1 H, J = 1.6 Hz), 9.46 (s, 1 H), 10.35 (s, 1 H), 11.81 (s, 1 H). + p ESI m/z (%): 530 [M+H⁺]⁺ (100), 531 [M+H⁺]⁺ (33), 532 [M+H⁺]⁺ (11); - p ESI m/z (%): 528 [M-H⁺]⁻ (40), 529 [M-H⁺]⁻ (11). Anal. (C₂₈H₂₇N₅O₄S x 5/2 H₂O) Calc. C 58.52, H 5.61, N 12.19; Found C 58.56, H 5.21, N 12.19.

### N¹-(3-(4-(pyridin-3-yl)thiazol-2-ylamino)phenyl)-N⁴-(tetrahydro-2H-pyran-2-yloxy)terephthalamide (58a):

Yield: 1 mmol (82%), brown solid; m.p. 223.9 °C; IR (KBr): v (cm-1)= 3290, 2945, 1641. ¹H-NMR (DMSO-[D₆]): 6 (ppm) = 1.57 (s, 3H), 1.74 (s, 3H), 3.56 (s, 1 H), 4.08 (m, 1 H), 5.04 (s, 1 H), 7.32 (m, 2H), 7.48 (m, 2H), 7.55 (s, 1 H), 7.92 (d, 2H, J = 8.4 Hz), 8.07 (d, 2H, J = 8.4 Hz), 8.37 (m, 1 H), 8.46 (m, 1 H), 8.51 (dd, 1H, J = 1.6 Hz, J = 4.7 Hz), 9.22 (d, 1 H, J = 1.7 Hz), 10.42 (s, 2H), 11.83 (s, 1 H). + p ESI m/z (%): 516 [M+H⁺]⁺ (100), 517 [M+H⁺]⁺ (33), 518 [M+H⁺]⁺ (10); - p ESI m/z (%): 574 [M+CH₃COO]⁻ (100), 514 [M-H⁺]⁻ (80), 515 [M-H⁺]⁻ (19), 516 [M-H⁺]⁻ (9). Anal. (C₂₇H₂₅N₅0₄S) Calc. C 62.90, H 4.89, N 13.58; Found C 62.60, H 4.79, N 13.43.

### N¹-(4-(pyridin-3-yl)pyrimidin-2-yl)benzene-1,3-diamine

A mixture of the (3-Nitro-phenyl)-(4-pyridin-3-yl-pyrimidin-2-yl)-amine (2.58 g, 8.8 mmol) and 5.0 eq of SnCl₂·H₂O (8.3 g, 44 mmol) in MeOH (88 ml) was stirred at reflux temperature for 8.5 h. After 3 h, 5.75 h and 7.5 h 2.5, 2.5 and 1.25 eq of SnCl₂·H₂O was added. MeOH was removed under reduced pressure and the crude product was diluted with water. The aqueous layer was neutralized with Na₂CO₃ and the precipitating salt was removed by filtration. Afterwards it was extracted with CH₂Cl₂. The combined organic phase was dried over Na₂SO₄ and evaporated under vacuo. The crude product was purified by silica gel flash chromatography [CH₂Cl₂/MeOH (100:0 to 98:02)] to give the title compound (388 mg, 16.7%) as yellow crystals. ¹H-NMR (DMSO-[D₆]): δ (ppm) = 4.96 (s, 2 H), 6.17 (m, 1 H), 6.94 (d, 2 H, J = 5.7 Hz), 7.06 (s, 1 H), 7.4 (d, 1 H, J = 5.1 Hz), 7.55 (dd, 1 H, J = 4.7 Hz, J = 7.9 Hz), 8.49 (d, 1 H, J = 8.1 Hz), 8.55 (d, 1 H, J = 5.1 Hz), 8.68 (dd, 1 H, J = 1.5 Hz, J = 4.8 Hz), 9.32 (d, 1 H, J = 2.0 Hz), 9.38 (s, 1 H).

### 6-Methyl-N¹-(4-(pyridin-3-yl)pyrimidin-2-yl)benzene-1,3-diamine

The corresponding methyl derivative was prepared in a similar fashion as outlined above. ¹H-NMR (DMSO-[D₆]): δ (ppm) = 2.09 (s, 3 H), 4.85 (bs, 2 H), 6.35 (dd, 1 H, J = 2.4 Hz, J = 8.1 Hz), 6.80 (m, 2 H), 7.35 (m, 1 H), 7.51 (dd, 1 H, J = 5.0 Hz, J = 8.0 Hz), 8.46 (m, 1 H), 8.69 (m, 2 H), 9.24 (d, 1 H, 2.4 Hz).

### 4-Chloro-N(3)-(4-pyridin-3-yl-pyrimidin-2-yl)-benzene-1,3-diamine

In a similar way the corresponding chloro derivative is prepared. The title compound is isolated as yellow oil. 298.2 [M+H⁺]⁺

### N-Methyl-N'-(4-pyridin-3-yl-pyrimidin-2-yl)-benzene-1,3-diamine

A mixture of the respective amine (100 mg, 0.38 mmol) and paraformaldehyde (57 mg, 1.9 mmol) were dissolved in MeOH (20 ml) and 5.4 M NaOMe in MeOH (352 µl, 1.9 mmol) was added. The mixture was refluxed for 2 h. Then it was cooled to 0°C and NaBH₄ (72 mg, 1.9 mmol) was added. After 1 h stirring at reflux temperature, the reaction mixture was poured into ice. The aqueous phase was extracted with CH₂Cl₂. The combined organic layer was dried over Na₂SO₄ and the solvent was evaporated in vacuum. The crude product was purified by silica gel flash chromatography [CH₂Cl₂/MeOH (100:0 to 98:02)] to give the title compound (98 mg, 93 %). ¹H-NMR (DMSO-[D₆]): δ (ppm) = 2.70 (d, 3 H, J = 5.0 Hz), 5.55 (m, 1 H), 6.20 (m, 1 H), 7.00 (m, 2 H), 7.15 (s, 1 H), 7.45 (d, 1 H, J = 5.2 Hz), 7.57 (dd, 1 H, J = 4.8 Hz, J = 8.0 Hz), 8.55 (m, 2 H), 8.72 (dd, 1 H, J = 1.7 Hz, J = 4.8 Hz), 9.34 (d, 1 H, J = 1.7 Hz), 9.45 (s, 1 H).

### 4-Chloro-N(1)-methyl-N(3)-(4-pyridin-3-yl-pyrimidin-2-yl)-benzene-1,3-diamine:

The title compound was prepared in a similar way as outlined above. It was isolated as a yellow solid. 312.2 [M+H⁺]⁺

### Methyl 4-(2-(tert-butoxycarbonylamino)phenylcarbamoyl)benzoate:

Yield: 31.05 mmol (94 %), colorless crystals; m.p. 145.5-146.5 °C; ¹H-NMR (DMSO-[D₆]): δ (ppm) = 1.44 (s, 9H), 3.91 (s, 3H), 7.22 (dt, 1H, J = 1.8 Hz, J = 7.7 Hz), 7.15 (dt, 1 H, J = 1.7 Hz, J = 7.6 Hz), 7.55 (m, 2H), 8.10 (m, 4H), 8.71 (s, 1 H), 9.98 (s, 1 H).

### 4-(2-(tert-butoxycarbonylamino)phenylcarbamoyl)benzoic acid:

Yield: 18.9 mmol (68 %), colorless crystals; m.p. 189.5-191.5 °C; ¹H-NMR (DMSO-[D₆]): δ (ppm) = 1.44 (s, 9H), 7.15 (dt, 1 H, J = 1.6 Hz, J = 7.6 Hz), 7.22 (dt, 1 H, J = 1.8 Hz, J = 7.7 Hz), 7.55 (dt, 2H, J = 1.5 Hz, J = 7.8 Hz), 8.07 (m, 4H), 8.73 (s, 1 H), 9.97 (s, 1H), 13.33 (s, 1 H).

### {2-[(1-{4-[3-(4-Pyridin-3-yl-pyrimidin-2-ylamino)-phenylcarbamoyl]-phenyl}-methanoyl)-amino]-phenyl}-carbamic acid tert-butyl ester:

The arylamine (100 mg, 0.38 mmol), 4-(2-(tert-butoxycarbonylamino) phenylcarbamoyl)benzoic acid (136 mg, 0.38 mmol), HBTU (165 mg, 0.4 mmol) and N-ethyldiisopropylamine (54 mg, 0.42 mmol) were dissolved in pyridine (1ml) and stirred at room temperature for 3 h. Then the solution was dried under reduced pressure and coevaporated with toluene. The crude product was suspended in CH2Cl2/MeOH. The suspension was filtrated and the solid dried in vacuum to give the title compound (93 mg, 41 %) as a pale red powder. ¹H-NMR (DMSO-[D6]): δ (ppm) = 1.46 (s, 9 H), 7.2 (m, 2 H), 7.33 (m, 2 H), 7.54 (m, 5 H), 8.13 (m, 4 H), 8.48 (s, 1 H), 8.65 (m, 2 H), 8.72 (m, 2 H), 9.4 (s, 1 H), 9.85 (s, 1 H), 9.96 (s, 1 H), 10.4 (s, 1 H).

### {2-[(1-{4-[4-Methyl-3-(4-pyridin-3-yl-pyrimidin-2-ylamino)-phenyl}-methanoyl)-amino]-phenyl}-carbamic acid tert-butyl ester

¹H-NMR (DMSO-[D6]): δ (ppm) = 1.45 (s, 9 H), 2.25 (s, 3 H), 7.22 (m, 3 H), 7.43 (d, 1 H, J = 5.6 Hz), 7.53 (m, 4 H), 8.11 (m, 5 H), 8.49 (m, 2 H), 8.68 (m, 2 H), 8.96 (s, 1 H), 9.28 (s, 1 H), 9.95 (s, 1 H), 10.4 (s, 1 H).

### (2-{[1-(4-{Methyl-[3-(4-pyridin-3-yl-pyrimidin-2-ylamino)-phenyl]carbamoyl}-phenyl)-methanoyl]-amino}-phenyl)-carbamic acid tert-butyl ester

4-(2-(Tert-butoxycarbonylamino)phenylcarbamoyl)benzoic acid (109 mg, 0.31 mmol) and HOBt (42 mg, 0.31 mmol) were dissolved in DMF (0.7 ml) and EDCxHCl (178 mg, 0.93 mmol) and triethylamine (129 µl, 0.93 mmol) was added. The suspension was stirred at room temperature for 1 h. The suspension was cooled to 0 °C and then a solution of the respective arylamin (85 mg, 0.31 mmol) and DMF (1.4 ml) was added. The mixture was stirred at room temoperature for 36 h. Afterwards it was poured into PE/EE (1:1) and the organic phase was washed with H₂O (2x). The organic phase was dried over Na₂SO₄ and the solvent evaporated. The crude product was purified by silica gel flash chromatography [CH₂Cl₂/MeOH (99:01 97:03)] to give a colorless solid (52 mg, 27 %). ¹H-NMR (DMSO-[D₆]): δ (ppm) = 1.35 (s, 9 H), 3.46 (s, 3 H), 6.80 (d, 1 H, J = 7.6 Hz), 7.10 (t, 1 H, J = 7.1 Hz), 7.17 (t, 1 H, 7.0 Hz), 7.23 (t, 1 H, J = 8.1 Hz), 7.50 (m, 4 H), 7.55 (m, 2 H), 7.60 (m, 2 H), 7.77 (m, 2 H), 7.85 (s, 1 H), 8.49 (d, 1 H, J = 8.0 Hz), 8.64 (m, 2 H), 8.74 (d, 1 H, J = 3.5 Hz), 9.34 (s, 1 H), 9.74 (s, 1 H), 9.83 (s, 1 H).

### {2-[(1-{4-[4-Chloro-3-(4-pyridin-3-yl-pyrimidin-2-ylamino)-phenylcarbamoyl]-phenyl}-methanoyl)-amino]-phenyl}-carbamic acid tert-butyl ester

The title compound was prepared in a similar way as outlined above and was obtained in 22% yield as a colorless solid. 636.12 [M+H⁺]⁺

### (2-{[1-(4-{[4-Chloro-3-(4-pyridin-3-yl-pyrimidin-2-ylamino)-phenyl]-methylcarbamoyl}-phenyl)-methanoyl]-amino}-phenyl)-carbamic acid tert-butyl ester

The title compound was prepared in a similar way as described above and was obtained in about 10% yield.

### N-(2-Amino-phenyl)-N'-[3-(4-pyridin-3-yl-pyrimidin-2-ylamino)-phenyl]-terephthalamide [31]

The carbamin acid-tert-butyl-ester (76.6 mg, 0.13 mmol) was suspended in 4 M HCl in dioxane (7.7 ml) and was stirred at room temperature for 24 h. Then the mixture was heated at 70°C for 1 h. The dioxane was removed under reduced pressure. Afterwards the crude product was suspended in H₂O and the aqueous phase was neutralized with an aqueous solution of Na₂CO₃. The aqueous layer was extracted with CH₂Cl₂. The combined organic layer was dried over Na₂SO₄ and evaporated in vacuum. The crude product was purified by silica gel flash chromatotgraphy [CH₂Cl₂/MeOH (95:05)] to give the title compound (12.3 mg, 19%) as a solid. ¹H-NMR (DMSO-[D₆]): δ (ppm) = 5.02 (bs, 2 H), 6.65 (t, 1 H, J = 7.4 Hz), 6.80 (d, 1 H, J = 7.9 Hz), 7.00 (t, 1 H, J = 7.5 Hz), 7.20 (d, 1 H, J = 7.6 Hz), 7.35 (m, 2 H), 7.54 (m, 3 H), 8.15 (s, 4 H), 8.48 (s, 1 H), 8.65 (m, 3 H), 8.72 (s, 1 H), 9.40 (s, 1 H), 9.83 (s, 2 H), 10.40 (s, 1 H). 502,1 [M+H⁺]⁺

### N-(2-Amino-phenyl)-N'-[4-methyl-3-(4-pyridin-3-yl-pyrimidin-2-ylamino)-phenyl]-terephthalamide compound with trifluoroacetic acid [14]

¹H-NMR (DMSO-[D₆]): δ (ppm) = 2.10 (s, 3 H), 4.40 (s, 2 H), 6.44 (dd, 1 H, J = 2.4 Hz, J = 8.4 Hz), 6.97 (m, 2 H), 7.21 (m, 3 H), 7.34 (m, 1 H), 7.43 (m, 1 H), 7.53 (m, 2 H), 7.69 (m, 1 H), 8.50 (d, 1 H, J = 5.2 Hz), 8.58 (m, 1 H), 8.78 (m, 2 H), 8.95 - 9.40 (bs, 2 H), 9.31 (s, 1 H), 10.1 (s, 1 H). 516,1[M+H⁺]⁺, m.p. 308-310 °C

### N-(2-Amino-phenyl)-N'-[4-methyl-3-(4-pyridin-3-yl-pyrimidin-2-ylamino)-phenyl]-terephthalamide compound with hydrochloric acid contains 2,79 HCl /mol [29]

The compound was prepared by treating the respective starting material with hydrochloric acid.

### N-(2-Amino-phenyl)-N'-methyl-N'-[3-(4-pyridin-3-yl-pyrimidin-2-ylamino)-phenyl]-terephthalamide [30]

The carbamin acid-tert-butyl-ester (41.7 mg, 0.07 mmol) was suspended in 4 M HCl in dioxane (4 ml) and stirred at room temperature for 3 h. Then the mixture was dried under vacuum. The crude product was dissolved in H₂O/acetonitrile and lyophylized to give the title compound (40.3 mg, 98 %) as a yellow solid. m.p. 204.6 °C (dec.); ¹H-NMR (DMSO-[D₆]): δ (ppm) = 3.45 (s, 3 H), 4.25 - 6.36 (bs, 2 H), 6.83 (d, 1 H, J = 7.7 Hz), 7.23 (t, 1 H, J = 8.1 Hz), 7.34 (m, 2 H), 7.49 (m, 4 H), 7.6 (m, 2 H), 7.81 (s, 1 H), 7.89 (m, 3 H), 8.70 (d, 1 H, J = 5.1 Hz), 8.91 (m, 2 H), 9.47 (s, 1 H), 9.94 (s, 1 H), 10.51 (s, 1 H). 516.1 [M+H⁺]⁺, m.p. = 204,6 °C.

### N-(2-Amino-phenyl)-N'[4-chloro-3-(4-pyridin-3-yl-pyrimidin-2-ylamino)-phenyl]-terephthalamide compound with hydrochloric acid [32]

The title compound was prepared in a similar way as outlined above. m.p: 269.7 °C.

### N-(2-Amino-phenyl)-N-[4-chloro-3-(4-pyridin-3-yl-pyrimidin-2-ylamino)-phenyl]-N'-methyl-terephthalamide [35]

The title compound was prepared in a similar way as outlined above and was obtained in 15 % yield as nearly colorless solid. 359.8 [M+H⁺]⁺

### (E)-3-(4-{[4-Methyl-3-(4-pyridin-3-yl-pyrimidin-2-ylamino)-phenylamino]-methyl}-phenyl)-acrylic acid

A solution of the respective arylamine (1.00g, 3.6 mmol) in MeOH (12 ml) was stirred with (E)-3-(4-formyl-phenyl)-acrylic acid (635 mg, 3.6 mmol), HOAc (217 mg, 3.6 mmol) and NaBH₃CN (272 mg, 4.3 mmol) for 14h at ambient temperature. After typical acidic workup, 1.11 g (71 %) of the title compound was obtained. Melting point 234-242°C. ¹H-NMR (DMSO-[D₆]): δ (ppm) = 2.06 (s, 3 H), 4.27 (s, 2 H),6.12 (bs, 1 H), 6.32 (d, 1 H), 6.50 (d, 1 H,), 6.88 (m, 2 H), 7.39 (m, 3 H), 7.57 (m, 4 H), 8.40 (m, 2 H), 8.69 (m, 2 H), 9.25 (s, 1 H), 12.2 (bs, 1 H).

### (E)-3-(4-{[4-Methyl-3-(4-pyridin-3-yl-pyrimidin-2-ylamino)-phenylamino]-methyl}-phenyl)-N-(tetrahydro-pyran-2-yloxy)-acrylamide

The title compound was prepared in a similar fashion as outlined above. The compound was obtained as an oil in 47% yield. ¹H-NMR (DMSO-d₆) 6/ppm: 1.53 (4 H, m); 1.68 (4 H, m); 2.06 (3 H, s); 3.52 (1 H, m); 3.95 (1 H, m); 4.25 (2 H, d); 4.89 (1 H, s); 6.15 (1 H, t); 6.34 (1 H, d); 6.48 (1 H, d); 6.85 (2 H, m); 7.40 (6 H, m); 8.39 (2 H, m); 8.71 (2 H, m); 9.25 (1 H, d); 11.2 (1 H, s).

### (E)-N-Hydroxy-3-(4-{[4-methyl-3-(4-pyridin-3-yl-pyrimidin-2-ylamino)-phenylamino]-methyl}-phenyl)-acrylamide [16]

By acid treatment, the title compound was prepared in 76% yield as solid with m.p. 201-205°C. ¹H-NMR (DMSO-d₆) δ/ppm: 2.25 (3 H, s); 4.52 (2 H, s); 4.50 (1 H, s); 6.50 (1 H, d); 7.14-7.68 (8 H, m); 7.86 (2 H, s); 8.16 (1 H, m); 8.66 (1 H, d); 9.01 (1 H, d); 9.17 (1 H, d); 9.31 (1 H, s); 9.55 (1 H, s); um 11.0 (2 H, broad). 453,3 [M+H⁺]⁺

### 4-{[4-Methyl-3-(4-pyridin-3-yl-pyrimidin-2-ylamino)-phenylamino]-methyl}-benzoic acid methyl ester

A mixture of 4-formyl-benzoic acid methyl ester (930mg, 5.1 mmol) and the respective arylamine (1.34 g, 5.1 mmol) in THF (40 ml) was treated with HOAc (900 mg, 15 mmol) and NaBH(OAc)₃ (1.7g, 8 mmol). The suspension was stirred at 40°C for 16h, evaporated and the residue was partitioned between CH₂Cl₂ and an aqueous 1 N Na₂CO₃ solution. After drying of the organic phase and evaporation, the residue was purified by silica gel chromatography. Yield: 1,18g (54%), m.p. 158°C. ¹H-NMR (CDCl₃) δ/ppm: 2.25 (3 H, s); 3.90 (3 H, s); 4.44 (2 H, s); 6.32 (1 H, d); 6.99 (2 H, m); 7.13 (1 H, d); 7.41 (3 H, m); 7.59 (1 H, s); 7.99 (2 H, m); 8.32 (1 H, m); 8.44 (1 H, d); 8.71 (1 H, d); 9.26 (1 H, m).

### 4-{[4-Methyl-3-(4-pyridin-3-yl-pyrimidin-2-ylamino)-phenylamino]-methyl}-benzoic acid

m.p.: 209-210 °C ¹H-NMR (DMSO-d₆) δ/ppm: 2.06 (3 H, s); 4.31 (2 H, d); 6.15 (1 H, bs); 6.33 (1 H, m); 6.88 (2 H, m); 7.51 (3 H, m); 7.86 (2 H, m); 8.38 (2 H, m); 8.70 (2 H, m); 9.24 (1 H, m).

### (2-{[1-(4-{[4-Methyl-3-(4-pyridin-3-yl-pyrimidin-2-ylamino)-phenylamino]-methyl}-phenyl)-methanoyl]-amino}-phenyl)-carbamic acid tert-butyl ester

By coupling with (2-aminophenyl)carbamic acid tert-butyl ester 555 mg (53%) the title compound was obtained as solid with m.p. = 125°C. ¹H-NMR (CDCl₃) δ/ppm: 1.49 (9 H, s); 2.25 (3 H, s); 4.18 (1 H, bs); 4.43 (2 H, s); 6.33 (1 H, m); 7.00 (3 H, m); 7.16 (4 H, m); 7.30 (1 H, m); 7.40 (1 H, m); 7.57 (2 H, m); 7.76 (1 H, d); 7.91 (2 H, m); 8.32 (1 H, m); 8.44 (1 H, d); 8.68 (1 H, d); 9.21 (1 H, bs); 9.24 (1 H, m).

### N-(2-Amino-phenyl)-4-{[4-methyl-3-(4-pyridin-3-yi-pyrimidin-2-ylamino)-phenylamino]-methyl}-benzamide [15]

Similar to above the title compound was obtained in 82% yield as solid with m.p. 112-120°C. ¹H-NMR (DMSO-d₆) 6/ppm: 2.10 (3 H, s); 4.40 (2 H, s); 6.44 (1 H, d); 6.97 (2 H, m); 7.21 (3 H, m); 7.34 (1 H, m); 7.43 (1 H, m); 7.53 (2 H, m); 7.69 (1 H, m); 7.94 (2 H, m); 8.50 (1 H, m); 8.58 (1 H, m); 8.78 (2 H, m); 8.95-9.40 (2 H, bs, NH); 9.31 (1 H, s); 10.1 (1 H, s, NH). 502,2 [M+H⁺]⁺

### IV. Synthesis of Erlotinib Hybrides

Preparation of **6-nitroquinazolin-4(3*H*)-one** and **7-nitroquinazolin-4(3*H*)-one** according to Rachid et al. (The Combi-Targeting Concept: Chemical Dissection of the Dual Targeting Properties of a Series of "Combi-Triazenes". Journal of Medicinal Chemistry 2003, 46, 4313-4321) and to Oerfi et al. (Hegymegi-Barakonyi; Houghten, B.; Richard, A.; Keri, G. Improved, high yield synthesis of 3H-quinazoliN-4-ones, the key intermediates of recently developed drugs. Current Medicinal Chemistry 2004, 11, 2549-2553), respectively.

### 6-nitroquinazolin-4(3H)-one

(Rachid et al.; The Combi-Targeting Concept: Chemical Dissection of the Dual Targeting Properties of a Series of "Combi-Triazenes". Journal of Medicinal Chemistry 2003, 46, 4313-4321)

### 7-nitroquinazolin-4(3H)-one

(Oerfi et al.; Hegymegi-Barakonyi; Houghten, B.; Richard, A.; Keri, G. Improved, high yield synthesis of 3H-quinazoliN-4-ones, the key intermediates of recently developed drugs (Current Medicinal Chemistry 2004, 11, 2549-2553).

Preparation of ***N*-(3-ethynylphenyl)-6-nitroquinazolin-4-amine** and ***N*-(3-ethynylphenyl)-7-nitroquinazolin-4-amine** according to Nishino et al (Process for producing 4-aminoquinazoline compound by chlorination of quinazolin-4-one or its derivative and amination. 2003)

### N-(3-ethynylphenyl)-6-nitroquinazolin-4-amine:

(Nishino et al.; Process for producing 4-aminoquinazoline compound by chlorination of quinazolin-4-one or its derivative and amination. 2003)

### N-3-ethynylphenyl)-7-nitroquinazolin-4-amine:

(Nishino et al.; Process for producing 4-aminoquinazoline compound by chlorination of quinazolin-4-one or its derivative and amination. 2003)

Reduction to the compounds ***N*4-(3-Ethynyi-phenyl)-quinazoline-4,6-diamine** (Schnur and Arnold; Preparation of alkynyl- and azido-substituted 4-anilinoquinazolines for the treatment of hyperproliferative diseases. US 5747498 A 19980505, 1998) and ***N*4-(3-Ethynyi-phenyl)-quinazoline-4,7-diamine** (Schnur and Arnold; Preparation of alkynyl- and azido-substituted 4-anilinoquinazolines for the treatment of hyperproliferative diseases. US 5,747,498 A 19980505) according to literature (Rachid et al.; The Combi-Targeting Concept: Chemical Dissection of the Dual Targeting Properties of a Series of "Combi-Triazenes". Journal of Medicinal Chemistry 2003, 46, 4313-4321).

### N4-(3-Ethynyi-phenyl)-quinazoline-4,6-diamine

(Schnur and Arnold; Preparation of alkynyl- and azido-substituted 4-anilinoquinazolines for the treatment of hyperproliferative diseases. US 5747498 A 19980505)

### N4-(3-Ethynyl-phenyl)-quinazoline-4,7-diamine

(Schnur and Arnold; Preparation of alkynyl- and azido-substituted 4-anilinoquinazolines for the treatment of hyperproliferative diseases. US 5,747,498 A 19980505)

### Preparation of carboxylic acid methylesters by amidation with mono-protected phenylendiamine

The respective methoxycarbonyl aroylic acid was dissolved in dry THF or DMF and 1.1 eq. BOP (benzotriazolyloxy-tris-(dimethylamino)phosphonium-hexafluorophosphate), 2.2 eq. NEt₃, and 1.1 eq. of the respective amine were added. After stirring at room temperature for 24h, the mixture was poured into water by stirring, the precipitating product filtered off, dried in vacuum and purified by cc (CH₂Cl₂/MeOH = 10/1).

### 6-Methyl- methyl 4-(2-(tert-butoxycarbonylamino)phenylcarbamoyl)benzoate:

(Delorme et al.; Preparation of triazinyl and other carboxamides as inhibitors of histone deacetylase. US 2005/288282 A1)

### Methyl 5-(2-(tert-butoxycarbonylamino)phenylcarbamoyl)thio-phene-2-carboxylate (72)

(Fertig et al.; Preparation of new mono-acylated o-phenylendiamines derivatives as HDAC inhibitors for treating cancer. WO 2004/069803)

### Methyl 6-(2-(tert-butoxycarbonylamino)phenylcarbamoyl)-nicotinate:

Yield: 9.23 mmol (84 %), colorless crystals m.p. 171.5-173.0 °C; IR (KBr): v (cm⁻¹)= 3342, 1732, 1690. ¹H-NMR (DMSO-[D₆]): δ (ppm) = 1.51 (s, 9H), 3.94 (s, 3H), 7.20 (m, 1 H), 7.28 (m, 2H), 7.97 (dd, 1 H, J = 1.3 Hz, J = 7.8 Hz), 8.31 (m, 1 H), 8.56 (dd, 1H,J=2.1 Hz,J=8.1 Hz), 9.08 (d,1H, J = 1.5 Hz), 9.19 (s, 1 H), 10.55 (s, 1H).+ p ESI m/z (%): 372 [M+H⁺]⁺ (100). Anal. (C₁₉H₂₁N₃O₅). Calc. C 61.45, H 5.70, N 11.31; Found C 61.45, H 5.90, N 11.32.

Preparation of suitable protected and substituted carboxylic acids by alkaline cleavage of the corresponding carboxylic acid methylesters:

The respective carboxylic acid methyl esters were dissolved in MeOH and 2 eq. LiOH in H₂O were added. After stirring at room temperature over night, MeOH was removed, the aqueous layer extracted with EE, cooled to 0°C and acidified with diluted acetic acid till pH = 5-6. The precipitating product was removed by filtration and dried in vacuum.

### 4-(2-(tert-butoxycarbonylamino)phenylcarbamoyl)benzoic acid:

(Schuppan et al.; Preparation of *N*-aryl benzamides as histone deacetylase inhibitors. WO 2004058234 A2)

### 5-(2-(tert-butoxycarbonylamino)phenylcarbamoyl)thiophene-2-carboxylic acid (WO 2004/069803)

### 6-(2-(tert-butoxycarbonylamino)phenylcarbamoyl)nicotinic acid:

Yield: 8.62 mmol (88 %), colorless crystals; m.p. 358.0-361.0 °C; IR (KBr): v (cm⁻¹)= 3345, 2986,1689. ¹H-NMR (DMSO-[D₆]): δ (ppm) =1.51 (s, 9H), 7.21 (dd, 1H, J = 6.6 Hz, J = 8.2 Hz), 7.28 (m, 2H), 7.98 (m, 1 H), 8.29 (d, 1H, J = 8.2 Hz), 8.53 (dd, 1H, J = 2.0 Hz, J = 8.1 Hz), 9.07 (d, 1H, J = 1.4 Hz), 9.18 (s, 1 H), 10.55 (s, 1 H), 13.74 (s, 1H). + p ESI m/z (%): 358 [M+H⁺]⁺ (100). Anal.(C₁₈H₁₉N₃O₅ x 0.5 H₂O) Calc. C 59.01; H 5.50; N 11.47; Found C 59.16, H 5.69, N 11.48.

Preparation of the amides by amidation of the respective carboxylic acids with the respective amines:
The respective carboxylic acid was dissolved in dry pyridine and 1.1 eq. SOCl₂ or alternatively SOBr₂ were added. The mixture was stirred at room temperature for 2h, and 1.0 eq. of the respective amine was added. After stirring at room temperature for 24h, the mixture was poured into water, extracted with EE dried in vacuo and purified by cc (EE) and crystallized from methanol. In case of *tert*-butyl 2-(5-(4-(3-ethynylphenylamino)-quinazolin-6-ylcarbamoyl)picolinamido)-phenylcarbamate dihydrate, the crude product precipitated while pouring into water, the precipitating product was removed by filtration, dried in vacuo and purified by cc (DCM/MeOH = 10/1) and crystallized from methanol.

### tert-butyl 2-(5-(4-(3-ethynylphenylamino)-quinazolin-6-ylcarbamoyl)picolinamido)-phenylcarbamate dihydrate:

m.p. 163.0 °C; IR (KBr): v (cm⁻¹)= 3324, 1686. ¹H-NMR (DMSO-[D₆]): δ (ppm) = 1.52 (s, 9H), 4.22 (s, 1 H), 7.25 (m, 4H), 7.42 (t, 1 H, J = 7.9 Hz), 7.87 (d, 1 H, J = 9.0 Hz), 7.91 (d, 1 H, J = 8.2 Hz), 8.03 (m, 3H), 8.37 (d, 1 H, J = 8.3 Hz), 8.62 (s, 1 H), 8.68 (dd, 1 H, J = 2.1 Hz, J = 8.1 Hz), 8.95 (d, 1 H, J = 1.8 Hz), 9.21 (d, 1 H, J = 1.6 Hz), 9.96 (s, 1 H), 10.59 (s, 1 H), 11.02 (s, 1H). ES-MS (CH₂Cl₂/CH₃OH/CH₃COONH₄) m/z (%): 600 [M+H⁺]. Anal.(C₃₄H₂₉N₇O₄) Calc. C 64.24; H 5.23; N 15.42; Found C 64.13, H 5.39, N 15.31.

### [2-({5-[4-(3-Ethynyl-phenylamino)-quinazolin-7-ylcarbamoyl]-thiophene-2-carbonyl}-amino)-phenyl]-carbamic acid tert-butyl ester:

m.p. 203 - 208°C; ¹H-NMR (DMSO-[D₆]): δ (ppm) = 1.47 (s, 9H), 4.21 (s, 1 H), 7.16 (t, 1 H, J = 7.4 Hz), 7.23 (m, 2H), 7.41 (t, 1 H, J = 7.9 Hz), 7.52 (d, 1 H, J = 7.4 Hz), 7.58 (d, 1H, J = 7.7 Hz), 7.98 (m, 3H), 8.11 (s, 1H), 8.15 (d, 1 H, J = 3.8 Hz), 8.28 (d, 1 H, J =0.7 Hz), 8.55 (d, 1H, J = 9.1 Hz), 8.63 (s, 1 H), 8.78 (s, 1H), 9.79 (s, 1 H), 10.04(s, 1 H), 10.80 (s, 1 H).

### [2-({5-[4-(3-Ethynyl-phenylamino)-quinazolin-6-ylcarbamoyl]-thiophene-2-carbonyl}-amino)-phenyl]-carbamic acid tert-butyl ester:

m.p. 163 - 167°C; ¹H-NMR (DMSO-[D₆]): δ (ppm) = 1.47 (s, 9H), 4.21 (s, 1H), 7.17 (dd, 1 H, J = 1.6 Hz, J = 7.7 Hz), 7.24 (m, 2H), 7.41 (t, 1 H, J = 7.9 Hz), 7.50 (dd, 1 H, J = 1.6 Hz, J = 7.8 Hz), 7.58 (dd, 1 H, J = 1.4 Hz, J = 8.0 Hz), 7.85 (d, 1 H, J = 9.0 Hz), 7.90 (m,1H), 8.01 (m,2H),8.05(t, 1 H, J = 1.7 Hz), 8.15 (d, 1 H, J = 4.1 Hz), 8.61 (s, 1 H), 8.78 (s, 1 H), 8.90 (d, 1 H, J = 2.0 Hz), 9.94 (s, 1 H), 10.00 (s, 1 H), 10.80 (s, 1 H).

### [2-({5-[4-(3-Ethynyl-phenylamino)-quinazolin 7-ylcarbamoyl]-pyridine-2-carbonyl}-amino)-phenyl]-carbamic acid tert-butyl ester:

m.p. 161-166 °C; ¹H-NMR (DMSO-[D₆]): δ (ppm) = 1.52 (s, 9H), 4.22 (s, 1 H), 7.21 (dd, 2H, J = 1.3 Hz, J = 7.5 Hz), 7.27 (m, 2H), 7.42 (t, 1 H, J = 6.9 Hz), 7.94 (d, 1 H, J = 8.7 Hz), 8.02 (m, 2H), 8.11 (m, 2H), 8.36 (m, 2H), 8.56 (d, 1 H, J = 6.8 Hz), 8.65 (m, 2H), 9.17 (d, 2H, J = 1.6 Hz), 9.80 (s, 1 H), 10.58 (s, 1 H), 11.04 (s, 1 H).

### (2-{4-[4-(3-Ethynyl-phenylamino)-quinazolin-7-ylcarbamoyl]-benzoylamino}-phenyl)-carbamic acid tert-butyl ester:

m.p. 167 - 171°C; ¹H-NMR (DMSO-[D₆]): δ (ppm) =1.46 (s, 9H), 4.21 (s, 1 H), 7.20 (m, 4H), 7.42 (t, 1 H, J = 8.0 Hz), 7.58 (d, 2H, J = 7.8 Hz), 7.94 (dd, 1 H, J = 1.1 Hz, J = 8.0 Hz), 8.04 (dd, 1 H, J = 1.9 Hz, J = 9.1 Hz), 8.11 (s, 1 H), 8.17 (q, 3H, J = 8.7Hz), 8.38 (d, 1 H, J = 1.9 Hz), 8.55 (d, 1 H, J = 9.1 Hz), 8.63 (s, 1 H), 8.74 (s, 1 H), 9.78 (s, 1 H), 10.00 (s, 1 H), 10.82 (s, 1 H).

### Preparation of (2-{4-[4-(3-Ethynyl-phenylamino)-quinazolin-6-ylcarbamoyl]-benzoylamino}-phenyl)-carbamic acid tert-butyl ester by amidation of the carboxylic acid with the amine:

The carboxylic acid was dissolved in dry THF and 1.1 eq. BOP, 2.0 eq. NEt₃, and 1.1 eq. of the amine were added. After stirring at room temperature for 72 h, the mixture was poured into water extracted with EE, dried in vacuum, and purified by cc (EE).

### (2-{4-[4-(3-Ethynyl-phenylamino)quinazolin-6-ylcarbamoyl]-benzoylamino}-phenyl)-carbamic acid tert-butyl ester:

m.p. 159 - 164 °C; ¹H-NMR (DMSO-[D₆]): δ (ppm) =1.46 (s, 9H), 4.20 (s, 1H), 7.15 (m, 2H), 7.21 (m, 1 H), 7.40 (t, 1 H, J = 7.9 Hz), 7.60 (m, 3H), 7.82 (d, 1 H, J = 9.0 Hz), 7.94 (d, 1H, J = 8.2 Hz), 8.09 (s, 1 H), 8.15 (m, 4H), 8.23 (d, 2H, J = 8.4 Hz), 8.57 (s, 1 H), 9.00 (d, 1 H, J = 1.6 Hz), 10.25 (s, 1 H).

Preparation of [36], [37], [38], [39], **[41]** and [40] by cleavage of the *tert*-butyl phenylcarbamate-group:
The respective acid-tert-butyl-ester was dissolved in formic acid and stirred at room temperature for 24h. In case of [36] and [37] TFA was used instead of formic acid. The solution was poured into water by stirring and the mixture alkalized with NH₃ (pH = 9). The precipitating product was filtered off, washed with H₂O, crystallized from methanol and dried *in vacuo.*

### N²-(2-aminophenyl)-N⁵-(4-(3-ethynylphenylamino)-quinazolin-6-yl)pyridine-2,5-dicarboxamide [36]:

m.p. 213-216 °C; IR (KBr): v (cm⁻¹)= 3290,1663. ¹H-NMR (DMSO-[D₆]): δ (ppm) = 4.22 (s, 1 H), 4.97 (s, 2H), 6.67 (dt, 1 H, J = 1.4 Hz, J = 7.7 Hz), 6.85 (dd, 1 H, J = 1.3 Hz, J = 8.0 Hz), 6.99 (dt, 1 H, J = 1.5 Hz, J = 7.9 Hz), 7.24 (m, 1 H), 7.42 (t, 1 H, J = 7.9 Hz), 7.49 (dd, 1 H, J = 1.3 Hz, J = 7.9 Hz), 7.87 (d, 1 H, J = 9.0 Hz), 7.92 (m, 1 H), 8.05 (m, 2H), 8.33 (dd, 1 H, J = 0.5 Hz, J = 8.2 Hz), 8.62 (s, 1 H), 8.65 (dd, 1 H, J = 2.2 Hz, J = 8.2 Hz), 8.95 (d, 1 H, J = 2.0 Hz), 9.31 (dd, 1 H, J = 0.6 Hz, J = 2.2 Hz), 9.96 (s, 1 H), 10.20 (s, 1 H), 11.01 (s, 1H). ES-MS (CH₂Cl₂/CH₃OH/CH₃COONH₄) m/z (%): 500 [M+H⁺].

### N-(2-Amino-phenyl)-N'-[4-(3-ethynyl-phenylamino)-quinazolin-6-yl]-terephthalamide [37]:

m.p. 253 - 258 °C; ¹H-NMR (DMSO-[D₆]): δ (ppm) = 4.22 (s, 1 H), 4.98 (s, 2H), 6.62 (t, 1 H, J = 7.4 Hz), 6.80 (d, 1 H, J = 7.1 Hz), 7.00 (dt, 1 H, J = 1.1 Hz, J = 7.8 Hz), 7.22 (m, 2H), 7.41 (t, 1 H, J = 7.9 Hz), 7.85 (d, 1 H, J = 8.9 Hz), 7.92 (d, 1 H, J = 8.1 Hz), 8.06 (m, 2H), 8.18 (s, 4H), 8.61 (s, 1 H), 8.94 (d, 1H, J = 1.5 Hz), 9.85 (s, 1 H), 9.93 (s, 1 H), 10.78 (s, 1 H).

### Thiophene-2,5-dicarboxylic acid 2-[(2-amino-phenyl)-amide]-5-{[4-(3-ethynyl-phenylamino)-qumazolin-6-yl]-amide} [38]:

m.p. 247 - 249 °C; ¹H-NMR (DMSO-[D₆]): δ (ppm) = 4.21 (s, 1 H), 5.00 (s, 2H), 6.61 (dt, 1 H, J = 1.1 Hz, J = 7.6 Hz), 6.80 (dd, 1 H, J = 1.0 Hz, J = 8.0 Hz), 7.01 (m, 1 H), 7.15 (dd, 1 H, J = 0.8 Hz, J = 7.7 Hz), 7.23 (d, 1 H, J = 7.6 Hz), 7.41 (t, 1 H, J = 7.9 Hz), 7.85 (d, 1H, J = 8.9 Hz), 7.91 (d, 1H, J = 8.1 Hz), 8.02 (dd, 1H, J = 1.9 Hz, J = 9.1 Hz), 8.05 (m, 2H), 8.12 (d,1H,J= 4.0 Hz), 8.61 (s, 1 H), 8.90 (d,1H.J= 1.3 Hz), 9.89 (s, 1 H), 9.94 (s, 1 H), 10.78 (s, 1 H).

### Pyridine-2,5-dicarboxylic acid 2-[(2-amino-phenyl)-amide]-5-{[4-(3-ethynyl-phenylamino)quinazolin-7-yl]-amide} [39]:

m.p. 249 - 252 °C; ¹H-NMR (DMSO-[D₆]): δ (ppm) = 4.22 (s, 1 H), 4.96 (s, 2H), 6.67 (t, 1 H, J = 7.5 Hz), 6.85 (d, 1 H, J = 7.8 Hz), 6.99 (t, 1 H, J = 7.5 Hz), 7.24 (d, 1 H, J = 7.5 Hz), 7.42 (t, 1H, J = 7.9 Hz), 7.50 (d, 1H, J = 7.7 Hz), 7.94 (d, 1H, J = 8.1 Hz), 8.02 (dd, 1 H, J = 1.3 Hz, J = 8.9 Hz), 8.11 (m, 1 H), 8.32 (d, 1 H, J = 8.2 Hz), 8.36 (d, 1 H, J = 1.6 Hz), 8.57 (d, 1 H, J = 9.1 Hz), 8.62 (m, 2H), 9.28 (d, 1 H, J = 1.2 Hz), 9.80 (s, 1 H), 10.20 (s, 1 H), 11.03 (s, 1 H).

### N-(2-Amino-phenyl)-N-[4-(3-ethynyl-phenylamino)-quinazolin-7-yl]-terephthalamide [41]:

m.p. 240 - 246 °C; ¹H-NMR (DMSO-[D₆]): δ (ppm) = 4.22 (s, 1 H), 4.98 (s, 2H), 6.62 (t, 1 H, J = 7.4 Hz), 6.80 (dd, 1 H, J = 0.8 Hz, J = 7.9 Hz), 7.00 (dt, 1 H, J = 1.3 Hz, J = 7.9 Hz), 7.21 (m, 2H), 7.42 (t, 1H, J = 7.9 Hz), 7.94 (d, 1H, J = 8.2 Hz), 8.04 (dd, 1H, J = 2.0 Hz, J = 9.1 Hz), 8.11 (m, 1 H), 8.17 (m, 5H), 8.38 (d, 1 H, J = 1.9 Hz), 8.55 (d, 1 H, J = 9.0 Hz), 8.62 (s, 1 H), 9.89 (s, 1H), 10.62 (s, 1H).

### Thiophene-2,5-dicarboxylic acid 2-[(2-amino-phenyl)-amide] 5-{[4-(3-ethynyl-phenylamino)-quinazolin-7-yi]-amidel [40]:

m.p. 220 - 228°C; ¹H-NMR (DMSO-[D₆]): δ (ppm) = 4.22 (s, 1 H), 5.02 (s, 2H), 6.61 (dt, 1 H, J = 1.2 Hz, J = 7.7 Hz), 6.80 (dd, 1 H, J = 1.2 Hz, J = 8.0 Hz), 7.01 (dt, 1 H, J = 1.4 Hz, J = 7.8 Hz), 7.15 (dd, 1 H, J = 1.1 Hz, J = 7.8 Hz), 7.23 (m, 1 H), 7.42 (t, 1 H, J = 7.9 Hz), 7.93 (dd, 1 H, J = 1.5 Hz, J = 7.8 Hz), 8.01 (dd, 1 H, J = 1.8 Hz, J = 9.2 Hz), 8.05 (d, 1 H, J = 3.9 Hz), 8.11 (m, 2H), 8.28 (d, 1 H, J = 2.0 Hz), 8.55 (d, 1 H, J = 9.2 Hz), 8.62 (s, 1H),9.79 (s, 1 H), 9.90 (s, 1 H), 10.77 (s, 1H).

### 6-Methoxyquinazolin-4(3H)-one:

according to Nishino et al. (Process for producing 3,4-dihydroquinazolin-4-one derivatives. 2003)

### 6-Methoxyquinazolin-4(3H)-one:

(Takase et al.; Cyclic GMP Phosphodiesterase Inhibitors. 2. Requirement of 6-Substitution of Quinazoline Derivatives for Potent and Selective Inhibitory Activity. Journal of Medicinal Chemistry 1994, 37)

### N-(3-bromophenyl)-6-methoxyquinazolin-4-amine:

according to Nishino et al. (Process for producing 4-aminoquinazoline compound by chlorination of quinazolin-4-one or its derivative and amination. 2003);

### N-(3-bromophenyl)-6-methoxyquinazolin-4-amine:

(Nishino et al.; Process for producing 4-aminoquinazoline compound by chlorination of quinazolin-4-one or its derivative and amination, 2003)

### 4-(3-bromophenylamino)quinazolin-6-ol:

Preparation analogous to a modified procedure described by Barker (Quinazoline derivatives. EP 0 566 226 A1, 1993) as follows: A mixture of N-(3-bromophenyl)-6-methoxyquinazolin-4-amine (8.66g; 26.23 mmol), sodium ethanethiolate (22.06 g; 262.3 mmol) and DMF was stirred and heated to 140°C for 8 hours. The solvent was removed in vacuum, the remaining solid dissolved in water (400 mL), the aqueous layer extracted with ethyl acetate (2 x 150 mL), acidified with acetic acid till pH = 5 and the precipitating product removed by filtration. There was thus obtained the title compound (5.67 g; 68%) as a light brownish solid.

### 4-(3-bromophenylamino)quinazolin-6-ol:

m.p. 297 - 300°C. ¹H-NMR (DMSO-[D₆]): δ (ppm) = 7.27 (m, 1 H), 7.34 (t, 1 H, J = 8.0 Hz), 7.45 (dd, 1 H, J = 2.5 Hz, J = 9.0 Hz), 7.71 (d, 1 H, J = 9.0 Hz), 7.80 (d, 1 H, J = 2.5 Hz), 7.94 (ddd, 1 H, J = 1.2 Hz, J = 1.9 Hz, J = 8.0 Hz), 8.28 (t, 1 H, J = 1.9 Hz), 8.52 (s, 1 H), 9.59 (s, 1 H), 10.12 (s, 1 H).

### Preparation of tert-butyl 2-(4-(bromomethyl)benzamido)phenylcarbamate:

4-(Bromomethyl)benzoic acid (3.0g; 13.95 mmol) (Acros) was dissolved in 20.0 mL SOCI2 and the mixture refluxed for half an hour. The excess of thionyl chloride was removed in vacuum, the resulting carboxylic acid chloride dissolved in methylene chloride (15.0 mL), *tert*-butyl 2-aminophenylcarbamate (2.91 g; 13.95 mmol) and pyridine (3.56 mL; 16.74 mmol) were added and the mixture stirred at room temperature for half an hour. The mixture was directly subjected to cc (SiO₂ CH₂Cl₂, ethyl acetate 10:1) without work up, thus obtaining the title compound (1.73g; 31 %) as colourless crystals after crystallisation from CH₂Cl₂/hexane.

### tert-butyl 2-(4-(bromomethyl)benzamido)phenylcarbamate:

m.p. 282 - 283 °C; IR (KBr): v (cm⁻¹)= 3327, 1656. ¹H-NMR (DMSO-[D₆]): δ (ppm) = 1.45 (s, 9H), 4.82 (d, 2H, J = 20.5 Hz), 7.18 (m, 2H), 7.55 (m, 2H), 7.61 (dd, 2H, J = 1.8 Hz, J = 8.3 Hz), 7.96 (dd, 2H, J = 6.6 Hz, J = 8.2 Hz), 8.69 (s, 1 H), 9.85 (s, 1 H).

### tert-butyl 2-(4-((4-(3-bromophenylamino)quinazolin-6-yloxy)methylr benzamido)phenylcarbamate:

4-(3-Bromophenylamino)quinazoli*N*-6-ol (1.50g; 3.17 mmol) was dissolved in DMF (15.0 mL), the solution cooled to 0°C and NaH (0.14g, 60% in paraffin; 3.49 mmol) was added by stirring. After half an hour *tert*-butyl 2-(4-(bromomethyl)benzamido)phenylcarbamate (1.41 g; 3.49 mmol) was added and the mixture stirred for 48 h at room temperature. The solution was poured into water (100 mL) by stirring the precipitating product collected by filtration, dried in vacuum and purified by cc (SiO₂, CH₂Cl₂, ethyl acetate 1:2). The solid which remained after removal of the solvent was crystallized by dissolving in a small amount of CH₂Cl₂ and drop wise adding to diethyl ether by stirring, thus obtaining the title compound (1.24 g; 61 %) as colourless crystals.

### tert-butyl 2-(4-((4-(3-bromophenylamino)quinazolin-6-yloxy)methyl)benzamido) phenyl-carbamate:

m.p. 208 - 210 °C; IR (KBr): v (cm⁻¹)= 3251, 1652. ¹H-NMR (DMSO-[D₆]): δ (ppm) = 1.44 (s, 9H), 5.40 (s, 2H), 7.19 (m, 2H), 7.32 (m, 1 H), 7.39 (t, 1H, J = 8.0 Hz), 7.56 (ddd, 2H, J = 1.7 Hz, J = 6.2 Hz, J = 8.0 Hz); 7.65 (dd, 1 H, J = 2.5 Hz, J = 9.1 Hz), 7.72 (d, 1 H, J = 8.3 Hz), 7.81 (d, 1 H, J = 9.1 Hz), 7.95 (m, 1 H), 8.03 (d, 2H, J = 8.2 Hz), 8.11 (d, 1 H, J = 2.5 Hz), 8.22 (t, 1 H, J = 1.9 Hz), 8.60 (s, 1 H), 8.70 (s, 1 H), 9.69 (s, 1 H), 9.86 (s, 1H). ES-MS (CH₂Cl₂/CH₃OH/CH₃COONH₄) m/z (%): 640 [M+H⁺]. Anal.(C₃₃H₃₀BrN₅O₄) Calc. C 61.88; H 4.72; N 10.93; Found C 61.83, H 4.84, N 10.81.

### N-(2-aminophenyl)-4-((4-(3-bromophenylamino)quinazolin-6-yloxy)-methyl)benzamide [43]:

*tert*-butyl 2-(4-((4-(3-bromophenylamino)quinazolin-6-yloxy)methyl)benzamido) phenylcarbamate (1.24 g; 19.35 mmol) was dissolved in trifluoro acetic acid (10.0 mL) and the solution stirred for 1 h at room temperature. The solution was poured into water by stirring, the mixture alkalized with conc. NH₃ till pH = 9, the precipitating product removed by filtration, washed with water and dried in vacuum. Purification by cc (SiO₂, ethyl acetate), removing most of the solvent under reduced pressure and storage of the solution at -18°C over night led to colourless crystals (0.75 g; 72 %).

### N-(2-aminophenyl)-4-((4-(3-bromophenylamino)quinazolin-6-yloxy)-methyl)benzamide [43]:

m.p. 215-218 °C; IR (KBr): v (cm⁻¹)= 3286, 1643.¹H-NMR (DMSO-[D₆]): δ (ppm) = 4.93 (s, 2H), 5.38 (s, 2H), 6.61 (dt, 1H, J = 1.2 Hz, J = 7.8 Hz), 6.79 (dd, 1H, J = 1.3 Hz, J = 8.0 Hz), 6.98 (dt, 1H, J = 1.5 Hz, J = 7.9 Hz), 7.18 (dd, 1H, J = 0.8 Hz, J = 8.1 Hz), 7.32 (m, 1 H), 7.39 (t, 1H, J = 8.0 Hz), 7.64 (d, 1H, J = 2.5 Hz), 7.69 (d, 2H, J = 8.6 Hz), 7.81 (d, 1H,J=9.1 Hz), 7.94 (m, 1 H), 8.05 (d,1H,J= 8.2 Hz), 8.12 (d, 1H, J = 2.5 Hz), 8.22 (t, 1 H, J = 1.9 Hz), 8.59 (s, 1 H), 9.70 (d, 2H, J = 2.1 Hz). EI-MS (70eV) *mlz* (%): 539 [M^{+.}] (2).

### N-(2-aminophenyl)-4-((4-(3-ethynylphenylamino)quinazolin-6-yloxy)methyl)benzamide [44]:

A mixture of *N*-(2-aminophenyl)-4-((4-(3-bromophenylamino)quinazolin-6-yloxy)methyl)benzamide [43] (0.54 g; 1.0 mmol), bis(triphenylphoshine)palladium(II)chloride (0.35g; 0.5 mmol), copper (I)iodide (95 mg; 0.5 mmol), trimethylsilylacetylene (1.0 mL; 7.22 mmol) and triethylamine (5.0 mL) in 1-methyl-2-pyrrolidinone (10 mL) was stirred in a sealed tube under nitrogen atmosphere at 40 °C for 24 hours. The reaction mixture was then cooled to room temperature and poured into water in a separatory funnel and the aqueous layer extracted with ethyl acetate (3 x 50 mL).The combined organic layers were washed with water (3x), dried (Na₂SO₄), filtered and the solvent was removed in vacuum. The resulting intermediate, N-(2-aminophenyl)-4-((4-(3-((trimethylsilyl)ethynyl)phenylamino)quinazolin-6-yloxy)methyl)benzamide, was purified by cc (SiO₂ ethyl acetate) (0.22 g, 39 %) and the coupled product characterized by ¹H-NMr spectroscopy. The TMS protected alkyne was in the following dissolved in THF (15 mL), MeOH (15 mL) and K₂CO₃ (60 mg, 0.43 mmol) were added and the mixture stirred over night at room temperature. The mixture was filtered, and the residue passed through a plug of silica gel by washing with THF and the solvent removed to afford the terminal alkyne (0.18) in an overall yield of 37 %.

### N-(2-aminophenyl)-4-((4-(3-ethynylphenylamino)-quinazolin-6-yloxy)methyl)benzamide [44]:

m.p. 164-168 °C; ¹H-NMR (DMSO-[D₆]): δ (ppm) = 4.23 (s, 1 H), 4.92 (s, 2H), 5.38 (s, 2H), 6.60 (dt, 1 H, J = 1.2 Hz, J = 7.8 Hz), 6.79 (dd, 1 H, J = 1.2 Hz, J = 8.0 Hz), 6.98 (dt, 1H, J = 1.4 Hz, J = 7.8 Hz), 7.18 (m, 1 H), 7.24 (td, 1H, J = 1.1 Hz, J = 7.6 Hz), 7.44 (t, 1 H, J = 7.9 Hz), 7.64 (dd, 1 H, J = 2.4 Hz, J = 9.1 Hz), 7.69 (d, 2H, J = 8.2 Hz), 7.79 (d, 1H, J=9.1 Hz), 7.95 (m, 1 H), 8.05 (m, 2H), 8.13 (d, 1 H, J = 2.3 Hz), 8.57 (s, 1H), 9.70 (s, 2H).

### Quinazolin-4(3H)-one:

according to Nishino et al. (Process for producing 4-aminoquinazoline compound by chlorination of quinazolin-4-one or its derivative and amination, 2003)

### Quinazolin-4(3H)-one:

(Nishino et al.; Process for producing 4-aminoquinazoline compound by chlorination of quinazolin-4-one or its derivative and amination, 2003)

### 4-Oxo-3,4-dihydroquinazoline-6-sulfonic acid:

was prepared as described by Maillard et al (Derives de La (3H) quinazoline-4-doues de proprietes anti-inflammatoires II. - Derives substitutes dans le noyau aromatique et produits voisins. Chimie Therapeutique 1967, 4)

### 4-Oxo-3,4-dihydroquinazoline-6-sulfonic acid:

(Maillard et al.; Derives de La (3H) quinazoline-4-doues de proprietes anti-inflammatoires II. - Derives substitutes dans le noyau aromatique et produits voisins. Chimie Therapeutique 1967, 4)

### 4-Oxo-3,4-dihydroquinazoline-6-sulfonyl chloride:

A mixture of 2.5g (12 mmol) of 4-oxo-3,4-dihydroquinazoline-6-sulfonic acid and 1 mL DMF in 100 mL of thionyl chloride was refluxed for 7h and stirred overnight at RT. The excess of thionyl chloride was distilled off in vacuo, the residue was suspended in CH₂Cl₂, filtered off and dried in a steam of air.

### 4-Oxo-3,4-dihydroquinazoline-6-sulfonyl chloride:

Yield: 8.1 mmol from 12.0 mmol (69 %), beige solid; m.p. > 400.°C; IR (KBr): v (cm⁻¹)= 3428,2623,1712. ¹H-NMR (DMSO-[D₆]): δ (ppm) = 9.07 (s, 1 H), 8.34 (d, J = 1.9 Hz, 1 H), 8.13 (dd, J1 = 8.3 Hz, J2 = 1.9 Hz, 1H), 7.77 (d, J = 8.3 Hz, 1H). ES-MS (CH₂Cl₂, CH₃OH CH₃COONH₄) m/z (%): 243 [M-H⁺]⁻ (100). Anal.(C₈H₅ClN₂O₃S x 1.1 H₂O) Calc. C 36.33, H 2.74, N 10.59; Found C 36.09, H 2.84, N 10.94.

### Preparation of 3-(1H-Pyrrol-3-yl)acrylic acid ethyl ester:

according to Karousis et al. (Synthesis of (E)-3-(1H-pyrrol-3-yl)prop-2-ene derivatives using organophosphorous reagents. Synthesis 2006, 9, 1494 - 1498).

### 3-(1H-Pyrrol-3-yl)acrylic acid ethyl ester

(Karousis et al.; Synthesis of (E)-3-(1H-pyrrol-3-yl)prop-2-ene derivatives using organophosphorous reagents. Synthesis 2006, 9,1494 - 1498)

### 3-[1-(4-Oxo-3,4-dihydroquinazoline-6-sulfonyl)-1H-pyrrol-3-yl]acrylic acid ethyl ester:

0.8 g (5 mmol) 3-(1*H*-pyrrol-3-yl)acrylic acid ethyl ester was dissolved in 20 mL THF. The mixture was stirred at -20°C after addition of 0.3 g (8.0 mmol) NaH (60% in paraffin) for 1 h. Then 1.22g (0.5 mmol) 4-oxo-3,4-dihydroquinazoline-6-sulfonyl chloride was added in small portions and the mixture was stirred over night while warming up to RT. Afterwards, the solution was treated with sat. aq. NH₄Cl and extracted with ethyl acetate, dried over Na₂SO₄ and subjected to CC (SiO₂, ethyl acetate).

### 3-[1-(4-Oxo-3,4-dihydroquinazoline-6-sulfonyl)-1H-pyrrol-3-yl]acrylic acid ethyl ester:

Yield:1.15 mmol from 5 mmol (23 %), beige solid; m.p. 207.4 - 209.6 °C; IR (KBr): v (cm⁻¹)= 3431, 2868, 1685. ¹H-NMR (DMSO-[D₆]): δ (ppm) =8.60 (d, J = 2.5 Hz, 1 H), 8.33-8.26 (m, 2H), 7.96-7.92 (m, 1 H), 7.87 (d, J = 8.7 Hz, 1 H), 7.54-7.44 (m, 2H), 6.87-6.83 (m, 1 H), 6.35 (d, J = 15.9 Hz, 1 H), 4.12 (q, J = 7.1 Hz, 2H), 1.20 (t, J = 7.1 Hz, 3H). Cl-MS (NH₃) *m*/*z* (%): 391 [M+NH₄⁺] (39), 374 [MH⁺] (2). Anal.(C₁₇H₁₅N₃O₅S x 0.05 CH₂Cl₂) Calc. C 54.23, H 4.03, N 11.13; Found C 54.17, H 4.23, N 11.27.

### 3-{1-[4-(3-Ethynylphenylamino)quinazoline-6-sulfonyl]-1H-pyrrol-3-yl}acrylic acid ethyl ester:

A mixture of 1.0g (2.6mmol) 3-[1-(4-oxo-3,4-dihydroquinazoline-6-sulfonyl)-1*H*-pyrrol-3-yl]acrylic acid ethyl ester, 0.5g POCl₃, 0.33g triethylamine and 1 mL toluene was stirred for 2h at 75°C. After cooling down to RT 0.8 mL 4-methyl-2-oxopentane was added together with 0.4g 3-ethynylaniline and the mixture was stirred for 1 h at 70 °C. The resulting precipitate was filtered off and the residue was stirred for 30 min in 15 mL of 1 M NaOH aq. The product was filtered off and dried in vacuo.

### 3-{1-[4-(3-Ethynylphenylamino)quinazoline-6-sulfon-yl]-1H-pyrrol-3-yl}acrylic acid ethyl ester:

Yield: 2.1 mmol from 2.6 mmol (78 %), beige solid; m.p. 153.5 - 156.2 °C; IR (KBr): v (cm⁻¹)= 3424, 3285, 1707. ¹H-NMR (DMSO-[D₆]): δ (ppm) = 10.50 (s, 1 H), 9.42 (s, 1 H), 8.71 (s, 1 H), 8.25 (dd; J₁ = 8.8 Hz, J₂ = 1.9 Hz, 1 H), 8.05-7.82 (m, 4H), 7.55-7.38 (m, 3H), 7.30 (d, J = 7.4 HZ, 1 H), 6.90-6.84 (m, 1 H), 6.35 (d, J = 15.9 Hz, 1 H), 4.25 (s, 1 H), 4.12 (q, J = 7.1 Hz, 2H), 1.20 (t, J = 7.1 Hz, 3H). CI-MS (NH₃) *m*/*z* (%): 473 [MH⁺] (14), 183 (100). Anal. (C₂₅H₂₀N₄O₄S x 1.1 H₂O) Calc. C 60.99, H 4.54, N 11.38; Found C 61.05, H 4.60, N 11.25.

### 3-{1-[4-(3-Ethynylphenylamino)quinazoline-6-sulfonyl]-1H-pyrrol-3-yl}acrylic acid:

A mixture of 470 mg (1.0 mmol) 3-{1-[4-(3-ethynylphenylamino)quinazoline-6-sulfonyl]-1*H*-pyrrol-3-yl}acrylic acid ethyl ester, 40 mg LiOH in 10 mL THF and 5mL H₂O was stirred for 15h at 75°C. After cooling down to RT, 150 mg acetic acid was added and the resulting precipitate was collected by filtration. The solid was treated with THF while the product was dissolved and the side product 4-(3-ethynylphenylamino)quinazoline-6-sulfonic acid remained as a solid and was removed by filtration. The THF-solution was directly subjected to CC (SiO₂ ethyl acetate).

### 3-{1-[4-(3-Ethynylphenylamino)quinazoline-6-sulfonyl]-1H-pyrrol-3-yl}acrylic acid:

Yield: 0.25 mmol from 1.0 mmol (25 %), beige solid; m.p. 286.4 - 289.0 °C; IR (KBr): v (cm⁻¹)= 3420, 3295, 1668. ¹H-NMR (DMSO-[D₆]): δ (ppm) = 12.30 (bs, 1 H), 10.45 (s, 1 H), 9.41 (s, 1 H), 8.72 (s, 1 H), 8.25 (dd, J1 = 8.8 Hz, J2 = 1.9 Hz, 1 H), 8.03-7.79 (m, 4H), 7.50-7.38 (m, 3H), 7.30 (d, J = 8.5 Hz, 1 H), 6.87-6.81 (m, 1 H), 6.25 (d, J = 15.9 Hz, 1 H), 4.25 (s, 1 H). CI-MS (NH₃) *m*/*z* (%): 445 [MH⁺] (12), 155 (100). Anal.(C₂₃H₁₆N₄O₄S x 0.66 H₂O) Calc. C 60.52, H 3.83, N 12.27; Found C 60.39, H 3.68, N 12.44.

### [2-(3-{1-[4-(3-Ethynylphenylamino)quinazoline-6-sulfonyl]-1H-pyrrol-3-yl}acryloylamino)phenyl]carbamic acid tert-butyl ester:

220 mg (0.5 mmol) 3-{1-[4-(3-ethynylphenylamino)quinazoline-6-sulfonyl]-1*H*-pyrrol-3-yl}acrylic acid was dissolved together with 100 mg *tert.*-butyl-2-aminophenyl carbamate in 7.5mL acetonitrile and 2 mL DMF. After addition of 220 mg BOP and 200 mg triethylamine the solution was stirred over night, concentrated in vacuo, treated with water and filtered off. The collected solid was dried in vacuo.

### [2-(3-{1-[4-(3-Ethynylphenylamino)quinazoline-6-sulfonyl]-1H-pyrrol-3-yl}acryloylamino)phenyl]carbamic acid tert-butyl ester:

Yield: 0.44 mmol from 0.50 mmol (89 %), beige solid; m.p. 146.2 - 149.8 °C; IR (KBr): v (cm⁻¹)= 3288, 1781, 1729. ¹H-NMR (DMSO-[D₆]): δ (ppm) = 10.50 (s, 1 H), 9.60 (s, 1 H), 9.45 (s, 1 H), 8.76 (s, 1 H), 8.30 (d, J = 7 Hz, 1 H), 8.05-7.80 (m, 6H), 7.63-7.40 (m, 5H), 7.34 (d, J = 7 Hz, 1 H), 7.10 (m, 1 H), 6.70 (d, 3.5 Hz, 1 H), 6.58 (d, J = 12 Hz, 1 H), 4.27 (s, 1 H), 1.40 (s, 9H). ES-MS (CH₂Cl₂, CH₃OH, CH₃COONH₄) *mlz* (%): 635 [M+H]⁺ (100). Anal.(C₃₄H₃₀N₆O₅S x DMF) Calc. C 62.79, H 5.27, N 13.85; Found C 62.11, H 4.76, N 14.49.

### N-(2-Amino-phenyl)-3-{1-[4-(3-ethynylphenylamino)quinazoline-6-sulfonyl]-1H-pyrrol-3-yl}acrylamide [46]:

450 mg (0.7 mmol) [2-(3-{1-[4-(3-ethynylphenylamino)quinazoline-6-sulfonyl]-1*H-*pyrrol-3-yl}acryloylamino)phenyl]carbamic acid *tert*-butyl ester was dissolved in 2 mL formic acid and stirred over night. The solution was poured into sat aq.NaCl and neutralized with aq ammonia. The resulting precipitate was filtered off, dissolved in ethyl acetate and subjected to CC (SiO₂, ethyl acetate).

### N-(2-Amino-phenyl)-3-{1-[4-(3-ethynylphenylamino)quinazoline-6-sulfonyl]-1H-pyrrol-3-yl}acryl-amide [46]:

Yield: 0.13 mmol from 0.7 mmol (18 %), beige solid; m.p. 149.5 - 153.2 °C; IR (KBr): v (cm⁻¹)= 3419, 1619. ¹H-NMR (DMSO-[D₆]): δ (ppm) = 10.45 (s, 1 H), 9.43 (d, J = 1.9 Hz, 1H), 9.26 (s, 1 H), 8.74 (s, 1 H), 8.28 (dd, J1 = 8.8 Hz, J2 = 1.9 Hz, 1 H), 8.02-7.87 (m, 3H), 7.79-7.77 (m, 1 H), 7.50-7.26 (m, 5H), 6.92-6.86 (m, 1 H), 6.75-6.51 (m, 4H), 4.90 (bs, 2H), 4.25 (s, 1 H). ES-MS (CH₂Cl₂, CH₃OH, CH₃COONH₄) m/z (%): 535 [M+H]⁺ (100). Anal.(C₂₉H₂₂N₆O₃S x HCl) Calc. C 61.00, H 4.06, N 14.72; Found C 61.29, H 3.80, N 15.19.

### 3-{1-[4-(3-Ethynylphenylamino)quinazoline-6-sulfonyl]-1H-pyrrol-3-yl}-N-

### (tetrahydropyran-2-yloxy)acrylamide:

220 mg (0.5 mmol) 3-{1-[4-(3-ethynylphenylamino)quinazoline-6-sulfonyl]-1*H*-pyrrol-3-yl}acrylic acid was dissolved together with 60 mg O-(tetrahydro-2*H*-pyran-2-yl)hydroxylamine in 7.5mL acetonitrile and 2.5 mL DMF. After addition of 200 mg BOP and 200 mg triethylamine the mixture was stirred over night. Volatiles were removed in vacuo and the residue was treated with water. The resulting precipitate was collected by filtration and dried in vacuo.

### 3-{1-[4-(3-Ethynylphenylamino)quinazoline-6-sulfonyl]-1H-pyrrol-3-yl}-N-(tetrahydro-pyran-2-yloxy)acrylamide:

Yield: 0.4 mmol from 0.5 mmol (82 %), beige solid; m.p. 152.0 - 155.3 °C; IR (KBr): v (cm⁻¹)= 3422, 3289, 1661. ¹H-NMR (DMSO-[D₆]): δ (ppm) = 11.12 (s, 1 H), 10.45 (s, 1 H), 9.40 (s, 1 H), 8.72 (s, 1 H), 8.26 (dd, J₁ = 8.8 Hz, J₂ = 1.9 Hz, 1 H), 8.05-7.72 (m, 4H), 7.50-7.25 (m, 4H), 6.62 (s, 1 H), 6.20 (d, J = 15.9 Hz, 1 H), 4.85 (s, 1 H), 4.25 (s, 1 H), 3.98-3.85 (m, 1 H), 3.57-3.44 (m, 1 H), 1.75-1.40 (m, 6H). ES-MS (CH₂Cl₂, CH₃OH, CH₃COONH₄) *mlz* (%): 544 [M+H]⁺ (100). Anal.(C₂₈H₂₅N₅O₅S x 1.25 H₂O) Calc. C 59.41, H 4.90, N 12.37; Found C 59.00, H 5.08, N 12.93.

### 3-{1-[4-(3-Ethynylphenylamino)quinazoline-6-sulfonyl]-1H-pyrrol-3-yl}-N-hydroxy-acrylamide [45]:

250 mg (0.45 mmol) 3-{1-[4-(3-ethynylphenylamino)quinazoline-6-sulfonyl]-1*H*-pyrrol-3-yl}-*N*-(tetrahydropyran-2-yloxy)acrylamide was dissolved in 20 mL of 0.35N HCl in methanole and stirred over night. The solution was concentrated in vacuo and the residue washed with diethylether.

### 3-{1-[4-(3-Ethynylphenylamino)quinazoline-6-sulfonyl]-1H-pyrrol-3-yl}-N-hydroxy-acrylamide [45]:

Yield: 0.37 mmol from 0.45 mmol (82 %), light brown solid; m.p. 184.4 °C (decomp.); IR (KBr): v (cm⁻¹)= 3425, 3271, 2542, 1612. ¹H-NMR (DMSO-[D₆]): δ (ppm) = 12.50 (bs, 1 H), 10.33 (bs, 1 H), 9.89 (s, 1 H), 9.00 (s, 1 H), 8.80 (bs, 1 H), 8.52 (d, J = 12 Hz, 1 H), 8.14 (d, J = 12 Hz, 1 H), 7.91-7.79 (m, 3H), 7.61-7.46 (m, 3H), 7.28 (d, J = 19 Hz, 1 H), 6.60 (s, 1 H), 6.19 (d, J = 20 Hz, 1 H), 4.30 (s, 1H). ES-MS (CH₂Cl₂, CH₃OH, CH₃COONH₄) *m*/*z* (%): 460 [M+H]⁺ (95). Anal.(C₂₃H₁₇N₅O₄S x 2 HCl x 1.5 H₂O) Calc. C 49.38, H 3.96, N 12.52; Found C 49.04, H 4.36, N 13.11.

Suitable salts for compounds of formula I according to this invention are acid addition salts or salts with bases. Particular mention may be made of the pharmacologically tolerable inorganic and organic acids and bases customarily used in pharmacy. Those suitable are, on the one hand, water-insoluble and, particularly, water-soluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid or 3-hydroxy-2-naphthoic acid, the acids being employed in salt preparation - depending on whether a mono- or polybasic acid is concerned and depending on which salt is desired - in an equimolar quantitative ratio or one differing therefrom.

On the other hand, salts with bases are - depending on substitution - also suitable. As examples of salts with bases are mentioned the lithium, sodium, potassium, calcium, aluminium, magnesium, titanium, ammonium, meglumine or guanidinium salts, here, too, the bases being employed in salt preparation in an equimolar quantitative ratio or one differing therefrom.

Pharmacologically intolerable salts, which can be obtained, for example, as process products during the preparation of the compounds of formula I according to this invention on an industrial scale, are converted into pharmacologically tolerable salts by processes known to the person skilled in the art.

According to expert's knowledge the compounds of formula I according to this invention as well as their salts may contain, e.g. when isolated in crystalline form, varying amounts of solvents. Included within the scope of the invention are therefore all solvates and in particular all hydrates of the compounds of formula I according to this invention as well as all solvates and in particular all hydrates of the salts of the compounds of formula I according to this invention.

Some of the compounds and salts according to the invention may exist in different crystalline forms (polymorphs), which are within the scope of the invention.

### Biological Investigations

### Isolation of nuclear extract HDAC activity

The purification of HDAC activity was done by using nuclei isolated from the cervical carcinoma cell line HeLa (ATCC CCL2) according to a modified method first described by Dignam et al. (Nucl. Acids Res. 11, pp1475, 1983). HeLa nuclei as provided by CIL SA, Belgium (Art. No. CC013050) and stored at -80°C were thawed on ice. By centrifugation (Sorvall SS34, 20 min at 20.000 xg, 4°C), the nuclei were pelleted and the supernatant discarded. 1 e 9 nuclei were resuspended in 3ml of buffer C (20 mM HEPES pH 7.9, 25 vol% glycerol, 0.42 M NaCl, 1.5 mM MgCl₂, 0.2 mM EDTA, 0.5 mM PefaBloc, 0.5 mM DTT) and stirred for 30min on ice. After centrifugation at 20.000xg / 4°C, the supernatant was dialysed against buffer D (40 mM Tris-HCl pH 7.4, 100 mM KCl, 0.2 mM EDTA, 0.5 mM DTT, 25 vol% glycerol) for 5h at 4°C. The dialysed solution containing nuclear HDAC activity was centrifuged (20 min, 20.000 x g, 4°C) and the supernatant, after aliquotation, frozen at -80°C.

### Isolation of rHDAC1

Human HDAC1 (Taunton et al., Science 272 (5260), p 408-11, 1996) fused with the flag epitope is stably expressed in Hek293 cells. After mass cultivation in DMEM with supplements and 2% fetal calf serum, cells are lysed and flag-HDAC1 purified by M2-agarose affinity chromatography as described (Sigma Art. No. A-2220). Fractions from the purification are analysed by Western blot as well as for enzymatic activity as described below.

### Fluorimetric HeLa nuclear extract HDAC and rHDAC1 activity assay

The HDAC enzyme activity assay was done as described by Wegener et al. (Chem. & Biol. 10, 61-68, 2003, Beckers et al. Int J Cancer 121, pp1138, 2007). Briefly 40 µl of a rHDAC1 dilution (about 10 ng/well rHDAC1) or a 1:100 dilution (= 0.4 µl) in an enzyme buffer (15 mM Tris HCl pH 8.1, 0.25 mM EDTA, 250 mM NaCl, 10% v:v glycerol) and 1 µl test compound were added to a well of a 96 well microtiter plate and reaction started by addition of 30 µl substrate (Ac-NH-GGK(Ac)-AMC; final concentration 25 µM). After incubation for 180 min at 30°C, the reaction was terminated by the addition of 25 µl stop solution (50 mM Tris HCl pH 8, 100 mM NaCl, 0.5 mg/ml trypsin and 2 µM trichostatin A /TSA). After incubation at room temperature for further 40 min, fluorescence was measured using a Wallac Victor 1420 multilabel counter (excitation λ=355 nm, emission λ=460 nm) for quantification of AMC (7-amino-4-methylcoumarin) generated by trypsin cleavage of the deacetylated peptide. For the calculation of IC₅₀ values the fluorescence in wells without test compound (1% DMSO, negative control) was set as 100% enzymatic activity and the fluorescence in wells with 2µM TSA (positive control) were set at 0% enzymatic activity. The corresponding IC₅₀ values of the compounds for HDAC inhibitory activity were determined from the concentration-effect curves by means of non-linear regression analysis using the program GraphPad prism (Version 4.0).

The HeLa nuclear extract HDAC inhibitory activity expressed by IC₅₀ values for selected compounds according to the present invention is shown in the following table 2, in which the numbers of the compounds correspond to the numbers of the examples.

**Table 2: HDAC inhibitory activity (HDAC activity isolated from HeLa nuclear extract)**

| **Chimera** | **Compound No.** | **IC₅₀ (M)** |
|---|---|---|
| Imatinib | [19], [22], [16], [30] | 5.1 e-8 to 7.5e-6 |
| Erlotinib | [36], [38], [42] | 6.4e-9 to >3e-5 |
| Lapatinib | [9], [2], [7], [8], [10], [11], [12], [13] | 4.65e-8 to >3.2e-5 |

The rHDAC1 inhibitory activity expressed by IC₅₀ values for selected compounds according to the present invention is shown in the following table 3, in which the numbers of the compounds correspond to the numbers of the examples.

**Table 3: rHDAC1 inhibitory activity (recombinant HDAC1 isolated from HEK293 cells with heterologous HDAC1 expression)**

| **Chimera** | **Compound No.** | **IC₅₀ (M)** |
|---|---|---|
| Imatinib | [19], [22], [16], [35] | 7.7e-8 to 1.4e-6 |
| Erlotinib | [44], [42] | 6.5e-9 to >3.3e-5 |
| Lapatinib | [9], [2], [6], [7], [8], [10], [11], [12], [13] | 3.53e-8 to >3.2e-5 |

### Biochemical protein kinase assays

Active kinase proteins were either obtained from commercial suppliers (AbI, PDGF-Rβ ProQinase, Freiburg/Germany; AblT³¹⁵I: Millipore/Upstate, Billerica/USA; PKA: InvitroGen/Panvera, Carlsbad / USA) or prepared in-house (KDR, c-terminal kinase domain AA790-1356, HER2 c-terminal kinase domain, EGFR/HER1 C-terminal kinase domain). As substrates, poly-AGKY for Abl, AblT³¹⁵I and PDGF-Rβ (#P-1152 Sigma, Munich /Germany), poly-GluTyr for KDR, HER2 and EGFR/HER1 (#P-0275, Sigma, Munich / Germany) or a PKA substrate peptide for PKA (#12-394, Millipore/Upstate, Billerica / USA) were used. Into each well of a 96-well flashplate (#SMP-200 in the case of Abl, AblT³¹⁵I, PDGF-Rβ, EGFR, HER2 and KDR, #SMP-103 in the case of PKA; Perkin Elmer, Waltham / USA) kinase protein was diluted into kinase assay buffer (50mM HEPES pH 7.5, 3mM MgCl₂, 3mM MnCl₂, 1 mM DTT, 3µM sodium orthovanadate, 5µg/ml PEG 8000) containing the appropriate substrate (final volume 89µl). DMSO vehicle or compounds are added as 1 µl/well prior to the initiation of the kinase reaction by 10µl 1mM [³³P]-7ATP (4x10⁵cpm). Reactions were allowed to proceed for a time predetermined to be linear on a time versus phosphorylation plot (30min for PKA, otherwise 80min) and terminated with the addition of an equal volume of phosphoric acid (2% H₃PO₄ for 5 minutes). Plates were washed three times with 0.9% w/v NaCl and quantitated by liquid scintillation counting. Representative IC₅₀ values for Bcr-abl, Bcr-abl T³¹⁵I and PDGFRβ inhibition as determined in the aforementioned assay follow from the following table 4, in which the numbers of the compound correspond to the numbers of the examples.

**Table 4: Bcr-abl, bcr-abl T³¹⁵I and PDGFRβ inhibition in a biochemical, flash-plate based kinase assay**

| **Imatinib Chimera** | | |
|---|---|---|
| | **Compound No** | IC₅₀ (M) |
| **Bcr-abl (wt)** | [15], [19], [33] | 2.0e-6 to 3.9e-5 |
| **Bcr-abl T³¹⁵I** | [15], [19], [33], [30] | 1.0e-6 to 3.4e-5 |
| **PDGFRβ** | [15], [19], [20], [18], [14] | 2.1 e-6 to 7.1 e-5 |

Representative IC₅₀ values for EGFR/HER1 and PKA inhibition as determined in the aforementioned assay follow the following tables 5 and 6, in whicht the numbers of the compounds correspond to the numbers of the examples.

**Table 5: EGFR/HER1 and HER2 inhibition in a biochemical, flash-plate based kinase assay**

| **Erlotinib & Lapatinib Chimera** | | |
|---|---|---|
| | **Compound No.** | **IC₅₀ (M)** |
| **EGFR/HER1** | [4], [2], [36] | 2.2e-9 to 1.5e-7 |
| **HER2** | [6], [39] | 2e-9 to 7.4e-7 |

**Table 6: Protein kinase A (PKA) inhibition in a biochemical, flash-plate based kinase assay**

| **Chimera** | **Compound No.** | **PKA** **IC₅₀ (M)** |
|---|---|---|
| Imatinib | [30] | 3.8e-5 |
| | all other examples | >1e-4 |
| Erlotinib & Lapatinib | [7] | 3.5e-5 |
| | [6] | 8.5e-5 |
| | all other examples | >1e-4 |

### Quantification of cellular HDAC activity in HeLa cells

To assess the cellular potency of histone deacetylase inhibitors, an assay recently described was applied (Ciossek et al. Anal Biochem e-pub 2007). 5x10³ HeLa cervical carcinoma cells/well (ATCC CCL-2) were cultivated in 200µl/well DMEM (containing 10% FCS) in cell culture microtiter plates (white, 96well flat bottom plates suitable for fluorimetric analysis; Costar Art. No. 3917). Cells were cultivated for 24h under standard cell culture conditions at 37°C and 5% CO₂ to allow cell attachment and proliferation. The compounds of the present invention were added at different concentrations (dilutions done in DMSO) and incubation continued for 4h at 37°C under cell culture conditions (each concentration was tested in quadruplicate). For determination of the background activity defined as deacetylase activity not associated with HDAC class I and II enzymes, 10 µM TSA were added to control wells. After discarding the culture medium, 100 µl/well substrate solution (200 µM Boc-K(Ac)-AMC in DMEM (containing 10% FCS), stock solution diluted 1:250) were added and incubation continued at 37°C under cell culture conditions for further 3h. After discarding the substrate solution, HeLa cells were washed once with 200 µl/well PBS before addition of 100µl/well developer solution (50 mM TRIS pH 8, 100mM NaCl with 0.5 mg/ml trypsin and 10µM TSA). After incubation for 5min at 37°C, cells were lysed by addition of 100 µl/well lysis buffer (50 mM TRIS pH 8, 137 mM NaCl, 2,7 mM KCl, 1 mM MgCl₂, 1 vol% NP-40) and further incubation for 20min at 37°C. Finally, the amount of AMC was quantified using the Wallac Victor device (extinction λ=355 nm, emission at λ=460 nm). For the calculation of IC₅₀ values, the fluorescence in wells without test compound (1% DMSO, negative control) was set as 100% enzymatic activity and the fluorescence in wells with 10 µM TSA (background control) were set at 0% enzymatic activity.

The inhibition of cellular HDAC enzymatic activity expressed by IC₅₀ values for selected compounds according to the present invention is shown in the follwing table 7, in which the numbers of the compounds correspond to the numbers of the examples.

**Table 7: Inhibition of cellular HDAC activity in HeLa cells**

| **Chimera** | **Compound No.** | **IC₅₀ (M)** |
|---|---|---|
| Erlotinib | [45], [36], [37], [38], [40], [42] | 3.42e-8 to >1.6e-5 |
| Lapatinib | [9] | 4.3 e-7 |
| | all other examples | >1e-6 |

### Cellular Histone H3 hyperacetylation assay

To assess the cellular efficacy of a histone deacetylase inhibitor in vitro, an assay was set up in black clear-bottom 96-well plates and optimized for use on the Cellomics "ArrayScan II" platform for a quantitative calculation of histone acetylation. The protocol uses a polyclonal rabbit antibody, specifically binding to acetylated lysine 1-20 of human histone H3 on fixed cells with an Alexa Fluor 488 labeled goat anti rabbit-IgG used for counterstaining (Beckers et al. Int J Cancer 121, 1138, 2007). 5x10³ HeLa cervical carcinoma cells/well (ATCC CCL-2) in 200 µl Dulbecco's modified Eagle's medium (DMEM) containing 10% fetal calf serum are seeded at day 1 in Packard view plates and incubated for 24 h under standard cell culture conditions. On day 2, 1 µl test compound (200x final concentration) is added and incubation continued for further 24 h. On day 3, the culture medium is discarded and attached cells fixed for 15min at room temperature by addition of 100 µl fixation buffer (3.7% v:v formaldehyde in phosphate buffered saline / PBS). After discarding the fixation buffer and one wash with blocking solution (1% BSA, 0,3% Tween 20 in PBS), cells are permeabilized at room temperature by addition of 100µl/well permeabilization buffer (30,8 mM NaCl, 0,54 mM Na₂HPO₄, 0,31 mM KH₂PO₄, 0,1 % v:v Triton X-100) for 15 min at room temperature. After discarding the permeabilization buffer and washing twice with 100µl/well blocking solution at room temperature, the 1^{st} antibody (anti-K¹⁻²⁰ histone H3 antibody, Calbiochem No. 382158) in blocking solution (50 µl/well) is added. After incubation for 1 h at room temperature, the wells are washed twice at room temperature with 100 µl/ well blocking solution before additon of the 2^{nd} antibody (goat-anti-rabbit Alexa Fluor 488; MoBiTec No. A-11008) in blocking solution (50 µl/ well). After further incubation for 1 h at room temperature, wells are washed twice with 100 µl/ well blocking solution at room temperature. Finally, 100 µl/well PBS are added and image analysis performed on the Cellomics "ArrayScan II" platform. For EC₅₀ determination, the percentage of positive cells showing nuclear fluorescence is determined and EC₅₀ calculation done from concentration-effect curves by means of non-linear regression. For calibration, a positive (reference HDAC inhibitors like SAHA or NVP LBH-589) and a negative control were included.

The histone hyperacetylating cellular potency expressed by EC₅₀ values for selected compounds according to the present invention is shown in the following table 8, in which the numbers of the compounds correspond to the numbers of the examples.

**Table 8: Induction of histone H3 hyperacetylation in HeLa cervical carcinoma cells**

| **Chimera** | **Compound No.** | **EC₅₀ (M)** |
|---|---|---|
| Imatinib | [16], [20] | 1.7e-6 to 1 e-5 |
| Lapatinib | [9], [2], [4], [5], [6] | 3.65e-6 to >1e-4 |

### Cellular cytotoxicity assay

The anti-proliferative activity of the chimeric HDAC and protein kinase inhibitory chimeric compounds of the present invention was evaluated using HeLa cervical carcinoma, ,A549 NSCLC, SKOV3 ovarian carcinoma, Cal27 head and neck cancer, K562 chronic myeloid leukemia (CML) and EOL1 acute hypereosinophilic myeloid leukemia, cell lines. For quantification of cellular proliferation / living cells the Alamar Blue (Resazurin) cell viability assay was applied (O'Brien et al. Eur J. Biochem. 267, 5421-5426, 2000). In this assay Resazurin is reduced to the fluorescent resorufin by cellular dehydrogenase activity, correlating with viable, proliferating cells. The examples were dissolved as 20mM solutions in dimethylsulfoxide (DMSO) and subsequently diluted in semi-logarithmic steps. Cell lines were seeded at respective density into 96 well flat bottom plates in a volume of 200µl per well. 24h after seeding 1 µl each of the compound dilutions were added into each well of the 96 well plate. Each compound dilution was tested as quadruplicates. Wells containing untreated control cells were filled with 200µl DMEM medium containing 0.5% v:v DMSO. The cells were then incubated with the substances for 72h at 37°C in a humidified athmosphere containing 5% carbon dioxide. To determine the viability of the cells, 20 µl of an Resazurin solution (Sigma; 90 mg/l) were added. After 4h incubation at 37°C the fluorescence was measured at an extinction of 544 nm and an emission of 590 nm. For the calculation of the cell viability the emission value from untreated cells was set as 100% viability and the emission rates of treated cells were set in relation to the values of untreated cells. Viabilities were expressed as % values. The corresponding IC₅₀ values of the compounds for cytotoxic activity are determined from the concentration-effect curves by means of non-linear regression.

The anti-proliferative / cytotoxic potency expressed by IC₅₀ values for selected compounds according to the present invention is shown in the following tables 9 to 11, in which the numbers of the compounds correspond to the numbers of the examples.

**Table 9: Cytotoxicity of chimera towards HeLa cervical and A549 NSCLC cell lines**

| **Chimera** | | **Compound No.** | **IC₅₀ (M)** |
|---|---|---|---|
| Imatinib | **A549** | [26], [16], [15], [34], [33],[32], [31], [17], [20] | 2.95e-6 to >5e-5 |
| | **HeLa** | [16], [15], [20], [14] | 1.15e-6 to >5e-5 |
| Erlotinib | **A549** | [37], [38], [45] | 2.4e-7 to >5e-5 |
| | **HeLa** | [37], [38], [45] | 5e-7 to >5e-5 |
| Lapatinib | **A549** | [8], [9], [2], [5], [10], [11], [12], [13] | 1.1e-6 to >1e-5 |
| | **HeLa** | [8], [9], [6] | 9.1e-7 to 0.94e-5 |

**Table 10: Cytotoxicity of Imatinib chimera towards EOL1 and K562 CML cell lines**

| **Imatinib Chimera** | | |
|---|---|---|
| | **Compound No.** | **IC₅₀ (M)** |
| **K562** | [15], [26], [24], [35] | 4.9e-7 to 4.07e-6 |
| **EOL1** | [26], [35], [19], [14] | 3.0e-9 to 7.8e-7 |

**Table 11: Cytotoxicity of Erlotinib and Lapatinib chimera towards Cal27 and SKOV3 cell lines**

| **Chimera** | | **Compound No.** | **IC₅₀ (M)** |
|---|---|---|---|
| Erlotinib | **Cal27** | [45], [36] | 1.6e-7 to 8.0e-6 |
| | **SKOV3** | [45], [37], [38], [39], [41] | 5.6e-7 to >5e-5 |
| Lapatinib | **Cal27** | [8], [9], [11] | 2.8e-7 to >1.5e-5 |
| | **SKOV3** | [8], [9], [5] | 1 e-6 to >5e-5 |

### Western blot analysis

For detection of histone hyperacetylation as well as protein phosphorylation (protein kinase autophosphorylation), K562 CML and Cal27 head and neck cancer cell lines were cultivated in 6 well cell culture plates and treated for 16h to 24h with selected chimeric compounds of the present invention. Non-adherent K562 cells were collected by short centrifugation at 1200 rpm, adherent Cal27 collected by using a cell scraper. Collected cells were washed with ice-cold PBS, centrifuged again and the supernatant was discarded. Subsequently, the cells were lysed in 300µl lysis buffer (50 mM Tris HCl pH 8, 150 mM NaCl, 1%v/v NP-40, 0.5% w/v Nadesoxycholate, 0.1% w/v disodiumdodecylsulfate (SDS), 0.02% w/v NaN₃, 1 mM Na-Vanadate, 10 mM sodium pyrophosphate, 20 mM NaF, 100 µg/ml phenylmethylsulfonyl fluoride (PMSF), protease inhibitor mix / Roche and 50 U/ml Benzonase) at 4°C. After incubation for 30 min under agitation at 4°C, the lysate was centrifuged (20 min at 14.000 rpm, 4°C) and the protein concentration of the supernatant quantified using the BCA assay (Pierce). Equal amounts of protein were separated by sodium dodecylsulfate polyacrylamide gel electrophoresis (SDS-PAGE, 10%) before transfer to a polyvinylidendifluoride (PVDF) membrane (Biorad Art. No.162-0177) by semi-dry blotting. The PVDF membrane was soaked in methanol before treatment with transfer buffer (tris/glycine buffer + 20% methanol). The blot was prepared (paper - membrane - acrylamide protein gel - paper) and transfer done by using a Biorad SemiDry Transfer Cell device (0,1 A / gel for 60min). After protein transfer, the membrane was treated for 60 min at RT with 3% w:v BSA in TBS-T (Tris buffered saline + 0,05vol% Tween 20). Next, the membrane was incubated with the 1st antibody specific for histone H3, histone AcH3-K¹⁻²⁰ or EGFR/HER1 phosphorylated EGFR^{Y1068} diluted in TBS-T with 3% w:v BSA / 3 mM NaN₃) overnight at 4°C. On the next day, the membrane was washed three times for 15min each with TBS-T, before addition of the respective 2^{nd} antibody (diluted in TBS-T with 3%w/v dry milk powder, respectively) and incubation for 1 h at RT. After washing three times with TBS-T, the blot was developed by using the ECL-reagent (Amersham / GE Healthcare). The blot was washed once with TBS-T and rehybridization was done with an antibody, eg specific for β-action.

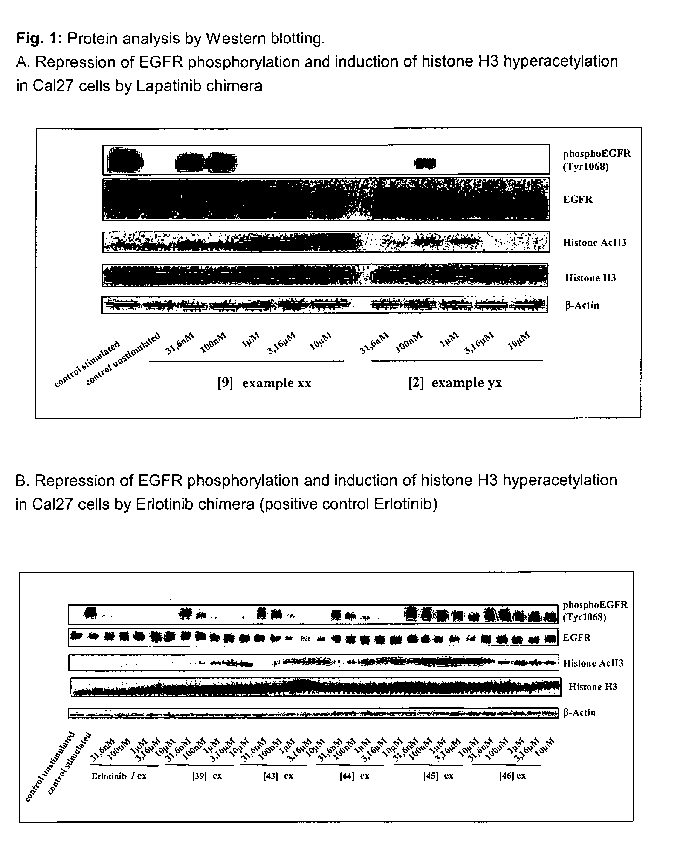

**Fig. 1****:** Protein analysis by Western blotting.

## Claims

1. A compound of formula I or its pharmaceutically acceptable salts or solvates
A-L-B (I)
wherein A is a histone deacetylase (HDAC) inhibitory moiety, L is a single bond or a linker group and B is a protein kinase inhibitory moiety.

2. Compound according to claim 1 wherein the HDAC inhibitory moiety A contains (i) a hydroxamic acid group, and (ii) a benzamide group.

3. Compound according to claim 2, wherein the HDAC inhibitory moiety A is 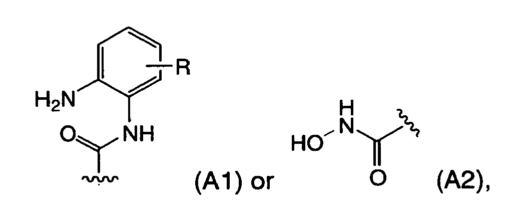 wherein R is hydrogen, halogen, (C₁-C₄)alkyl, (C₁-C₄)alkoxy, thienyl, or N(R¹²)R¹³
wherein R¹² and R¹³ independently of one another are hydrogen, (C₁-C₄)alkyl, (C₁-C₄)alkylcarbonyl, or R¹² and R¹³ together and with inclusion of the nitrogen atom to which they are bonded form an azetidinyl-, pyrrolidinyl-, piperidinyl-, piperazinyl-, 4-methylpiperazinyl-, morpholinyl- or thiomorpholinyl-ring;

4. Compound according to claim 3 wherein R is hydrogen, halogen, (C₁-C₄)alkyl, (C₁-C₄)alkoxy, thienyl, or N(R¹²)R¹³, wherein R¹² and R¹³ independently of one another are hydrogen, (C₁-C₄)alkyl, or R¹² and R¹³ together and with inclusion of the nitrogen atom to which they are bonded form a pyrrolidinyl-ring.

5. Compound according to claim 3 wherein R is hydrogen, fluorine, chlorine, methyl, ethyl, propyl, isopropyl, methoxy, ethoxy, thienyl, N(R¹²)R¹³ wherein R¹² and R¹³ independently of one another are hydrogen, methyl, ethyl, or R¹² and R¹³ together and with inclusion of the nitrogen atom to which they are bonded form a pyrrolidinyl-ring.

6. Compound according to claim 3 wherein R is hydrogen.

7. Compound according to any of the preceding claims, wherein L is a single bond or a linker group selected from (L1) a straight or branched C₁-C₆ alkylene group, a C₂-C₆ alkenylene group, a C₂-C₆ alkinylene group, a C₃-C₆ cycloalkylene group, each of which may optionally be interrupted by -0-, -S-, -COO-, -NHCO- or arylene, (L2) a group of the formula
D-Ar-E
wherein D and E may be the same or different and are selected from a bond, a straight or branched C₁-C₆ alkylene group, C₂-C₆ alkenylene group or C₂-C₆ alkinylene group, a C₃-C₆ cycloalkylene group, an amide group, a sulfinyl group, a sulfonyl group, -O-, -NH-, -N(C₁-C₆ alkyl)- and -S-; Ar is an aryl group with 5-10 carbon atoms, an alkylaryl group wherein alkyl is C₁-C₆ and aryl is C₅-C₁₀, a heteroaryl group with 5-10 carbon atoms and 1-3 heteroatoms, selected from O, S and N.

8. Compound according to claim 7, wherein L is an ethylene group, a trans-ethenylene group, or L is
D-Ar-E
wherein D is selected from a bond or a trans-ethenylene group, and E is selected from a bond, an amide group, a sulfonyl group, and -O-, and Ar is selected from a phenyl group, a benzyl group, a pyridinyl group, a pyrimidinyl group, a thienyl group or a pyrrolyl group.

9. Compound according to any preceding claims wherein the protein kinase inhibitory moiety B has an enzyme specificity for at least one kinase selected from (i) tyrosine kinase,(ii) serine kinase and (iii) dual-specificity kinases.

10. Compound according to any preceding claims wherein the protein kinase inhibitory moiety B inhibits a protein kinase having pathophysiological relevance.

11. Compound according to any preceding claims wherein the protein kinase inhibitory moiety B inhibits the protein kinase bcr-abl, PDGFR, HER1 and/or HER2 protein kinases as well as mutants of these protein kinases, including but not limited to point mutations and fusion proteins.

12. Compound according to any preceding claim, wherein the protein kinase inhibitory moiety B is selected from 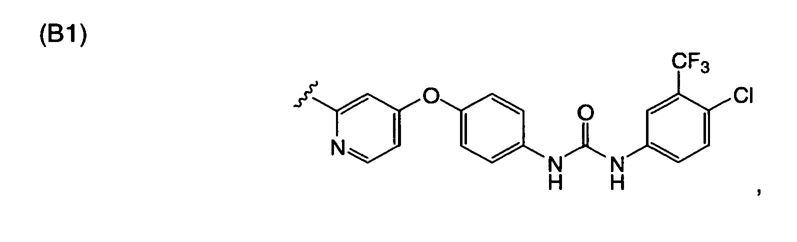 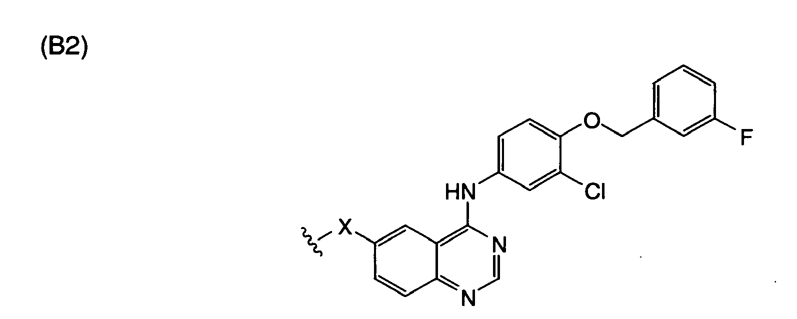 wherein X is a 5- or 6-membered carbocyclic or heterocyclic aromatic ring; 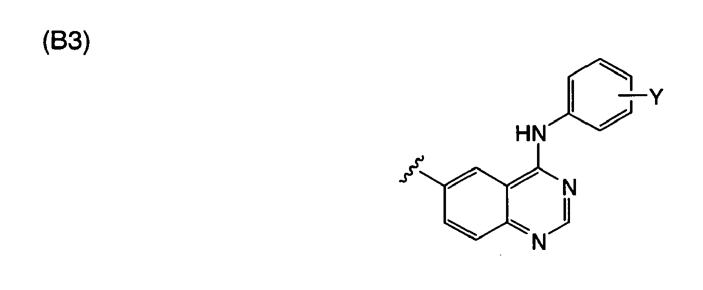 wherein Y is F, Cl, Br, I, an optionally substituted C₁-C₆ alkyl group, an optionally substituted C₂-C₆ alkenyl group, an optionally substituted C₂-C₆ alkinyl group, and 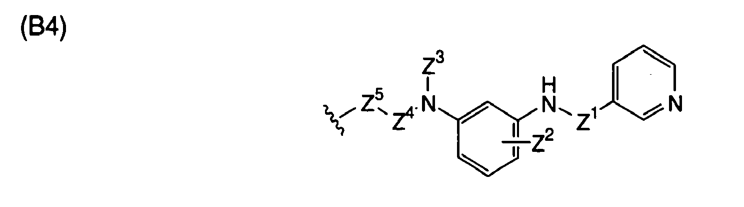 wherein Z¹ is a 5- or 6-membered aromatic or heteroaromatic ring,
Z² is a hydrogen atom, a C₁-C₆ straight or branched alkyl group or a halogen atom, Z³ is a hydrogen atom, a C₁-C₆ straight or branched alkyl group, or a C₃-C₆ cycloalkyl group,
Z⁴ is a -CH₂- group or a -CO- group, and
Z⁵ is a 5- or 6-membered aromatic or heteroaromatic ring.

13. Compound according to claim 12 wherein the protein kinase inhibitory moiety B is selected from 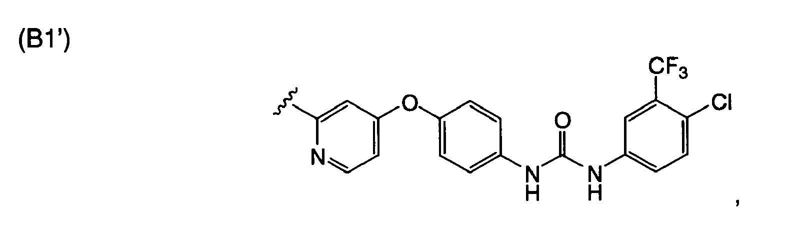 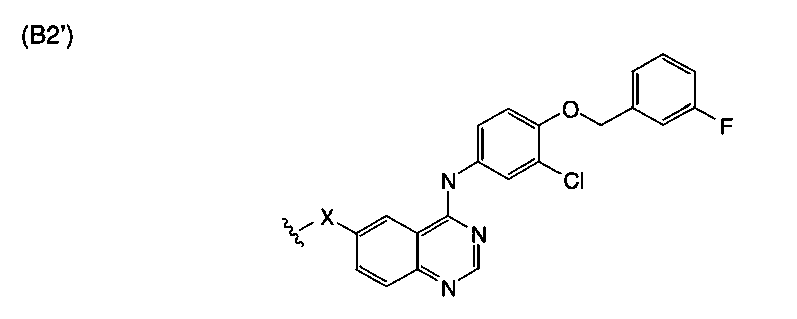 wherein X is a furanyl moiety, a thienyl moiety, or a phenyl moiety, 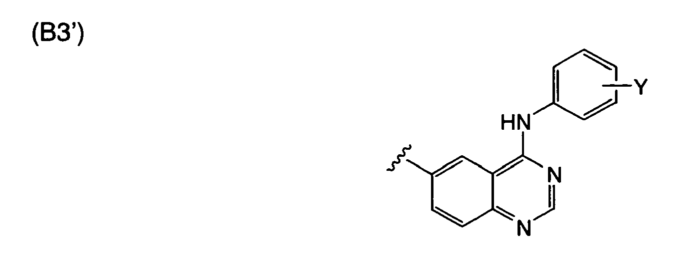 wherein Y is Br or an ethinyl group, 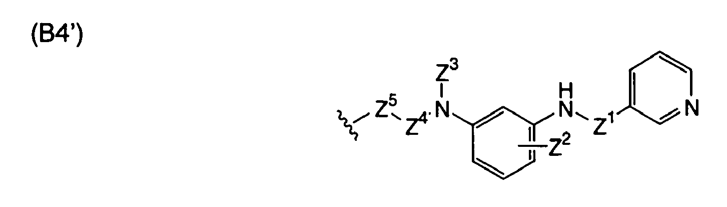 wherein Z¹ is a pyrimidinyl moiety or a thiazolyl moiety,
Z² is selected from a hydrogen atom, a methyl group or chlorine atom,
Z3 is selected from a hydrogen atom or a methyl group,
Z⁴ is a -CH₂- group or a -CO- group, and
Z⁵ is a phenyl moiety, a pyrimidyl moiety or a thienyl moiety.

14. Compound according to claim 13, wherein Y is in meta-position and wherein Z² is in ortho-position of the ring.

15. Compound of formula I or its pharmaceutically acceptable salts or solvates
A-L-B (I)
wherein A is a HDAC inhibitory moiety as defined in claim 3, L is a group as defined in claim 7, and the protein kinase inhibitory moiety B is defined as in claim 12.

16. Compound of formula I or its pharmaceutically acceptable salts or solvates
A-L-B (I)
according to claim 1, wherein A is either 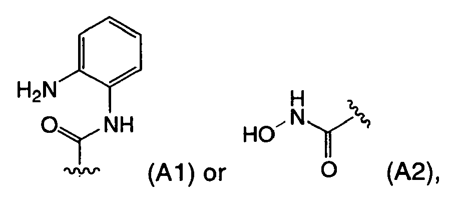 wherein the L is an ethylene group, a trans-ethenylene group, or wherein L is
D-Ar-E
wherein D, E and Ar are as defined in claim 7, and
wherein B is selected from a compound (B1'), (B2'), (B3') or (B4') as defined in claim 13.

17. Compound of formula I or its pharmaceutically acceptable salts or solvates according to claim 1, wherein (B3) is represented by the following formula (B3") 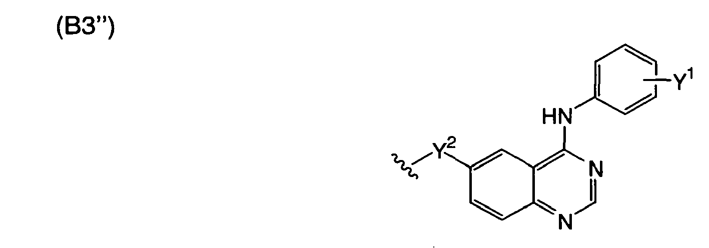 wherein Y¹ has the same meaning as Y in claim 12, and
Y² is selected from a -NHCO-Ar moiety, a -OCH₂-Ar moiety, and a -SO₂-Ar moiety, Ar is an aryl group with 5-10 carbon atoms, an alkylaryl group wherein alkyl is C₁-C₆ and aryl is C₅-C₁₀, a heteroaryl group with 5-10 carbon atoms and 1-3 heteroatoms, selected from O, S and N.

18. A compound according to claim 1 selected from
[1] *E*-3-(4-{4-[3-(Chloro-trifluoromethyl-phenyl)-ureido]-phenoxy}-pyridin-2-yl)-N-hydroxy-acrylamide
[2] *E*-3-(5-{4-[3-Chloro-4-(3-fluorobenzyloxy)-phenylamino]quinazolin-6-yl}furan-2-yl)-*N*-hydroxy-acrylamide hydrochloride monohydrate
[9] *E*-3-(3-(4-(3-chloro-4-(3-fluorobenzyloxy)phenylamino)quinazolin-6-yl)phenyl)-*N*-hydroxy-acrylamide
[39] Pyridine-2,5-dicarboxylic acid 2-[(2-amino-phenyl)-amide]-5-{[4-(3-ethynyl-phenylamino)-quinazolin-7-yl]-amide}
[43] *N*-(2-aminophenyl)-4-((4-(3-bromophenylamino)quinazolin-6-yloxy)-methyl)benzamide
[44] *N*-(2-aminophenyl)-4-((4-(3-ethynylphenylamino)-quinazolin-6-yloxy)methyl)benzamide
[45] 3-{1-[4-(3-Ethynylphenylamino)quinazoline-6-sulfonyl]-1*H*-pyrrol-3-yl}-*N-*hydroxy-acrylamide
[46] *N*-(2-Amino-phenyl)-3-{1-[4-(3-ethynylphenylamino)quinazoline-6-sulfonyl]-1*H*-pyrrol-3-yl}acryl-amide
or a pharmaceutically acceptable salt or solvate thereof.

19. A compound according to any of the preceding claims, **characterized in that** the compound is in crystalline form.

20. Pharmaceutical composition comprising a compound according to any of claims 1 to 19 and a pharmaceutically acceptable carrier.

21. A compound according to any of claims 1 to 19 for use in a method for the treatment of malignant neoplasia.

22. A compound according to any of claims 1 to 19 for use in a method for the treatment of a non-malignant disease selected from
(i) arthropathies and osteopathological conditions or diseases such as rheumatoid arthritis, osteoarthritis, gout, polyarthritis, and psoriatic arthritis,
(ii) autoimmune diseases like systemic lupus erythematosus and transplant rejection,
(iii) hyperproliferative diseases such as smooth muscle cell proliferation including vascular proliferative disorders, atherosclerosis, restenosis and proliferative fibrosis such as lung fibrosis,
(iv) acute and chronic inflammatory conditions or diseases and dermal conditions such as psoriasis, ulcerative colitis, Crohn's disease, allergic rhinitis, allergic dermatitis, cystic fibrosis, chronic obstructive bronchitis and asthma, and
(v) endometriosis, uterine fibroids, endometrial hyperplasia and benign prostate hyperplasia.

23. The compound according to claim 21 wherein the malignant neoplasia is a cancer disease selected from solid and hematological tumors, myelodysplastic syndrome, plasma cell neoplasia, paraneoplastic syndromes, cancers of unknown primary site as well as AIDS related malignancies.

24. Pharmaceutical composition comprising a combination of a compound according to any of claims 1 to 19 and a further anti-cancer agent.

25. A method for the treatment of cancer, comprising administration of a therapeutically effective amount of a compound according to any of claims 1 to 19 to a human patient in need thereof.
